# EUROPÄISCHE PATENTSCHRIFT

(11) **EP 1 233 980 B1**
(45) Veröffentlichungstag und Bekanntmachung des Hinweises auf die Patenterteilung: **15.10.2008**
(21) Anmeldenummer: 00956469.1
(22) Anmeldetag: 21.08.2000
(51) Int. Cl.: C07K 14/00, A61K 39/00, C12N 15/00

(54) **NUKLEINSÄUREMOLEKÜL, UMFASSEND EINE FÜR EIN HÄMOCYANIN KODIERENDE NUKLEINSÄURESEQUENZ UND MINDESTENS EINE INTRONSEQUENZ**
NUCLEIC ACID MOLECULE COMPRISING A NUCLEIC ACID SEQUENCE WHICH CODES FOR A HAEMOCYANIN, AND COMPRISING AT LEAST ONE INTRON SEQUENCE
MOLECULE D'ACIDE NUCLEIQUE COMPORTANT UNE SEQUENCE D'ACIDE NUCLEIQUE CODANT POUR UNE HEMOCYANINE ET AU MOINS UN INTRON

(30) Priorität: 20.08.1999 DE 19939578
(43) Veröffentlichungstag der Anmeldung: 28.08.2002
(73) Patentinhaber: Biosyn Arzneimittel GmbH, 70734 Fellbach (DE)
(72) Erfinder: MARKL, Jürgen, D-55296 Gau-Bischofsheim (DE); ALTENHEIN, Benjamin, D-65195 Wiesbaden (DE); LIEB, Bernhard, D-55129 Mainz (DE); STIEFEL, Thomas, D-70184 Stuttgart (DE)
(74) Vertreter: Grünecker, Kinkeldey, Stockmair & Schwanhäusser Anwaltssozietät
(86) Internationale Anmeldenummer: PCT/EP2000/008129
(87) Internationale Veröffentlichungsnummer: WO 2001/014536

(56) Entgegenhaltungen:
- EP-A- 0 244 295
- EP-A- 0 252 829
- EP-A- 0 621 039
- WO-A-94/11019
- US-A- 5 831 033
- US-A- 5 888 775
- SWERDLOW RICHARD D ET AL: "Keyhole limpet hemocyanin: Structural and functional characterization of two different subunits and multimers." COMPARATIVE BIOCHEMISTRY AND PHYSIOLOGY B, Bd. 113, Nr. 3, 1996, Seiten 537-548, XP000900921 ISSN: 0305-0491
- HAMILTON J V ET AL: "Periodate-sensitive immunological cross-reactivity between keyhole limpet haemocyanin (KLH) and serodiagnostic Schistosoma mansoni egg antigens." PARASITOLOGY, Bd. 118, Nr. 1, Januar 1999 (1999-01), Seiten 83-89, XP000912289 ISSN: 0031-1820
- MILLER KAREN I ET AL: "Sequence of the Octopus dofleini hemocyanin subunit: Structural and evolutionary implications." JOURNAL OF MOLECULAR BIOLOGY, Bd. 278, Nr. 4, 15. Mai 1998 (1998-05-15), Seiten 827-842, XP002164204 ISSN: 0022-2836
- STOEVA STANKA ET AL: "Primary structure and unusual carbohydrate moiety of functional unit 2-c of keyhole limpet hemocyanin (KLH)." BIOCHIMICA ET BIOPHYSICA ACTA, Bd. 1435, Nr. 1-2, 16. November 1999 (1999-11-16), Seiten 94-109, XP000937695 ISSN: 0006-3002
- GEBAUER WOLFGANG ET AL: "Keyhole limpet hemocyanin type 2 (KLH2): Detection and immunolocalization of a labile functional unit h." JOURNAL OF STRUCTURAL BIOLOGY., Bd. 128, Nr. 3, 30. Dezember 1999 (1999-12-30), Seiten 280-286, XP000937601 ISSN: 1047-8477
- HARRIS J ROBIN ET AL: "Immunoelectron microscopy of hemocyanin from the keyhole limpet (Megathura crenulata): A parallel subunit model." JOURNAL OF STRUCTURAL BIOLOGY, Bd. 111, Nr. 2, 1993, Seiten 96-104, XP000900919 ISSN: 1047-8477
- CARRERA M ROCIO A ET AL: "Cocaine vaccines: Antibody protection against relapse in a rat model." PROCEEDINGS OF THE NATIONAL ACADEMY OF SCIENCES OF THE UNITED STATES, Bd. 97, Nr. 11, 23. Mai 2000 (2000-05-23), Seiten 6202-6206, XP002148844 May 23, 2000 ISSN: 0027-8424
- SOEHNGEN SABINE M ET AL: "Mass determination, subunit organization and control of oligomerization states of keyhole limpet hemocyanin (KLH)." EUROPEAN JOURNAL OF BIOCHEMISTRY, Bd. 248, Nr. 2, 1997, Seiten 602-614, XP000912288 ISSN: 0014-2956 in der Anmeldung erwähnt
- CARRERA M ROCIO A ET AL: "Suppression of psychoactive effects of cocaine by active immunization." NATURE (LONDON), Bd. 378, Nr. 6558, 1995, Seiten 727-730, XP000946580 ISSN: 0028-0836
- LIEB BERNHARD ET AL: "The sequence of a gastropod hemocyanin (HtH1 from Haliotis tuberculata)." JOURNAL OF BIOLOGICAL CHEMISTRY, Bd. 275, Nr. 8, 25. Februar 2000 (2000-02-25), Seiten 5675-5681, XP000946778 ISSN: 0021-9258
- LIEB BERNHARD ET AL: "Subunit organization of the abalone Haliotis tuberculata hemocyanin type 2 (HtH2), and the cDNA sequence encoding its functional units d, e, f, g and h." EUROPEAN JOURNAL OF BIOCHEMISTRY, Bd. 265, Nr. 1, Oktober 1999 (1999-10), Seiten 134-144, XP000952187 ISSN: 0014-2956
- KELLER HENNING ET AL: "Abalone (Haliotis tuberculata) hemocyanin type 1 (HtH1): Organization of the apprxeq400 kDa subunit, and amino acid sequence of its functional units f, g and h." EUROPEAN JOURNAL OF BIOCHEMISTRY, Bd. 264, Nr. 1, August 1999 (1999-08), Seiten 27-38, XP000952186 ISSN: 0014-2956
- DREXEL R ET AL: "COMPLETE AMINO ACID SEQUENCE OF A FUNCTIONAL UNIT FROM A MOLLUSCAN HEMOCYANIN HELIX-POMATIA" BIOLOGICAL CHEMISTRY HOPPE-SEYLER, Bd. 368, Nr. 6, 1987, Seiten 617-636, XP000997484 ISSN: 0177-3593
- LIEB BERNHARD ET AL: "Structures of two molluscan hemocyanin genes: Significance for gene evolution." PROCEEDINGS OF THE NATIONAL ACADEMY OF SCIENCES OF THE UNITED STATES, Bd. 98, Nr. 8, 10. April 2001 (2001-04-10), Seiten 4546-4551, XP002190023 April 10, 2001 ISSN: 0027-8424

### SEQUENZPROTOKOLL

<110> Biosyn Arzneimittel GmbH
<120> Nukleinsäuremolekül, umfassend eine für ein Hämocyanin kodierende Nukleinsäuresequenz und mindestens eine Intronsequenz
<130> PCT1220-01966
<140>
   <141>
<160> 108
<170> PatentIn Ver. 2.1
<210> 1
   <211> 1269
   <212> DNA
   <213> Haliotis tuberculata
<400> 1
   ggcttgttca gtttctactc gtcgcccttg tggcgggggc tggagcagac aacgtcgtca 60
   gaaaggacgt gagtcacctc acggatgacg aggtgcaagc tctccacggc gccctccatg 120
   acgtcactgc atctacaggg cctctgagtt tcgaagacat aacatcttac catgccgcac 180
   cagcgtcgtg tgactacaag ggacggaaga tcgcctgctg tgtccacggt atgcccagtt 240
   tccccttctg gcacagggca tatgtcgtcc aagccgagcg ggcactgttg tccaaacgga 300
   agactgtcgg aatgccttac tgggactgga cgcaaacgct gactcactta ccatctcttg 360
   tgactgaacc catctacatt gacagtaaag gtggaaaggc tcaaaccaac tactggtacc 420
   gcggcgagat agcgttcatc aataagaaga ctgcgcgagc tgtagatgat cgcctattcg 480
   agaaggtgga gcctggtcac tacacacatc ttatggagac tgtcctcgac gctctcgaac 540
   aggacgaatt ctgtaaattt gaaatccagt tcgagttggc tcataatgct atccattact 600
   tggttggcgg taaatttgaa tattcaatgt caaacttgga atacacctcc tacgacccca 660
   tcttcttcct ccaccactcc aacgttgacc gcctcttcgc catctggcag cgtcttcagg 720
   aactgcgagg aaagaatccc aatgcaatgg actgtgcaca tgaactcgct caccagcaac 780
   tccaaccctt caacagggac agcaatccag tccagctcac aaaggaccac tcgacacctg 840
   ctgacctctt tgattacaaa caacttggat acagctacga cagcttaaac ctgaatggaa 900
   tgacgccaga acagctgaaa acagaactag acgaacgcca ctccaaagaa cgtgcgtttg 960
   caagcttccg actcagtggc tttgggggtt ctgccaacgt tgttgtctat gcatgtgtcc 1020
   ctgatgatga tccacgcagt gatgactact gcgagaaagc aggcgacttc ttcattcttg 1080
   ggggtcaaag cgaaatgccg tggagattct acagaccctt cttctatgat gtaactgaag 1140
   cggtacatca ccttggagtc ccgctaagtg gccactacta tgtgaaaaca gaactcttca 1200
   gcgtgaatgg cacagcactt tcacctgatc ttcttcctca accaactgtt gcctaccgac 1260
   ctgggaaag                                                         1269
<210> 2
   <211> 1257
   <212> DNA
   <213> Haliotis tuberculata
<400> 2
   gtcaccttga cccacctgtg catcatcgcc acgatgacga tcttattgtt cgaaaaaata 60
   tagatcattt gactcgtgaa gaggaatacg agctaaggat ggctctggag agattccagg 120
   ccgacacatc cgttgatggg taccaggcta cagtagagta ccatggcctt cctgctcgtt 180
   gtccacgacc agatgcaaaa gtcaggttcg cctgttgtat gcatggcatg gcatccttcc 240
   ctcactggca ccggctgttc gttacccagg tggaagatgc tcttgtacgg cgtggatcgc 300
   ctatcggtgt tccttattgg gactggacaa aacctatgac tcaccttcca gacttggcat 360
   caaatgagac gtacgtagac ccgtatggac atacacatca taatccattc ttcaatgcaa 420
   atatatcttt tgaggaggga caccatcaca cgagcaggat gatagattcg aaactgtttg 480
   ccccagtcgc ttttggggag cattcccatc tgtttgatgg aatcctgtac gcatttgagc 540
   aggaagattt ctgcgacttt gagattcagt ttgagttagt ccataattct attcatgcgt 600
   ggataggcgg ttccgaagat tactccatgg ccaccctgca ttacacagcc tttgacccca 660
   ttttctacct tcatcattcc aatgtcgatc gtctatgggc aatctggcaa gctcttcaaa 720
   tcaggagaca caagccatat caagcccact gtgcacagtc tgtggaacag ttgccaatga 780
   agccatttgc tttcccatca cctcttaaca acaacgagaa gacacatagt cattcagtcc 840
   cgactgacat ttatgactac gaggaagtgc tgcactacag ctacgatgat ctaacgtttg 900
   gtgggatgaa ccttgaagaa atagaagaag ctatacatct cagacaacag catgaacgag 960
   tcttcgcggg atttctcctt gctggaatag gaacatctgc acttgttgac attttcataa 1020
   ataaaccggg gaaccaacca ctcaaagctg gagatattgc cattcttggt ggtgccaagg 1080
   aaatgccttg ggcgtttgac cgcttgtata aggtcgaaat aactgactca ttgaagacac 1140
   tttctctcga tgtcgatgga gattatgaag tcacttttaa aattcatgat atgcacggaa 1200
   acgctcttga tacggacctg attccacacg cagcagttgt ttctgagcca gctcacc    1257
<210> 3
   <211> 1242
   <212> DNA
   <213> Haliotis tuberculata
<400> 3
   ctacctttga ggatgaaaag cacagcttac gaatcagaaa aaatgtcgac agcttgactc 60
   ctgaagaaac aaatgaactg cgtaaagccc tggagcttct tgaaaatgat catactgcag 120
   gtggattcaa tcagcttggc gccttccatg gagagcctaa atggtgccct aatcctgaag 180
   cggagcacaa ggttgcatgc tgtgttcatg gcatggctgt tttccctcat tggcacaggc 240
   ttcttgctct ccaggcggag aatgctctta gaaagcatgg gtacagtggt gctctaccat 300
   actgggattg gactcgcccc ctttcccaac ttcctgatct ggttagtcat gagcagtata 360
   cagatccttc cgaccatcac gtgaagcata acccgtggtt caatggccac atcgatacag 420
   taaatcagga taccaccaga agcgtacggg aggatcttta tcaacaacct gaatttggac 480
   atttcacgga tattgctcaa caagtcctct tagcattaga acaagatgac ttctgttcgt 540
   ttgaagtgca gtatgagatt tcccataatt ttatccatgc acttgtagga ggaaccgacg 600
   cttatggcat ggcatcgctg agatatacag catacgatcc aatctttttc ttgcatcatt 660
   caaacaccga caggatctgg gctatttggc aatccctgca aaaatacaga ggcaaaccgt 720
   acaacactgc caactgcgcc atagaatcta tgagaaggcc cctgcaacca tttggactaa 780
   gcagtgccat taaccctgac agaatcacca gagagcatgc tatcccgttt gatgtcttca 840
   actatagaga taaccttcat tacgtatatg ataccctgga atttaatggt ttgtcgattt 900
   cacaacttga tagagagctg gaaaaaatca agagtcacga aagagtattt gctggattct 960
   tgctgtcggg gattaaaaaa tctgctcttg tgaaattcga agtttgtact ccacctgata 1020
   attgtcataa agcaggggag ttttatctac tcggggacga aaacgagatg gcttgggcct 1080
   atgaccgact tttcaagtat gatattactc aggttctgga agcaaaccat ctacacttct 1140
   atgatcatct cttcattcgc tacgaagtct ttgatcttaa aggagtgagt ttgggaactg 1200
   acctgttcca cactgcaaat gtggtacatg attccggcac ag                    1242
<210> 4
   <211> 1239
   <212> DNA
   <213> Haliotis tuberculata
<400> 4
   gcacccgtga tcgtgataac tacgttgaag aagttactgg ggccagtcat atcaggaaga 60
   atttgaacga cctcaatacc ggagaaatgg aaagccttag agctgctttc ctgcatattc 120
   aggacgacgg aacatatgaa tctattgccc agtaccatgg caaaccaggc aaatgtcaat 180
   tgaatgatca taatattgcg tgttgtgtcc atggtatgcc taccttcccc cagtggcaca 240
   gactgtatgt ggttcaggtg gagaatgctc tcctaaacag gggatctggt gtggctgttc 300
   cttactggga gtggactgct cccatagacc atctacctca tttcattgat gatgcaacat 360
   acttcaattc ccgacaacag cggtacgacc ctaacccttt cttcagggga aaggttactt 420
   ttgaaaacgc agtcacaaca agggacccac aagccgggct cttcaactca gattatatgt 480
   atgagaatgt tttacttgca ctggagcagg aaaattattg tgactttgaa attcagtttg 540
   agcttgttca taacgcactt cattccatgc tgggaggtaa agggcagtac tccatgtcct 600
   ccctggacta ttctgcgttt gatcccgtct tcttcctaca tcatgccaac acggacagac 660
   tgtgggcaat ctggcaggaa ctacaaagat tccgagaact gccttatgaa gaagcgaact 720
   gtgcaatcaa cctcatgcat caaccactga agccgttcag tgatccacat gagaatcacg 780
   acaatgtcac tttgaaatac tcaaaaccac aggacggatt cgactaccag aaccacttcg 840
   gatacaagta tgacaacctt gagttccatc acttatctat cccaagtctt gatgctaccc 900
   tgaagcaaag gagaaatcac gacagagtgt ttgcgggctt ccttcttcat aacataggaa 960
   cttctgctga cataactatc tacatatgtc tgcctgacgg acggcgtggc aatgactgca 1020
   gtcatgaggc gggaacattc tatatcctcg gaggcgaaac agagatgcct tttatctttg 1080
   accgtttgta taaatttgaa atcaccaaac cactgcaaca gttaggagtc aagctgcatg 1140
   gtggagtttt cgaactggag cttgagatca aggcatacaa cggttcctat ctggatcccc 1200
   atacctttga tccaactatc atctttgaac ctggaacag 1239
<210> 5
   <211> 1260
   <212> DNA
   <213> Haliotis tuberculata
<400> 5
   atacccatat cttggaccac gaccatgagg aagagatact tgtcaggaag aatataattg 60
   atttgagccc aagggagagg gtttctctag tcaaagcttt gcaaagaatg aagaatgatc 120
   gctccgctga tgggtaccaa gccattgcct ctttccatgc cctgccacca ctctgtccca 180
   atccatctgc agctcaccgt tatgcttgct gtgtccatgg catggctaca tttccccagt 240
   ggcacagact gtacactgtt caggttcagg atgccctgag gagacatggt tcacttgttg 300
   gtattcctta ctgggactgg acaaaaccag tcaacgagtt acccgagctt ctttcttcag 360
   caacatttta tcatccaatc cggaatatta atatttcaaa tccattcctc ggggctgaca 420
   tagaatttga aggaccgggc gttcatacag agaggcacat aaatactgag cgcctgtttc 480
   acagtgggga tcatgacgga taccacaact ggttcttcga aactgttctc tttgctttgg 540
   aacaggaaga ttactgcgat tttgaaatac aatttgagat agcccataat ggcatccaca 600
   catggattgg tggaagcgca gtatatggca tgggacacct tcactatgca tcatatgatc 660
   caattttcta catccaccat tcacagacgg acagaatatg ggctatttgg caagagctgc 720
   agaagtacag gggtctatct ggttcggaag caaactgtgc cattgaacat atgagaacac 780
   ccttgaagcc tttcagcttt gggccaccct acaatttgaa tagtcatacg caagaatatt 840
   caaagcctga ggacacgttt gactataaga agtttggata cagatatgat agtctggaat 900
   tggaggggcg atcaatttct cgcattgatg aacttatcca gcagagacag gagaaagaca 960
   gaacttttgc agggttcctc cttaaaggtt ttggtacatc cgcatctgtg tcattgcaag 1020
   tttgcagagt tgatcacacc tgtaaagatg cgggctattt cactattctg ggaggatcag 1080
   ccgaaatgcc atgggcattc gacaggcttt ataagtatga cattactaaa actcttcacg 1140
   acatgaacct gaggcacgag gacactttct ctatagacgt aactatcacg tcttacaatg 1200
   gaacagtact ctcgggagac ctcattcaga cgccctccat tatatttgta cctggacgcc 1260
<210> 6
   <211> 1251
   <212> DNA
   <213> Haliotis tuberculata
<400> 6
   ataaactcaa ctcacggaaa catacaccta acagagtccg ccatgagcta agtagcctta 60
   gttcccgtga catagcaagc ttgaaggcag ctttgacaag ccttcaacat gataatggga 120
   ctgatggtta tcaagctatt gctgccttcc atggcgttcc tgcgcagtgc cacgagccat 180
   ctggacgtga gatcgcctgt tgcatccacg gcatggcgac gtttcctcac tggcaccggt 240
   tgtacactct gcagttggag caagcgctgc gcagacacgg gtccagtgtt gctgttccat 300
   actgggactg gaccaagcca atcaccgaac tgccacacat tctgacagac ggagaatatt 360
   atgacgtttg gcaaaatgcc gtcttggcca atccgtttgc aagaggttat gtgaaaatta 420
   aagatgcatt tacggtgaga aatgtccagg aaagtctgtt caaaatgtca agttttggaa 480
   agcactcgct tctgtttgac caggctttgt tggctcttga acaaactgac tactgtgact 540
   tcgaagttca gtttgaagtg atgcataaca cgatccatta tctcgtagga gggcgtcaaa 600
   cgtacgcctt ctcctctctc gagtattcct catacgatcc aatcttcttt attcaccact 660
   cgtttgttga caaaatatgg gctgtatggc aagaactgca aagcaggaga catctacagt 720
   ttagaacagc tgattgtgct gtgggcctca tgggtcaggc aatgaggcct ttcaacaagg 780
   atttcaacca caactcgttc accaagaagc acgcagtccc taatacagta tttgattatg 840
   aagatcttgg ctataactat gacaaccttg aaatcagtgg tttaaactta aatgagatcg 900
   aggcgttaat agcaaaacgc aagtcacatg ctagagtctt tgctgggttc ctgttgtttg 960
   gattaggaac ttcggctgat atacatctgg aaatttgcaa gacatcggaa aactgccatg 1020
   atgctggtgt gattttcatc cttggaggtt ctgcagagat gcattgggca tacaaccgcc 1080
   tctacaagta tgacattaca gaagcattgc aggaatttga catcaaccct gaagatgttt 1140
   tccatgctga tgaaccattt ttcctgaggc tgtcggttgt tgctgtgaat ggaactgtca 1200
   ttccatcgtc tcatcttcac cagccaacga taatctatga accaggcgaa g          1251
<210> 7
   <211> 1209
   <212> DNA
   <213> Haliotis tuberculata
<400> 7
   atcaccatga cgaccatcag tcgggaagca tagcaggatc cggggtccgc aaggacgtga 60
   acaccttgac taaggctgag accgacaacc tgagggaggc gctgtggggt gtcatggcag 120
   accacggtcc caatggcttt caagctattg ctgctttcca tggaaaacca gctttgtgtc 180
   ccatgcctga tggccacaac tactcatgtt gtactcacgg catggctacc ttcccacact 240
   ggcatcgcct ctacaccaag cagatggagg atgcaatgag ggcgcatggg tctcatgtcg 300
   gcctgcccta ctgggactgg actgctgcct tcacccacct gccaacactg gtcaccgaca 360
   cggacaacaa cccccttcca catggacaca ttgattatct caatgtcagc acaactcgat 420
   ctccccgaga catgctgttc aacgaccccg agcatggatc agagtcgttc ttctacagac 480
   aagtcctctt agctctggaa caaactgatt tctgcaaatt cgaagttcag tttgagataa 540
   cccacaatgc catccattcc tggacaggtg gccacagccc ctacggaatg tccactctcg 600
   acttcactgc ctacgatcct ctcttctggc ttcaccactc caacaccgac agaatctggg 660
   ctgtctggca agctttgcaa gaatacagag gacttccata caaccatgcc aattgtgaga 720
   tccaggcaat gaaaacgccc ctgaggcctt tcagtgacga tatcaaccac aacccagtca 780
   caaaggctaa cgcgaagcca ttagatgtgt tcgagtataa tcggttgagc ttccagtacg 840
   acaacctcat cttccatgga tacagtattc cggaacttga tcgcgtgctt gaagaaagaa 900
   aggaggagga cagaatattt gctgccttcc ttctcagtgg aatcaagcgt agtgctgatg 960
   tagtgttcga catatgccag ccagaacacg aatgtgtgtt cgcagggact tttgcgattt 1020
   tgggagggga gctagaaatg ccctggtcct tcgacagact gttccgctat gatatcacca 1080
   aggtgatgaa gcagctacac ctgaggcatg actctgactt taccttcagg gtgaagattg 1140
   tcggcaccga cgaccacgag cttccttcag acagtgtcaa agcaccaact attgaatttg 1200
   aaccgggcg                                                         1209
<210> 8
   <211> 1535
   <212> DNA
   <213> Haliotis tuberculata
<400> 8
   tgcacagagg cggaaaccac gaagatgaac accatgatga cagactcgca gatgtcctga 60
   tcaggaaaga agttgacttc ctctccctgc aagaggccaa cgcaattaag gatgcactgt 120
   acaagctcca gaatgacgac agtaaagggg gctttgaggc catagctggc tatcacgggt 180
   atcctaatat gtgtccagaa agaggtaccg acaagtatcc ctgctgtgtc cacggaatgc 240
   ccgtgttccc ccactggcac cgcctgcata ccattcagat ggagagagct ctgaaaaacc 300
   atggctctcc aatgggcatt ccttactggg attggacaaa gaagatgtcg agtcttccat 360
   ctttctttgg agattccagc aacaacaacc ctttctacaa atattacatc cggggcgtgc 420
   agcacgaaac aaccagggac attaatcaga gactctttaa tcaaaccaag tttggtgaat 480
   ttgattacct atattaccta actctgcaag tcctggagga aaactcgtac tgtgactttg 540
   aagttcagta tgagatcctc cataacgccg tccactcctg gcttggagga actggaaagt 600
   attccatgtc taccctggag cattcggcct ttgaccctgt cttcatgatt caccactcga 660
   gtttggatag aatctggatc ctttggcaga agttgcaaaa gataagaatg aagccttact 720
   acgcattgga ttgtgctggc gacagactta tgaaagaccc cctgcatccc ttcaactacg 780
   aaaccgttaa tgaagatgaa ttcacccgca tcaactcttt cccaagcata ctgtttgacc 840
   actacaggtt caactatgaa tacgataaca tgagaatcag gggtcaggac atacatgaac 900
   ttgaagaggt aattcaggaa ttaagaaaca aagatcgcat atttgctggt tttgttttgt 960
   cgggcttacg gatatcagct acagtgaaag tattcattca ttcgaaaaac gatacaagtc 1020
   acgaagaata tgcaggagaa tttgcagttt tgggaggtga gaaggagatg ccgtgggcat 1080
   atgaaagaat gctgaaattg gacatctccg atgctgtaca caagcttcac gtgaaagatg 1140
   aagacatccg ttttagagtg gttgttactg cctacaacgg tgacgttgtt accaccaggc 1200
   tgtctcagcc attcatcgtc caccgtccag cccatgtggc tcacgacatc ttggtaatcc 1260
   cagtaggtgc gggccatgac cttccgccta aagtcgtagt aaagagcggc accaaagtcg 1320
   agtttacacc aatagattcg tcggtgaaca aagcaatggt ggagctgggc agctatactg 1380
   ctatggctaa atgcatcgtt ccccctttct cttaccacgg ctttgaactg gacaaagtct 1440
   acagcgtcga tcacggagac tactacattg ctgcaggtac ccacgcgttg tgtgagcaga 1500
   acctcaggct ccacatccac gtggaacacg agtag 1535
<210> 9
   <211> 1003
   <212> DNA
   <213> Haliotis tuberculata
<400> 9
   cacagactgt tcgtcaccca ggtggaagat gctctgatca ggcgaggatc gcctataggg 60
   gtcccctact gggactggac tcagcctatg gcgcatctcc caggacttgc agacaacgcc 120
   acctatagag atcccatcag cggggacagc agacacaacc ccttccacga tgttgaagtt 180
   gcctttgaaa atggacgtac agaacgtcac ccagatagta gattgtttga acaaccttta 240
   tttggcaaac atacgcgtct cttcgacagt atagtctatg cttttgagca ggaggacttc 300
   tgcgattttg aagttcaatt tgagatgacc cataataata ttcacgcctg gattggtggc 360
   ggcgagaagt attccatgtc ttctctacac tacacagcct tcgaccctat cttctacctt 420
   cgtcactcca acactgaccg gctctgggca atttggcaag cgttgcagat acgaagaaac 480
   aggccttaca aggctcattg tgcttggtct gaggaacgcc agcctctcaa acctttcgcc 540
   ttcagttccc cactgaacaa caacgaaaaa acctacgaaa actcggtgcc caccaacgtt 600
   tacgactacg aaggagtcct tggctatact tatgatgacc tcaacttcgg gggcatggac 660
   ctgggtcagc ttgaggaata catccagagg cagagacaga gagacaggac ctttgctggt 720
   ttctttctgt cacatattgg tacatcagcg aatgttgaaa tcattataga ccatgggact 780
   cttcatacct ccgtgggcac gtttgctgtt cttggcggag agaaggagat gaaatgggga 840
   tttgaccgtt tgtacaaata tgagattaca gatgaactga ggcaacttaa tctccgtgct 900
   gatgatgttt tcagcatctc tgttaaagta actgatgttg atggcagtga gctgtcctct 960
   gaactcatcc catctgctgc tatcatcttc gaacgaagcc ata                  1003
<210> 10
   <211> 1251
   <212> DNA
   <213> Haliotis tuberculata
<400> 10
   ttgaccatca ggacccgcat catgacacaa tcattaggaa aaatgttgat aatcttacac 60
   ccgaggaaat taattctctg aggcgggcaa tggcagacct tcaatcagac aaaaccgccg 120
   gtggattcca gcaaattgct gcttttcacg gggaacccaa atggtgccca agtcccgatg 180
   ctgagaagaa gttctcctgc tgtgtccatg gaatggctgt cttccctcac tggcacagac 240
   tcctgaccgt gcaaggcgag aatgccctga gaaagcatgg atgtctcgga gctctcccct 300
   actgggactg gactcggccc ctgtctcacc tacctgattt ggttttggta agtagcagaa 360
   ctacaccgat gccatattcc accgtggaag cccgaaaccc ctggtacagc ggccatattg 420
   atacagttgg tgttgacaca acaagaagcg tccgtcaaga actgtatgaa gctcctggat 480
   ttggccatta tactggggtc gctaagcaag tgcttctggc tttggagcag gatgacttct 540
   gtgattttga agtccagttt gagatagctc acaatttcat tcacgctctt gtcggcggaa 600
   gcgagccata tggtatggcg tcactccgtt acactactta tgatccaatt ttctacctcc 660
   atcattctaa cactgacaga ctctgggcta tatggcaggc tctacaaaag tacaggggca 720
   aaccttacaa ttccgccaac tgcgccattg cttctatgag aaaaccccta caaccctttg 780
   gtctgactga tgagatcaac ccggatgatg agacaagaca gcatgctgtt cctttcagtg 840
   tctttgatta caagaacaac ttcaattatg aatatgacac ccttgacttc aacggactat 900
   caatctccca gctggaccgt gaactgtcac ggagaaagtc tcatgacaga gtatttgccg 960
   gatttttgct gcatggtatt cagcagtctg cactagttaa attctttgtc tgcaaatcag 1020
   atgatgactg tgaccactat gctggtgaat tctacatcct tggtgatgaa gctgaaatgc 1080
   catggggcta tgatcgtctt tacaaatatg agatcactga gcagctcaat gccctggatc 1140
   tacacatcgg agatagattc ttcatcagat acgaagcgtt tgatcttcat ggtacaagtc 1200
   ttggaagcaa catcttcccc aaaccttctg tcatacatga cgaaggggca g          1251
<210> 11
   <211> 1244
   <212> DNA
   <213> Haliotis tuberculata
<400> 11
   gtcaccatca ggctgacgag tacgacgaag ttgtaactgc tgcaagccac atcagaaaga 60
   atttaaaaga tctgtcaaag ggagaagtag agagcctaag gtctgccttc ctgcaacttc 120
   agaacgacgg agtctatgag aatattgcca agttccacgg caagcctggg ttgtgtgatg 180
   ataacggtcg caaggttgcc tgttgtgtcc atggaatgcc caccttcccc cagtggcaca 240
   ggctctatgt cctccaggtg gagaatgctt tgctggagag aggatctgcc gtctctgtgc 300
   catactggga ctggactgaa acatttacag agctgccatc tttgattgct gaggctacct 360
   atttcaattc ccgtcaacaa acgtttgacc ctaatccttt cttcagaggt aaaatcagtt 420
   ttgagaatgc tgttacaaca cgtgatcccc agcctgagct gtacgttaac aggtactact 480
   accaaaacgt catgttggtt tttgaacagg acaactactg cgacttcgag atacagtttg 540
   agatggttca caatgttctc catgcttggc ttggtggaag agctacttat tctatttctt 600
   ctcttgatta ttctgcattc gaccctgtgt ttttccttca ccatgcgaac acagatagat 660
   tgtgggccat ctggcaggag ctgcagaggt acaggaagaa gccatacaat gaagcggatt 720
   gtgccattaa cctaatgcgc aaacctctac atcccttcga caacagtgat ctcaatcatg 780
   atcctgtaac ctttaaatac tcaaaaccca ctgatggctt tgactaccag aacaactttg 840
   gatacaagta tgacaacctt gagttcaatc atttcagtat tcccaggctt gaagaaatca 900
   ttcgtattag acaacgtcaa gatcgtgtgt ttgcaggatt cctccttcac aacattggga 960
   catccgcaac tgttgagata ttcgtctgtg tccctaccac cagcggtgag caaaactgtg 1020
   aaaacaaagc cggaacattt gccgtactcg gaggagaaac agagatggcg tttcattttg 1080
   acagactcta caggtttgac atcagtgaaa cactgaggga cctcggcata cagctggaca 1140
   gccatgactt tgacctcagc atcaagattc aaggagtaaa tggatcctac cttgatccac 1200
   acatcctgcc agagccatcc ttgatttttg tgcctggttc aagt                   1244
<210> 12
   <211> 1255
   <212> DNA
   <213> Haliotis tuberculata
<400> 12
   tctttcctgc gtcctgatgg gcattcagat gacatccttg tgagaaaaga agtgaacagc 60
   ctgacaacca gggagactgc atctctgatc catgctctga aaagtatgca ggaagaccat 120
   tcacctgacg ggttccaagc cattgcctct ttccatgctc tgccaccact ctgcccttca 180
   ccatctgcag ctcaccgtta tgcttgctgt gtccacggca tggctacatt tccccagtgg 240
   cacagattgt acactgtaca gttccaggat gcactgagga gacatggagc tacggtaggt 300
   gtaccgtatt gggattggct gcgaccgcag tctcacctac cagagcttgt caccatggag 360
   acataccatg atatttggag taacagagat ttccccaatc ctttctacca agccaatatt 420
   gagtttgaag gagaaaacat tacaacagag agagaagtca ttgcagacaa actttttgtc 480
   aaaggtggac acgtttttga taaactggtt cttcaaacaa gccatcctag cgctgagcag 540
   gaaaactact gtgactttga gattcagttt gaaattcttc acaacggcgt tcacacgtgg 600
   gtcggaggca gtcgtaccta ctctatcgga catcttcatt acgcattcta cgaccctctt 660
   ttctaccttc accatttcca gacagaccgt atttgggcaa tctggcaaga actccaggaa 720
   cagagagggc tctcgggtga tgaggctcac tgtgctctcg agcaaatgag agaaccattg 780
   aagcctttca gcttcggcgc tccttataac tggaatcagc tcacacagga tttctcccga 840
   cccgaggaca ccttcgacta caggaagttt ggttatgaat atgacaattt agaattcctg 900
   ggaatgtcag ttgctgaact ggatcaatac attattgaac atcaagaaaa tgatagagta 960
   ttcgctgggt tcctgttgag tggattcgga ggttccgcat cagttaattt ccaggtttgt 1020
   agagctgatt ccacatgtca ggatgctggg tacttcaccg ttcttggtgg cagtgctgag 1080
   atggcgtggg catttgacag gctttacaaa tatgacatta ctgaaactct ggagaaaatg 1140
   caccttcgat atgatgatga cttcacaatc tctgtcagtc tgaccgccaa caacggaact 1200
   gtcctgagca gcagtctaat cccaacaccg agtgtcatat tccagcgggg acatc       1255
<210> 13
   <211> 1248
   <212> DNA
   <213> Haliotis tuberculata
<400> 13
   gtgacataaa taccaggagc atgtcaccga accgtgttcg ccgtgagctg agcgatctgt 60
   ctgcgaggga cctgtctagt ctcaagtctg ctctgcgaga cctacaggag gatgatggcc 120
   ccaacggata ccaggctctt gcagccttcc atgggctacc agcaggctgc catgatagcc 180
   ggggaaatga gatcgcatgt tgcattcacg ggatgccgac cttcccccag tggcacagac 240
   tgtacaccct gcagttggag atggctctga ggagacatgg atcatctgtc gccatcccct 300
   actgggactg gacaaagcct atctccgaac tcccctcgct cttcaccagc cctgagtatt 360
   atgacccatg gcatgatgct gtggtaaaca acccattctc caaaggtttt gtcaaatttg 420
   caaataccta cacagtaaga gacccacagg agatgctgtt ccagctttgt gaacatggag 480
   agtcaatcct ctatgagcaa actcttcttg ctcttgagca aaccgactac tgtgattttg 540
   aggtacagtt tgaggtcctc cataacgtga tccactacct tgttggtgga cgtcagacct 600
   acgcattgtc ttctctgcat tatgcctcct acgacccatt cttctttata caccattcct 660
   ttgtggataa gatgtgggta gtatggcaag ctcttcaaaa gaggaggaaa cttccataca 720
   agcgagctga ctgtgctgtc aacctaatga ctaaaccaat gaggccattt gactccgata 780
   tgaatcagaa cccattcaca aagatgcacg cagttcccaa cacactctat gactacgaga 840
   cactgtacta cagctacgat aatctcgaaa taggtggcag gaatctcgac cagcttcagg 900
   ctgaaattga cagaagcaga agccacgatc gcgtttttgc tggattcttg cttcgtggaa 960
   tcggaacttc tgctgatgtc aggttttgga tttgtagaaa tgaaaatgac tgccacaggg 1020
   gtggaataat tttcatctta ggtggagcca aggaaatgcc atggtcattt gacagaaact 1080
   tcaagtttga tatcacccat gtactcgaga atgctggcat tagcccagag gacgtgtttg 1140
   atgctgagga gccattttat atcaaggttg agatccatgc tgttaacaag accatgatac 1200
   cgtcgtctgt gatcccagcc ccaactatca tctattctcc tggggaag 1248
<210> 14
   <211> 1207
   <212> DNA
   <213> Haliotis tuberculata
<400> 14
   gtcgcgctgc tgacagtgcg cactctgcca acattgctgg ctctggggtg aggaaggacg 60
   tcacgaccct cactgtgtct gagaccgaga acctaagaca ggctcttcaa ggtgtcatcg 120
   atgatactgg tcccaatggt taccaagcaa tagcatcctt ccacggaagt cctccaatgt 180
   gcgagatgaa cggccgcaag gttgcctgtt gtgctcacgg tatggcctcc ttcccacact 240
   ggcacagact gtatgtgaag cagatggaag atgccctggc tgaccacggg tcacatatcg 300
   gcatccctta ctgggactgg acaactgcct tcacagagtt acccgccctt gtcacagact 360
   ccgagaacaa tcccttccat gagggtcgca ttgatcatct cggtgtaacc acgtcacgtt 420
   cccccagaga catgctgttt aacgacccag agcaaggatc agagtcgttc ttctatagac 480
   aagtcctcct ggctttggag cagactgact actgccagtt cgaagtccag tttgagctga 540
   cccacaacgc cattcactcc tggacaggtg gacgtagccc ttacggaatg tcgaccctcg 600
   agttcacagc ctacgatcct ctcttctggc ttcaccactc caacaccgac agaatctggg 660
   ctgtctggca agcactgcag aaataccgag gactcccata caacgaagca cactgtgaaa 720
   tccaggttct gaaacagccc ttgaggccat tcaacgatga catcaaccac aatccaatca 780
   ccaagactaa tgccaggcct atcgattcat ttgattatga gaggtttaac tatcagtatg 840
   acacccttag cttccatggt aagagcatcc ctgaactgaa tgacctgctc gaggaaagaa 900
   aaagagaaga gagaacattt gctgccttcc ttcttcgtgg aatcggttgc agtgctgatg 960
   tcgtctttga catctgccgg cccaatggtg actgtgtctt tgcaggaacc tttgctgtgc 1020
   tgggagggga gctagaaatg ccttggtcct tcgacagact gttccgctat gacatcacca 1080
   gagtcatgaa tcagctccat ctccagtatg attcagattt cagtttcagg gtgaagcttg 1140
   ttgccaccaa tggcactgag ctttcatcag accttctcaa gtcaccaaca attgaacatg 1200
   aacttgg 1207
<210> 15
   <211> 1546
   <212> DNA
   <213> Haliotis tuberculata
<400> 15
   agcccacaga ggaccagttg aagaaacaga agtcactcgc caacatactg acggcaatgc 60
   acactttcat cgtaaggaag ttgattcgct gtccctggat gaagcaaaca acttgaagaa 120
   tgccctttac aagctacaga acgaccacag tctaacggga tacgaagcaa tctctggtta 180
   ccatggatac cccaatctgt gtccggaaga aggcgatgac aaaatacccc tgctgcgtcc 240
   ccggatgggc atctttcctt actggcacag actcttgacc attcaactgg aaagagctct 300
   tgagcacaat ggtgcactgc ttggtgttcc ttactgggac tggaacaagg acctgtcgtc 360
   actgccggcg ttcttctccg actccagcaa caacaatccc tacttcaagt accacatcgc 420
   cggtgttggt cacgacaccg tcagagagcc aactagtctt atatataacc agccccaaat 480
   ccatggttat gattatctct attacctagc attgaccacg cttgaagaaa acaattactg 540
   ggactttgag gttcagtatg agatcctcca caacgccgtc cactcctggc ttggaggatc 600
   ccagaagtat tccatgtcta ccctggagta ttcggccttt gaccctgtct ttatgatcct 660
   tcactcgggt ctagacagac tttggatcat ctggcaagaa cttcagaaga tcaggagaaa 720
   gccctacaac ttcgctaaat gtgcttatca tatgatggaa gagccactgg cgcccttcag 780
   ctatccatct atcaaccagg acgagttcac ccgtgccaac tccaagcctt ctacagtttt 840
   tgacagccat aagttcggct accattacga taacctgaat gttagaggtc acagcatcca 900
   agaactcaac acaatcatca atgacttgag aaacacagac agaatctacg caggatttgt 960
   tttgtcaggc atcggtacgt ctgctagtgt caagatctat ctccgaacag atgacaatga 1020
   cgaagaagtt ggaactttca ctgtcctggg aggagagagg gaaatgccat gggcctacga 1080
   gcgagttttc aagtatgaca tcacagaggt tgcagataga cttaaaatta agttatgggg 1140
   acacccttta acttccggaa ctggagatca catccttacg aatggaatcg gtggtaaaca 1200
   agagcctacc caaatccttt catcatctac agacctgcca atcatgacta cgatgttctt 1260
   gttatcccag tanggaagaa accttcacat ccctcccaaa gttgtcgtca agaaaggcac 1320
   ccgcatcgag ttccacccag tcgatgattc agttacgaga ccagttgttg atcttggaag 1380
   ctacactgca ctcttcaact gtgtggtacc accgttcaca taccacggat tcgaactgaa 1440
   ccacgtctat tctgtcaagc ctggtgacta ctatgttact ggacccacga gagacctttg 1500
   ccagaatgca gatgtcagga ttcatatcca tgttgaggat gagtaa                1546
<210> 16
   <211> 967
   <212> DNA
   <213> Megathura crenulata
<400> 16
   ggcctaccgt actgggactg gactgaaccc atgacacaca ttccgggtct ggcaggaaac 60
   aaaacttatg tggattctca tggtgcatcc cacacaaatc cttttcatag ttcagtgatt 120
   gcatttgaag aaaatgctcc ccacaccaaa agacaaatag atcaaagact ctttaaaccc 180
   gctacctttg gacaccacac agacctgttc aaccagattt tgtatgcctt tgaacaagaa 240
   gattactgtg actttgaagt ccaatttgag attacccata acacgattca cgcttggaca 300
   ggaggaagcg aacatttctc aatgtcgtcc ctacattaca cagctttcga tcctttgttt 360
   tactttcacc attctaacgt tgatcgtctt tgggccgttt ggcaagcctt acagatgaga 420
   cggcataaac cctacagggc ccactgcgcc atatctctgg aacatatgca tctgaaacca 480
   ttcgcctttt catctcccct taacaataac gaaaagactc atgccaatgc catgccaaac 540
   aagatctacg actatgaaaa tgtcctccat tacacatacg aagatttaac atttggaggc 600
   atctctctgg aaaacataga aaagatgatc cacgaaaacc agcaagaaga cagaatatat 660
   gccggttttc tcctggctgg catacgtact tcagcaaatg ttgatatctt cattaaaact 720
   accgattccg tgcaacataa ggctggaaca tttgcagtgc tcggtggaag caaggaaatg 780
   aagtggggat ttgatcgcgt tttcaagttt gacatcacgc acgttttgaa agatctcgat 840
   ctcactgctg atggcgattt cgaagttact gttgacatca ctgaagtcga tggaactaaa 900
   cttgcatcca gtcttattcc acatgcttct gtcattcgtg agcatgcacg tggtaagctg 960
   aatagag                                                           967
<210> 17
   <211> 1242
   <212> DNA
   <213> Megathura crenulata
<400> 17
   ttaaatttga caaagtgcca aggagtcgtc ttattcgaaa aaatgtagac cgtttgagcc 60
   ccgaggagat gaatgaactt cgtaaagccc tagccttact gaaagaggac aaaagtgccg 120
   gtggatttca gcagcttggt gcattccatg gggagccaaa atggtgtcct agtcccgaag 180
   catctaaaaa atttgcctgc tgtgttcacg gcatgtctgt gttccctcac tggcatcgac 240
   tgttgacggt tcagagtgaa aatgctttga gacgacatgg ctacgatgga gctttgccgt 300
   actgggattg gacctctcct cttaatcacc ttcccgaact ggcagatcat gagaagtacg 360
   tcgaccctga agatggggta gagaagcata acccttggtt cgatggtcat atagatacag 420
   tcgacaaaac aacaacaaga agtgttcaga ataaactctt cgaacagcct gagtttggtc 480
   attatacaag cattgccaaa caagtactgc tagcgttgga acaggacaat ttctgtgact 540
   ttgaaatcca atatgagatt gcccataact acatccatgc acttgtagga ggcgctcagc 600
   cttatggtat ggcatcgctt cgctacactg cttttgatcc actattctac ttgcatcact 660
   ctaatacaga tcgtatatgg gcaatatggc aggctttaca gaagtacaga ggaaaaccgt 720
   acaacgttgc taactgtgct gttacatcga tgagagaacc tttgcaacca tttggcctct 780
   ctgccaatat caacacagac catgtaacca aggagcattc agtgccattc aacgtttttg 840
   attacaagac caatttcaat tatgaatatg acactttgga atttaacggt ctctcaatct 900
   ctcagttgaa taaaaagctc gaagcgataa agagccaaga caggttcttt gcaggcttcc 960
   tgttatctgg tttcaagaaa tcatctcttg ttaaattcaa tatttgcacc gatagcagca 1020
   actgtcaccc cgctggagag ttttaccttc tgggtgatga aaacgagatg ccatgggcat 1080
   acgatagagt cttcaaatat gacataaccg aaaaactcca cgatctaaag ctgcatgcag 1140
   aagaccactt ctacattgac tatgaagtat ttgaccttaa accagcaagc ctgggaaaag 1200
   atttgttcaa gcagccttca gtcattcatg aaccaagaat ag                    1242
<210> 18
   <211> 1236
   <212> DNA
   <213> Megathura crenulata
<400> 18
   gtcaccatga aggcgaagta tatcaagctg aagtaacttc tgccaaccgt attcgaaaaa 60
   acattgaaaa tctgagcctt ggtgaactcg aaagtctgag agctgccttc ctggaaattg 120
   aaaacgatgg aacttacgaa tcaatagcta aattccatgg tagccctggt ttgtgccagt 180
   taaatggtaa ccccatctct tgttgtgtcc atggcatgcc aactttccct cactggcaca 240
   gactgtacgt ggttgtcgtt gagaatgccc tcctgaaaaa aggatcatct gtagctgttc 300
   cctattggga ctggacaaaa cgaatcgaac atttacctca cctgatttca gacgccactt 360
   actacaattc caggcaacat cactatgaga caaacccatt ccatcatggc aaaatcacac 420
   acgagaatga aatcactact agggatccca aggacagcct cttccattca gactactttt 480
   acgagcaggt cctttacgcc ttggagcagg ataacttctg tgatttcgag attcagttgg 540
   agatattaca caatgcattg cattctttac ttggtggcaa aggtaaatat tccatgtcaa 600
   accttgatta cgctgctttt gatcctgtgt tcttccttca tcacgcaacg actgacagaa 660
   tctgggcaat ctggcaagac cttcagaggt tccgaaaacg gccataccga gaagcgaatt 720
   gcgctatcca attgatgcac acgccactcc agccgtttga taagagcgac aacaatgacg 780
   aggcaacgaa aacgcatgcc actccacatg atggttttga atatcaaaac agctttggtt 840
   atgcttacga taatctggaa ctgaatcact actcgattcc tcagcttgat cacatgctgc 900
   aagaaagaaa aaggcatgac agagtattcg ctggcttcct ccttcacaat attggaacat 960
   ctgccgatgg ccatgtattt gtatgtctcc caactgggga acacacgaag gactgcagtc 1020
   atgaggctgg tatgttctcc atcttaggcg gtcaaacgga gatgtccttt gtatttgaca 1080
   gactttacaa acttgacata actaaagcct tgaaaaagaa cggtgtgcac ctgcaagggg 1140
   atttcgatct ggaaattgag attacggctg tgaatggatc tcatctagac agtcatgtca 1200
   tccactctcc cactatactg tttgaggccg gaacag                            1236
<210> 19
   <211> 241
   <212> DNA
   <213> Megathura crenulata
<400> 19
   attctgccca cacagatgat ggacacactg aaccagtgat gattcgcaaa gatatcacac 60
   aattggacaa gcgtcaacaa ctgtcactgg tgaaagccct cgagtccatg aaagccgacc 120
   attcatctga tgggttccag gcaatcgctt ccttccatgc tcttcctcct ctttgtccat 180
   caccagctgc ttcaaagagg tttgcgtgct gcgtccatgg catgccaacc ttcccgcaat 240
   g                                                                 241
<210> 20
   <211> 949
   <212> DNA
   <213> Megathura crenulata
<400> 20
   ggcctgccct actgggattg gaccatgcca atgagtcatt tgccagaact ggctacaagt 60
   gagacctacc tcgatccagt tactggggaa actaaaaaca accctttcca tcacgcccaa 120
   gtggcgtttg aaaatggtgt aacaagcagg aatcctgatg ccaaactttt tatgaaacca 180
   acttacggag accacactta cctcttcgac agcatgatct acgcatttga gcaggaagac 240
   ttctgcgact ttgaagtcca atatgagctc acgcataatg caatacatgc atgggttgga 300
   ggcagtgaaa agtattcaat gtcttctctt cactacactg cttttgatcc tatattttac 360
   ctccatcact caaatgttga tcgtctctgg gccatttggc aagctcttca aatcaggaga 420
   ggcaagtctt acaaggccca ctgcgcctcg tctcaagaaa gagaaccatt aaagcctttt 480
   gcattcagtt ccccactgaa caacaacgag aaaacgtacc acaactctgt ccccactaac 540
   gtttatgact atgtgggagt tttgcactat cgatatgatg accttcagtt tggcggtatg 600
   accatgtcag aacttgagga atatattcac aagcagacac aacatgatag aacctttgca 660
   ggattcttcc tttcatatat tggaacatca gcaagcgtag atatcttcat caatcgagaa 720
   ggtcatgata aatacaaagt gggaagtttt gtagtacttg gtggatccaa agaaatgaaa 780
   tggggctttg atagaatgta caagtatgag atcactgagg ctctgaagac gctgaatgtt 840
   gcagtggatg atgggttcag cattactgtt gagatcaccg atgttgatgg atctccccca 900
   tctgcagatc tcattccacc tcctgctata atctttgaac gtggtcatg             949
<210> 21
   <211> 760
   <212> DNA
   <213> Megathura crenulata
<400> 21
   ctgatgccaa agactttggc catagcagaa aaatcaggaa agccgttgat tctctgacag 60
   tcgaagaaca aacttcgttg aggcgagcta tggcagatct acaggacgac aaaacatcag 120
   ggggtttcca gcagattgca gcattccacg gagaaccaaa atggtgtcca agccccgaag 180
   cggagaaaaa atttgcatgc tgtgttcatg gaatggctgt tttccctcac tggcacagat 240
   tgctgacagt tcaaggagaa aatgctctga ggaaacatgg ctttactggt ggactgccct 300
   actgggactg gactcgatca atgagcgccc ttccacattt tgttgctgat cctacttaca 360
   atgatgctat ttccagccag gaagaagata acccatggca tcatggtcac atagactctg 420
   ttgggcatga tactacaaga gatgtgcgtg atgatcttta tcaatctcct ggtttcggtc 480
   actacacaga tattgcacaa caagtccttc tggcctttga gcaggacagt ttctgtgatt 540
   ttgaggtaca atttgaaatt gcccataatt tcatacatgc actgattggt ggtaacgaac 600
   catacagtat gtcatctttg aggtatacta catacgatcc aatcttcttc ttgcaccact 660
   ccagtacaga ccgactttgg gccatctggc aagcaatcac tagtgcggcc gcctgcaggt 720
   cgaccataag ggagagctcc caacgcgttg gatgcaatct                       760
<210> 22
   <211> 323
   <212> DNA
   <213> Megathura crenulata
<400> 22
   gttcacacca ggctgatgaa tatcgtgagg cagtaacaag cgctagccac ataagaaaaa 60
   atatccggga cctctcagag ggagaaattg agagcatcag atctgctttc ctccaaattc 120
   aaaaagaggg tatatatgaa aacattgcaa agttccatgg aaaaccagga ctttgtgaac 180
   atgatggaca tcctgttgct tgttgtgtcc atggcatgcc cacctttccc cactggcaca 240
   gactgtacgt tcttcaggtg gagaatgcgc tcttagaacg agggtctgca gttgctgttc 300
   cttactggga ctggacccta cct                                         323
<210> 23
   <211> 988
   <212> DNA
   <213> Megathura crenulata
<400> 23
   atggctgtgt ttccgcactg gcacagactg tttgtgaaac agatggagga cgcacttgct 60
   gctcatggag ctcatattgg cataccatac tgggattgga caagtgcgtt tagtcatctg 120
   cccgccctag tgactgacca cgagaacaat cccttccacc acggccatat tggtcatctg 180
   aatgtggata catctcgatc tccaagagac atgctgttta atgatcctga acaaggctca 240
   gaatcattct tctacagaca ggttctcttg actctagaac agacagactt ctgccaattt 300
   gaagttcagt ttgaacttac acacaatgcc atccactctt ggactggagg acatactcca 360
   tatggaatgt catcactgga atatacagca tatgatccac tcttttatct ccaccattcc 420
   aacactgatc gtatctgggc catctggcag gcactccaga aatatagagg tcttccatac 480
   aacgcagctc actgcgatat ccaagttctg aaacaacctc ttaaaccatt cagcgagtcc 540
   aggaatccaa acccagtcac cagagccaat tctagggccg ttgattcatt tgattatgag 600
   aaattcaatt atcaatatga cacacttacc ttccacggac tttctatccc agaacttgat 660
   gccatgcttc aagagagaaa gaaggaagag agaacatttg cagccttcct gttgcacgga 720
   tttggcgcca gtgctgatgt ttcgtttgat gtctgcacac ctgatggtca ttgtgccttt 780
   gctggaacct tcgcggtact tggtggggag cttgagatgc cctggtcctt tgaaagattg 840
   ttccgttacg atatcacaaa ggttctcaag cagatgaatc ttcactatga ttctgagttc 900
   cactttgagt tgaagattgt tggcacagat ggaacagaac tgccatcgga tcgtatcaag 960
   agccctacca ttgaacacca tggaggag                                   988
<210> 24
   <211> 310
   <212> DNA
   <213> Megathura crenulata
<400> 24
   gtcacgatca cagtgaacgt cacgatggat ttttcaggaa ggaagtcggt tccctgtccc 60
   tggatgaagc caatgacctt aaaaatgcac tgtacaagct gcagaatgat cagggtccca 120
   atggatatga atcaatagcc ggttaccatg gctatccatt cctctgccct gaacatggtg 180
   aagaccagta cgcatgctgt gtccacggaa tgcctgtatt tccacattgg cacagacttc 240
   atacaatcca gtttgagaga gctctcaaag aacatggttc tcatttgggt ctgccatact 300
   gggactggac                                                       310
<210> 25
   <211> 422
   <212> PRT
   <213> Haliotis tuberculata
<220>
   <221> SIGNAL
   <222> (1)..(15)
<400> 25
   Leu Val Gln Phe Leu Leu Val Ala Leu Val Ala Gly Ala Gly Ala Asp
    1               5                  10                  15
   Asn Val Val Arg Lys Asp Val Ser His Leu Thr Asp Asp Glu Val Gln
               20                  25                  30
   Ala Leu His Gly Ala Leu His Asp Val Thr Ala Ser Thr Gly Pro Leu
           35                  40                  45
   Ser Phe Glu Asp Ile Thr Ser Tyr His Ala Ala Pro Ala Ser Cys Asp
       50                  55                  60
   Tyr Lys Gly Arg Lys Ile Ala Cys Cys Val His Gly Met Pro Ser Phe
   65                  70                  75                  80
   Pro Phe Trp His Arg Ala Tyr Val Val Gln Ala Glu Arg Ala Leu Leu
                   85                  90                  95
   Ser Lys Arg Lys Thr Val Gly Met Pro Tyr Trp Asp Trp Thr Gln Thr
               100                 105                 110
   Leu Thr His Leu Pro Ser Leu Val Thr Glu Pro Ile Tyr Ile Asp Ser
           115                 120                 125
   Lys Gly Gly Lys Ala Gln Thr Asn Tyr Trp Tyr Arg Gly Glu Ile Ala
       130                 135                 140
   Phe Ile Asn Lys Lys Thr Ala Arg Ala Val Asp Asp Arg Leu Phe Glu
   145                 150                 155                 160
   Lys Val Glu Pro Gly His Tyr Thr His Leu Met Glu Thr Val Leu Asp
                   165                 170                 175
   Ala Leu Glu Gln Asp Glu Phe Cys Lys Phe Glu Ile Gln Phe Glu Leu
               180                 185                 190
   Ala His Asn Ala Ile His Tyr Leu Val Gly Gly Lys Phe Glu Tyr Ser
           195                 200                 205
   Met Ser Asn Leu Glu Tyr Thr Ser Tyr Asp Pro Ile Phe Phe Leu His
       210                 215                 220
   His Ser Asn Val Asp Arg Leu Phe Ala Ile Trp Gln Arg Leu Gln Glu
   225                 230                 235                 240
   Leu Arg Gly Lys Asn Pro Asn Ala Met Asp Cys Ala His Glu Leu Ala
   245 250 255
   His Gln Gln Leu Gln Pro Phe Asn Arg Asp Ser Asn Pro Val Gln Leu
   260 265 270
   Thr Lys Asp His Ser Thr Pro Ala Asp Leu Phe Asp Tyr Lys Gln Leu
   275 280 285
   Gly Tyr Ser Tyr Asp Ser Leu Asn Leu Asn Gly Met Thr Pro Glu Gln
   290 295 300
   Leu Lys Thr Glu Leu Asp Glu Arg His Ser Lys Glu Arg Ala Phe Ala
   305 310 315 320
   Ser Phe Arg Leu Ser Gly Phe Gly Gly Ser Ala Asn Val Val Val Tyr
   325 330 335
   Ala Cys Val Pro Asp Asp Asp Pro Arg Ser Asp Asp Tyr Cys Glu Lys
   340 345 350
   Ala Gly Asp Phe Phe Ile Leu Gly Gly Gln Ser Glu Met Pro Trp Arg
   355 360 365
   Phe Tyr Arg Pro Phe Phe Tyr Asp Val Thr Glu Ala Val His His Leu
   370 375 380
   Gly Val Pro Leu Ser Gly His Tyr Tyr Val Lys Thr Glu Leu Phe Ser
   385 390 395 400
   Val Asn Gly Thr Ala Leu Ser Pro Asp Leu Leu Pro Gln Pro Thr Val
   405 410 415
   Ala Tyr Arg Pro Gly Lys
   420
<210> 26
   <211> 419
   <212> PRT
   <213> Haliotis tuberculata
<400> 26
   Gly His Leu Asp Pro Pro Val His His Arg His Asp Asp Asp Leu Ile
   1 5 10 15
   Val Arg Lys Asn Ile Asp His Leu Thr Arg Glu Glu Glu Tyr Glu Leu
   20 25 30
   Arg Met Ala Leu Glu Arg Phe Gln Ala Asp Thr Ser Val Asp Gly Tyr
   35 40 45
   Gln Ala Thr Val Glu Tyr His Gly Leu Pro Ala Arg Cys Pro Arg Pro
   50 55 60
   Asp Ala Lys Val Arg Phe Ala Cys Cys Met His Gly Met Ala Ser Phe
   65 70 75 80
   Pro His Trp His Arg Leu Phe Val Thr Gln Val Glu Asp Ala Leu Val
   85 90 95
   Arg Arg Gly Ser Pro Ile Gly Val Pro Tyr Trp Asp Trp Thr Lys Pro
   100 105 110
   Met Thr His Leu Pro Asp Leu Ala Ser Asn Glu Thr Tyr Val Asp Pro
   115 120 125
   Tyr Gly His Thr His His Asn Pro Phe Phe Asn Ala Asn Ile Ser Phe
   130 135 140
   Glu Glu Gly His His His Thr Ser Arg Met Ile Asp Ser Lys Leu Phe
   145 150 155 160
   Ala Pro Val Ala Phe Gly Glu His Ser His Leu Phe Asp Gly Ile Leu
   165 170 175
   Tyr Ala Phe Glu Gln Glu Asp Phe Cys Asp Phe Glu Ile Gln Phe Glu
   180 185 190
   Leu Val His Asn Ser Ile His Ala Trp Ile Gly Gly Ser Glu Asp Tyr
   195 200 205
   Ser Met Ala Thr Leu His Tyr Thr Ala Phe Asp Pro Ile Phe Tyr Leu
   210 215 220
   His His Ser Asn Val Asp Arg Leu Trp Ala Ile Trp Gln Ala Leu Gln
   225 230 235 240
   Ile Arg Arg His Lys Pro Tyr Gln Ala His Cys Ala Gln Ser Val Glu
   245 250 255
   Gln Leu Pro Met Lys Pro Phe Ala Phe Pro Ser Pro Leu Asn Asn Asn
   260 265 270
   Glu Lys Thr His Ser His Ser Val Pro Thr Asp Ile Tyr Asp Tyr Glu
   275 280 285
   Glu Val Leu His Tyr Ser Tyr Asp Asp Leu Thr Phe Gly Gly Met Asn
   290 295 300
   Leu Glu Glu Ile Glu Glu Ala Ile His Leu Arg Gln Gln His Glu Arg
   305 310 315 320
   Val Phe Ala Gly Phe Leu Leu Ala Gly Ile Gly Thr Ser Ala Leu Val
   325 330 335
   Asp Ile Phe Ile Asn Lys Pro Gly Asn Gln Pro Leu Lys Ala Gly Asp
   340 345 350
   Ile Ala Ile Leu Gly Gly Ala Lys Glu Met Pro Trp Ala Phe Asp Arg
   355 360 365
   Leu Tyr Lys Val Glu Ile Thr Asp Ser Leu Lys Thr Leu Ser Leu Asp
   370 375 380
   Val Asp Gly Asp Tyr Glu Val Thr Phe Lys Ile His Asp Met His Gly
   385 390 395 400
   Asn Ala Leu Asp Thr Asp Leu Ile Pro His Ala Ala Val Val Ser Glu
   405 410 415
   Pro Ala His
<210> 27
   <211> 414
   <212> PRT
   <213> Haliotis tuberculata
<400> 27
   Pro Thr Phe Glu Asp Glu Lys His Ser Leu Arg Ile Arg Lys Asn Val
   1 5 10 15
   Asp Ser Leu Thr Pro Glu Glu Thr Asn Glu Leu Arg Lys Ala Leu Glu
   20 25 30
   Leu Leu Glu Asn Asp His Thr Ala Gly Gly Phe Asn Gln Leu Gly Ala
   35 40 45
   Phe His Gly Glu Pro Lys Trp Cys Pro Asn Pro Glu Ala Glu His Lys
   50 55 60
   Val Ala Cys Cys Val His Gly Met Ala Val Phe Pro His Trp His Arg
   65 70 75 80
   Leu Leu Ala Leu Gln Ala Glu Asn Ala Leu Arg Lys His Gly Tyr Ser
   85 90 95
   Gly Ala Leu Pro Tyr Trp Asp Trp Thr Arg Pro Leu Ser Gln Leu Pro
   100 105 110
   Asp Leu Val Ser His Glu Gln Tyr Thr Asp Pro Ser Asp His His Val
   115 120 125
   Lys His Asn Pro Trp Phe Asn Gly His Ile Asp Thr Val Asn Gln Asp
   130 135 140
   Thr Thr Arg Ser Val Arg Glu Asp Leu Tyr Gln Gln Pro Glu Phe Gly
   145 150 155 160
   His Phe Thr Asp Ile Ala Gln Gln Val Leu Leu Ala Leu Glu Gln Asp
   165 170 175
   Asp Phe Cys Ser Phe Glu Val Gln Tyr Glu Ile Ser His Asn Phe Ile
   180 185 190
   His Ala Leu Val Gly Gly Thr Asp Ala Tyr Gly Met Ala Ser Leu Arg
   195 200 205
   Tyr Thr Ala Tyr Asp Pro Ile Phe Phe Leu His His Ser Asn Thr Asp
   210 215 220
   Arg Ile Trp Ala Ile Trp Gln Ser Leu Gln Lys Tyr Arg Gly Lys Pro
   225 230 235 240
   Tyr Asn Thr Ala Asn Cys Ala Ile Glu Ser Met Arg Arg Pro Leu Gln
   245 250 255
   Pro Phe Gly Leu Ser Ser Ala Ile Asn Pro Asp Arg Ile Thr Arg Glu
   260 265 270
   His Ala Ile Pro Phe Asp Val Phe Asn Tyr Arg Asp Asn Leu His Tyr
   275 280 285
   Val Tyr Asp Thr Leu Glu Phe Asn Gly Leu Ser Ile Ser Gln Leu Asp
   290 295 300
   Arg Glu Leu Glu Lys Ile Lys Ser His Glu Arg Val Phe Ala Gly Phe
   305 310 315 320
   Leu Leu Ser Gly Ile Lys Lys Ser Ala Leu Val Lys Phe Glu Val Cys
   325 330 335
   Thr Pro Pro Asp Asn Cys His Lys Ala Gly Glu Phe Tyr Leu Leu Gly
   340 345 350
   Asp Glu Asn Glu Met Ala Trp Ala Tyr Asp Arg Leu Phe Lys Tyr Asp
   355 360 365
   Ile Thr Gln Val Leu Glu Ala Asn His Leu His Phe Tyr Asp His Leu
   370 375 380
   Phe Ile Arg Tyr Glu Val Phe Asp Leu Lys Gly Val Ser Leu Gly Thr
   385 390 395 400
   Asp Leu Phe His Thr Ala Asn Val Val His Asp Ser Gly Thr
   405 410
<210> 28
   <211> 413
   <212> PRT
   <213> Haliotis tuberculata
<400> 28
   Gly Thr Arg Asp Arg Asp Asn Tyr Val Glu Glu Val Thr Gly Ala Ser
    1               5                  10                  15
   His Ile Arg Lys Asn Leu Asn Asp Leu Asn Thr Gly Glu Met Glu Ser
               20                  25                  30
   Leu Arg Ala Ala Phe Leu His Ile Gln Asp Asp Gly Thr Tyr Glu Ser
           35                  40                  45
   Ile Ala Gln Tyr His Gly Lys Pro Gly Lys Cys Gln Leu Asn Asp His
       50                  55                  60
   Asn Ile Ala Cys Cys Val His Gly Met Pro Thr Phe Pro Gln Trp His
    65                 70                  75                  80
   Arg Leu Tyr Val Val Gln Val Glu Asn Ala Leu Leu Asn Arg Gly Ser
                   85                  90                  95
   Gly Val Ala Val Pro Tyr Trp Glu Trp Thr Ala Pro Ile Asp His Leu
               100                 105                 110
   Pro His Phe Ile Asp Asp Ala Thr Tyr Phe Asn Ser Arg Gln Gln Arg
           115                 120                 125
   Tyr Asp Pro Asn Pro Phe Phe Arg Gly Lys Val Thr Phe Glu Asn Ala
       130                 135                 140
   Val Thr Thr Arg Asp Pro Gln Ala Gly Leu Phe Asn Ser Asp Tyr Met
   145                 150                 155                 160
   Tyr Glu Asn Val Leu Leu Ala Leu Glu Gln Glu Asn Tyr Cys Asp Phe
                   165                 170                 175
   Glu Ile Gln Phe Glu Leu Val His Asn Ala Leu His Ser Met Leu Gly
               180                 185                 190
   Gly Lys Gly Gln Tyr Ser Met Ser Ser Leu Asp Tyr Ser Ala Phe Asp
           195                 200                 205
   Pro Val Phe Phe Leu His His Ala Asn Thr Asp Arg Leu Trp Ala Ile
       210                 215                 220
   Trp Gln Glu Leu Gln Arg Phe Arg Glu Leu Pro Tyr Glu Glu Ala Asn
   225                 230                 235                 240
   Cys Ala Ile Asn Leu Met His Gln Pro Leu Lys Pro Phe Ser Asp Pro
                   245                 250                 255
   His Glu Asn His Asp Asn Val Thr Leu Lys Tyr Ser Lys Pro Gln Asp
               260                 265                 270
   Gly Phe Asp Tyr Gln Asn His Phe Gly Tyr Lys Tyr Asp Asn Leu Glu
           275                 280                 285
   Phe His His Leu Ser Ile Pro Ser Leu Asp Ala Thr Leu Lys Gln Arg
       290                 295                 300
   Arg Asn His Asp Arg Val Phe Ala Gly Phe Leu Leu His Asn Ile Gly
   305                 310                 315                 320
   Thr Ser Ala Asp Ile Thr Ile Tyr Ile Cys Leu Pro Asp Gly Arg Arg
                   325                 330                 335
   Gly Asn Asp Cys Ser His Glu Ala Gly Thr Phe Tyr Ile Leu Gly Gly
               340                 345                 350
   Glu Thr Glu Met Pro Phe Ile Phe Asp Arg Leu Tyr Lys Phe Glu Ile
           355                 360                 365
   Thr Lys Pro Leu Gln Gln Leu Gly Val Lys Leu His Gly Gly Val Phe
       370                 375                 380
   Glu Leu Glu Leu Glu Ile Lys Ala Tyr Asn Gly Ser Tyr Leu Asp Pro
   385                 390                 395                 400
   His Thr Phe Asp Pro Thr Ile Ile Phe Glu Pro Gly Thr
                   405                 410
<210> 29
   <211> 420
   <212> PRT
   <213> Haliotis tuberculata
<400> 29
   Asp Thr His Ile Leu Asp His Asp His Glu Glu Glu Ile Leu Val Arg
    1               5                  10                  15
   Lys Asn Ile Ile Asp Leu Ser Pro Arg Glu Arg Val Ser Leu Val Lys
               20                  25                  30
   Ala Leu Gln Arg Met Lys Asn Asp Arg Ser Ala Asp Gly Tyr Gln Ala
           35                  40                  45
   Ile Ala Ser Phe His Ala Leu Pro Pro Leu Cys Pro Asn Pro Ser Ala
       50                  55                  60
   Ala His Arg Tyr Ala Cys Cys Val His Gly Met Ala Thr Phe Pro Gln
   65                  70                  75                  80
   Trp His Arg Leu Tyr Thr Val Gln Val Gln Asp Ala Leu Arg Arg His
                   85                  90                  95
   Gly Ser Leu Val Gly Ile Pro Tyr Trp Asp Trp Thr Lys Pro Val Asn
               100                 105                 110
   Glu Leu Pro Glu Leu Leu Ser Ser Ala Thr Phe Tyr His Pro Ile Arg
           115                 120                 125
   Asn Ile Asn Ile Ser Asn Pro Phe Leu Gly Ala Asp Ile Glu Phe Glu
       130                 135                 140
   Gly Pro Gly Val His Thr Glu Arg His Ile Asn Thr Glu Arg Leu Phe
   145                 150                 155                 160
   His Ser Gly Asp His Asp Gly Tyr His Asn Trp Phe Phe Glu Thr Val
                   165                 170                 175
   Leu Phe Ala Leu Glu Gln Glu Asp Tyr Cys Asp Phe Glu Ile Gln Phe
               180                 185                 190
   Glu Ile Ala His Asn Gly Ile His Thr Trp Ile Gly Gly Ser Ala Val
           195                 200                 205
   Tyr Gly Met Gly His Leu His Tyr Ala Ser Tyr Asp Pro Ile Phe Tyr
       210                 215                 220
   Ile His His Ser Gln Thr Asp Arg Ile Trp Ala Ile Trp Gln Glu Leu
   225                 230                 235                 240
   Gln Lys Tyr Arg Gly Leu Ser Gly Ser Glu Ala Asn Cys Ala Ile Glu
                   245                 250                 255
   His Met Arg Thr Pro Leu Lys Pro Phe Ser Phe Gly Pro Pro Tyr Asn
               260                 265                 270
   Leu Asn Ser His Thr Gln Glu Tyr Ser Lys Pro Glu Asp Thr Phe Asp
           275                 280                 285
   Tyr Lys Lys Phe Gly Tyr Arg Tyr Asp Ser Leu Glu Leu Glu Gly Arg
       290                 295                 300
   Ser Ile Ser Arg Ile Asp Glu Leu Ile Gln Gln Arg Gln Glu Lys Asp
   305                 310                 315                 320
   Arg Thr Phe Ala Gly Phe Leu Leu Lys Gly Phe Gly Thr Ser Ala Ser
                   325                 330                 335
   Val Ser Leu Gln Val Cys Arg Val Asp His Thr Cys Lys Asp Ala Gly
               340                 345                 350
   Tyr Phe Thr Ile Leu Gly Gly Ser Ala Glu Met Pro Trp Ala Phe Asp
           355                 360                 365
   Arg Leu Tyr Lys Tyr Asp Ile Thr Lys Thr Leu His Asp Met Asn Leu
       370                 375                 380
   Arg His Glu Asp Thr Phe Ser Ile Asp Val Thr Ile Thr Ser Tyr Asn
   385                 390                 395                 400
   Gly Thr Val Leu Ser Gly Asp Leu Ile Gln Thr Pro Ser Ile Ile Phe
                   405                 410                 415
   Val Pro Gly Arg
               420
<210> 30
   <211> 417
   <212> PRT
   <213> Haliotis tuberculata
<400> 30
   His Lys Leu Asn Ser Arg Lys His Thr Pro Asn Arg Val Arg His Glu
   1 5 10 15
   Leu Ser Ser Leu Ser Ser Arg Asp Ile Ala Ser Leu Lys Ala Ala Leu
   20 25 30
   Thr Ser Leu Gln His Asp Asn Gly Thr Asp Gly Tyr Gln Ala Ile Ala
   35 40 45
   Ala Phe His Gly Val Pro Ala Gln Cys His Glu Pro Ser Gly Arg Glu
   50 55 60
   Ile Ala Cys Cys Ile His Gly Met Ala Thr Phe Pro His Trp His Arg
   65 70 75 80
   Leu Tyr Thr Leu Gln Leu Glu Gln Ala Leu Arg Arg His Gly Ser Ser
   85 90 95
   Val Ala Val Pro Tyr Trp Asp Trp Thr Lys Pro Ile Thr Glu Leu Pro
   100 105 110
   His Ile Leu Thr Asp Gly Glu Tyr Tyr Asp Val Trp Gln Asn Ala Val
   115 120 125
   Leu Ala Asn Pro Phe Ala Arg Gly Tyr Val Lys Ile Lys Asp Ala Phe
   130 135 140
   Thr Val Arg Asn Val Gln Glu Ser Leu Phe Lys Met Ser Ser Phe Gly
   145 150 155 160
   Lys His Ser Leu Leu Phe Asp Gln Ala Leu Leu Ala Leu Glu Gln Thr
   165 170 175
   Asp Tyr Cys Asp Phe Glu Val Gln Phe Glu Val Met His Asn Thr Ile
   180 185 190
   His Tyr Leu Val Gly Gly Arg Gln Thr Tyr Ala Phe Ser Ser Leu Glu
   195 200 205
   Tyr Ser Ser Tyr Asp Pro Ile Phe Phe Ile His His Ser Phe Val Asp
   210 215 220
   Lys Ile Trp Ala Val Trp Gln Glu Leu Gln Ser Arg Arg His Leu Gln
   225 230 235 240
   Phe Arg Thr Ala Asp Cys Ala Val Gly Leu Met Gly Gln Ala Met Arg
   245 250 255
   Pro Phe Asn Lys Asp Phe Asn His Asn Ser Phe Thr Lys Lys His Ala
   260 265 270
   Val Pro Asn Thr Val Phe Asp Tyr Glu Asp Leu Gly Tyr Asn Tyr Asp
   275 280 285
   Asn Leu Glu Ile Ser Gly Leu Asn Leu Asn Glu Ile Glu Ala Leu Ile
   290 295 300
   Ala Lys Arg Lys Ser His Ala Arg Val Phe Ala Gly Phe Leu Leu Phe
   305 310 315 320
   Gly Leu Gly Thr Ser Ala Asp Ile His Leu Glu Ile Cys Lys Thr Ser
   325 330 335
   Glu Asn Cys His Asp Ala Gly Val Ile Phe Ile Leu Gly Gly Ser Ala
   340 345 350
   Glu Met His Trp Ala Tyr Asn Arg Leu Tyr Lys Tyr Asp Ile Thr Glu
           355                 360                 365
   Ala Leu Gln Glu Phe Asp Ile Asn Pro Glu Asp Val Phe His Ala Asp
       370                 375                 380
   Glu Pro Phe Phe Leu Arg Leu Ser Val Val Ala Val Asn Gly Thr Val
   385                 390                 395                 400
   Ile Pro Ser Ser His Leu His Gln Pro Thr Ile Ile Tyr Glu Pro Gly
                   405                 410                 415
   Glu
<210> 31
   <211> 403
   <212> PRT
   <213> Haliotis tuberculata
<400> 31
   Asp His His Asp Asp His Gln Ser Gly Ser Ile Ala Gly Ser Gly Val
   1               5                   10                  15
   Arg Lys Asp Val Asn Thr Leu Thr Lys Ala Glu Thr Asp Asn Leu Arg
               20                  25                  30
   Glu Ala Leu Trp Gly Val Met Ala Asp His Gly Pro Asn Gly Phe Gln
           35                  40                  45
   Ala Ile Ala Ala Phe His Gly Lys Pro Ala Leu Cys Pro Met Pro Asp
       50                  55                  60
   Gly His Asn Tyr Ser Cys Cys Thr His Gly Met Ala Thr Phe Pro His
   65                  70                  75                  80
   Trp His Arg Leu Tyr Thr Lys Gln Met Glu Asp Ala Met Arg Ala His
                   85                  90                  95
   Gly Ser His Val Gly Leu Pro Tyr Trp Asp Trp Thr Ala Ala Phe Thr
               100                 105                 110
   His Leu Pro Thr Leu Val Thr Asp Thr Asp Asn Asn Pro Phe Gln His
           115                 120                 125
   Gly His Ile Asp Tyr Leu Asn Val Ser Thr Thr Arg Ser Pro Arg Asp
       130                 135                 140
   Met Leu Phe Asn Asp Pro Glu His Gly Ser Glu Ser Phe Phe Tyr Arg
   145                 150                 155                 160
   Gln Val Leu Leu Ala Leu Glu Gln Thr Asp Phe Cys Lys Phe Glu Val
                   165                 170                 175
   Gln Phe Glu Ile Thr His Asn Ala Ile His Ser Trp Thr Gly Gly His
               180                 185                 190
   Ser Pro Tyr Gly Met Ser Thr Leu Asp Phe Thr Ala Tyr Asp Pro Leu
           195                 200                 205
   Phe Trp Leu His His Ser Asn Thr Asp Arg Ile Trp Ala Val Trp Gln
       210                 215                 220
   Ala Leu Gln Glu Tyr Arg Gly Leu Pro Tyr Asn His Ala Asn Cys Glu
   225                 230                 235                 240
   Ile Gln Ala Met Lys Thr Pro Leu Arg Pro Phe Ser Asp Asp Ile Asn
                   245                 250                 255
   His Asn Pro Val Thr Lys Ala Asn Ala Lys Pro Leu Asp Val Phe Glu
               260                 265                 270
   Tyr Asn Arg Leu Ser Phe Gln Tyr Asp Asn Leu Ile Phe His Gly Tyr
           275                 280                 285
   Ser Ile Pro Glu Leu Asp Arg Val Leu Glu Glu Arg Lys Glu Glu Asp
       290                 295                 300
   Arg Ile Phe Ala Ala Phe Leu Leu Ser Gly Ile Lys Arg Ser Ala Asp
   305                 310                 315                 320
   Val Val Phe Asp Ile Cys Gln Pro Glu His Glu Cys Val Phe Ala Gly
                   325                 330                 335
   Thr Phe Ala Ile Leu Gly Gly Glu Leu Glu Met Pro Trp Ser Phe Asp
               340                 345                 350
   Arg Leu Phe Arg Tyr Asp Ile Thr Lys Val Met Lys Gln Leu His Leu
           355                 360                 365
   Arg His Asp Ser Asp Phe Thr Phe Arg Val Lys Ile Val Gly Thr Asp
       370                 375                 380
   Asp His Glu Leu Pro Ser Asp Ser Val Lys Ala Pro Thr Ile Glu Phe
   385                 390                 395                 400
   Glu Pro Gly
<210> 32
   <211> 511
   <212> PRT
   <213> Haliotis tuberculata
<400> 32
   Val His Arg Gly Gly Asn His Glu Asp Glu His His Asp Asp Arg Leu
   1               5                   10                  15
   Ala Asp Val Leu Ile Arg Lys Glu Val Asp Phe Leu Ser Leu Gln Glu
               20                  25                  30
   Ala Asn Ala Ile Lys Asp Ala Leu Tyr Lys Leu Gln Asn Asp Asp Ser
           35                  40                  45
   Lys Gly Gly Phe Glu Ala Ile Ala Gly Tyr His Gly Tyr Pro Asn Met
       50                  55                  60
   Cys Pro Glu Arg Gly Thr Asp Lys Tyr Pro Cys Cys Val His Gly Met
   65                  70                  75                  80
   Pro Val Phe Pro His Trp His Arg Leu His Thr Ile Gln Met Glu Arg
                   85                  90                  95
   Ala Leu Lys Asn His Gly Ser Pro Met Gly Ile Pro Tyr Trp Asp Trp
               100                 105                 110
   Thr Lys Lys Met Ser Ser Leu Pro Ser Phe Phe Gly Asp Ser Ser Asn
           115                 120                 125
   Asn Asn Pro Phe Tyr Lys Tyr Tyr Ile Arg Gly Val Gln His Glu Thr
       130                 135                 140
   Thr Arg Asp Val Asn Gln Arg Leu Phe Asn Gln Thr Lys Phe Gly Glu
   145                 150                 155                 160
   Phe Asp Tyr Leu Tyr Tyr Leu Thr Leu Gln Val Leu Glu Glu Asn Ser
                   165                 170                 175
   Tyr Cys Asp Phe Glu Val Gln Tyr Glu Ile Leu His Asn Ala Val His
               180                 185                 190
   Ser Trp Leu Gly Gly Thr Gly Gln Tyr Ser Met Ser Thr Leu Glu Tyr
           195                 200                 205
   Ser Ala Phe Asp Pro Val Phe Met Ile His His Ser Ser Leu Asp Arg
       210                 215                 220
   Ile Trp Ile Leu Trp Gln Lys Leu Gln Lys Ile Arg Met Lys Pro Tyr
   225                 230                 235                 240
   Tyr Ala Leu Asp Cys Ala Gly Asp Arg Leu Met Lys Asp Pro Leu His
                   245                 250                 255
   Pro Phe Asn Tyr Glu Thr Val Asn Glu Asp Glu Phe Thr Arg Ile Asn
               260                 265                 270
   Ser Phe Pro Ser Ile Leu Phe Asp His Tyr Arg Phe Asn Tyr Glu Tyr
           275                 280                 285
   Asp Asn Met Arg Ile Arg Gly Gln Asp Ile His Glu Leu Glu Glu Val
       290                 295                 300
   Ile Gln Glu Leu Arg Asn Lys Asp Arg Ile Phe Ala Gly Phe Val Leu
   305                 310                 315                 320
   Ser Gly Leu Arg Ile Ser Ala Thr Val Lys Val Phe Ile His Ser Lys
                   325                 330                 335
   Asn Asp Thr Ser His Glu Glu Tyr Ala Gly Glu Phe Ala Val Leu Gly
   340 345 350
   Gly Glu Lys Glu Met Pro Trp Ala Tyr Glu Arg Met Leu Lys Leu Asp
   355 360 365
   Ile Ser Asp Ala Val His Lys Leu His Val Lys Asp Glu Asp Ile Arg
   370 375 380
   Phe Arg Val Val Val Thr Ala Tyr Asn Gly Asp Val Val Thr Thr Arg
   385 390 395 400
   Leu Ser Gln Pro Phe Ile Val His Arg Pro Ala His Val Ala His Asp
   405 410 415
   Ile Leu Val Ile Pro Val Gly Ala Gly His Asp Leu Pro Pro Lys Val
   420 425 430
   Val Val Lys Ser Gly Thr Lys Val Glu Phe Thr Pro Ile Asp Ser Ser
   435 440 445
   Val Asn Lys Ala Met Val Glu Leu Gly Ser Tyr Thr Ala Met Ala Lys
   450 455 460
   Cys Ile Val Pro Pro Phe Ser Tyr His Gly Phe Glu Leu Asp Lys Val
   465 470 475 480
   Tyr Ser Val Asp His Gly Asp Tyr Tyr Ile Ala Ala Gly Thr His Ala
   485 490 495
   Leu Cys Glu Gln Asn Leu Arg Leu His Ile His Val Glu His Glu
   500 505 510
<210> 33
   <211> 334
   <212> PRT
   <213> Haliotis tuberculata
<400> 33
   His Arg Leu Phe Val Thr Gln Val Glu Asp Ala Leu Ile Arg Arg Gly
   1 5 10 15
   Ser Pro Ile Gly Val Pro Tyr Trp Asp Trp Thr Gln Pro Met Ala His
   20 25 30
   Leu Pro Gly Leu Ala Asp Asn Ala Thr Tyr Arg Asp Pro Ile Ser Gly
   35 40 45
   Asp Ser Arg His Asn Pro Phe His Asp Val Glu Val Ala Phe Glu Asn
   50 55 60
   Gly Arg Thr Glu Arg His Pro Asp Ser Arg Leu Phe Glu Gln Pro Leu
   65 70 75 80
   Phe Gly Lys His Thr Arg Leu Phe Asp Ser Ile Val Tyr Ala Phe Glu
                   85                  90                  95
   Gln Glu Asp Phe Cys Asp Phe Glu Val Gln Phe Glu Met Thr His Asn
               100                 105                 110
   Asn Ile His Ala Trp Ile Gly Gly Gly Glu Lys Tyr Ser Met Ser Ser
           115                 120                 125
   Leu His Tyr Thr Ala Phe Asp Pro Ile Phe Tyr Leu Arg His Ser Asn
       130                 135                 140
   Thr Asp Arg Leu Trp Ala Ile Trp Gln Ala Leu Gln Ile Arg Arg Asn
   145                 150                 155                 160
   Arg Pro Tyr Lys Ala His Cys Ala Trp Ser Glu Glu Arg Gln Pro Leu
                   165                 170                 175
   Lys Pro Phe Ala Phe Ser Ser Pro Leu Asn Asn Asn Glu Lys Thr Tyr
               180                 185                 190
   Glu Asn Ser Val Pro Thr Asn Val Tyr Asp Tyr Glu Gly Val Leu Gly
           195                 200                 205
   Tyr Thr Tyr Asp Asp Leu Asn Phe Gly Gly Met Asp Leu Gly Gln Leu
       210                 215                 220
   Glu Glu Tyr Ile Gln Arg Gln Arg Gln Arg Asp Arg Thr Phe Ala Gly
   225                 230                 235                 240
   Phe Phe Leu Ser His Ile Gly Thr Ser Ala Asn Val Glu Ile Ile Ile
                   245                 250                 255
   Asp His Gly Thr Leu His Thr Ser Val Gly Thr Phe Ala Val Leu Gly
               260                 265                 270
   Gly Glu Lys Glu Met Lys Trp Gly Phe Asp Arg Leu Tyr Lys Tyr Glu
           275                 280                 285
   Ile Thr Asp Glu Leu Arg Gln Leu Asn Leu Arg Ala Asp Asp Val Phe
       290                 295                 300
   Ser Ile Ser Val Lys Val Thr Asp Val Asp Gly Ser Glu Leu Ser Ser
   305                 310                 315                 320
   Glu Leu Ile Pro Ser Ala Ala Ile Ile Phe Glu Arg Ser His
                   325                 330
<210> 34
   <211> 417
   <212> PRT
   <213> Haliotis tuberculata
<400> 34
   Ile Asp His Gln Asp Pro His His Asp Thr Ile Ile Arg Lys Asn Val
    1               5                   10                  15
   Asp Asn Leu Thr Pro Glu Glu Ile Asn Ser Leu Arg Arg Ala Met Ala
               20                  25                  30
   Asp Leu Gln Ser Asp Lys Thr Ala Gly Gly Phe Gln Gln Ile Ala Ala
           35                  40                  45
   Phe His Gly Glu Pro Lys Trp Cys Pro Ser Pro Asp Ala Glu Lys Lys
       50                  55                  60
   Phe Ser Cys Cys Val His Gly Met Ala Val Phe Pro His Trp His Arg
   65                  70                  75                  80
   Leu Leu Thr Val Gln Gly Glu Asn Ala Leu Arg Lys His Gly Cys Leu
                   85                  90                  95
   Gly Ala Leu Pro Tyr Trp Asp Trp Thr Arg Pro Leu Ser His Leu Pro
               100                 105                 110
   Asp Leu Val Leu Val Ser Ser Arg Thr Thr Pro Met Pro Tyr Ser Thr
           115                 120                 125
   Val Glu Ala Arg Asn Pro Trp Tyr Ser Gly His Ile Asp Thr Val Gly
       130                 135                 140
   Val Asp Thr Thr Arg Ser Val Arg Gln Glu Leu Tyr Glu Ala Pro Gly
   145                 150                 155                 160
   Phe Gly His Tyr Thr Gly Val Ala Lys Gln Val Leu Leu Ala Leu Glu
                   165                 170                 175
   Gln Asp Asp Phe Cys Asp Phe Glu Val Gln Phe Glu Ile Ala His Asn
               180                 185                 190
   Phe Ile His Ala Leu Val Gly Gly Ser Glu Pro Tyr Gly Met Ala Ser
           195                 200                 205
   Leu Arg Tyr Thr Thr Tyr Asp Pro Ile Phe Tyr Leu His His Ser Asn
       210                 215                 220
   Thr Asp Arg Leu Trp Ala Ile Trp Gln Ala Leu Gln Lys Tyr Arg Gly
   225                 230                 235                 240
   Lys Pro Tyr Asn Ser Ala Asn Cys Ala Ile Ala Ser Met Arg Lys Pro
                   245                 250                 255
   Leu Gln Pro Phe Gly Leu Thr Asp Glu Ile Asn Pro Asp Asp Glu Thr
               260                 265                 270
   Arg Gln His Ala Val Pro Phe Ser Val Phe Asp Tyr Lys Asn Asn Phe
           275                 280                 285
   Asn Tyr Glu Tyr Asp Thr Leu Asp Phe Asn Gly Leu Ser Ile Ser Gln
       290                 295                 300
   Leu Asp Arg Glu Leu Ser Arg Arg Lys Ser His Asp Arg Val Phe Ala
   305                 310                 315                 320
   Gly Phe Leu Leu His Gly Ile Gln Gln Ser Ala Leu Val Lys Phe Phe
                   325                 330                 335
   Val Cys Lys Ser Asp Asp Asp Cys Asp His Tyr Ala Gly Glu Phe Tyr
               340                 345                 350
   Ile Leu Gly Asp Glu Ala Glu Met Pro Trp Gly Tyr Asp Arg Leu Tyr
           355                 360                 365
   Lys Tyr Glu Ile Thr Glu Gln Leu Asn Ala Leu Asp Leu His Ile Gly
       370                 375                 380
   Asp Arg Phe Phe Ile Arg Tyr Glu Ala Phe Asp Leu His Gly Thr Ser
   385                 390                 395                 400
   Leu Gly Ser Asn Ile Phe Pro Lys Pro Ser Val Ile His Asp Glu Gly
                   405                 410                 415
   Ala
<210> 35
   <211> 415
   <212> PRT
   <213> Haliotis tuberculata
<400> 35
   Gly His His Gln Ala Asp Glu Tyr Asp Glu Val Val Thr Ala Ala Ser
   1               5                   10                  15
   His Ile Arg Lys Asn Leu Lys Asp Leu Ser Lys Gly Glu Val Glu Ser
               20                  25                  30
   Leu Arg Ser Ala Phe Leu Gln Leu Gln Asn Asp Gly Val Tyr Glu Asn
           35                  40                  45
   Ile Ala Lys Phe His Gly Lys Pro Gly Leu Cys Asp Asp Asn Gly Arg
       50                  55                  60
   Lys Val Ala Cys Cys Val His Gly Met Pro Thr Phe Pro Gln Trp His
   65                  70                  75                  80
   Arg Leu Tyr Val Leu Gln Val Glu Asn Ala Leu Leu Glu Arg Gly Ser
                   85                  90                  95
   Ala Val Ser Val Pro Tyr Trp Asp Trp Thr Glu Thr Phe Thr Glu Leu
               100                 105                 110
   Pro Ser Leu Ile Ala Glu Ala Thr Tyr Phe Asn Ser Arg Gln Gln Thr
           115                 120                 125
   Phe Asp Pro Asn Pro Phe Phe Arg Gly Lys Ile Ser Phe Glu Asn Ala
       130                 135                 140
   Val Thr Thr Arg Asp Pro Gln Pro Glu Leu Tyr Val Asn Arg Tyr Tyr
   145                 150                 155                 160
   Tyr Gln Asn Val Met Leu Val Phe Glu Gln Asp Asn Tyr Cys Asp Phe
                   165                 170                 175
   Glu Ile Gln Phe Glu Met Val His Asn Val Leu His Ala Trp Leu Gly
               180                 185                 190
   Gly Arg Ala Thr Tyr Ser Ile Ser Ser Leu Asp Tyr Ser Ala Phe Asp
           195                 200                 205
   Pro Val Phe Phe Leu His His Ala Asn Thr Asp Arg Leu Trp Ala Ile
       210                 215                 220
   Trp Gln Glu Leu Gln Arg Tyr Arg Lys Lys Pro Tyr Asn Glu Ala Asp
   225                 230                 235                 240
   Cys Ala Ile Asn Leu Met Arg Lys Pro Leu His Pro Phe Asp Asn Ser
                   245                 250                 255
   Asp Leu Asn His Asp Pro Val Thr Phe Lys Tyr Ser Lys Pro Thr Asp
               260                 265                 270
   Gly Phe Asp Tyr Gln Asn Asn Phe Gly Tyr Lys Tyr Asp Asn Leu Glu
           275                 280                 285
   Phe Asn His Phe Ser Ile Pro Arg Leu Glu Glu Ile Ile Arg Ile Arg
       290                 295                 300
   Gln Arg Gln Asp Arg Val Phe Ala Gly Phe Leu Leu His Asn Ile Gly
   305                 310                 315                 320
   Thr Ser Ala Thr Val Glu Ile Phe Val Cys Val Pro Thr Thr Ser Gly
                   325                 330                 335
   Glu Gln Asn Cys Glu Asn Lys Ala Gly Thr Phe Ala Val Leu Gly Gly
               340                 345                 350
   Glu Thr Glu Met Ala Phe His Phe Asp Arg Leu Tyr Arg Phe Asp Ile
           355                 360                 365
   Ser Glu Thr Leu Arg Asp Leu Gly Ile Gln Leu Asp Ser His Asp Phe
       370                 375                 380
   Asp Leu Ser Ile Lys Ile Gln Gly Val Asn Gly Ser Tyr Leu Asp Pro
   385                 390                 395                 400
   His Ile Leu Pro Glu Pro Ser Leu Ile Phe Val Pro Gly Ser Ser
                   405                 410                 415
<210> 36
   <211> 418
   <212> PRT
   <213> Haliotis tuberculata
<400> 36
   Ser Phe Leu Arg Pro Asp Gly His Ser Asp Asp Ile Leu Val Arg Lys
   1               5                   10                  15
   Glu Val Asn Ser Leu Thr Thr Arg Glu Thr Ala Ser Leu Ile His Ala
               20                  25                  30
   Leu Lys Ser Met Gln Glu Asp His Ser Pro Asp Gly Phe Gln Ala Ile
           35                  40                  45
   Ala Ser Phe His Ala Leu Pro Pro Leu Cys Pro Ser Pro Ser Ala Ala
       50                  55                  60
   His Arg Tyr Ala Cys Cys Val His Gly Met Ala Thr Phe Pro Gln Trp
   65                  70                  75                  80
   His Arg Leu Tyr Thr Val Gln Phe Gln Asp Ala Leu Arg Arg His Gly
                   85                  90                  95
   Ala Thr Val Gly Val Pro Tyr Trp Asp Trp Leu Arg Pro Gln Ser His
               100                 105                 110
   Leu Pro Glu Leu Val Thr Met Glu Thr Tyr His Asp Ile Trp Ser Asn
           115                 120                 125
   Arg Asp Phe Pro Asn Pro Phe Tyr Gln Ala Asn Ile Glu Phe Glu Gly
       130                 135                 140
   Glu Asn Ile Thr Thr Glu Arg Glu Val Ile Ala Asp Lys Leu Phe Val
   145                 150                 155                 160
   Lys Gly Gly His Val Phe Asp Lys Leu Val Leu Gln Thr Ser His Pro
                   165                 170                 175
   Ser Ala Glu Gln Glu Asn Tyr Cys Asp Phe Glu Ile Gln Phe Glu Ile
               180                 185                 190
   Leu His Asn Gly Val His Thr Trp Val Gly Gly Ser Arg Thr Tyr Ser
           195                 200                 205
   Ile Gly His Leu His Tyr Ala Phe Tyr Asp Pro Leu Phe Tyr Leu His
       210                 215                 220
   His Phe Gln Thr Asp Arg Ile Trp Ala Ile Trp Gln Glu Leu Gln Glu
   225                 230                 235                 240
   Gln Arg Gly Leu Ser Gly Asp Glu Ala His Cys Ala Leu Glu Gln Met
                   245                 250                 255
   Arg Glu Pro Leu Lys Pro Phe Ser Phe Gly Ala Pro Tyr Asn Trp Asn
               260                 265                 270
   Gln Leu Thr Gln Asp Phe Ser Arg Pro Glu Asp Thr Phe Asp Tyr Arg
           275                 280                 285
   Lys Phe Gly Tyr Glu Tyr Asp Asn Leu Glu Phe Leu Gly Met Ser Val
       290                 295                 300
   Ala Glu Leu Asp Gln Tyr Ile Ile Glu His Gln Glu Asn Asp Arg Val
   305 310 315 320
   Phe Ala Gly Phe Leu Leu Ser Gly Phe Gly Gly Ser Ala Ser Val Asn
   325 330 335
   Phe Gln Val Cys Arg Ala Asp Ser Thr Cys Gln Asp Ala Gly Tyr Phe
   340 345 350
   Thr Val Leu Gly Gly Ser Ala Glu Met Ala Trp Ala Phe Asp Arg Leu
   355 360 365
   Tyr Lys Tyr Asp Ile Thr Glu Thr Leu Glu Lys Met His Leu Arg Tyr
   370 375 380
   Asp Asp Asp Phe Thr Ile Ser Val Ser Leu Thr Ala Asn Asn Gly Thr
   385 390 395 400
   Val Leu Ser Ser Ser Leu Ile Pro Thr Pro Ser Val Ile Phe Gln Arg
   405 410 415
   Gly His
<210> 37
   <211> 416
   <212> PRT
   <213> Haliotis tuberculata
<400> 37
   Arg Asp Ile Asn Thr Arg Ser Met Ser Pro Asn Arg Val Arg Arg Glu
   1 5 10 15
   Leu Ser Asp Leu Ser Ala Arg Asp Leu Ser Ser Leu Lys Ser Ala Leu
   20 25 30
   Arg Asp Leu Gln Glu Asp Asp Gly Pro Asn Gly Tyr Gln Ala Leu Ala
   35 40 45
   Ala Phe His Gly Leu Pro Ala Gly Cys His Asp Ser Arg Gly Asn Glu
   50 55 60
   Ile Ala Cys Cys Ile His Gly Met Pro Thr Phe Pro Gln Trp His Arg
   65 70 75 80
   Leu Tyr Thr Leu Gln Leu Glu Met Ala Leu Arg Arg His Gly Ser Ser
   85 90 95
   Val Ala Ile Pro Tyr Trp Asp Trp Thr Lys Pro Ile Ser Glu Leu Pro
   100 105 110
   Ser Leu Phe Thr Ser Pro Glu Tyr Tyr Asp Pro Trp His Asp Ala Val
   115 120 125
   Val Asn Asn Pro Phe Ser Lys Gly Phe Val Lys Phe Ala Asn Thr Tyr
       130                 135                 140
   Thr Val Arg Asp Pro Gln Glu Met Leu Phe Gln Leu Cys Glu His Gly
   145                 150                 155                 160
   Glu Ser Ile Leu Tyr Glu Gln Thr Leu Leu Ala Leu Glu Gln Thr Asp
                   165                 170                 175
   Tyr Cys Asp Phe Glu Val Gln Phe Glu Val Leu His Asn Val Ile His
               180                 185                 190
   Tyr Leu Val Gly Gly Arg Gln Thr Tyr Ala Leu Ser Ser Leu His Tyr
           195                 200                 205
   Ala Ser Tyr Asp Pro Phe Phe Phe Ile His His Ser Phe Val Asp Lys
       210                 215                 220
   Met Trp Val Val Trp Gln Ala Leu Gln Lys Arg Arg Lys Leu Pro Tyr
   225                 230                 235                 240
   Lys Arg Ala Asp Cys Ala Val Asn Leu Met Thr Lys Pro Met Arg Pro
                   245                 250                 255
   Phe Asp Ser Asp Met Asn Gln Asn Pro Phe Thr Lys Met His Ala Val
               260                 265                 270
   Pro Asn Thr Leu Tyr Asp Tyr Glu Thr Leu Tyr Tyr Ser Tyr Asp Asn
           275                 280                 285
   Leu Glu Ile Gly Gly Arg Asn Leu Asp Gln Leu Gln Ala Glu Ile Asp
       290                 295                 300
   Arg Ser Arg Ser His Asp Arg Val Phe Ala Gly Phe Leu Leu Arg Gly
   305                 310                 315                 320
   Ile Gly Thr Ser Ala Asp Val Arg Phe Trp Ile Cys Arg Asn Glu Asn
                   325                 330                 335
   Asp Cys His Arg Gly Gly Ile Ile Phe Ile Leu Gly Gly Ala Lys Glu
               340                 345                 350
   Met Pro Trp Ser Phe Asp Arg Asn Phe Lys Phe Asp Ile Thr His Val
           355                 360                 365
   Leu Glu Asn Ala Gly Ile Ser Pro Glu Asp Val Phe Asp Ala Glu Glu
       370                 375                 380
   Pro Phe Tyr Ile Lys Val Glu Ile His Ala Val Asn Lys Thr Met Ile
   385                 390                 395                 400
   Pro Ser Ser Val Ile Pro Ala Pro Thr Ile Ile Tyr Ser Pro Gly Glu
                   405                 410                 415
<210> 38
   <211> 402
   <212> PRT
   <213> Haliotis tuberculata
<400> 38
   Gly Arg Ala Ala Asp Ser Ala His Ser Ala Asn Ile Ala Gly Ser Gly
   1               5                   10                  15
   Val Arg Lys Asp Val Thr Thr Leu Thr Val Ser Glu Thr Glu Asn Leu
               20                  25                  30
   Arg Gln Ala Leu Gln Gly Val Ile Asp Asp Thr Gly Pro Asn Gly Tyr
           35                  40                  45
   Gln Ala Ile Ala Ser Phe His Gly Ser Pro Pro Met Cys Glu Met Asn
       50                  55                  60
   Gly Arg Lys Val Ala Cys Cys Ala His Gly Met Ala Ser Phe Pro His
   65                  70                  75                  80
   Trp His Arg Leu Tyr Val Lys Gln Met Glu Asp Ala Leu Ala Asp His
                   85                  90                  95
   Gly Ser His Ile Gly Ile Pro Tyr Trp Asp Trp Thr Thr Ala Phe Thr
               100                 105                 110
   Glu Leu Pro Ala Leu Val Thr Asp Ser Glu Asn Asn Pro Phe His Glu
           115                 120                 125
   Gly Arg Ile Asp His Leu Gly Val Thr Thr Ser Arg Ser Pro Arg Asp
       130                 135                 140
   Met Leu Phe Asn Asp Pro Glu Gln Gly Ser Glu Ser Phe Phe Tyr Arg
   145                 150                 155                 160
   Gln Val Leu Leu Ala Leu Glu Gln Thr Asp Tyr Cys Gln Phe Glu Val
                   165                 170                 175
   Gln Phe Glu Leu Thr His Asn Ala Ile His Ser Trp Thr Gly Gly Arg
               180                 185                 190
   Ser Pro Tyr Gly Met Ser Thr Leu Glu Phe Thr Ala Tyr Asp Pro Leu
           195                 200                 205
   Phe Trp Leu His His Ser Asn Thr Asp Arg Ile Trp Ala Val Trp Gln
       210                 215                 220
   Ala Leu Gln Lys Tyr Arg Gly Leu Pro Tyr Asn Glu Ala His Cys Glu
   225                 230                 235                 240
   Ile Gln Val Leu Lys Gln Pro Leu Arg Pro Phe Asn Asp Asp Ile Asn
                   245                 250                 255
   His Asn Pro Ile Thr Lys Thr Asn Ala Arg Pro Ile Asp Ser Phe Asp
               260                 265                 270
   Tyr Glu Arg Phe Asn Tyr Gln Tyr Asp Thr Leu Ser Phe His Gly Lys
   275 280 285
   Ser Ile Pro Glu Leu Asn Asp Leu Leu Glu Glu Arg Lys Arg Glu Glu
   290 295 300
   Arg Thr Phe Ala Ala Phe Leu Leu Arg Gly Ile Gly Cys Ser Ala Asp
   305 310 315 320
   Val Val Phe Asp Ile Cys Arg Pro Asn Gly Asp Cys Val Phe Ala Gly
   325 330 335
   Thr Phe Ala Val Leu Gly Gly Glu Leu Glu Met Pro Trp Ser Phe Asp
   340 345 350
   Arg Leu Phe Arg Tyr Asp Ile Thr Arg Val Met Asn Gln Leu His Leu
   355 360 365
   Gln Tyr Asp Ser Asp Phe Ser Phe Arg Val Lys Leu Val Ala Thr Asn
   370 375 380
   Gly Thr Glu Leu Ser Ser Asp Leu Leu Lys Ser Pro Thr Ile Glu His
   385 390 395 400
   Glu Leu
<210> 39
   <211> 515
   <212> PRT
   <213> Haliotis tuberculata
<400> 39
   Gly Ala His Arg Gly Pro Val Glu Glu Thr Glu Val Thr Arg Gln His
   1 5 10 15
   Thr Asp Gly Asn Ala His Phe His Arg Lys Glu Val Asp Ser Leu Ser
   20 25 30
   Leu Asp Glu Ala Asn Asn Leu Lys Asn Ala Leu Tyr Lys Leu Gln Asn
   35 40 45
   Asp His Ser Leu Thr Gly Tyr Glu Ala Ile Ser Gly Tyr His Gly Tyr
   50 55 60
   Pro Asn Leu Cys Pro Glu Glu Gly Asp Asp Lys Ile Pro Leu Leu Arg
   65 70 75 80
   Pro Arg Met Gly Ile Phe Pro Tyr Trp His Arg Leu Leu Thr Ile Gln
   85 90 95
   Leu Glu Arg Ala Leu Glu His Asn Gly Ala Leu Leu Gly Val Pro Tyr
   100 105 110
   Trp Asp Trp Asn Lys Asp Leu Ser Ser Leu Pro Ala Phe Phe Ser Asp
           115                 120                 125
   Ser Ser Asn Asn Asn Pro Tyr Phe Lys Tyr His Ile Ala Gly Val Gly
       130                 135                 140
   His Asp Thr Val Arg Glu Pro Thr Ser Leu Ile Tyr Asn Gln Pro Gln
   145                 150                 155                 160
   Ile His Gly Tyr Asp Tyr Leu Tyr Tyr Leu Ala Leu Thr Thr Leu Glu
                   165                 170                 175
   Glu Asn Asn Tyr Trp Asp Phe Glu Val Gln Tyr Glu Ile Leu His Asn
               180                 185                 190
   Ala Val His Ser Trp Leu Gly Gly Ser Gln Lys Tyr Ser Met Ser Thr
           195                 200                 205
   Leu Glu Tyr Ser Ala Phe Asp Pro Val Phe Met Ile Leu His Ser Gly
       210                 215                 220
   Leu Asp Arg Leu Trp Ile Ile Trp Gln Glu Leu Gln Lys Ile Arg Arg
   225                 230                 235                 240
   Lys Pro Tyr Asn Phe Ala Lys Cys Ala Tyr His Met Met Glu Glu Pro
                   245                 250                 255
   Leu Ala Pro Phe Ser Tyr Pro Ser Ile Asn Gln Asp Glu Phe Thr Arg
               260                 265                 270
   Ala Asn Ser Lys Pro Ser Thr Val Phe Asp Ser His Lys Phe Gly Tyr
           275                 280                 285
   His Tyr Asp Asn Leu Asn Val Arg Gly His Ser Ile Gln Glu Leu Asn
       290                 295                 300
   Thr Ile Ile Asn Asp Leu Arg Asn Thr Asp Arg Ile Tyr Ala Gly Phe
   305                 310                 315                 320
   Val Leu Ser Gly Ile Gly Thr Ser Ala Ser Val Lys Ile Tyr Leu Arg
                   325                 330                 335
   Thr Asp Asp Asn Asp Glu Glu Val Gly Thr Phe Thr Val Leu Gly Gly
               340                 345                 350
   Glu Arg Glu Met Pro Trp Ala Tyr Glu Arg Val Phe Lys Tyr Asp Ile
           355                 360                 365
   Thr Glu Val Ala Asp Arg Leu Lys Ile Lys Leu Trp Gly His Pro Leu
       370                 375                 380
   Thr Ser Gly Thr Gly Asp His Ile Leu Thr Asn Gly Ile Gly Gly Lys
   385                 390                 395                 400
   Gln Glu Pro Thr Gln Ile Leu Ser Ser Ser Thr Asp Leu Pro Ile Met
                   405                 410                 415
   Thr Thr Met Phe Leu Leu Ser Gln Xaa Gly Arg Asn Leu His Ile Pro
   420 425 430
   Pro Lys Val Val Val Lys Lys Gly Thr Arg Ile Glu Phe His Pro Val
   435 440 445
   Asp Asp Ser Val Thr Arg Pro Val Val Asp Leu Gly Ser Tyr Thr Ala
   450 455 460
   Leu Phe Asn Cys Val Val Pro Pro Phe Thr Tyr His Gly Phe Glu Leu
   465 470 475 480
   Asn His Val Tyr Ser Val Lys Pro Gly Asp Tyr Tyr Val Thr Gly Pro
   485 490 495
   Thr Arg Asp Leu Cys Gln Asn Ala Asp Val Arg Ile His Ile His Val
   500 505 510
   Glu Asp Glu
   515
<210> 40
   <211> 322
   <212> PRT
   <213> Megathura crenulata
<400> 40
   Gly Leu Pro Tyr Trp Asp Trp Thr Glu Pro Met Thr His Ile Pro Gly
   1 5 10 15
   Leu Ala Gly Asn Lys Thr Tyr Val Asp Ser His Gly Ala Ser His Thr
   20 25 30
   Asn Pro Phe His Ser Ser Val Ile Ala Phe Glu Glu Asn Ala Pro His
   35 40 45
   Thr Lys Arg Gln Ile Asp Gln Arg Leu Phe Lys Pro Ala Thr Phe Gly
   50 55 60
   His His Thr Asp Leu Phe Asn Gln Ile Leu Tyr Ala Phe Glu Gln Glu
   65 70 75 80
   Asp Tyr Cys Asp Phe Glu Val Gln Phe Glu Ile Thr His Asn Thr Ile
   85 90 95
   His Ala Trp Thr Gly Gly Ser Glu His Phe Ser Met Ser Ser Leu His
   100 105 110
   Tyr Thr Ala Phe Asp Pro Leu Phe Tyr Phe His His Ser Asn Val Asp
   115 120 125
   Arg Leu Trp Ala Val Trp Gln Ala Leu Gln Met Arg Arg His Lys Pro
   130 135 140
   Tyr Arg Ala His Cys Ala Ile Ser Leu Glu His Met His Leu Lys Pro
   145 150 155 160
   Phe Ala Phe Ser Ser Pro Leu Asn Asn Asn Glu Lys Thr His Ala Asn
   165 170 175
   Ala Met Pro Asn Lys Ile Tyr Asp Tyr Glu Asn Val Leu His Tyr Thr
   180 185 190
   Tyr Glu Asp Leu Thr Phe Gly Gly Ile Ser Leu Glu Asn Ile Glu Lys
   195 200 205
   Met Ile His Glu Asn Gln Gln Glu Asp Arg Ile Tyr Ala Gly Phe Leu
   210 215 220
   Leu Ala Gly Ile Arg Thr Ser Ala Asn Val Asp Ile Phe Ile Lys Thr
   225 230 235 240
   Thr Asp Ser Val Gln His Lys Ala Gly Thr Phe Ala Val Leu Gly Gly
   245 250 255
   Ser Lys Glu Met Lys Trp Gly Phe Asp Arg Val Phe Lys Phe Asp Ile
   260 265 270
   Thr His Val Leu Lys Asp Leu Asp Leu Thr Ala Asp Gly Asp Phe Glu
   275 280 285
   Val Thr Val Asp Ile Thr Glu Val Asp Gly Thr Lys Leu Ala Ser Ser
   290 295 300
   Leu Ile Pro His Ala Ser Val Ile Arg Glu His Ala Arg Gly Lys Leu
   305 310 315 320
   Asn Arg
<210> 41
   <211> 414
   <212> PRT
   <213> Megathura crenulata
<400> 41
   Val Lys Phe Asp Lys Val Pro Arg Ser Arg Leu Ile Arg Lys Asn Val
   1 5 10 15
   Asp Arg Leu Ser Pro Glu Glu Met Asn Glu Leu Arg Lys Ala Leu Ala
   20 25 30
   Leu Leu Lys Glu Asp Lys Ser Ala Gly Gly Phe Gln Gln Leu Gly Ala
   35 40 45
   Phe His Gly Glu Pro Lys Trp Cys Pro Ser Pro Glu Ala Ser Lys Lys
   50 55 60
   Phe Ala Cys Cys Val His Gly Met Ser Val Phe Pro His Trp His Arg
   65 70 75 80
   Leu Leu Thr Val Gln Ser Glu Asn Ala Leu Arg Arg His Gly Tyr Asp
   85 90 95
   Gly Ala Leu Pro Tyr Trp Asp Trp Thr Ser Pro Leu Asn His Leu Pro
   100 105 110
   Glu Leu Ala Asp His Glu Lys Tyr Val Asp Pro Glu Asp Gly Val Glu
   115 120 125
   Lys His Asn Pro Trp Phe Asp Gly His Ile Asp Thr Val Asp Lys Thr
   130 135 140
   Thr Thr Arg Ser Val Gln Asn Lys Leu Phe Glu Gln Pro Glu Phe Gly
   145 150 155 160
   His Tyr Thr Ser Ile Ala Lys Gln Val Leu Leu Ala Leu Glu Gln Asp
   165 170 175
   Asn Phe Cys Asp Phe Glu Ile Gln Tyr Glu Ile Ala His Asn Tyr Ile
   180 185 190
   His Ala Leu Val Gly Gly Ala Gln Pro Tyr Gly Met Ala Ser Leu Arg
   195 200 205
   Tyr Thr Ala Phe Asp Pro Leu Phe Tyr Leu His His Ser Asn Thr Asp
   210 215 220
   Arg Ile Trp Ala Ile Trp Gln Ala Leu Gln Lys Tyr Arg Gly Lys Pro
   225 230 235 240
   Tyr Asn Val Ala Asn Cys Ala Val Thr Ser Met Arg Glu Pro Leu Gln
   245 250 255
   Pro Phe Gly Leu Ser Ala Asn Ile Asn Thr Asp His Val Thr Lys Glu
   260 265 270
   His Ser Val Pro Phe Asn Val Phe Asp Tyr Lys Thr Asn Phe Asn Tyr
   275 280 285
   Glu Tyr Asp Thr Leu Glu Phe Asn Gly Leu Ser Ile Ser Gln Leu Asn
   290 295 300
   Lys Lys Leu Glu Ala Ile Lys Ser Gln Asp Arg Phe Phe Ala Gly Phe
   305 310 315 320
   Leu Leu Ser Gly Phe Lys Lys Ser Ser Leu Val Lys Phe Asn Ile Cys
   325 330 335
   Thr Asp Ser Ser Asn Cys His Pro Ala Gly Glu Phe Tyr Leu Leu Gly
   340 345 350
   Asp Glu Asn Glu Met Pro Trp Ala Tyr Asp Arg Val Phe Lys Tyr Asp
   355 360 365
   Ile Thr Glu Lys Leu His Asp Leu Lys Leu His Ala Glu Asp His Phe
   370 375 380
   Tyr Ile Asp Tyr Glu Val Phe Asp Leu Lys Pro Ala Ser Leu Gly Lys
   385 390 395 400
   Asp Leu Phe Lys Gln Pro Ser Val Ile His Glu Pro Arg Ile
   405 410
<210>
   <211> 411
   <212> PRT
   <213> Megathura crenulata
<400> 42
<210> 43
   <211> 111
   <212> PRT
   <213> Megathura crenulata
<400> 43
   Asp Ser Ala His Thr Asp Asp Gly His Thr Glu Pro Val Met Ile Arg
   1 5 10 15
   Lys Asp Ile Thr Gln Leu Asp Lys Arg Gln Gln Leu Ser Leu Val Lys
   20 25 30
   Ala Leu Glu Ser Met Lys Ala Asp His Ser Ser Asp Gly Phe Gln Ala
   35 40 45
   Ile Ala Ser Phe His Ala Leu Pro Pro Leu Cys Pro Ser Pro Ala Ala
   50 55 60
   Ser Lys Arg Phe Ala Cys Cys Val His Gly Met Pro Thr Phe Pro Gln
   65 70 75 80
   Trp His Arg Leu Tyr Thr Val Gln Phe Gln Asp Ser Leu Arg Lys His
   85 90 95
   Gly Ala Val Val Gly Leu Pro Tyr Trp Asp Trp Thr Leu Pro Arg
   100 105 110
<210> 44
   <211> 317
   <212> PRT
   <213> Megathura crenulata
<400> 44
   Gly Leu Pro Tyr Trp Asp Trp Thr Met Pro Met Ser His Leu Pro Glu
    1               5                  10                  15
   Leu Ala Thr Ser Glu Thr Tyr Leu Asp Pro Val Thr Gly Glu Thr Lys
               20                  25                  30
   Asn Asn Pro Phe His His Ala Gln Val Ala Phe Glu Asn Gly Val Thr
           35                  40                  45
   Ser Arg Asn Pro Asp Ala Lys Leu Phe Met Lys Pro Thr Tyr Gly Asp
       50                  55                  60
   His Thr Tyr Leu Phe Asp Ser Met Ile Tyr Ala Phe Glu Gln Glu Asp
   65                  70                  75                  80
   Phe Cys Asp Phe Glu Val Gln Tyr Glu Leu Thr His Asn Ala Ile His
                   85                  90                  95
   Ala Trp Val Gly Gly Ser Glu Lys Tyr Ser Met Ser Ser Leu His Tyr
               100                 105                 110
   Thr Ala Phe Asp Pro Ile Phe Tyr Leu His His Ser Asn Val Asp Arg
           115                 120                 125
   Leu Trp Ala Ile Trp Gln Ala Leu Gln Ile Arg Arg Gly Lys Ser Tyr
       130                 135                 140
   Lys Ala His Cys Ala Ser Ser Gln Glu Arg Glu Pro Leu Lys Pro Phe
   145                 150                 155                 160
   Ala Phe Ser Ser Pro Leu Asn Asn Asn Glu Lys Thr Tyr His Asn Ser
                   165                 170                 175
   Val Pro Thr Asn Val Tyr Asp Tyr Val Gly Val Leu His Tyr Arg Tyr
               180                 185                 190
   Asp Asp Leu Gln Phe Gly Gly Met Thr Met Ser Glu Leu Glu Glu Tyr
           195                 200                 205
   Ile His Lys Gln Thr Gln His Asp Arg Thr Phe Ala Gly Phe Phe Leu
       210                 215                 220
   Ser Tyr Ile Gly Thr Ser Ala Ser Val Asp Ile Phe Ile Asn Arg Glu
   225                 230                 235                 240
   Gly His Asp Lys Tyr Lys Val Gly Ser Phe Val Val Leu Gly Gly Ser
                   245                 250                 255
   Lys Glu Met Lys Trp Gly Phe Asp Arg Met Tyr Lys Tyr Glu Ile Thr
               260                 265                 270
   Glu Ala Leu Lys Thr Leu Asn Val Ala Val Asp Asp Gly Phe Ser Ile
           275                 280                 285
   Thr Val Glu Ile Thr Asp Val Asp Gly Ser Pro Pro Ser Ala Asp Leu
       290                 295                 300
   Ile Pro Pro Pro Ala Ile Ile Phe Glu Arg Gly His Ala
   305                 310                 315
<210> 45
   <211> 411
   <212> PRT
   <213> Megathura crenulata
<400> 45
   Asp Ala Lys Asp Phe Gly His Ser Arg Lys Ile Arg Lys Ala Val Asp
    1               5                  10                  15
   Ser Leu Thr Val Glu Glu Gln Thr Ser Leu Arg Arg Ala Met Ala Asp
               20                  25                  30
   Leu Gln Asp Asp Lys Thr Ser Gly Gly Phe Gln Gln Ile Ala Ala Phe
           35                  40                  45
   His Gly Glu Pro Lys Trp Cys Pro Ser Pro Glu Ala Glu Lys Lys Phe
       50                  55                  60
   Ala Cys Cys Val His Gly Met Ala Val Phe Pro His Trp His Arg Leu
   65                  70                  75                  80
   Leu Thr Val Gln Gly Glu Asn Ala Leu Arg Lys His Gly Phe Thr Gly
                   85                  90                  95
   Gly Leu Pro Tyr Trp Asp Trp Thr Arg Ser Met Ser Ala Leu Pro His
               100                 105                 110
   Phe Val Ala Asp Pro Thr Tyr Asn Asp Ala Ile Ser Ser Gln Glu Glu
           115                 120                 125
   Asp Asn Pro Trp His His Gly His Ile Asp Ser Val Gly His Asp Thr
       130                 135                 140
   Thr Arg Asp Val Arg Asp Asp Leu Tyr Gln Ser Pro Gly Phe Gly His
   145                 150                 155                 160
   Tyr Thr Asp Ile Ala Gln Gln Val Leu Leu Ala Phe Glu Gln Asp Ser
                   165                 170                 175
   Phe Cys Asp Phe Glu Val Gln Phe Glu Ile Ala His Asn Phe Ile His
               180                 185                     190
   Ala Leu Ile Gly Gly Asn Glu Pro Tyr Ser Met Ser Ser Leu Arg Tyr
           195                 200                 205
   Thr Thr Tyr Asp Pro Ile Phe Phe Leu His His Ser Ser Thr Asp Arg
       210                 215                 220
   Leu Trp Ala Ile Trp Gln Ala Leu Gln Lys Tyr Arg Gly Lys Pro Tyr
   225                 230                 235                 240
   Asn Thr Ala Asn Cys Ala Ile Ala Ser Met Arg Lys Pro Leu Gln Pro
                   245                 250                 255
   Phe Gly Leu Asp Ser Val Ile Asn Pro Asp Asp Glu Thr Arg Glu His
               260                 265                 270
   Ser Val Pro Phe Arg Val Phe Asp Tyr Lys Asn Asn Phe Asp Tyr Glu
           275                 280                 285
   Tyr Glu Ser Leu Ala Phe Asn Gly Leu Ser Ile Ala Gln Leu Asp Arg
       290                 295                 300
   Glu Leu Gln Arg Arg Lys Ser His Asp Arg Val Phe Ala Gly Phe Leu
   305                 310                 315                 320
   Leu His Glu Ile Gly Gln Ser Ala Lys His Asn Val Ser Asp Cys Asp
                   325                 330                 335
   His Tyr Ala Gly Glu Phe Tyr Ile Leu Gly Asp Glu Ala Glu Met Pro
               340                 345                 350
   Trp Arg Tyr Asp Arg Val Tyr Lys Tyr Glu Ile Thr Gln Gln Leu His
           355                 360                 365
   Asp Leu Asp Leu His Val Gly Asp Asn Phe Phe Leu Lys Tyr Glu Ala
       370                 375                 380
   Phe Asp Leu Asn Gly Gly Ser Leu Gly Gly Ser Ile Phe Ser Gln Pro
   385                 390                 395                 400
   Ser Val Ile Phe Glu Pro Ala Ala Gly Met Phe
                   405                 410
<210> 46
   <211> 109
   <212> PRT
   <213> Megathura crenulata
<400> 46
   Gly Ser His Gln Ala Asp Glu Tyr Arg Glu Ala Val Thr Ser Ala Ser
    1               5                  10                  15
   His Ile Arg Lys Asn Ile Arg Asp Leu Ser Glu Gly Glu Ile Glu Ser
               20                  25                  30
   Ile Arg Ser Ala Phe Leu Gln Ile Gln Lys Glu Gly Ile Tyr Glu Asn
           35                  40                  45
   Ile Ala Lys Phe His Gly Lys Pro Gly Leu Cys Glu His Asp Gly His
       50                  55                  60
   Pro Val Ala Cys Cys Val His Gly Met Pro Thr Phe Pro His Trp His
   65                  70                  75                  80
   Arg Leu Tyr Val Leu Gln Val Glu Asn Ala Leu Leu Glu Arg Gly Ser
                   85                  90                  95
   Ala Val Ala Val Pro Tyr Trp Asp Trp Thr Leu Pro Arg
               100                 105
<210> 47
   <211> 329
   <212> PRT
   <213> Megathura crenulata
<400> 47
   Met Ala Val Phe Pro His Trp His Arg Leu Phe Val Lys Gln Met Glu
    1               5                  10                  15
   Asp Ala Leu Ala Ala His Gly Ala His Ile Gly Ile Pro Tyr Trp Asp
               20                  25                  30
   Trp Thr Ser Ala Phe Ser His Leu Pro Ala Leu Val Thr Asp His Glu
           35                  40                  45
   Asn Asn Pro Phe His His Gly His Ile Gly His Leu Asn Val Asp Thr
       50                  55                  60
   Ser Arg Ser Pro Arg Asp Met Leu Phe Asn Asp Pro Glu Gln Gly Ser
   65                  70                  75                  80
   Glu Ser Phe Phe Tyr Arg Gln Val Leu Leu Thr Leu Glu Gln Thr Asp
                   85                  90                  95
   Phe Cys Gln Phe Glu Val Gln Phe Glu Leu Thr His Asn Ala Ile His
               100                 105                 110
   Ser Trp Thr Gly Gly His Thr Pro Tyr Gly Met Ser Ser Leu Glu Tyr
           115                 120                 125
   Thr Ala Tyr Asp Pro Leu Phe Tyr Leu His His Ser Asn Thr Asp Arg
       130                 135                 140
   Ile Trp Ala Ile Trp Gln Ala Leu Gln Lys Tyr Arg Gly Leu Pro Tyr
   145                 150                 155                 160
   Asn Ala Ala His Cys Asp Ile Gln Val Leu Lys Gln Pro Leu Lys Pro
                   165                 170                 175
   Phe Ser Glu Ser Arg Asn Pro Asn Pro Val Thr Arg Ala Asn Ser Arg
               180                 185                 190
   Ala Val Asp Ser Phe Asp Tyr Glu Lys Phe Asn Tyr Gln Tyr Asp Thr
           195                 200                 205
   Leu Thr Phe His Gly Leu Ser Ile Pro Glu Leu Asp Ala Met Leu Gln
       210                 215                 220
   Glu Arg Lys Lys Glu Glu Arg Thr Phe Ala Ala Phe Leu Leu His Gly
   225                 230                 235                 240
   Phe Gly Ala Ser Ala Asp Val Ser Phe Asp Val Cys Thr Pro Asp Gly
                   245                 250                 255
   His Cys Ala Phe Ala Gly Thr Phe Ala Val Leu Gly Gly Glu Leu Glu
               260                 265                 270
   Met Pro Trp Ser Phe Glu Arg Leu Phe Arg Tyr Asp Ile Thr Lys Val
           275                 280                 285
   Leu Lys Gln Met Asn Leu His Tyr Asp Ser Glu Phe His Phe Glu Leu
       290                 295                 300
   Lys Ile Val Gly Thr Asp Gly Thr Glu Leu Pro Ser Asp Arg Ile Lys
   305                 310                 315                 320
   Ser Pro Thr Ile Glu His His Gly Gly
                   325
<210> 48
   <211> 103
   <212> PRT
   <213> Megathura crenulata
<400> 48
   Gly His Asp His Ser Glu Arg His Asp Gly Phe Phe Arg Lys Glu Val
   1               5                   10                  15
   Gly Ser Leu Ser Leu Asp Glu Ala Asn Asp Leu Lys Asn Ala Leu Tyr
               20                  25                  30
   Lys Leu Gln Asn Asp Gln Gly Pro Asn Gly Tyr Glu Ser Ile Ala Gly
           35                  40                  45
   Tyr His Gly Tyr Pro Phe Leu Cys Pro Glu His Gly Glu Asp Gln Tyr
       50                  55                  60
   Ala Cys Cys Val His Gly Met Pro Val Phe Pro His Trp His Arg Leu
   65                  70                  75                  80
   His Thr Ile Gln Phe Glu Arg Ala Leu Lys Glu His Gly Ser His Leu
                   85                  90                  95
   Gly Leu Pro Tyr Trp Asp Trp
               100
<210> 49
   <211> 1269
   <212> DNA
   <213> Haliotis tuberculata
<400> 49
   ggcttgttca gtttctactc gtcgcccttg tggtgggggc tggagcagac aacgtcgtca 60
   gaaaggacgt gagtcacctc acggatgacg aggtgcaagc tctccacggc gccctccatg 120
   acgtcactgc atctacaggg cctctgagtt tcgaagacat aacatcttac catgccgcac 180
   cagcgtcgtg tgactacaag ggacggaaga tcgcctgctg tgtccacggt atgcccagtt 240
   tccccttctg gcacagggca tatgtcgtcc aagccgagcg ggcactgttg tccaaacgga 300
   agactgtcgg aatgccttac tgggactgga cgcaaacgct gactcactta ccatctcttg 360
   tgactgaacc catctacatt gacagtaaag gtggaaaggc tcaaaccaac tactggtacc 420
   gcggcgagat agcgttcatc aataagaaga ctgcgcgagc tgtagatgat cgcctattcg 480
   agaaggtgga gcctggtcac tacacacatc ttatggagac tgtcctcgac gctctcgaac 540
   aggacgaatt ctgtaaattt gaaatccagt tcgagttggc tcataatgct atccattact 600
   tggttggcgg taaatttgaa tattcaatgt caaacttgga atacacctcc tacgacccca 660
   tcttcttcct ccaccactcc aacgttgacc gcctcttcgc catctggcag cgtcttcagg 720
   aactgcgagg aaagaatccc aatgcaatgg actgtgcaca tgaactcgct caccagcaac 780
   tccaaccctt caacagggac agcaatccag tccagctcac aaaggaccac tcgacacctg 840
   ctgacctctt tgattacaaa caacttggat acagctacga cagcttaaac ctgaatggaa 900
   tgacgccaga acagctgaaa acagaactag acgaacgcca ctccaaagaa cgtgcgtttg 960
   caagcttccg actcagtggc tttgggggtt ctgccaacgt tgttgtctat gcatgtgtcc 1020
   ctgatgatga tccacgcagt gatgactact gcgagaaagc aggcgacttc ttcattcttg 1080
   ggggtcaaag cgaaatgccg tggagattct acagaccctt cttctatgat gtaactgaag 1140
   cggtacatca ccttggagtc ccgctaagtg gccactacta tgtgaaaaca gaactcttca 1200
   gcgtgaatgg cacagcactt tcacctgatc ttcttcctca accaactgtt gcctaccgac 1260
   ctgggaaag                                                         1269
<210> 50
   <211> 569
   <212> DNA
   <213> Haliotis tuberculata
<400> 50
   ggtcttccgt actgggactg gacgcagcat ctgactcaac tcccagatct ggtgtcagac 60
   cccttgtttg tcgacccgga aggaggaaag gcccatgaca acgcatggta tcgtggaaac 120
   atcaagtttg agaataagaa gactgcaaga gctgttgacg atcgcctttt cgagaaggtt 180
   ggaccaggag agaatacccg actctttgaa ggaattctcg atgctcttga acaggatgaa 240
   ttctgcaact tcgagatcca gtttgagttg gctcacaacg ctatccacta cctggttggc 300
   ggccgtcaca cgtactccat gtctcatctc gagttacacc ctcctacgac cccctcttct 360
   tcctccatca ctccaacacc ggaccgcatc ttcgccatct gggaacgtct tcaggtactc 420
   agaggaaagg accccaacac cgccgactgc gcacacaacc tcatccatga gcccatggaa 480
   ccgttccgtc gggactcgaa ccctcttgac ctcaccaggg aaaactccaa accaattgac 540
   agctttgatt atgcccacct tggctacca                                   569
<210> 51
   <211> 1246
   <212> DNA
   <213> Haliotis tuberculata
<400> 51
   gttacagagg ccccagctcc ctcctcggat gctcacctcg ccgtcaggaa ggatatcaac 60
   catctgacac gcgaggaggt gtacgagctg cgcagagcta tggagagatt ccaggccgac 120
   acatccgttg atgggtacca ggctacggtt gagtatcacg gcttacctgc tcgatgtcca 180
   ttccccgagg ccacaaatag gttcgcctgt tgcatccacg gcatggcgac attccctcat 240
   tggcacagac tgttcgtcac ccaggtggaa gatgctctga tcaggcgagg atcgcctata 300
   ggggtcccct actgggactg gactcagcct atggcgcatc tcccaggact tgcagacaac 360
   gccacctata gagatcccat cagcggggac agcagacaca accccttcca cgatgttgaa 420
   gttgcctttg aaaatggacg tacagaacgt cacccagata gtagattgtt tgaacaacct 480
   ttatttggca aacatacgcg tctcttcgac agtatagtct atgcttttga gcaggaggac 540
   ttctgcgatt ttgaagttca atttgagatg acccataata atattcacgc ctggattggt 600
   ggcggcgaga agtattccat gtcttctcta cactacacag ccttcgaccc tatcttctac 660
   cttcgtcact ccaacactga ccggctctgg gcaatttggc aagcgttgca gatacgaaga 720
   aacaggcctt acaaggctca ttgtgcttgg tctgaggaac gccagcctct caaacctttc 780
   gccttcagtt ccccactgaa caacaacgaa aaaacctacg aaaactcggt gcccaccaac 840
   gtttacgact acgaaggagt ccttggctat acttatgatg acctcaactt cgggggcatg 900
   gacctgggtc agcttgagga atacatccag aggcagagac agagagacag gacctttgct 960
   ggtttctttc tgtcacatat tggtacatca gcgaatgttg aaatcattat agaccatggg 1020
   actcttcata cctccgtggg cacgtttgct gttcttggcg gagagaagga gatgaaatgg 1080
   ggatttgacc gtttgtacaa atatgagatt acagatgaac tgaggcaact taatctccgt 1140
   gctgatgatg ttttcagcat ctctgttaaa gtaactgatg ttgatggcag tgagctgtcc 1200
   tctgaactca tcccatctgc tgctatcatc ttcgaacgaa gccata 1246
<210> 52
   <211> 1242
   <212> DNA
   <213> Haliotis tuberculata
<400> 52
   gtcaccatca ggctgacgag tacgacgaag ttgtaactgc tgcaagccac atcagaaaga 60
   atttaaaaga tctgtcaaag ggagaagtag agagcctaag gtctgccttc ctgcaacttc 120
   agaacgacgg agtctatgag aatattgcca agttccacgg caagcctggg ttgtgtgatg 180
   ataacggtcg caaggttgcc tgttgtgtcc atggaatgcc caccttcccc cagtggcaca 240
   ggctctatgt cctccaggtg gagaatgctt tgctggagag aggatctgcc gtctctgtgc 300
   catactggga ctggactgaa acatttacag agctgccatc tttgattgct gaggctacct 360
   atttcaattc ccgtcaacaa acgtttgacc ctaatccttt cttcagaggt aaaatcagtt 420
   ttgagaatgc tgttacaaca cgtgatcccc agcctgagct gtacgttaac aggtactact 480
   accaaaacgt catgttggtt tttgaacagg acaactactg cgacttcgag atacagtttg 540
   agatggttca caatgttctc catgcttggc ttggtggaag agctacttat tctatttctt 600
   ctcttgatta ttctgcattc gaccctgtgt ttttccttca ccatgcgaac acagatagat 660
   tgtgggccat ctggcaggag ctgcagaggt acaggaagaa gccatacaat gaagcggatt 720
   gtgccattaa cctaatgcgc aaacctctac atcccttcga caacagtgat ctcaatcatg 780
   atcctgtaac ctttaaatac tcaaaaccca ctgatggctt tgactaccag aacaactttg 840
   gatacaagta tgacaacctt gagttcaatc atttcagtat tcccaggctt gaagaaatca 900
   ttcgtattag acaacgtcaa gatcgtgtgt ttgcaggatt cctccttcac aacattggga 960
   catccgcaac tgttgagata ttcgtctgtg tccctaccac cagcggtgag caaaactgtg 1020
   aaaacaaagc cggaacattt gccgtactcg gaggagaaac agagatggcg tttcattttg 1080
   acagactcta caggtttgac atcagtgaaa cactgaggga cctcggcata cagctggaca 1140
   gccatgactt tgacctcagc atcaagattc aaggagtaaa tggatcctac cttgatccac 1200
   acatcctgcc agagccatcc ttgatttttg tgcctggttc aa 1242
<210> 53
   <211> 1257
   <212> DNA
   <213> Haliotis tuberculata
<400> 53
   gttctttcct gcgtcctgat gggcattcag atgacatcct tgtgagaaaa gaagtgaaca 60
   gcctgacaac cagggagact gcatctctga tccatgctct gaaaagtatg caggaagacc 120
   attcacctga cgggttccaa gccattgcct ctttccatgc tctgccacca ctctgccctt 180
   caccatctgc agctcaccgt tatgcttgct gtgtccacgg catggctaca tttccccagt 240
   ggcacagatt gtacactgta cagttccagg atgcactgag gagacatgga gctacggtag 300
   gtgtaccgta ttgggattgg ctgcgaccgc agtctcacct accagagctt gtcaccatgg 360
   agacatacca tgatatttgg agtaacagag atttccccaa tcctttctac caagccaata 420
   ttgagtttga aggagaaaac attacaacag agagagaagt cattgcagac aaactttttg 480
   tcaaaggtgg acacgttttt gataaactgg ttcttcaaac aagccatcct agcgctgagc 540
   aggaaaacta ctgtgacttt gagattcagt ttgaaattct tcacaacggc gttcacacgt 600
   gggtcggagg cagtcgtacc tactctatcg gacatcttca ttacgcattc tacgaccctc 660
   ttttctacct tcaccatttc cagacagacc gtatttgggc aatctggcaa gaactccagg 720
   aacagagagg gctctcgggt gatgaggctc actgtgctct cgagcaaatg agagaaccat 780
   tgaagccttt cagcttcggc gctccttata actggaatca gctcacacag gatttctccc 840
   gacccgagga caccttcgac tacaggaagt ttggttatga atatgacaat ttagaattcc 900
   tgggaatgtc agttgctgaa ctggatcaat acattattga acatcaagaa aatgatagag 960
   tattcgctgg gttcctgttg agtggattcg gaggttccgc atcagttaat ttccaggttt 1020
   gtagagctga ttccacatgt caggatgctg ggtacttcac cgttcttggt ggcagtgctg 1080
   agatggcgtg ggcatttgac aggctttaca aatatgacat tactgaaact ctggagaaaa 1140
   tgcaccttcg atatgatgat gacttcacaa tctctgtcag tctgaccgcc aacaacggaa 1200
   ctgtcctgag cagcagtcta atcccaacac cgagtgtcat attccagcgg ggacatc    1257
<210> 54
   <211> 1257
   <212> DNA
   <213> Megathura crenulata
<400> 54
   attctgccca cacagatgat ggacacactg aaccagtgat gattcgcaaa gatatcacac 60
   aattggacaa gcgtcaacaa ctgtcactgg tgaaagccct cgagtccatg aaagccgacc 120
   attcatctga tgggttccag gcaatcgctt ccttccatgc tcttcctcct ctttgtccat 180
   caccagctgc ttcaaagagg tttgcgtgct gcgtccatgg catggcaacg ttcccacaat 240
   ggcaccgtct gtacacagtc caattccaag attctctcag aaaacatggt gcagtcgttg 300
   gacttccgta ctgggactgg accctacctc gttctgaatt accagagctc ctgaccgtct 360
   caactattca tgacccggag acaggcagag atataccaaa tccatttatt ggttctaaaa 420
   tagagtttga aggagaaaac gtacatacta aaagagatat caatagggat cgtctcttcc 480
   agggatcaac aaaaacacat cataactggt ttattgagca agcactgctt gctcttgaac 540
   aaaccaacta ctgcgacttc gaggttcagt ttgaaattat gcataatggt gttcatacct 600
   gggttggagg caaggagccc tatggaattg gccatctgca ttatgcttcc tatgatccac 660
   ttttctacat ccatcactcc caaactgatc gtatttgggc tatatggcaa tcgttgcagc 720
   gtttcagagg actttctgga tctgaggcta actgtgctgt aaatctcatg aaaactcctc 780
   tgaagccttt cagctttgga gcaccatata atcttaatga tcacacgcat gatttctcaa 840
   agcctgaaga tacattcgac taccaaaagt ttggatacat atatgacact ctggaatttg 900
   cagggtggtc aattcgtggc attgaccata ttgtccgtaa caggcaggaa cattcaaggg 960
   tctttgccgg attcttgctt gaaggatttg gcacctctgc cactgtcgat ttccaggtct 1020
   gtcgcacagc gggagactgt gaagatgcag ggtacttcac cgtgttggga ggtgaaaaag 1080
   aaatgccttg ggcctttgat cggctttaca agtacgacat aacagaaacc ttagacaaga 1140
   tgaaccttcg acatgacgaa atcttccaga ttgaagtaac cattacatcc tacgatggaa 1200
   ctgtactcga tagtggcctt attcccacac cgtcaatcat ctatgatcct gctcatc 1257
<210> 55
   <211> 1254
   <212> DNA
   <213> Megathura crenulata
<400> 55
   atgatattag ttcgcaccac ctgtcgctca acaaggttcg tcatgatctg agtacactga 60
   gtgagcgaga tattggaagc cttaaatatg ctttgagcag cttgcaggca gatacctcag 120
   cagatggttt tgctgccatt gcatccttcc atggtctgcc tgccaaatgt aatgacagcc 180
   acaataacga ggtggcatgc tgtatccatg gaatgcctac attcccccac tggcacagac 240
   tctacaccct ccaatttgag caagctctaa gaagacatgg ctctagtgta gcagtaccct 300
   actgggactg gacaaagcca atacataata ttccacatct gttcacagac aaagaatact 360
   acgatgtctg gagaaataaa gtaatgccaa atccatttgc ccgagggtat gtcccctcac 420
   acgatacata cacggtaaga gacgtccaag aaggcctgtt ccacctgaca tcaacgggtg 480
   aacactcagc gcttctgaat caagctcttt tggcgctgga acagcacgac tactgcgatt 540
   ttgcagtcca gtttgaagtc atgcacaaca caatccatta cctagtggga ggacctcaag 600
   tctattcttt gtcatccctt cattatgctt catatgatcc gatcttcttc atacaccact 660
   cctttgtaga caaggtttgg gctgtctggc aggctcttca agaaaagaga ggccttccat 720
   cagaccgtgc tgactgcgct gttagtctga tgactcagaa catgaggcct ttccattacg 780
   aaattaacca taaccagttc accaagaaac atgcagttcc aaatgatgtt ttcaagtacg 840
   aactcctggg ttacagatac gacaatctgg aaatcggtgg catgaatttg catgaaattg 900
   aaaaggaaat caaagacaaa cagcaccatg tgagagtgtt tgcagggttc ctccttcacg 960
   gaattagaac ctcagctgat gtccaattcc agatttgtaa aacatcagaa gattgtcacc 1020
   atggaggcca aatcttcgtt cttgggggga ctaaagagat ggcctgggct tataaccgtt 1080
   tattcaagta cgatattacc catgctcttc atgacgcaca catcactcca gaagacgtat 1140
   tccatccctc tgaaccattc ttcatcaagg tgtcagtgac agccgtcaac ggaacagttc 1200
   ttccggcttc aatcctgcat gcaccaacca ttatctatga acctggtctc ggtg 1254
<210> 56
   <211> 509
   <212> DNA
   <213> Megathura crenulata
<400> 56
   accatcacga agatcatcat tcttcttcta tggctggaca tggtgtcaga aaggaaatca 60
   acacacttac cactgcagag gtggacaatc tcaaagatgc catgagagcc gtcatggcag 120
   accacggtcc aaatggatac caggctatag cagcgttcca tggaaaccca ccaatgtgcc 180
   ctatgccaga tggaaagaat tactcgtgtt gtacacatgg catggctact ttcccccact 240
   ggcacagact gtacacaaaa cagatggaag atgccttgac cgcccatggt gccagagtcg 300
   gccttcctta ctgggacggg acaactgcct ttacagcttt gccaactttt gtcacagatg 360
   aagaggacaa tcctttccat catggtcaca tagactattt gggagtggat acaactcggt 420
   cgccccgaga caagttgttc aatgatccag agcgaggatc agaatcgttc ttctacaggc 480
   aggttctctt ggctttggag cagacagat                                  509
<210> 57
   <211> 943
   <212> DNA
   <213> Megathura crenulata
<400> 57
   ggcctgccct actgggattg gaccatgcca atgagtcatt tgccagaact ggctacaagt 60
   gagacctacc tcgatccagt tactggggaa actaaaaaca accctttcca tcacgcccaa 120
   gtggcgtttg aaaatggtgt aacaagcagg aatcctgatg ccaaactttt tatgaaacca 180
   acttacggag accacactta cctcttcgac agcatgatct acgcatttga gcaggaagac 240
   ttctgcgact ttgaagtcca atatgagctc acgcataatg caatacatgc atgggttgga 300
   ggcagtgaaa agtattcaat gtcttctctt cactacactg cttttgatcc tatattttac 360
   ctccatcact caaatgttga tcgtctctgg gccatttggc aagctcttca aatcaggaga 420
   ggcaagtctt acaaggccca ctgcgcctcg tctcaagaaa gagaaccatt aaagcctttt 480
   gcattcagtt ccccactgaa caacaacgag aaaacgtacc acaactctgt ccccactaac 540
   gtttatgact atgtgggagt tttgcactat cgatatgatg accttcagtt tggcggtatg 600
   accatgtcag aacttgagga atatattcac aagcagacac aacatgatag aacctttgca 660
   ggattcttcc tttcatatat tggaacatca gcaagcgtag atatcttcat caatcgagaa 720
   ggtcatgata aatacaaagt gggaagtttt gtagtacttg gtggatccaa agaaatgaaa 780
   tggggctttg atagaatgta caagtatgag atcactgagg ctctgaagac gctgaatgtt 840
   gcagtggatg atgggttcag cattactgtt gagatcaccg atgttgatgg atctccccca 900
   tctgcagatc tcattccacc tcctgctata atctttgaac gtg                  943
<210> 58
   <211> 1248
   <212> DNA
   <213> Megathura crenulata
<400> 58
   ctgatgccaa agactttggc catagcagaa aaatcaggaa agccgttgat tctctgacag 60
   tcgaagaaca aacttcgttg aggcgagcta tggcagatct acaggacgac aaaacatcag 120
   ggggtttcca gcagattgca gcattccacg gagaaccaaa atggtgtcca agccccgaag 180
   cggagaaaaa atttgcatgc tgtgttcatg gaatggctgt tttccctcac tggcacagat 240
   tgctgacagt tcaaggagaa aatgctctga ggaaacatgg ctttactggt ggactgccct 300
   actgggactg gactcgatca atgagcgccc ttccacattt tgttgctgat cctacttaca 360
   atgatgctat ttccagccag gaagaagata acccatggca tcatggtcac atagactctg 420
   ttgggcatga tactacaaga gatgtgcgtg atgatcttta tcaatctcct ggtttcggtc 480
   actacacaga tattgcaaaa caagtccttc tggcctttga gcaggacgat ttctgtgatt 540
   ttgaggtaca atttgaaatt gcccataatt tcatacatgc tctggttggt ggtaacgaac 600
   catacagtat gtcatctttg aggtatacta catacgatcc aatcttcttc ttgcaccgct 660
   ccaatacaga ccgactttgg gccatttggc aagctttgca aaaataccgg gggaaaccat 720
   acaacactgc aaactgtgcc attgcatcca tgagaaaacc acttcagcca tttggtcttg 780
   atagtgtcat aaatccagat gacgaaactc gtgaacattc ggttcctttc cgagtcttcg 840
   actacaagaa caacttcgac tatgagtatg agagcctggc atttaatggt ctgtctattg 900
   cccaactgga ccgagagttg cagagaagaa agtcacatga cagagtcttt gcaggattcc 960
   ttcttcatga aattggacag tctgcactcg tgaaattcta cgtttgcaaa cacaatgtat 1020
   ctgactgtga ccattatgct ggagaattct acattttggg agatgaagct gagatgcctt 1080
   ggaggtatga ccgtgtgtac aagtacgaga taacacagca gctgcacgat ttagatctac 1140
   atgttggaga taatttcttc cttaaatatg aagcctttga tctgaatggc ggaagtcttg 1200
   gtggaagtat cttttctcag ccttcggtga ttttcgagcc agctgcag             1248
<210> 59
   <211> 1257
   <212> DNA
   <213> Megathura crenulata
<400> 59
   gttcacacca ggctgatgaa tatcgtgagg cagtaacaag cgctagccac ataagaaaaa 60
   atatccggga cctctcagag ggagaaattg agagcatcag atctgctttc ctccaaattc 120
   aaaaagaggg tatatatgaa aacattgcaa agttccatgg aaaaccagga ctttgtgaac 180
   atgatggaca tcctgttgct tgttgtgtcc atggcatgcc cacctttccc cactggcaca 240
   gactgtacgt tcttcaggtg gagaatgcgc tcttagaacg agggtctgca gttgctgttc 300
   cttactggga ctggaccgag aaagctgact ctctgccatc attaatcaat gatgcaactt 360
   atttcaattc acgatcccag acctttgatc ctaatccttt cttcagggga catattgcct 420
   tcgagaatgc tgtgacgtcc agagatcctc agccagaact atgggacaat aaggacttct 480
   acgagaatgt catgctggct cttgagcaag acaacttctg tgactttgag attcagcttg 540
   agctgataca caacgccctt cattctagac ttggaggaag ggctaaatac tccctttcgt 600
   ctcttgatta taccgcattt gatcctgtat ttttccttca ccatgcaaac gttgacagaa 660
   tctgggccat ctggcaggac ttgcagagat atagaaagaa accatacaat gaggctgact 720
   gcgcagtcaa cgagatgcgt aaacctcttc aaccatttaa taacccagaa cttaacagtg 780
   attccatgac gcttaaacac aacctcccac aagacagttt tgattatcaa aaccgcttca 840
   ggtaccaata tgataacctt caatttaacc acttcagcat acaaaagcta gaccaaacta 900
   ttcaggctag aaaacaacac gacagagttt ttgctggctt tattcttcac aacattggga 960
   catctgctgt tgtagatatt tatatttgcg ttgaacaagg aggagaacaa aactgcaaga 1020
   caaaggcggg ttccttcacg attctggggg gagaaacaga aatgccattc cactttgacc 1080
   gcttgtacaa atttgacata acgtctgctc tgcataaact tggtgttccc ttggacggac 1140
   atggattcga catcaaagtt gacgtcagag ctgtcaatgg atcgcatctt gatcaacaca 1200
   tcctcaacga accgagtctg ctttttgttc ctggtgaacg taagaatata tattatg    1257
<210> 60
   <211> 1239
   <212> DNA
   <213> Megathura crenulata
<400> 60
   atgggctttc acaacataat cttgtgcgaa aagaagtaag ctctcttaca acactggaga 60
   aacatttttt gaggaaagct ctcaagaaca tgcaagcaga tgattctcca gacggatatc 120
   aagctattgc ttctttccac gctttgcctc ctctttgtcc aagtccatct gctgcacata 180
   gacacgcttg ttgcctccat ggtatggcta ccttccctca gtggcacaga ctctacacag 240
   ttcagttcga agattctttg aaacgacatg gttctattgt cggacttcca tattgggatt 300
   ggctgaaacc gcagtctgca ctccctgatt tggtgacaca ggagacatac gagcacctgt 360
   tttcacacaa aaccttccca aatccgttcc tcaaggcaaa tatagaattt gagggagagg 420
   gagtaacaac agagagggat gttgatgctg aacacctctt tgcaaaagga aatctggttt 480
   acaacaactg gttttgcaat caggcactat atgcactaga acaagaaaat tactgtgact 540
   ttgaaataca gttcgaaatt ttgcataatg gaattcattc atgggttgga ggatcaaaga 600
   cccattcaat aggtcatctt cattacgcat catacgatcc actgttctat atccaccatt 660
   cgcagacaga tcgcatttgg gctatctggc aagctctcca ggagcacaga ggtctttcag 720
   ggaaggaagc acactgcgcc ctggagcaaa tgaaagaccc tctcaaacct ttcagctttg 780
   gaagtcccta taatttgaac aaacgcactc aagagttctc caagcctgaa gacacatttg 840
   attatcaccg attcgggtat gagtatgatt ccctcgaatt tgttggcatg tctgtttcaa 900
   gtttacataa ctatataaaa caacaacagg aagctgatag agtcttcgca ggattccttc 960
   ttaaaggatt tggacaatca gcatccgtat cgtttgatat ctgcagacca gaccagagtt 1020
   gccaagaagc tggatacttc tcagttctcg gtggaagttc agaaatgccg tggcagtttg 1080
   acaggcttta caagtacgac attacaaaaa cgttgaaaga catgaaactg cgatacgatg 1140
   acacatttac catcaaggtt cacataaagg atatagctgg agctgagttg gacagcgatc 1200
   tgattccaac tccttctgtt ctccttgaag aaggaaagc                        1239
<210> 61
   <211> 1251
   <212> DNA
   <213> Haliotis tuberculata
<400> 61
   atgggatcaa tgtacgtcac gttggtcgta atcggattcg tatggaacta tctgaactca 60
   ccgagagaga tctcgccagc ctgaaatctg caatgaggtc tctacaagct gacgatgggg 120
   tgaacggtta tcaagccatt gcatcattcc acggtctccc ggcttcttgt catgatgatg 180
   agggacatga gattgcctgt tgtatccacg gaatgccagt attcccacac tggcacaggc 240
   tttacaccct gcaaatggac atggctctgt tatctcacgg atctgctgtt gctattccat 300
   actgggactg gaccaaacct atcagcaaac tgcctgatct cttcaccagc cctgaatatt 360
   acgatccttg gagggatgca gttgtcaata atccatttgc taaaggctac attaaatccg 420
   aggacgctta cacggttagg gatcctcagg acattttgta ccacttgcag gacgaaacgg 480
   gaacatctgt tttgttagat caaactcttt tagccttaga gcagacagat ttctgtgatt 540
   ttgaggttca atttgaggtc gtccataatg ctattcacta cttggtgggt ggtcgacaag 600
   tttatgctct ttcttctcaa cactatgctt catatgaccc agccttcttt attcatcact 660
   cctttgttga caaaatatgg gcagtctggc aagctctgca aaagaagaga aagcgtccct 720
   atcataaagc ggattgtgct cttaacatga tgaccaaacc aatgcgacca tttgcacacg 780
   atttcaatca caatggattc acaaaaatgc acgcagtccc caacactcta tttgactttc 840
   aggacctttt ctacacgtat gacaacttag aaattgctgg catgaatgtt aatcagttgg 900
   aagcggaaat caaccggcga aaaagccaaa caagagtctt tgccgggttc cttctacatg 960
   gcattggaag atcagctgat gtacgatttt ggatttgcaa gacagctgac gactgccacg 1020
   catctggcat gatctttatc ttaggaggtt ctaaagagat gcactgggcc tatgacagga 1080
   actttaaata cgacatcacc caagctttga aggctcagtc catacaccct gaagatgtgt 1140
   ttgacactga tgctcctttc ttcattaaag tggaggtcca tggtgtaaac aagactgctc 1200
   tcccatcttc agctatccca gcacctacta taatctactc agctggtgaa g          1251
<210> 62
   <211> 1185
   <212> DNA
   <213> Haliotis tuberculata
<400> 62
   atcatattgc tggcagtgga gtcaggaaag acgtgacgtc tcttaccgca tctgagatag 60
   agaacctgag gcatgctctg caaagcgtga tggatgatga tggacccaat ggattccagg 120
   caattgctgc ttatcacgga agtcctccca tgtgtcacat gcntgatggt agagacgttg 180
   catgttgtac tcatggaatg gcatctttcc ctcactggca cagactgttt gtgaaacaga 240
   tggaggatgc actggctgcg catggagctc acattggcat accatactgg gattggacaa 300
   gtgcgtttag tcatctgcct gccctagtga ctgaccacga gcacaatccc ttccaccacg 360
   gacatattgc tcatcggaat gtggatacat ctcgatctcc gagagacatg ctgttcaatg 420
   accccgaaca cgggtcagaa tcattcttct atagacaggt tctcttggct ctagaacaga 480
   cagacttctg ccaatttgaa gttcagtttg aaataacaca caatgcaatc cactcttgga 540
   ctggaggaca tactccatat ggaatgtcat cactggaata tacagcatat gatccactct 600
   tttatctcca ccattccaac actgatcgta tctgggccat ctggcaggca ctccagaaat 660
   acagaggttt tcaatacaac gcagctcatt gcgatatcca ggttctgaaa caacctctta 720
   aaccattcag cgagtccagg aatccaaacc cagtcaccag agccaattct agggcagtcg 780
   attcatttga ttatgagaga ctcaattatc aatatgacac acttaccttc cacggacatt 840
   ctatctcaga acttgatgcc atgcttcaag agagaaagaa ggaagagaga acatttgcag 900
   ccttcctgtt gcacggattt ggcgccagtg ctgatgtttc gtttgatgtc tgcacacctg 960
   atggtcattg tgcctttgct ggaaccttcg cggtacttgg tggggagctt gagatgccct 1020
   ggtcctttga aagattgttc cgttacgata tcacaaaggt tctcaagcag atgaatcttc 1080
   actatgattc tgagttccac tttgagttga agattgttgg cacagatgga acagaactgc 1140
   catcggatcg tatcaagagc cctaccattg aacaccatgg aggag                 1185
<210> 63
   <211> 422
   <212> PRT
   <213> Haliotis tuberculata
<220>
   <221> SIGNAL
   <222> (1)..(15)
<400> 63
   Leu Val Gln Phe Leu Leu Val Ala Leu Val Val Gly Ala Gly Ala Asp
    1               5                  10                  15
   Asn Val Val Arg Lys Asp Val Ser His Leu Thr Asp Asp Glu Val Gln
               20                  25                  30
   Ala Leu His Gly Ala Leu His Asp Val Thr Ala Ser Thr Gly Pro Leu
           35                  40                  45
   Ser Phe Glu Asp Ile Thr Ser Tyr His Ala Ala Pro Ala Ser Cys Asp
       50                  55                  60
   Tyr Lys Gly Arg Lys Ile Ala Cys Cys Val His Gly Met Pro Ser Phe
   65                  70                  75                  80
   Pro Phe Trp His Arg Ala Tyr Val Val Gln Ala Glu Arg Ala Leu Leu
                   85                  90                  95
   Ser Lys Arg Lys Thr Val Gly Met Pro Tyr Trp Asp Trp Thr Gln Thr
               100                 105                 110
   Leu Thr His Leu Pro Ser Leu Val Thr Glu Pro Ile Tyr Ile Asp Ser
           115                 120                 125
   Lys Gly Gly Lys Ala Gln Thr Asn Tyr Trp Tyr Arg Gly Glu Ile Ala
       130                 135                 140
   Phe Ile Asn Lys Lys Thr Ala Arg Ala Val Asp Asp Arg Leu Phe Glu
   145                 150                 155                 160
   Lys Val Glu Pro Gly His Tyr Thr His Leu Met Glu Thr Val Leu Asp
                   165                 170                 175
   Ala Leu Glu Gln Asp Glu Phe Cys Lys Phe Glu Ile Gln Phe Glu Leu
               180                 185                 190
   Ala His Asn Ala Ile His Tyr Leu Val Gly Gly Lys Phe Glu Tyr Ser
           195                 200                 205
   Met Ser Asn Leu Glu Tyr Thr Ser Tyr Asp Pro Ile Phe Phe Leu His
       210                 215                 220
   His Ser Asn Val Asp Arg Leu Phe Ala Ile Trp Gln Arg Leu Gln Glu
   225                 230                 235                 240
   Leu Arg Gly Lys Asn Pro Asn Ala Met Asp Cys Ala His Glu Leu Ala
                   245                 250                 255
   His Gln Gln Leu Gln Pro Phe Asn Arg Asp Ser Asn Pro Val Gln Leu
               260                 265                 270
   Thr Lys Asp His Ser Thr Pro Ala Asp Leu Phe Asp Tyr Lys Gln Leu
           275                 280                 285
   Gly Tyr Ser Tyr Asp Ser Leu Asn Leu Asn Gly Met Thr Pro Glu Gln
       290                 295                 300
   Leu Lys Thr Glu Leu Asp Glu Arg His Ser Lys Glu Arg Ala Phe Ala
   305                 310                 315                 320
   Ser Phe Arg Leu Ser Gly Phe Gly Gly Ser Ala Asn Val Val Val Tyr
                   325                 330                 335
   Ala Cys Val Pro Asp Asp Asp Pro Arg Ser Asp Asp Tyr Cys Glu Lys
               340                 345                 350
   Ala Gly Asp Phe Phe Ile Leu Gly Gly Gln Ser Glu Met Pro Trp Arg
           355                 360                 365
   Phe Tyr Arg Pro Phe Phe Tyr Asp Val Thr Glu Ala Val His His Leu
       370                 375                 380
   Gly Val Pro Leu Ser Gly His Tyr Tyr Val Lys Thr Glu Leu Phe Ser
   385                 390                 395                 400
   Val Asn Gly Thr Ala Leu Ser Pro Asp Leu Leu Pro Gln Pro Thr Val
                   405                 410                 415
   Ala Tyr Arg Pro Gly Lys
   420
<210> 64
   <211> 511
   <212> PRT
   <213> Haliotis tuberculata
<400> 64
   Val His Arg Gly Gly Asn His Glu Asp Glu His His Asp Asp Arg Leu
   1               5                   10                  15
   Ala Asp Val Leu Ile Arg Lys Glu Val Asp Phe Leu Ser Leu Gln Glu
               20                  25                  30
   Ala Asn Ala Ile Lys Asp Ala Leu Tyr Lys Leu Gln Asn Asp Asp Ser
           35                  40                  45
   Lys Gly Gly Phe Glu Ala Ile Ala Gly Tyr His Gly Tyr Pro Asn Met
       50                  55                  60
   Cys Pro Glu Arg Gly Thr Asp Lys Tyr Pro Cys Cys Val His Gly Met
   65                  70                  75                  80
   Pro Val Phe Pro His Trp His Arg Leu His Thr Ile Gln Met Glu Arg
                   85                  90                  95
   Ala Leu Lys Asn His Gly Ser Pro Met Gly Ile Pro Tyr Trp Asp Trp
               100                 105                 110
   Thr Lys Lys Met Ser Ser Leu Pro Ser Phe Phe Gly Asp Ser Ser Asn
           115                 120                 125
   Asn Asn Pro Phe Tyr Lys Tyr Tyr Ile Arg Gly Val Gln His Glu Thr
       130                 135                 140
   Thr Arg Asp Val Asn Gln Arg Leu Phe Asn Gln Thr Lys Phe Gly Glu
   145                 150                 155                 160
   Phe Asp Tyr Leu Tyr Tyr Leu Thr Leu Gln Val Leu Glu Glu Asn Ser
                   165                 170                 175
   Tyr Cys Asp Phe Glu Val Gln Tyr Glu Ile Leu His Asn Ala Val His
               180                 185                 190
   Ser Trp Leu Gly Gly Thr Gly Gln Tyr Ser Met Ser Thr Leu Glu His
           195                 200                 205
   Ser Ala Phe Asp Pro Val Phe Met Ile His His Ser Ser Leu Asp Arg
       210                 215                 220
   Ile Trp Ile Leu Trp Gln Lys Leu Gln Lys Ile Arg Met Lys Pro Tyr
   225                 230                 235                 240
   Tyr Ala Leu Asp Cys Ala Gly Asp Arg Leu Met Lys Asp Pro Leu His
                   245                 250                 255
   Pro Phe Asn Tyr Glu Thr Val Asn Glu Asp Glu Phe Thr Arg Ile Asn
               260                 265                 270
   Ser Phe Pro Ser Ile Leu Phe Asp His Tyr Arg Phe Asn Tyr Glu Tyr
           275                 280                 285
   Asp Asn Met Arg Ile Arg Gly Gln Asp Ile His Glu Leu Glu Glu Val
       290                 295                 300
   Ile Gln Glu Leu Arg Asn Lys Asp Arg Ile Phe Ala Gly Phe Val Leu
   305                 310                 315                 320
   Ser Gly Leu Arg Ile Ser Ala Thr Val Lys Val Phe Ile His Ser Lys
                   325                 330                 335
   Asn Asp Thr Ser His Glu Glu Tyr Ala Gly Glu Phe Ala Val Leu Gly
               340                 345                 350
   Gly Glu Lys Glu Met Pro Trp Ala Tyr Glu Arg Met Leu Lys Leu Asp
           355                 360                 365
   Ile Ser Asp Ala Val His Lys Leu His Val Lys Asp Glu Asp Ile Arg
       370                 375                 380
   Phe Arg Val Val Val Thr Ala Tyr Asn Gly Asp Val Val Thr Thr Arg
   385                 390                 395                 400
   Leu Ser Gln Pro Phe Ile Val His Arg Pro Ala His Val Ala His Asp
                   405                 410                 415
   Ile Leu Val Ile Pro Val Gly Ala Gly His Asp Leu Pro Pro Lys Val
               420                 425                 430
   Val Val Lys Ser Gly Thr Lys Val Glu Phe Thr Pro Ile Asp Ser Ser
           435                 440                 445
   Val Asn Lys Ala Met Val Glu Leu Gly Ser Tyr Thr Ala Met Ala Lys
       450                 455                 460
   Cys Ile Val Pro Pro Phe Ser Tyr His Gly Phe Glu Leu Asp Lys Val
   465                 470                 475                 480
   Tyr Ser Val Asp His Gly Asp Tyr Tyr Ile Ala Ala Gly Thr His Ala
                   485                 490                 495
   Leu Cys Glu Gln Asn Leu Arg Leu His Ile His Val Glu His Glu
               500                 505                 510
<210> 65
   <211> 197
   <212> PRT
   <213> Haliotis tuberculata
<400> 65
   Gly Leu Pro Tyr Trp Asp Trp Thr Gln His Leu Thr Gln Leu Pro Asp
    1               5                  10                  15
   Leu Val Ser Asp Pro Leu Phe Val Asp Pro Glu Gly Gly Lys Ala His
               20                  25                  30
   Asp Asn Ala Trp Tyr Arg Gly Asn Ile Lys Phe Glu Asn Lys Lys Thr
           35                  40                  45
   Ala Arg Ala Val Asp Asp Arg Leu Phe Glu Lys Val Gly Pro Gly Glu
       50                  55                  60
   Asn Thr Arg Leu Phe Glu Gly Ile Leu Asp Ala Leu Glu Gln Asp Glu
   65                  70                  75                  80
   Phe Cys Asn Phe Glu Ile Gln Phe Glu Leu Ala His Asn Ala Ile His
                   85                  90                  95
   Tyr Leu Val Gly Gly Arg His Thr Tyr Ser Met Ser His Leu Glu Tyr
               100                 105                 110
   Thr Ser Tyr Asp Pro Leu Phe Phe Leu His His Ser Asn Pro Asp Arg
           115                 120                 125
   Ile Phe Ala Ile Trp Glu Arg Leu Gln Val Leu Arg Gly Lys Asp Pro
       130                 135                 140
   Asn Thr Ala Asp Cys Ala His Asn Leu Ile His Glu Pro Met Glu Pro
   145                 150                 155                 160
   Phe Arg Arg His Glu Pro Met Glu Pro Phe Arg Arg Asp Ser Asn Pro
                   165                 170                 175
   Leu Asp Leu Thr Arg Glu Asn Ser Lys Pro Ile Asp Ser Phe Asp Tyr
               180                 185                 190
   Ala His Leu Gly Tyr
           195
<210> 66
   <211> 415
   <212> PRT
   <213> Haliotis tuberculata
<400> 66
   Val Thr Glu Ala Pro Ala Pro Ser Ser Asp Ala His Leu Ala Val Arg
    1               5                  10                  15
   Lys Asp Ile Asn His Leu Thr Arg Glu Glu Val Tyr Glu Leu Arg Arg
               20                  25                  30
   Ala Met Glu Arg Phe Gln Ala Asp Thr Ser Val Asp Gly Tyr Gln Ala
           35                  40                  45
   Thr Val Glu Tyr His Gly Leu Pro Ala Arg Cys Pro Phe Pro Glu Ala
        50                  55                  60
   Thr Asn Arg Phe Ala Cys Cys Ile His Gly Met Ala Thr Phe Pro His
   65                  70                  75                  80
   Trp His Arg Leu Phe Val Thr Gln Val Glu Asp Ala Leu Ile Arg Arg
                   85                  90                  95
   Gly Ser Pro Ile Gly Val Pro Tyr Trp Asp Trp Thr Gln Pro Met Ala
               100                 105                 110
   His Leu Pro Gly Leu Ala Asp Asn Ala Thr Tyr Arg Asp Pro Ile Ser
           115                 120                 125
   Gly Asp Ser Arg His Asn Pro Phe His Asp Val Glu Val Ala Phe Glu
       130                 135                 140
   Asn Gly Arg Thr Glu Arg His Pro Asp Ser Arg Leu Phe Glu Gln Pro
   145                 150                 155                 160
   Leu Phe Gly Lys His Thr Arg Leu Phe Asp Ser Ile Val Tyr Ala Phe
                   165                 170                 175
   Glu Gln Glu Asp Phe Cys Asp Phe Glu Val Gln Phe Glu Met Thr His
               180                 185                 190
   Asn Asn Ile His Ala Trp Ile Gly Gly Gly Glu Lys Tyr Ser Met Ser
           195                 200                 205
   Ser Leu His Tyr Thr Ala Phe Asp Pro Ile Phe Tyr Leu Arg His Ser
       210                 215                 220
   Asn Thr Asp Arg Leu Trp Ala Ile Trp Gln Ala Leu Gln Ile Arg Arg
   225                 230                 235                 240
   Asn Arg Pro Tyr Lys Ala His Cys Ala Trp Ser Glu Glu Arg Gln Pro
                   245                 250                 255
   Leu Lys Pro Phe Ala Phe Ser Ser Pro Leu Asn Asn Asn Glu Lys Thr
               260                 265                 270
   Tyr Glu Asn Ser Val Pro Thr Asn Val Tyr Asp Tyr Glu Gly Val Leu
           275                 280                 285
   Gly Tyr Thr Tyr Asp Asp Leu Asn Phe Gly Gly Met Asp Leu Gly Gln
       290                 295                 300
   Leu Glu Glu Tyr Ile Gln Arg Gln Arg Gln Arg Asp Arg Thr Phe Ala
   305                 310                 315                 320
   Gly Phe Phe Leu Ser His Ile Gly Thr Ser Ala Asn Val Glu Ile Ile
                   325                 330                 335
   Ile Asp His Gly Thr Leu His Thr Ser Val Gly Thr Phe Ala Val Leu
               340                 345                 350
   Gly Gly Glu Lys Glu Met Lys Trp Gly Phe Asp Arg Leu Tyr Lys Tyr
           355                 360                 365
   Glu Ile Thr Asp Glu Leu Arg Gln Leu Asn Leu Arg Ala Asp Asp Val
       370                 375                 380
   Phe Ser Ile Ser Val Lys Val Thr Asp Val Asp Gly Ser Glu Leu Ser
   385                 390                 395                 400
   Ser Glu Leu Ile Pro Ser Ala Ala Ile Ile Phe Glu Arg Ser His
                   405                 410                 415
<210> 67
   <211> 414
   <212> PRT
   <213> Haliotis tuberculata
<400> 67
   Gly His His Gln Ala Asp Glu Tyr Asp Glu Val Val Thr Ala Ala Ser
    1               5                  10                  15
   His Ile Arg Lys Asn Leu Lys Asp Leu Ser Lys Gly Glu Val Glu Ser
               20                  25                  30
   Leu Arg Ser Ala Phe Leu Gln Leu Gln Asn Asp Gly Val Tyr Glu Asn
           35                  40                  45
   Ile Ala Lys Phe His Gly Lys Pro Gly Leu Cys Asp Asp Asn Gly Arg
       50                  55                  60
   Lys Val Ala Cys Cys Val His Gly Met Pro Thr Phe Pro Gln Trp His
    65                  70                  75                  80
   Arg Leu Tyr Val Leu Gln Val Glu Asn Ala Leu Leu Glu Arg Gly Ser
                   85                  90                  95
   Ala Val Ser Val Pro Tyr Trp Asp Trp Thr Glu Thr Phe Thr Glu Leu
               100                 105                 110
   Pro Ser Leu Ile Ala Glu Ala Thr Tyr Phe Asn Ser Arg Gln Gln Thr
           115                 120                 125
   Phe Asp Pro Asn Pro Phe Phe Arg Gly Lys Ile Ser Phe Glu Asn Ala
       130                 135                 140
   Val Thr Thr Arg Asp Pro Gln Pro Glu Leu Tyr Val Asn Arg Tyr Tyr
   145                 150                 155                 160
   Tyr Gln Asn Val Met Leu Val Phe Glu Gln Asp Asn Tyr Cys Asp Phe
                   165                 170                 175
   Glu Ile Gln Phe Glu Met Val His Asn Val Leu His Ala Trp Leu Gly
               180                 185                 190
   Gly Arg Ala Thr Tyr Ser Ile Ser Ser Leu Asp Tyr Ser Ala Phe Asp
           195                 200                 205
   Pro Val Phe Phe Leu His His Ala Asn Thr Asp Arg Leu Trp Ala Ile
       210                 215                 220
   Trp Gln Glu Leu Gln Arg Tyr Arg Lys Lys Pro Tyr Asn Glu Ala Asp
   225                 230                 235                 240
   Cys Ala Ile Asn Leu Met Arg Lys Pro Leu His Pro Phe Asp Asn Ser
                   245                 250                 255
   Asp Leu Asn His Asp Pro Val Thr Phe Lys Tyr Ser Lys Pro Thr Asp
               260                 265                 270
   Gly Phe Asp Tyr Gln Asn Asn Phe Gly Tyr Lys Tyr Asp Asn Leu Glu
           275                 280                 285
   Phe Asn His Phe Ser Ile Pro Arg Leu Glu Glu Ile Ile Arg Ile Arg
       290                 295                 300
   Gln Arg Gln Asp Arg Val Phe Ala Gly Phe Leu Leu His Asn Ile Gly
   305                 310                 315                 320
   Thr Ser Ala Thr Val Glu Ile Phe Val Cys Val Pro Thr Thr Ser Gly
                   325                 330                 335
   Glu Gln Asn Cys Glu Asn Lys Ala Gly Thr Phe Ala Val Leu Gly Gly
               340                 345                 350
   Glu Thr Glu Met Ala Phe His Phe Asp Arg Leu Tyr Arg Phe Asp Ile
           355                 360                 365
   Ser Glu Thr Leu Arg Asp Leu Gly Ile Gln Leu Asp Ser His Asp Phe
       370                 375                 380
   Asp Leu Ser Ile Lys Ile Gln Gly Val Asn Gly Ser Tyr Leu Asp Pro
   385                 390                 395                 400
   His Ile Leu Pro Glu Pro Ser Leu Ile Phe Val Pro Gly Ser
                   405                 410
<210> 68
   <211> 419
   <212> PRT
   <213> Haliotis tuberculata
<400> 68
   Ser Ser Phe Leu Arg Pro Asp Gly His Ser Asp Asp Ile Leu Val Arg
    1               5                  10                  15
   Lys Glu Val Asn Ser Leu Thr Thr Arg Glu Thr Ala Ser Leu Ile His
               20                  25                  30
   Ala Leu Lys Ser Met Gln Glu Asp His Ser Pro Asp Gly Phe Gln Ala
           35                  40                  45
   Ile Ala Ser Phe His Ala Leu Pro Pro Leu Cys Pro Ser Pro Ser Ala
        50                  55                  60
   Ala His Arg Tyr Ala Cys Cys Val His Gly Met Ala Thr Phe Pro Gln
   65                  70                  75                  80
   Trp His Arg Leu Tyr Thr Val Gln Phe Gln Asp Ala Leu Arg Arg His
                   85                  90                  95
   Gly Ala Thr Val Gly Val Pro Tyr Trp Asp Trp Leu Arg Pro Gln Ser
               100                 105                 110
   His Leu Pro Glu Leu Val Thr Met Glu Thr Tyr His Asp Ile Trp Ser
           115                 120                 125
   Asn Arg Asp Phe Pro Asn Pro Phe Tyr Gln Ala Asn Ile Glu Phe Glu
       130                 135                 140
   Gly Glu Asn Ile Thr Thr Glu Arg Glu Val Ile Ala Asp Lys Leu Phe
   145                 150                 155                 160
   Val Lys Gly Gly His Val Phe Asp Lys Leu Val Leu Gln Thr Ser His
                   165                 170                 175
   Pro Ser Ala Glu Gln Glu Asn Tyr Cys Asp Phe Glu Ile Gln Phe Glu
               180                 185                 190
   Ile Leu His Asn Gly Val His Thr Trp Val Gly Gly Ser Arg Thr Tyr
           195                 200                 205
   Ser Ile Gly His Leu His Tyr Ala Phe Tyr Asp Pro Leu Phe Tyr Leu
       210                 215                 220
   His His Phe Gln Thr Asp Arg Ile Trp Ala Ile Trp Gln Glu Leu Gln
   225                 230                 235                 240
   Glu Gln Arg Gly Leu Ser Gly Asp Glu Ala His Cys Ala Leu Glu Gln
                   245                 250                 255
   Met Arg Glu Pro Leu Lys Pro Phe Ser Phe Gly Ala Pro Tyr Asn Trp
               260                 265                 270
   Asn Gln Leu Thr Gln Asp Phe Ser Arg Pro Glu Asp Thr Phe Asp Tyr
           275                 280                 285
   Arg Lys Phe Gly Tyr Glu Tyr Asp Asn Leu Glu Phe Leu Gly Met Ser
       290                 295                 300
   Val Ala Glu Leu Asp Gln Tyr Ile Ile Glu His Gln Glu Asn Asp Arg
   305                 310                 315                 320
   Val Phe Ala Gly Phe Leu Leu Ser Gly Phe Gly Gly Ser Ala Ser Val
                   325                 330                 335
   Asn Phe Gln Val Cys Arg Ala Asp Ser Thr Cys Gln Asp Ala Gly Tyr
               340                 345                 350
   Phe Thr Val Leu Gly Gly Ser Ala Glu Met Ala Trp Ala Phe Asp Arg
   355 360 365
   Leu Tyr Lys Tyr Asp Ile Thr Glu Thr Leu Glu Lys Met His Leu Arg
   370 375 380
   Tyr Asp Asp Asp Phe Thr Ile Ser Val Ser Leu Thr Ala Asn Asn Gly
   385 390 395 400
   Thr Val Leu Ser Ser Ser Leu Ile Pro Thr Pro Ser Val Ile Phe Gln
   405 410 415
   Arg Gly His
<210> 69
   <211> 378
   <212> PRT
   <213> Megathura crenulata
<400> 69
   Arg Tyr Gln Ala Thr Ala Glu Tyr His Gly Leu Pro Ala Arg Cys Pro
   1 5 10 15
   Arg Pro Asp Ala Lys Asp Arg Tyr Ala Cys Cys Val His Gly Met Pro
   20 25 30
   Ile Phe Pro His Trp His Arg Leu Phe Val Thr Gln Val Glu Asp Ala
   35 40 45
   Leu Val Gly Arg Gly Ala Thr Ile Gly Ile Pro Tyr Trp Asp Trp Thr
   50 55 60
   Glu Pro Met Thr His Ile Pro Gly Leu Ala Gly Asn Lys Thr Tyr Val
   65 70 75 80
   Asp Ser His Gly Ala Ser His Thr Asn Pro Phe His Ser Ser Val Ile
   85 90 95
   Ala Phe Glu Glu Asn Ala Pro His Thr Lys Arg Gln Ile Asp Gln Arg
   100 105 110
   Leu Phe Lys Pro Ala Thr Phe Gly His His Thr Asp Leu Phe Asn Gln
   115 120 125
   Ile Leu Tyr Ala Phe Glu Gln Glu Asp Tyr Cys Asp Phe Glu Val Gln
   130 135 140
   Phe Glu Ile Thr His Asn Thr Ile His Ala Trp Thr Gly Gly Ser Glu
   145 150 155 160
   His Phe Ser Met Ser Ser Leu His Tyr Thr Ala Phe Asp Pro Leu Phe
   165 170 175
   Tyr Phe His His Ser Asn Val Asp Arg Leu Trp Ala Val Trp Gln Ala
   180 185 190
   Leu Gln Met Arg Arg His Lys Pro Tyr Arg Ala His Cys Ala Ile Ser
           195                 200                 205
   Leu Glu His Met His Leu Lys Pro Phe Ala Phe Ser Ser Pro Leu Asn
       210                 215                 220
   Asn Asn Glu Lys Thr His Ala Asn Ala Met Pro Asn Lys Ile Tyr Asp
   225                 230                 235                     240
   Tyr Glu Asn Val Leu His Tyr Thr Tyr Glu Asp Leu Thr Phe Gly Gly
                   245                 250                 255
   Ile Ser Leu Glu Asn Ile Glu Lys Met Ile His Glu Asn Gln Gln Glu
               260                 265                 270
   Asp Arg Ile Tyr Ala Gly Phe Leu Leu Ala Gly Ile Arg Thr Ser Ala
           275                 280                 285
   Asn Val Asp Ile Phe Ile Lys Thr Thr Asp Ser Val Gln His Lys Ala
       290                 295                 300
   Gly Thr Phe Ala Val Leu Gly Gly Ser Lys Glu Met Lys Trp Gly Phe
   305                 310                 315                     320
   Asp Arg Val Phe Lys Phe Asp Ile Thr His Val Leu Lys Asp Leu Asp
                   325                 330                 335
   Leu Thr Ala Asp Gly Asp Phe Glu Val Thr Val Asp Ile Thr Glu Val
               340                 345                 350
   Asp Gly Thr Lys Leu Ala Ser Ser Leu Ile Pro His Ala Ser Val Ile
           355                 360                 365
   Arg Glu His Ala Arg Gly Lys Leu Asn Arg
       370                 375
<210> 70
   <211> 419
   <212> PRT
   <213> Megathura crenulata
<400> 70
   Asp Ser Ala His Thr Asp Asp Gly His Thr Glu Pro Val Met Ile Arg
    1               5                  10                  15
   Lys Asp Ile Thr Gln Leu Asp Lys Arg Gln Gln Leu Ser Leu Val Lys
               20                  25                  30
   Ala Leu Glu Ser Met Lys Ala Asp His Ser Ser Asp Gly Phe Gln Ala
           35                  40                  45
   Ile Ala Ser Phe His Ala Leu Pro Pro Leu Cys Pro Ser Pro Ala Ala
       50                  55                  60
   Ser Lys Arg Phe Ala Cys Cys Val His Gly Met Ala Thr Phe Pro Gln
    65                  70                  75                  80
   Trp His Arg Leu Tyr Thr Val Gln Phe Gln Asp Ser Leu Arg Lys His
                    85                  90                  95
   Gly Ala Val Val Gly Leu Pro Tyr Trp Asp Trp Thr Leu Pro Arg Ser
               100                 105                 110
   Glu Leu Pro Glu Leu Leu Thr Val Ser Thr Ile His Asp Pro Glu Thr
           115                 120                 125
   Gly Arg Asp Ile Pro Asn Pro Phe Ile Gly Ser Lys Ile Glu Phe Glu
       130                 135                 140
   Gly Glu Asn Val His Thr Lys Arg Asp Ile Asn Arg Asp Arg Leu Phe
   145                 150                 155                 160
   Gln Gly Ser Thr Lys Thr His His Asn Trp Phe Ile Glu Gln Ala Leu
                   165                 170                 175
   Leu Ala Leu Glu Gln Thr Asn Tyr Cys Asp Phe Glu Val Gln Phe Glu
               180                 185                 190
   Ile Met His Asn Gly Val His Thr Trp Val Gly Gly Lys Glu Pro Tyr
           195                 200                 205
   Gly Ile Gly His Leu His Tyr Ala Ser Tyr Asp Pro Leu Phe Tyr Ile
       210                 215                 220
   His His Ser Gln Thr Asp Arg Ile Trp Ala Ile Trp Gln Ser Leu Gln
   225                 230                 235                 240
   Arg Phe Arg Gly Leu Ser Gly Ser Glu Ala Asn Cys Ala Val Asn Leu
                   245                 250                 255
   Met Lys Thr Pro Leu Lys Pro Phe Ser Phe Gly Ala Pro Tyr Asn Leu
               260                 265                 270
   Asn Asp His Thr His Asp Phe Ser Lys Pro Glu Asp Thr Phe Asp Tyr
           275                 280                 285
   Gln Lys Phe Gly Tyr Ile Tyr Asp Thr Leu Glu Phe Ala Gly Trp Ser
       290                 295                 300
   Ile Arg Gly Ile Asp His Ile Val Arg Asn Arg Gln Glu His Ser Arg
   305                 310                 315                 320
   Val Phe Ala Gly Phe Leu Leu Glu Gly Phe Gly Thr Ser Ala Thr Val
                   325                 330                 335
   Asp Phe Gln Val Cys Arg Thr Ala Gly Asp Cys Glu Asp Ala Gly Tyr
               340                 345                 350
   Phe Thr Val Leu Gly Gly Glu Lys Glu Met Pro Trp Ala Phe Asp Arg
           355                 360                 365
   Leu Tyr Lys Tyr Asp Ile Thr Glu Thr Leu Asp Lys Met Asn Leu Arg
       370                 375                 380
   His Asp Glu Ile Phe Gln Ile Glu Val Thr Ile Thr Ser Tyr Asp Gly
   385                 390                 395                 400
   Thr Val Leu Asp Ser Gly Leu Ile Pro Thr Pro Ser Ile Ile Tyr Asp
                   405                 410                 415
   Pro Ala His
<210> 71
   <211> 418
   <212> PRT
   <213> Megathura crenulata
<400> 71
   His Asp Ile Ser Ser His His Leu Ser Leu Asn Lys Val Arg His Asp
     1               5                  10                  15
   Leu Ser Thr Leu Ser Glu Arg Asp Ile Gly Ser Leu Lys Tyr Ala Leu
                20                  25                  30
   Ser Ser Leu Gln Ala Asp Thr Ser Ala Asp Gly Phe Ala Ala Ile Ala
           35                  40                  45
   Ser Phe His Gly Leu Pro Ala Lys Cys Asn Asp Ser His Asn Asn Glu
       50                  55                  60
   Val Ala Cys Cys Ile His Gly Met Pro Thr Phe Pro His Trp His Arg
   65                  70                  75                  80
   Leu Tyr Thr Leu Gln Phe Glu Gln Ala Leu Arg Arg His Gly Ser Ser
                   85                  90                  95
   Val Ala Val Pro Tyr Trp Asp Trp Thr Lys Pro Ile His Asn Ile Pro
               100                 105                 110
   His Leu Phe Thr Asp Lys Glu Tyr Tyr Asp Val Trp Arg Asn Lys Val
           115                 120                 125
   Met Pro Asn Pro Phe Ala Arg Gly Tyr Val Pro Ser His Asp Thr Tyr
       130                 135                 140
   Thr Val Arg Asp Val Gln Glu Gly Leu Phe His Leu Thr Ser Thr Gly
   145                 150                 155                 160
   Glu His Ser Ala Leu Leu Asn Gln Ala Leu Leu Ala Leu Glu Gln His
                   165                 170                 175
   Asp Tyr Cys Asp Phe Ala Val Gln Phe Glu Val Met His Asn Thr Ile
               180                 185                 190
   His Tyr Leu Val Gly Gly Pro Gln Val Tyr Ser Leu Ser Ser Leu His
           195                 200                 205
   Tyr Ala Ser Tyr Asp Pro Ile Phe Phe Ile His His Ser Phe Val Asp
       210                 215                 220
   Lys Val Trp Ala Val Trp Gln Ala Leu Gln Glu Lys Arg Gly Leu Pro
   225                 230                 235                 240
   Ser Asp Arg Ala Asp Cys Ala Val Ser Leu Met Thr Gln Asn Met Arg
                   245                 250                 255
   Pro Phe His Tyr Glu Ile Asn His Asn Gln Phe Thr Lys Lys His Ala
               260                 265                 270
   Val Pro Asn Asp Val Phe Lys Tyr Glu Leu Leu Gly Tyr Arg Tyr Asp
           275                 280                 285
   Asn Leu Glu Ile Gly Gly Met Asn Leu His Glu Ile Glu Lys Glu Ile
       290                 295                 300
   Lys Asp Lys Gln His His Val Arg Val Phe Ala Gly Phe Leu Leu His
   305                 310                 315                 320
   Gly Ile Arg Thr Ser Ala Asp Val Gln Phe Gln Ile Cys Lys Thr Ser
                   325                 330                 335
   Glu Asp Cys His His Gly Gly Gln Ile Phe Val Leu Gly Gly Thr Lys
               340                 345                 350
   Glu Met Ala Trp Ala Tyr Asn Arg Leu Phe Lys Tyr Asp Ile Thr His
           355                 360                 365
   Ala Leu His Asp Ala His Ile Thr Pro Glu Asp Val Phe His Pro Ser
       370                 375                 380
   Glu Pro Phe Phe Ile Lys Val Ser Val Thr Ala Val Asn Gly Thr Val
   385                 390                 395                 400
   Leu Pro Ala Ser Ile Leu His Ala Pro Thr Ile Ile Tyr Glu Pro Gly
                   405                 410                 415
   Leu Gly
<210> 72
   <211> 241
   <212> PRT
   <213> Megathura crenulata
<400> 72
   Asp His His Glu Asp His His Ser Ser Ser Met Ala Gly His Gly Val
   1               5                   10                  15
   Arg Lys Glu Ile Asn Thr Leu Thr Thr Ala Glu Val Asp Asn Leu Lys
               20                  25                  30
   Asp Ala Met Arg Ala Val Met Ala Asp His Gly Pro Asn Gly Tyr Gln
   35 40 45
   Ala Ile Ala Ala Phe His Gly Asn Pro Pro Met Cys Pro Met Pro Asp
   50 55 60
   Gly Lys Asn Tyr Ser Cys Cys Thr His Gly Met Ala Thr Phe Pro His
   65 70 75 80
   Trp His Arg Leu Tyr Thr Lys Gln Met Glu Asp Ala Leu Thr Ala His
   85 90 95
   Gly Ala Arg Val Gly Leu Pro Tyr Trp Asp Gly Thr Thr Ala Phe Thr
   100 105 110
   Ala Leu Pro Thr Phe Val Thr Asp Glu Glu Asp Asn Pro Phe His His
   115 120 125
   Gly His Ile Asp Tyr Leu Gly Val Asp Thr Thr Arg Ser Pro Arg Asp
   130 135 140
   Lys Leu Phe Asn Asp Pro Glu Arg Gly Ser Glu Ser Phe Phe Tyr Arg
   145 150 155 160
   Gln Val Leu Leu Ala Leu Glu Gln Thr Asp Phe Cys Gln Phe Glu Val
   165 170 175
   Gln Phe Glu Ile Thr His Asn Ala Ile His Ser Trp Thr Gly Gly Leu
   180 185 190
   Thr Pro Tyr Gly Met Ser Thr Leu Glu Tyr Thr Thr Tyr Asp Pro Leu
   195 200 205
   Phe Trp Leu His His Ala Asn Thr Asp Arg Ile Trp Ala Ile Trp Gln
   210 215 220
   Ala Leu Gln Glu Tyr Arg Gly Leu Pro Tyr Asp His Ala Asn Cys Glu
   225 230 235 240
   Ile
<210> 73
   <211> 98
   <212> PRT
   <213> Megathura crenulata
<400> 73
   Lys His His Glu Lys His His Glu Asp His His Glu Asp Ile Leu Val
   1 5 10 15
   Arg Lys Asn Ile His Ser Leu Ser His His Glu Ala Glu Glu Leu Arg
   20 25 30
   Asp Ala Leu Tyr Lys Leu Gln Asn Asp Glu Ser His Gly Gly Tyr Glu
   35 40 45
   His Ile Ala Gly Phe His Gly Tyr Pro Asn Leu Cys Pro Glu Lys Gly
   50 55 60
   Asp Glu Lys Tyr Pro Cys Cys Val His Gly Met Ser Ile Phe Pro His
   65 70 75 80
   Trp His Arg Leu His Thr Ile Gln Leu Glu Arg Ala Leu Lys Lys His
   85 90 95
   Gly Ser
<210> 74
   <211> 314
   <212> PRT
   <213> Megathura crenulata
<400> 74
   Gly Leu Pro Tyr Trp Asp Trp Thr Met Pro Met Ser His Leu Pro Glu
   1 5 10 15
   Leu Ala Thr Ser Glu Thr Tyr Leu Asp Pro Val Thr Gly Glu Thr Lys
   20 25 30
   Asn Asn Pro Phe His His Ala Gln Val Ala Phe Glu Asn Gly Val Thr
   35 40 45
   Ser Arg Asn Pro Asp Ala Lys Leu Phe Met Lys Pro Thr Tyr Gly Asp
   50 55 60
   His Thr Tyr Leu Phe Asp Ser Met Ile Tyr Ala Phe Glu Gln Glu Asp
   65 70 75 80
   Phe Cys Asp Phe Glu Val Gln Tyr Glu Leu Thr His Asn Ala Ile His
   85 90 95
   Ala Trp Val Gly Gly Ser Glu Lys Tyr Ser Met Ser Ser Leu His Tyr
   100 105 110
   Thr Ala Phe Asp Pro Ile Phe Tyr Leu His His Ser Asn Val Asp Arg
   115 120 125
   Leu Trp Ala Ile Trp Gln Ala Leu Gln Ile Arg Arg Gly Lys Ser Tyr
   130 135 140
   Lys Ala His Cys Ala Ser Ser Gln Glu Arg Glu Pro Leu Lys Pro Phe
   145 150 155 160
   Ala Phe Ser Ser Pro Leu Asn Asn Asn Glu Lys Thr Tyr His Asn Ser
   165 170 175
   Val Pro Thr Asn Val Tyr Asp Tyr Val Gly Val Leu His Tyr Arg Tyr
   180 185 190
   Asp Asp Leu Gln Phe Gly Gly Met Thr Met Ser Glu Leu Glu Glu Tyr
           195                 200                 205
   Ile His Lys Gln Thr Gln His Asp Arg Thr Phe Ala Gly Phe Phe Leu
       210                 215                 220
   Ser Tyr Ile Gly Thr Ser Ala Ser Val Asp Ile Phe Ile Asn Arg Glu
   225                 230                 235                 240
   Gly His Asp Lys Tyr Lys Val Gly Ser Phe Val Val Leu Gly Gly Ser
                   245                 250                 255
   Lys Glu Met Lys Trp Gly Phe Asp Arg Met Tyr Lys Tyr Glu Ile Thr
               260                 265                 270
   Glu Ala Leu Lys Thr Leu Asn Val Ala Val Asp Asp Gly Phe Ser Ile
           275                 280                 285
   Thr Val Glu Ile Thr Asp Val Asp Gly Ser Pro Pro Ser Ala Asp Leu
       290                 295                 300
   Ile Pro Pro Pro Ala Ile Ile Phe Glu Arg
   305                 310
<210> 75
   <211> 416
   <212> PRT
   <213> Megathura crenulata
<400> 75
   Ala Asp Ala Lys Asp Phe Gly His Ser Arg Lys Ile Arg Lys Ala Val
     1               5                  10                  15
   Asp Ser Leu Thr Val Glu Glu Gln Thr Ser Leu Arg Arg Ala Met Ala
               20                  25                  30
   Asp Leu Gln Asp Asp Lys Thr Ser Gly Gly Phe Gln Gln Ile Ala Ala
           35                  40                  45
   Phe His Gly Glu Pro Lys Trp Cys Pro Ser Pro Glu Ala Glu Lys Lys
       50                  55                  60
   Phe Ala Cys Cys Val His Gly Met Ala Val Phe Pro His Trp His Arg
   65                  70                  75                  80
   Leu Leu Thr Val Gln Gly Glu Asn Ala Leu Arg Lys His Gly Phe Thr
                   85                  90                  95
   Gly Gly Leu Pro Tyr Trp Asp Trp Thr Arg Ser Met Ser Ala Leu Pro
               100                 105                 110
   His Phe Val Ala Asp Pro Thr Tyr Asn Asp Ala Ile Ser Ser Gln Glu
           115                 120                 125
   Glu Asp Asn Pro Trp His His Gly His Ile Asp Ser Val Gly His Asp
       130                 135                 140
   Thr Thr Arg Asp Val Arg Asp Asp Leu Tyr Gln Ser Pro Gly Phe Gly
   145 150 155 160
   His Tyr Thr Asp Ile Ala Lys Gln Val Leu Leu Ala Phe Glu Gln Asp
   165 170 175
   Asp Phe Cys Asp Phe Glu Val Gln Phe Glu Ile Ala His Asn Phe Ile
   180 185 190
   His Ala Leu Val Gly Gly Asn Glu Pro Tyr Ser Met Ser Ser Leu Arg
   195 200 205
   Tyr Thr Thr Tyr Asp Pro Ile Phe Phe Leu His Arg Ser Asn Thr Asp
   210 215 220
   Arg Leu Trp Ala Ile Trp Gln Ala Leu Gln Lys Tyr Arg Gly Lys Pro
   225 230 235 240
   Tyr Asn Thr Ala Asn Cys Ala Ile Ala Ser Met Arg Lys Pro Leu Gln
   245 250 255
   Pro Phe Gly Leu Asp Ser Val Ile Asn Pro Asp Asp Glu Thr Arg Glu
   260 265 270
   His Ser Val Pro Phe Arg Val Phe Asp Tyr Lys Asn Asn Phe Asp Tyr
   275 280 285
   Glu Tyr Glu Ser Leu Ala Phe Asn Gly Leu Ser Ile Ala Gln Leu Asp
   290 295 300
   Arg Glu Leu Gln Arg Arg Lys Ser His Asp Arg Val Phe Ala Gly Phe
   305 310 315 320
   Leu Leu His Glu Ile Gly Gln Ser Ala Leu Val Lys Phe Tyr Val Cys
   325 330 335
   Lys His Asn Val Ser Asp Cys Asp His Tyr Ala Gly Glu Phe Tyr Ile
   340 345 350
   Leu Gly Asp Glu Ala Glu Met Pro Trp Arg Tyr Asp Arg Val Tyr Lys
   355 360 365
   Tyr Glu Ile Thr Gln Gln Leu His Asp Leu Asp Leu His Val Gly Asp
   370 375 380
   Asn Phe Phe Leu Lys Tyr Glu Ala Phe Asp Leu Asn Gly Gly Ser Leu
   385 390 395 400
   Gly Gly Ser Ile Phe Ser Gln Pro Ser Val Ile Phe Glu Pro Ala Ala
   405 410 415
<210> 76
   <211> 419
   <212> PRT
   <213> Megathura crenulata
<400> 76
   Gly Ser His Gln Ala Asp Glu Tyr Arg Glu Ala Val Thr Ser Ala Ser
    1               5                  10                  15
   His Ile Arg Lys Asn Ile Arg Asp Leu Ser Glu Gly Glu Ile Glu Ser
               20                  25                  30
   Ile Arg Ser Ala Phe Leu Gln Ile Gln Lys Glu Gly Ile Tyr Glu Asn
           35                  40                  45
   Ile Ala Lys Phe His Gly Lys Pro Gly Leu Cys Glu His Asp Gly His
       50                  55                  60
   Pro Val Ala Cys Cys Val His Gly Met Pro Thr Phe Pro His Trp His
   65                  70                  75                  80
   Arg Leu Tyr Val Leu Gln Val Glu Asn Ala Leu Leu Glu Arg Gly Ser
                   85                  90                  95
   Ala Val Ala Val Pro Tyr Trp Asp Trp Thr Glu Lys Ala Asp Ser Leu
               100                 105                 110
   Pro Ser Leu Ile Asn Asp Ala Thr Tyr Phe Asn Ser Arg Ser Gln Thr
           115                 120                 125
   Phe Asp Pro Asn Pro Phe Phe Arg Gly His Ile Ala Phe Glu Asn Ala
       130                 135                 140
   Val Thr Ser Arg Asp Pro Gln Pro Glu Leu Trp Asp Asn Lys Asp Phe
   145                 150                 155                 160
   Tyr Glu Asn Val Met Leu Ala Leu Glu Gln Asp Asn Phe Cys Asp Phe
                   165                 170                 175
   Glu Ile Gln Leu Glu Leu Ile His Asn Ala Leu His Ser Arg Leu Gly
               180                 185                 190
   Gly Arg Ala Lys Tyr Ser Leu Ser Ser Leu Asp Tyr Thr Ala Phe Asp
           195                 200                 205
   Pro Val Phe Phe Leu His His Ala Asn Val Asp Arg Ile Trp Ala Ile
       210                 215                 220
   Trp Gln Asp Leu Gln Arg Tyr Arg Lys Lys Pro Tyr Asn Glu Ala Asp
   225                 230                 235                 240
   Cys Ala Val Asn Glu Met Arg Lys Pro Leu Gln Pro Phe Asn Asn Pro
                   245                 250                 255
   Glu Leu Asn Ser Asp Ser Met Thr Leu Lys His Asn Leu Pro Gln Asp
               260                 265                 270
   Ser Phe Asp Tyr Gln Asn Arg Phe Arg Tyr Gln Tyr Asp Asn Leu Gln
           275                 280                 285
   Phe Asn His Phe Ser Ile Gln Lys Leu Asp Gln Thr Ile Gln Ala Arg
   290 295 300
   Lys Gln His Asp Arg Val Phe Ala Gly Phe Ile Leu His Asn Ile Gly
   305 310 315 320
   Thr Ser Ala Val Val Asp Ile Tyr Ile Cys Val Glu Gln Gly Gly Glu
   325 330 335
   Gln Asn Cys Lys Thr Lys Ala Gly Ser Phe Thr Ile Leu Gly Gly Glu
   340 345 350
   Thr Glu Met Pro Phe His Phe Asp Arg Leu Tyr Lys Phe Asp Ile Thr
   355 360 365
   Ser Ala Leu His Lys Leu Gly Val Pro Leu Asp Gly His Gly Phe Asp
   370 375 380
   Ile Lys Val Asp Val Arg Ala Val Asn Gly Ser His Leu Asp Gln His
   385 390 395 400
   Ile Leu Asn Glu Pro Ser Leu Leu Phe Val Pro Gly Glu Arg Lys Asn
   405 410 415
   Ile Tyr Tyr
<210> 77
   <211> 413
   <212> PRT
   <213> Megathura crenulata
<400> 77
   Asp Gly Leu Ser Gln His Asn Leu Val Arg Lys Glu Val Ser Ser Leu
   1 5 10 15
   Thr Thr Leu Glu Lys His Phe Leu Arg Lys Ala Leu Lys Asn Met Gln
   20 25 30
   Ala Asp Asp Ser Pro Asp Gly Tyr Gln Ala Ile Ala Ser Phe His Ala
   35 40 45
   Leu Pro Pro Leu Cys Pro Ser Pro Ser Ala Ala His Arg His Ala Cys
   50 55 60
   Cys Leu His Gly Met Ala Thr Phe Pro Gln Trp His Arg Leu Tyr Thr
   65 70 75 80
   Val Gln Phe Glu Asp Ser Leu Lys Arg His Gly Ser Ile Val Gly Leu
   85 90 95
   Pro Tyr Trp Asp Trp Leu Lys Pro Gln Ser Ala Leu Pro Asp Leu Val
   100 105 110
   Thr Gln Glu Thr Tyr Glu His Leu Phe Ser His Lys Thr Phe Pro Asn
   115 120 125
   Pro Phe Leu Lys Ala Asn Ile Glu Phe Glu Gly Glu Gly Val Thr Thr
       130                 135                 140
   Glu Arg Asp Val Asp Ala Glu His Leu Phe Ala Lys Gly Asn Leu Val
   145                 150                 155                 160
   Tyr Asn Asn Trp Phe Cys Asn Gln Ala Leu Tyr Ala Leu Glu Gln Glu
                   165                 170                 175
   Asn Tyr Cys Asp Phe Glu Ile Gln Phe Glu Ile Leu His Asn Gly Ile
               180                 185                 190
   His Ser Trp Val Gly Gly Ser Lys Thr His Ser Ile Gly His Leu His
           195                 200                 205
   Tyr Ala Ser Tyr Asp Pro Leu Phe Tyr Ile His His Ser Gln Thr Asp
       210                 215                 220
   Arg Ile Trp Ala Ile Trp Gln Ala Leu Gln Glu His Arg Gly Leu Ser
   225                 230                 235                 240
   Gly Lys Glu Ala His Cys Ala Leu Glu Gln Met Lys Asp Pro Leu Lys
                   245                 250                 255
   Pro Phe Ser Phe Gly Ser Pro Tyr Asn Leu Asn Lys Arg Thr Gln Glu
               260                 265                 270
   Phe Ser Lys Pro Glu Asp Thr Phe Asp Tyr His Arg Phe Gly Tyr Glu
           275                 280                 285
   Tyr Asp Ser Leu Glu Phe Val Gly Met Ser Val Ser Ser Leu His Asn
       290                 295                 300
   Tyr Ile Lys Gln Gln Gln Glu Ala Asp Arg Val Phe Ala Gly Phe Leu
   305                 310                 315                 320
   Leu Lys Gly Phe Gly Gln Ser Ala Ser Val Ser Phe Asp Ile Cys Arg
                   325                 330                 335
   Pro Asp Gln Ser Cys Gln Glu Ala Gly Tyr Phe Ser Val Leu Gly Gly
               340                 345                 350
   Ser Ser Glu Met Pro Trp Gln Phe Asp Arg Leu Tyr Lys Tyr Asp Ile
           355                 360                 365
   Thr Lys Thr Leu Lys Asp Met Lys Leu Arg Tyr Asp Asp Thr Phe Thr
       370                 375                 380
   Ile Lys Val His Ile Lys Asp Ile Ala Gly Ala Glu Leu Asp Ser Asp
   385                 390                 395                 400
   Leu Ile Pro Thr Pro Ser Val Leu Leu Glu Glu Gly Lys
                   405                 410
<210> 78
   <211> 417
   <212> PRT
   <213> Megathura crenulata
<400> 78
   His Gly Ile Asn Val Arg His Val Gly Arg Asn Arg Ile Arg Met Glu
   1               5                   10                  15
   Leu Ser Glu Leu Thr Glu Arg Asp Leu Ala Ser Leu Lys Ser Ala Met
               20                  25                  30
   Arg Ser Leu Gln Ala Asp Asp Gly Val Asn Gly Tyr Gln Ala Ile Ala
           35                  40                  45
   Ser Phe His Gly Leu Pro Ala Ser Cys His Asp Asp Glu Gly His Glu
       50                  55                  60
   Ile Ala Cys Cys Ile His Gly Met Pro Val Phe Pro His Trp His Arg
   65                  70                  75                  80
   Leu Tyr Thr Leu Gln Met Asp Met Ala Leu Leu Ser His Gly Ser Ala
                   85                  90                  95
   Val Ala Ile Pro Tyr Trp Asp Trp Thr Lys Pro Ile Ser Lys Leu Pro
               100                 105                 110
   Asp Leu Phe Thr Ser Pro Glu Tyr Tyr Asp Pro Trp Arg Asp Ala Val
           115                 120                 125
   Val Asn Asn Pro Phe Ala Lys Gly Tyr Ile Lys Ser Glu Asp Ala Tyr
       130                 135                 140
   Thr Val Arg Asp Pro Gln Asp Ile Leu Tyr His Leu Gln Asp Glu Thr
   145                 150                 155                 160
   Gly Thr Ser Val Leu Leu Asp Gln Thr Leu Leu Ala Leu Glu Gln Thr
                   165                 170                 175
   Asp Phe Cys Asp Phe Glu Val Gln Phe Glu Val Val His Asn Ala Ile
               180                 185                 190
   His Tyr Leu Val Gly Gly Arg Gln Val Tyr Ala Leu Ser Ser Gln His
           195                 200                 205
   Tyr Ala Ser Tyr Asp Pro Ala Phe Phe Ile His His Ser Phe Val Asp
       210                 215                 220
   Lys Ile Trp Ala Val Trp Gln Ala Leu Gln Lys Lys Arg Lys Arg Pro
   225                 230                 235                 240
   Tyr His Lys Ala Asp Cys Ala Leu Asn Met Met Thr Lys Pro Met Arg
                   245                 250                 255
   Pro Phe Ala His Asp Phe Asn His Asn Gly Phe Thr Lys Met His Ala
               260                 265                 270
   Val Pro Asn Thr Leu Phe Asp Phe Gln Asp Leu Phe Tyr Thr Tyr Asp
           275                 280                 285
   Asn Leu Glu Ile Ala Gly Met Asn Val Asn Gln Leu Glu Ala Glu Ile
       290                 295                 300
   Asn Arg Arg Lys Ser Gln Thr Arg Val Phe Ala Gly Phe Leu Leu His
   305                 310                 315                 320
   Gly Ile Gly Arg Ser Ala Asp Val Arg Phe Trp Ile Cys Lys Thr Ala
                   325                 330                 335
   Asp Asp Cys His Ala Ser Gly Met Ile Phe Ile Leu Gly Gly Ser Lys
               340                 345                 350
   Glu Met His Trp Ala Tyr Asp Arg Asn Phe Lys Tyr Asp Ile Thr Gln
           355                 360                 365
   Ala Leu Lys Ala Gln Ser Ile His Pro Glu Asp Val Phe Asp Thr Asp
       370                 375                 380
   Ala Pro Phe Phe Ile Lys Val Glu Val His Gly Val Asn Lys Thr Ala
   385                 390                 395                 400
   Leu Pro Ser Ser Ala Ile Pro Ala Pro Thr Ile Ile Tyr Ser Ala Gly
                   405                 410                 415
   Glu
<210> 79
   <211> 395
   <212> PRT
   <213> Megathura crenulata
<400> 79
   Asp His Ile Ala Gly Ser Gly Val Arg Lys Asp Val Thr Ser Leu Thr
   1               5                   10                  15
   Ala Ser Glu Ile Glu Asn Leu Arg His Ala Leu Gln Ser Val Met Asp
               20                  25                  30
   Asp Asp Gly Pro Asn Gly Phe Gln Ala Ile Ala Ala Tyr His Gly Ser
           35                  40                  45
   Pro Pro Met Cys His Met Xaa Asp Gly Arg Asp Val Ala Cys Cys Thr
       50                  55                  60
   His Gly Met Ala Ser Phe Pro His Trp His Arg Leu Phe Val Lys Gln
   65                  70                  75                  80
   Met Glu Asp Ala Leu Ala Ala His Gly Ala His Ile Gly Ile Pro Tyr
                   85                  90                  95
   Trp Asp Trp Thr Ser Ala Phe Ser His Leu Pro Ala Leu Val Thr Asp
               100                 105                 110
   His Glu His Asn Pro Phe His His Gly His Ile Ala His Arg Asn Val
   115 120 125
   Asp Thr Ser Arg Ser Pro Arg Asp Met Leu Phe Asn Asp Pro Glu His
   130 135 140
   Gly Ser Glu Ser Phe Phe Tyr Arg Gln Val Leu Leu Ala Leu Glu Gln
   145 150 155 160
   Thr Asp Phe Cys Gln Phe Glu Val Gln Phe Glu Ile Thr His Asn Ala
   165 170 175
   Ile His Ser Trp Thr Gly Gly His Thr Pro Tyr Gly Met Ser Ser Leu
   180 185 190
   Glu Tyr Thr Ala Tyr Asp Pro Leu Phe Tyr Leu His His Ser Asn Thr
   195 200 205
   Asp Arg Ile Trp Ala Ile Trp Gln Ala Leu Gln Lys Tyr Arg Gly Phe
   210 215 220
   Gln Tyr Asn Ala Ala His Cys Asp Ile Gln Val Leu Lys Gln Pro Leu
   225 230 235 240
   Lys Pro Phe Ser Glu Ser Arg Asn Pro Asn Pro Val Thr Arg Ala Asn
   245 250 255
   Ser Arg Ala Val Asp Ser Phe Asp Tyr Glu Arg Leu Asn Tyr Gln Tyr
   260 265 270
   Asp Thr Leu Thr Phe His Gly His Ser Ile Ser Glu Leu Asp Ala Met
   275 280 285
   Leu Gln Glu Arg Lys Lys Glu Glu Arg Thr Phe Ala Ala Phe Leu Leu
   290 295 300
   His Gly Phe Gly Ala Ser Ala Asp Val Ser Phe Asp Val Cys Thr Pro
   305 310 315 320
   Asp Gly His Cys Ala Phe Ala Gly Thr Phe Ala Val Leu Gly Gly Glu
   325 330 335
   Leu Glu Met Pro Trp Ser Phe Glu Arg Leu Phe Arg Tyr Asp Ile Thr
   340 345 350
   Lys Val Leu Lys Gln Met Asn Leu His Tyr Asp Ser Glu Phe His Phe
   355 360 365
   Glu Leu Lys Ile Val Gly Thr Asp Gly Thr Glu Leu Pro Ser Asp Arg
   370 375 380
   Ile Lys Ser Pro Thr Ile Glu His His Gly Gly
   385 390 395
<210> 80
   <211> 1266
   <212> DNA
   <213> Haliotis tuberculata
<400> 80
   cttgttcagt ttctactcgt cgcccttgtg gtgggggctg gagcagacaa cgtcgtcaga 60
   aaggacgtga gtcacctcac ggatgacgag gtgcaagctc tccacggcgc cctccatgac 120
   gtcactgcat ctacagggcc tctgagtttc gaagacataa catcttacca tgccgcacca 180
   gcgtcgtgtg actacaaggg acggaagatc gcctgctgtg tccacggtat gcccagtttc 240
   cccttctggc acagggcata tgtcgtccaa gccgagcggg cactgttgtc caaacggaag 300
   actgtcggaa tgccttactg ggactggacg caaacgctga ctcacttacc atctcttgtg 360
   actgaaccca tctacattga cagtaaaggt ggaaaggctc aaaccaacta ctggtaccgc 420
   ggcgagatag cgttcatcaa taagaagact gcgcgagctg tagatgatcg cctattcgag 480
   aaggtggagc ctggtcacta cacacatctt atggagactg tcctcgacgc tctcgaacag 540
   gacgaattct gtaaatttga aatccagttc gagttggctc ataatgctat ccattacttg 600
   gttggcggta aatttgaata ttcaatgtca aacttggaat acacctccta cgaccccatc 660
   ttcttcctcc accactccaa cgttgaccgc ctcttcgcca tctggcagcg tcttcaggaa 720
   ctgcgaggaa agaatcccaa tgcaatggac tgtgcacatg aactcgctca ccagcaactc 780
   caacccttca acagggacag caatccagtc cagctcacaa aggaccactc gacacctgct 840
   gacctctttg attacaaaca acttggatac agctacgaca gcttaaacct gaatggaatg 900
   acgccagaac agctgaaaac agaactagac gaacgccact ccaaagaacg tgcgtttgca 960
   agcttccgac tcagtggctt tgggggttct gccaacgttg ttgtctatgc atgtgtccct 1020
   gatgatgatc cacgcagtga tgactactgc gagaaagcag gcgacttctt cattcttggg 1080
   ggtcaaagcg aaatgccgtg gagattctac agacccttct tctatgatgt aactgaagcg 1140
   gtacatcacc ttggagtccc gctaagtggc cactactatg tgaaaacaga actcttcagc 1200
   gtgaatggca cagcactttc acctgatctt cttcctcaac caactgttgc ctaccgacct 1260
   gggaaa                                                            1266
<210> 81
   <211> 1257
   <212> DNA
   <213> Haliotis tuberculata
<400> 81
   ggtcaccttg acccacctgt gcatcatcgc cacgatgacg atcttattgt tcgaaaaaat 60
   atagatcatt tgactcgtga agaggaatac gagctaagga tggctctgga gagattccag 120
   gccgacacat ccgttgatgg gtaccaggct acagtagagt accatggcct tcctgctcgt 180
   tgtccacgac cagatgcaaa agtcaggttc gcctgttgta tgcatggcat ggcatccttc 240
   cctcactggc accggctgtt cgttacccag gtggaagatg ctcttgtacg gcgtggatcg 300
   cctatcggtg ttccttattg ggactggaca aaacctatga ctcaccttcc agacttggca 360
   tcaaatgaga cgtacgtaga cccgtatgga catacacatc ataatccatt cttcaatgca 420
   aatatatctt ttgaggaggg acaccatcac acgagcagga tgatagattc gaaactgttt 480
   gccccagtcg cttttgggga gcattcccat ctgtttgatg gaatcctgta cgcatttgag 540
   caggaagatt tctgcgactt tgagattcag tttgagttag tccataattc tattcatgcg 600
   tggataggcg gttccgaaga ttactccatg gccaccctgc attacacagc ctttgacccc 660
   attttctacc ttcatcattc caatgtcgat cgtctatggg caatctggca agctcttcaa 720
   atcaggagac acaagccata tcaagcccac tgtgcacagt ctgtggaaca gttgccaatg 780
   aagccatttg ctttcccatc acctcttaac aacaacgaga agacacatag tcattcagtc 840
   ccgactgaca tttatgacta cgaggaagtg ctgcactaca gctacgatga tctaacgttt 900
   ggtgggatga accttgaaga aatagaagaa gctatacatc tcagacaaca gcatgaacga 960
   gtcttcgcgg gatttctcct tgctggaata ggaacatctg cacttgttga cattttcata 1020
   aataaaccgg ggaaccaacc actcaaagct ggagatattg ccattcttgg tggtgccaag 1080
   gaaatgcctt gggcgtttga ccgcttgtat aaggtcgaaa taactgactc attgaagaca 1140
   ctttctctcg atgtcgatgg agattatgaa gtcactttta aaattcatga tatgcacgga 1200
   aacgctcttg atacggacct gattccacac gcagcagttg tttctgagcc agctcac    1257
<210> 82
   <211> 1242
   <212> DNA
   <213> Haliotis tuberculata
<400> 82
   cctacctttg aggatgaaaa gcacagctta cgaatcagaa aaaatgtcga cagcttgact 60
   cctgaagaaa caaatgaact gcgtaaagcc ctggagcttc ttgaaaatga tcatactgca 120
   ggtggattca atcagcttgg cgccttccat ggagagccta aatggtgccc taatcctgaa 180
   gcggagcaca aggttgcatg ctgtgttcat ggcatggctg ttttccctca ttggcacagg 240
   cttcttgctc tccaggcgga gaatgctctt agaaagcatg ggtacagtgg tgctctacca 300
   tactgggatt ggactcgccc cctttcccaa cttcctgatc tggttagtca tgagcagtat 360
   acagatcctt ccgaccatca cgtgaagcat aacccgtggt tcaatggcca catcgataca 420
   gtaaatcagg ataccaccag aagcgtacgg gaggatcttt atcaacaacc tgaatttgga 480
   catttcacgg atattgctca acaagtcctc ttagcattag aacaagatga cttctgttcg 540
   tttgaagtgc agtatgagat ttcccataat tttatccatg cacttgtagg aggaaccgac 600
   gcttatggca tggcatcgct gagatataca gcatacgatc caatcttttt cttgcatcat 660
   tcaaacaccg acaggatctg ggctatttgg caatccctgc aaaaatacag aggcaaaccg 720
   tacaacactg ccaactgcgc catagaatct atgagaaggc ccctgcaacc atttggacta 780
   agcagtgcca ttaaccctga cagaatcacc agagagcatg ctatcccgtt tgatgtcttc 840
   aactatagag ataaccttca ttacgtatat gataccctgg aatttaatgg tttgtcgatt 900
   tcacaacttg atagagagct ggaaaaaatc aagagtcacg aaagagtatt tgctggattc 960
   ttgctgtcgg ggattaaaaa atctgctctt gtgaaattcg aagtttgtac tccacctgat 1020
   aattgtcata aagcagggga gttttatcta ctcggggacg aaaacgagat ggcttgggcc 1080
   tatgaccgac ttttcaagta tgatattact caggttctgg aagcaaacca tctacacttc 1140
   tatgatcatc tcttcattcg ctacgaagtc tttgatctta aaggagtgag tttgggaact 1200
   gacctgttcc acactgcaaa tgtggtacat gattccggca ca                    1242
<210> 83
   <211> 1239
   <212> DNA
   <213> Haliotis tuberculata
<400> 83
   ggcacccgtg atcgtgataa ctacgttgaa gaagttactg gggccagtca tatcaggaag 60
   aatttgaacg acctcaatac cggagaaatg gaaagcctta gagctgcttt cctgcatatt 120
   caggacgacg gaacatatga atctattgcc cagtaccatg gcaaaccagg caaatgtcaa 180
   ttgaatgatc ataatattgc gtgttgtgtc catggtatgc ctaccttccc ccagtggcac 240
   agactgtatg tggttcaggt ggagaatgct ctcctaaaca ggggatctgg tgtggctgtt 300
   ccttactggg agtggactgc tcccatagac catctacctc atttcattga tgatgcaaca 360
   tacttcaatt cccgacaaca gcggtacgac cctaaccctt tcttcagggg aaaggttact 420
   tttgaaaacg cagtcacaac aagggaccca caagccgggc tcttcaactc agattatatg 480
   tatgagaatg ttttacttgc actggagcag gaaaattatt gtgactttga aattcagttt 540
   gagcttgttc ataacgcact tcattccatg ctgggaggta aagggcagta ctccatgtcc 600
   tccctggact attctgcgtt tgatcccgtc ttcttcctac atcatgccaa cacggacaga 660
   ctgtgggcaa tctggcagga actacaaaga ttccgagaac tgccttatga agaagcgaac 720
   tgtgcaatca acctcatgca tcaaccactg aagccgttca gtgatccaca tgagaatcac 780
   gacaatgtca ctttgaaata ctcaaaacca caggacggat tcgactacca gaaccacttc 840
   ggatacaagt atgacaacct tgagttccat cacttatcta tcccaagtct tgatgctacc 900
   ctgaagcaaa ggagaaatca cgacagagtg tttgcgggct tccttcttca taacatagga 960
   acttctgctg acataactat ctacatatgt ctgcctgacg gacggcgtgg caatgactgc 1020
   agtcatgagg cgggaacatt ctatatcctc ggaggcgaaa cagagatgcc ttttatcttt 1080
   gaccgtttgt ataaatttga aatcaccaaa ccactgcaac agttaggagt caagctgcat 1140
   ggtggagttt tcgaactgga gcttgagatc aaggcataca acggttccta tctggatccc 1200
   catacctttg atccaactat catctttgaa cctggaaca                       1239
<210> 84
   <211> 1260
   <212> DNA
   <213> Haliotis tuberculata
<400> 84
   gatacccata tcttggacca cgaccatgag gaagagatac ttgtcaggaa gaatataatt 60
   gatttgagcc caagggagag ggtttctcta gtcaaagctt tgcaaagaat gaagaatgat 120
   cgctccgctg atgggtacca agccattgcc tctttccatg ccctgccacc actctgtccc 180
   aatccatctg cagctcaccg ttatgcttgc tgtgtccatg gcatggctac atttccccag 240
   tggcacagac tgtacactgt tcaggttcag gatgccctga ggagacatgg ttcacttgtt 300
   ggtattcctt actgggactg gacaaaacca gtcaacgagt tacccgagct tctttcttca 360
   gcaacatttt atcatccaat ccggaatatt aatatttcaa atccattcct cggggctgac 420
   atagaatttg aaggaccggg cgttcataca gagaggcaca taaatactga gcgcctgttt 480
   cacagtgggg atcatgacgg ataccacaac tggttcttcg aaactgttct ctttgctttg 540
   gaacaggaag attactgcga ttttgaaata caatttgaga tagcccataa tggcatccac 600
   acatggattg gtggaagcgc agtatatggc atgggacacc ttcactatgc atcatatgat 660
   ccaatttctt acatccacca ttcacagacg gacagaatat gggctatttg gcaagagctg 720
   cagaagtaca ggggtctatc tggttcggaa gcaaactgtg ccattgaaca tatgagaaca 780
   cccttgaagc ctttcagctt tgggccaccc tacaatttga atagtcatac gcaagaatat 840
   tcaaagcctg aggacacgtt tgactataag aagtttggat acagatatga tagtctggaa 900
   ttggaggggc gatcaatttc tcgcattgat gaacttatcc agcagagaca ggagaaagac 960
   agaacttttg cagggttcct ccttaaaggt tttggtacat ccgcatctgt gtcattgcaa 1020
   gtttgcagag ttgatcacac ctgtaaagat gcgggctatt tcactattct gggaggatca 1080
   gccgaaatgc catgggcatt cgacaggctt tataagtatg acattactaa aactcttcac 1140
   gacatgaacc tgaggcacga ggacactttc tctatagacg taactatcac gtcttacaat 1200
   ggaacagtac tctcgggaga cctcattcag acgccctcca ttatatttgt acctggacgc 1260
<210> 85
   <211> 1251
   <212> DNA
   <213> Haliotis tuberculata
<400> 85
   cataaactca actcacggaa acatacacct aacagagtcc gccatgagct aagtagcctt 60
   agttcccgtg acatagcaag cttgaaggca gctttgacaa gccttcaaca tgataatggg 120
   actgatggtt atcaagctat tgctgccttc catggcgttc ctgcgcagtg ccacgagcca 180
   tctggacgtg agatcgcctg ttgcatccac ggcatggcga cgtttcctca ctggcaccgg 240
   ttgtacactc tgcagttgga gcaagcgctg cgcagacacg ggtccagtgt tgctgttcca 300
   tactgggact ggaccaagcc aatcaccgaa ctgccacaca ttctgacaga cggagaatat 360
   tatgacgttt ggcaaaatgc cgtcttggcc aatccgtttg caagaggtta tgtgaaaatt 420
   aaagatgcat ttacggtgag aaatgtccag gaaagtctgt tcaaaatgtc aagttttgga 480
   aagcactcgc ttctgtttga ccaggctttg ttggctcttg aacaaactga ctactgtgac 540
   ttcgaagttc agtttgaagt gatgcataac acgatccatt atctcgtagg agggcgtcaa 600
   acgtacgcct tctcctctct cgagtattcc tcatacgatc caatcttctt tattcaccac 660
   tcgtttgttg acaaaatatg ggctgtatgg caagaactgc aaagcaggag acatctacag 720
   tttagaacag ctgattgtgc tgtgggcctc atgggtcagg caatgaggcc tttcaacaag 780
   gatttcaacc acaactcgtt caccaagaag cacgcagtcc ctaatacagt atttgattat 840
   gaagatcttg gctataacta tgacaacctt gaaatcagtg gtttaaactt aaatgagatc 900
   gaggcgttaa tagcaaaacg caagtcacat gctagagtct ttgctgggtt cctgttgttt 960
   ggattaggaa cttcggctga tatacatctg gaaatttgca agacatcgga aaactgccat 1020
   gatgctggtg tgattttcat ccttggaggt tctgcagaga tgcattgggc atacaaccgc 1080
   ctctacaagt atgacattac agaagcattg caggaatttg acatcaaccc tgaagatgtt 1140
   ttccatgctg atgaaccatt tttcctgagg ctgtcggttg ttgctgtgaa tggaactgtc 1200
   attccatcgt ctcatcttca ccagccaacg ataatctatg aaccaggcga a          1251
<210> 86
   <211> 1209
   <212> DNA
   <213> Haliotis tuberculata
<400> 86
   gatcaccatg acgaccatca gtcgggaagc atagcaggat ccggggtccg caaggacgtg 60
   aacaccttga ctaaggctga gaccgacaac ctgagggagg cgctgtgggg tgtcatggca 120
   gaccacggtc ccaatggctt tcaagctatt gctgctttcc atggaaaacc agctttgtgt 180
   cccatgcctg atggccacaa ctactcatgt tgtactcacg gcatggctac cttcccacac 240
   tggcatcgcc tctacaccaa gcagatggag gatgcaatga gggcgcatgg gtctcatgtc 300
   ggcctgccct actgggactg gactgctgcc ttcacccacc tgccaacact ggtcaccgac 360
   acggacaaca accccttcca acatggacac attgattatc tcaatgtcag cacaactcga 420
   tctccccgag acatgctgtt caacgacccc gagcatggat cagagtcgtt cttctacaga 480
   caagtcctct tagctctgga acaaactgat ttctgcaaat tcgaagttca gtttgagata 540
   acccacaatg ccatccattc ctggacaggt ggccacagcc cctacggaat gtccactctc 600
   gacttcactg cctacgatcc tctcttctgg cttcaccact ccaacaccga cagaatctgg 660
   gctgtctggc aagctttgca agaatacaga ggacttccat acaaccatgc caattgtgag 720
   atccaggcaa tgaaaacgcc cctgaggcct ttcagtgacg atatcaacca caacccagtc 780
   acaaaggcta acgcgaagcc attagatgtg ttcgagtata atcggttgag cttccagtac 840
   gacaacctca tcttccatgg atacagtatt ccggaacttg atcgcgtgct tgaagaaaga 900
   aaggaggagg acagaatatt tgctgccttc cttctcagtg gaatcaagcg tagtgctgat 960
   gtagtgttcg acatatgcca gccagaacac gaatgtgtgt tcgcagggac ttttgcgatt 1020
   ttgggagggg agctagaaat gccctggtcc ttcgacagac tgttccgcta tgatatcacc 1080
   aaggtgatga agcagctaca cctgaggcat gactctgact ttaccttcag ggtgaagatt 1140
   gtcggcaccg acgaccacga gcttccttca gacagtgtca aagcaccaac tattgaattt 1200
   gaaccgggc                                                         1209
<210> 87
   <211> 1536
   <212> DNA
   <213> Haliotis tuberculata
<400> 87
   gtgcacagag gcggaaacca cgaagatgaa caccatgatg acagactcgc agatgtcctg 60
   atcaggaaag aagttgactt cctctccctg caagaggcca acgcaattaa ggatgcactg 120
   tacaagctcc agaatgacga cagtaaaggg ggctttgagg ccatagctgg ctatcacggg 180
   tatcctaata tgtgtccaga aagaggtacc gacaagtatc cctgctgtgt ccacggaatg 240
   cccgtgttcc cccactggca ccgcctgcat accattcaga tggagagagc tctgaaaaac 300
   catggctctc caatgggcat tccttactgg gattggacaa agaagatgtc gagtcttcca 360
   tctttctttg gagattccag caacaacaac cctttctaca aatattacat ccggggcgtg 420
   cagcacgaaa caaccaggga cattaatcag agactcttta atcaaaccaa gtttggtgaa 480
   tttgattacc tatattacct aactctgcaa gtcctggagg aaaactcgta ctgtgacttt 540
   gaagttcagt atgagatcct ccataacgcc gtccactcct ggcttggagg aactggaaag 600
   tattccatgt ctaccctgga gcattcggcc tttgaccctg tcttcatgat tcaccactcg 660
   agtttggata gaatctggat cctttggcag aagttgcaaa agataagaat gaagccttac 720
   tacgcattgg attgtgctgg cgacagactt atgaaagacc ccctgcatcc cttcaactac 780
   gaaaccgtta atgaagatga attcacccgc atcaactctt tcccaagcat actgtttgac 840
   cactacaggt tcaactatga atacgataac atgagaatca ggggtcagga catacatgaa 900
   cttgaagagg taattcagga attaagaaac aaagatcgca tatttgctgg ttttgttttg 960
   tcgggcttac ggatatcagc tacagtgaaa gtattcattc attcgaaaaa cgatacaagt 1020
   cacgaagaat atgcaggaga atttgcagtt ttgggaggtg agaaggagat gccgtgggca 1080
   tatgaaagaa tgctgaaatt ggacatctcc gatgctgtac acaagcttca cgtgaaagat 1140
   gaagacatcc gttttagagt ggttgttact gcctacaacg gtgacgttgt taccaccagg 1200
   ctgtctcagc cattcatcgt ccaccgtcca gcccatgtgg ctcacgacat cttggtaatc 1260
   ccagtaggtg cgggccatga ccttccgcct aaagtcgtag taaagagcgg caccaaagtc 1320
   gagtttacac caatagattc gtcggtgaac aaagcaatgg tggagctggg cagctatact 1380
   gctatggcta aatgcatcgt tccccctttc tcttaccacg gctttgaact ggacaaagtc 1440
   tacagcgtcg atcacggaga ctactacatt gctgcaggta cccacgcgtt gtgtgagcag 1500
   aacctcaggc tccacatcca cgtggaacac gagtag                            1536
<210> 88
   <211> 591
   <212> DNA
   <213> Haliotis tuberculata
<400> 88
   ggtcttccgt actgggactg gacgcagcat ctgactcaac tcccagatct ggtgtcagac 60
   cccttgtttg tcgacccgga aggaggaaag gcccatgaca acgcatggta tcgtggaaac 120
   atcaagtttg agaataagaa gactgcaaga gctgttgacg atcgcctttt cgagaaggtt 180
   ggaccaggag agaatacccg actctttgaa ggaattctcg atgctcttga acaggatgaa 240
   ttctgcaact tcgagatcca gtttgagttg gctcacaacg ctatccacta cctggttggc 300
   ggccgtcaca cgtactccat gtctcatctc gagtacacct cctacgaccc cctcttcttc 360
   ctccatcact ccaacccgga ccgcatcttc gccatctggg aacgtcttca ggtactcaga 420
   ggaaaggacc ccaacaccgc cgactgcgca cacaacctca tccatgagcc catggaaccg 480
   ttccgtcggc atgagcccat ggaaccgttc cgtcgggact cgaaccctct tgacctcacc 540
   agggaaaact ccaaaccaat tgacagcttt gattatgccc accttggcta c           591
<210> 89
   <211> 1245
   <212> DNA
   <213> Haliotis tuberculata
<400> 89
   gttacagagg ccccagctcc ctcctcggat gctcacctcg ccgtcaggaa ggatatcaac 60
   catctgacac gcgaggaggt gtacgagctg cgcagagcta tggagagatt ccaggccgac 120
   acatccgttg atgggtacca ggctacggtt gagtatcacg gcttacctgc tcgatgtcca 180
   ttccccgagg ccacaaatag gttcgcctgt tgcatccacg gcatggcgac attccctcat 240
   tggcacagac tgttcgtcac ccaggtggaa gatgctctga tcaggcgagg atcgcctata 300
   ggggtcccct actgggactg gactcagcct atggcgcatc tcccaggact tgcagacaac 360
   gccacctata gagatcccat cagcggggac agcagacaca accccttcca cgatgttgaa 420
   gttgcctttg aaaatggacg tacagaacgt cacccagata gtagattgtt tgaacaacct 480
   ttatttggca aacatacgcg tctcttcgac agtatagtct atgcttttga gcaggaggac 540
   ttctgcgatt ttgaagttca atttgagatg acccataata atattcacgc ctggattggt 600
   ggcggcgaga agtattccat gtcttctcta cactacacag ccttcgaccc tatcttctac 660
   cttcgtcact ccaacactga ccggctctgg gcaatttggc aagcgttgca gatacgaaga 720
   aacaggcctt acaaggctca ttgtgcttgg tctgaggaac gccagcctct caaacctttc 780
   gccttcagtt ccccactgaa caacaacgaa aaaacctacg aaaactcggt gcccaccaac 840
   gtttacgact acgaaggagt ccttggctat acttatgatg acctcaactt cgggggcatg 900
   gacctgggtc agcttgagga atacatccag aggcagagac agagagacag gacctttgct 960
   ggtttctttc tgtcacatat tggtacatca gcgaatgttg aaatcattat agaccatggg 1020
   actcttcata cctccgtggg cacgtttgct gttcttggcg gagagaagga gatgaaatgg 1080
   ggatttgacc gtttgtacaa atatgagatt acagatgaac tgaggcaact taatctccgt 1140
   gctgatgatg ttttcagcat ctctgttaaa gtaactgatg ttgatggcag tgagctgtcc 1200
   tctgaactca tcccatctgc tgctatcatc ttcgaacgaa gccat 1245
<210> 90
   <211> 1251
   <212> DNA
   <213> Haliotis assimilis
<400> 90
   attgaccatc aggacccgca tcatgacaca atcattagga aaaatgttga taatcttaca 60
   cccgaggaaa ttaattctct gaggcgggca atggcagacc ttcaatcaga caaaaccgcc 120
   ggtggattcc agcaaattgc tgcttttcac ggggaaccca aatggtgccc aagtcccgat 180
   gctgagaaga agttctcctg ctgtgtccat ggaatggctg tcttccctca ctggcacaga 240
   ctcctgaccg tgcaaggcga gaatgccctg agaaagcatg gatgtctcgg agctctcccc 300
   tactgggact ggactcggcc cctgtctcac ctacctgatt tggttttggt aagtagcaga 360
   actacaccga tgccatattc caccgtggaa gcccgaaacc cctggtacag cggccatatt 420
   gatacagttg gtgttgacac aacaagaagc gtccgtcaag aactgtatga agctcctgga 480
   tttggccatt atactggggt cgctaagcaa gtgcttctgg ctttggagca ggatgacttc 540
   tgtgattttg aagtccagtt tgagatagct cacaatttca ttcacgctct tgtcggcgga 600
   agcgagccat atggtatggc gtcactccgt tacactactt atgatccaat tttctacctc 660
   catcattcta acactgacag actctgggct atatggcagg ctctacaaaa gtacaggggc 720
   aaaccttaca attccgccaa ctgcgccatt gcttctatga gaaaacccct acaacccttt 780
   ggtctgactg atgagatcaa cccggatgat gagacaagac agcatgctgt tcctttcagt 840
   gtctttgatt acaagaacaa cttcaattat gaatatgaca cccttgactt caacggacta 900
   tcaatctccc agctggaccg tgaactgtca cggagaaagt ctcatgacag agtatttgcc 960
   ggatttttgc tgcatggtat tcagcagtct gcactagtta aattctttgt ctgcaaatca 1020
   gatgatgact gtgaccacta tgctggtgaa ttctacatcc ttggtgatga agctgaaatg 1080
   ccatggggct atgatcgtct ttacaaatat gagatcactg agcagctcaa tgccctggat 1140
   ctacacatcg gagatagatt cttcatcaga tacgaagcgt ttgatcttca tggtacaagt 1200
   cttggaagca acatcttccc caaaccttct gtcatacatg acgaaggggc a          1251
<210> 91
   <211> 1242
   <212> DNA
   <213> Haliotis tuberculata
<400> 91
   ggtcaccatc aggctgacga gtacgacgaa gttgtaactg ctgcaagcca catcagaaag 60
   aatttaaaag atctgtcaaa gggagaagta gagagcctaa ggtctgcctt cctgcaactt 120
   cagaacgacg gagtctatga gaatattgcc aagttccacg gcaagcctgg gttgtgtgat 180
   gataacggtc gcaaggttgc ctgttgtgtc catggaatgc ccaccttccc ccagtggcac 240
   aggctctatg tcctccaggt ggagaatgct ttgctggaga gaggatctgc cgtctctgtg 300
   ccatactggg actggactga aacatttaca gagctgccat ctttgattgc tgaggctacc 360
   tatttcaatt cccgtcaaca aacgtttgac cctaatcctt tcttcagagg taaaatcagt 420
   tttgagaatg ctgttacaac acgtgatccc cagcctgagc tgtacgttaa caggtactac 480
   taccaaaacg tcatgttggt ttttgaacag gacaactact gcgacttcga gatacagttt 540
   gagatggttc acaatgttct ccatgcttgg cttggtggaa gagctactta ttctatttct 600
   tctcttgatt attctgcatt cgaccctgtg tttttccttc accatgcgaa cacagataga 660
   ttgtgggcca tctggcagga gctgcagagg tacaggaaga agccatacaa tgaagcggat 720
   tgtgccatta acctaatgcg caaacctcta catcccttcg acaacagtga tctcaatcat 780
   gatcctgtaa cctttaaata ctcaaaaccc actgatggct ttgactacca gaacaacttt 840
   ggatacaagt atgacaacct tgagttcaat catttcagta ttcccaggct tgaagaaatc 900
   attcgtatta gacaacgtca agatcgtgtg tttgcaggat tcctccttca caacattggg 960
   acatccgcaa ctgttgagat attcgtctgt gtccctacca ccagcggtga gcaaaactgt 1020
   gaaaacaaag ccggaacatt tgccgtactc ggaggagaaa cagagatggc gtttcatttt 1080
   gacagactct acaggtttga catcagtgaa acactgaggg acctcggcat acagctggac 1140
   agccatgact ttgacctcag catcaagatt caaggagtaa atggatccta ccttgatcca 1200
   cacatcctgc cagagccatc cttgattttt gtgcctggtt ca                    1242
<210> 92
   <211> 1257
   <212> DNA
   <213> Haliotis tuberculata
<400> 92
   agttctttcc tgcgtcctga tgggcattca gatgacatcc ttgtgagaaa agaagtgaac 60
   agcctgacaa ccagggagac tgcatctctg atccatgctc tgaaaagtat gcaggaagac 120
   cattcacctg acgggttcca agccattgcc tctttccatg ctctgccacc actctgccct 180
   tcaccatctg cagctcaccg ttatgcttgc tgtgtccacg gcatggctac atttccccag 240
   tggcacagat tgtacactgt acagttccag gatgcactga ggagacatgg agctacggta 300
   ggtgtaccgt attgggattg gctgcgaccg cagtctcacc taccagagct tgtcaccatg 360
   gagacatacc atgatatttg gagtaacaga gatttcccca atcctttcta ccaagccaat 420
   attgagtttg aaggagaaaa cattacaaca gagagagaag tcattgcaga caaacttttt 480
   gtcaaaggtg gacacgtttt tgataaactg gttcttcaaa caagccatcc tagcgctgag 540
   caggaaaact actgtgactt tgagattcag tttgaaattc ttcacaacgg cgttcacacg 600
   tgggtcggag gcagtcgtac ctactctatc ggacatcttc attacgcatt ctacgaccct 660
   cttttctacc ttcaccattt ccagacagac cgtatttggg caatctggca agaactccag 720
   gaacagagag ggctctcggg tgatgaggct cactgtgctc tcgagcaaat gagagaacca 780
   ttgaagcctt tcagcttcgg cgctccttat aactggaatc agctcacaca ggatttctcc 840
   cgacccgagg acaccttcga ctacaggaag tttggttatg aatatgacaa tttagaattc 900
   ctgggaatgt cagttgctga actggatcaa tacattattg aacatcaaga aaatgataga 960
   gtattcgctg ggttcctgtt gagtggattc ggaggttccg catcagttaa tttccaggtt 1020
   tgtagagctg attccacatg tcaggatgct gggtacttca ccgttcttgg tggcagtgct 1080
   gagatggcgt gggcatttga caggctttac aaatatgaca ttactgaaac tctggagaaa 1140
   atgcaccttc gatatgatga tgacttcaca atctctgtca gtctgaccgc caacaacgga 1200
   actgtcctga gcagcagtct aatcccaaca ccgagtgtca tattccagcg gggacat    1257
<210> 93
   <211> 1248
   <212> DNA
   <213> Haliotis tuberculata
<400> 93
   cgtgacataa ataccaggag catgtcaccg aaccgtgttc gccgtgagct gagcgatctg 60
   tctgcgaggg acctgtctag tctcaagtct gctctgcgag acctacagga ggatgatggc 120
   cccaacggat accaggctct tgcagccttc catgggctac cagcaggctg ccatgatagc 180
   cggggaaatg agatcgcatg ttgcattcac gggatgccga ccttccccca gtggcacaga 240
   ctgtacaccc tgcagttgga gatggctctg aggagacatg gatcatctgt cgccatcccc 300
   tactgggact ggacaaagcc tatctccgaa ctcccctcgc tcttcaccag ccctgagtat 360
   tatgacccat ggcatgatgc tgtggtaaac aacccattct ccaaaggttt tgtcaaattt 420
   gcaaatacct acacagtaag agacccacag gagatgctgt tccagctttg tgaacatgga 480
   gagtcaatcc tctatgagca aactcttctt gctcttgagc aaaccgacta ctgtgatttt 540
   gaggtacagt ttgaggtcct ccataacgtg atccactacc ttgttggtgg acgtcagacc 600
   tacgcattgt cttctctgca ttatgcctcc tacgacccat tcttctttat acaccattcc 660
   tttgtggata agatgtgggt agtatggcaa gctcttcaaa agaggaggaa acttccatac 720
   aagcgagctg actgtgctgt caacctaatg actaaaccaa tgaggccatt tgactccgat 780
   atgaatcaga acccattcac aaagatgcac gcagttccca acacactcta tgactacgag 840
   acactgtact acagctacga taatctcgaa ataggtggca ggaatctcga ccagcttcag 900
   gctgaaattg acagaagcag aagccacgat cgcgtttttg ctggattctt gcttcgtgga 960
   atcggaactt ctgctgatgt caggttttgg atttgtagaa atgaaaatga ctgccacagg 1020
   ggtggaataa ttttcatctt aggtggagcc aaggaaatgc catggtcatt tgacagaaac 1080
   ttcaagtttg atatcaccca tgtactcgag aatgctggca ttagcccaga ggacgtgttt 1140
   gatgctgagg agccatttta tatcaaggtt gagatccatg ctgttaacaa gaccatgata 1200
   ccgtcgtctg tgatcccagc cccaactatc atctattctc ctggggaa             1248
<210> 94
   <211> 1206
   <212> DNA
   <213> Haliotis tuberculata
<400> 94
   ggtcgcgctg ctgacagtgc gcactctgcc aacattgctg gctctggggt gaggaaggac 60
   gtcacgaccc tcactgtgtc tgagaccgag aacctaagac aggctcttca aggtgtcatc 120
   gatgatactg gtcccaatgg ttaccaagca atagcatcct tccacggaag tcctccaatg 180
   tgcgagatga acggccgcaa ggttgcctgt tgtgctcacg gtatggcctc cttcccacac 240
   tggcacagac tgtatgtgaa gcagatggaa gatgccctgg ctgaccacgg gtcacatatc 300
   ggcatccctt actgggactg gacaactgcc ttcacagagt tacccgccct tgtcacagac 360
   tccgagaaca atcccttcca tgagggtcgc attgatcatc tcggtgtaac cacgtcacgt 420
   tcccccagag acatgctgtt taacgaccca gagcaaggat cagagtcgtt cttctataga 480
   caagtcctcc tggctttgga gcagactgac tactgccagt tcgaagtcca gtttgagctg 540
   acccacaacg ccattcactc ctggacaggt ggacgtagcc cttacggaat gtcgaccctc 600
   gagttcacag cctacgatcc tctcttctgg cttcaccact ccaacaccga cagaatctgg 660
   gctgtctggc aagcactgca gaaataccga ggactcccat acaacgaagc acactgtgaa 720
   atccaggttc tgaaacagcc cttgaggcca ttcaacgatg acatcaacca caatccaatc 780
   accaagacta atgccaggcc tatcgattca tttgattatg agaggtttaa ctatcagtat 840
   gacaccctta gcttccatgg taagagcatc cctgaactga atgacctgct cgaggaaaga 900
   aaaagagaag agagaacatt tgctgccttc cttcttcgtg gaatcggttg cagtgctgat 960
   gtcgtctttg acatctgccg gcccaatggt gactgtgtct ttgcaggaac ctttgctgtg 1020
   ctgggagggg agctagaaat gccttggtcc ttcgacagac tgttccgcta tgacatcacc 1080
   agagtcatga atcagctcca tctccagtat gattcagatt tcagtttcag ggtgaagctt 1140
   gttgccacca atggcactga gctttcatca gaccttctca agtcaccaac aattgaacat 1200
   gaactt                                                            1206
<210> 95
   <211> 1548
   <212> DNA
   <213> Haliotis tuberculata
<400> 95
   ggagcccaca gaggaccagt tgaagaaaca gaagtcactc gccaacatac tgacggcaat 60
   gcacactttc atcgtaagga agttgattcg ctgtccctgg atgaagcaaa caacttgaag 120
   aatgcccttt acaagctaca gaacgaccac agtctaacgg gatacgaagc aatctctggt 180
   taccatggat accccaatct gtgtccggaa gaaggcgatg acaaaatacc cctgctgcgt 240
   ccccggatgg gcatctttcc ttactggcac agactcttga ccattcaact ggaaagagct 300
   cttgagcaca atggtgcact gcttggtgtt ccttactggg actggaacaa ggacctgtcg 360
   tcactgccgg cgttcttctc cgactccagc aacaacaatc cctacttcaa gtaccacatc 420
   gccggtgttg gtcacgacac cgtcagagag ccaactagtc ttatatataa ccagccccaa 480
   atccatggtt atgattatct ctattaccta gcattgacca cgcttgaaga aaacaattac 540
   tgggactttg aggttcagta tgagatcctc cacaacgccg tccactcctg gcttggagga 600
   tcccagaagt attccatgtc taccctggag tattcggcct ttgaccctgt ctttatgatc 660
   cttcactcgg gtctagacag actttggatc atctggcaag aacttcagaa gatcaggaga 720
   aagccctaca acttcgctaa atgtgcttat catatgatgg aagagccact ggcgcccttc 780
   agctatccat ctatcaacca ggacgagttc acccgtgcca actccaagcc ttctacagtt 840
   tttgacagcc ataagttcgg ctaccattac gataacctga atgttagagg tcacagcatc 900
   caagaactca acacaatcat caatgacttg agaaacacag acagaatcta cgcaggattt 960
   gttttgtcag gcatcggtac gtctgctagt gtcaagatct atctccgaac agatgacaat 1020
   gacgaagaag ttggaacttt cactgtcctg ggaggagaga gggaaatgcc atgggcctac 1080
   gagcgagttt tcaagtatga catcacagag gttgcagata gacttaaaat taagttatgg 1140
   ggacaccctt taacttccgg aactggagat cacatcctta cgaatggaat cggtggtaaa 1200
   caagagccta cccaaatcct ttcatcatct acagacctgc caatcatgac tacgatgttc 1260
   ttgttatccc agtanggaag aaaccttcac atccctccca aagttgtcgt caagaaaggc 1320
   acccgcatcg agttccaccc agtcgatgat tcagttacga gaccagttgt tgatcttgga 1380
   agctacactg cactcttcaa ctgtgtggta ccaccgttca cataccacgg attcgaactg 1440
   aaccacgtct attctgtcaa gcctggtgac tactatgtta ctggacccac gagagacctt 1500
   tgccagaatg cagatgtcag gattcatatc catgttgagg atgagtaa 1548
<210> 96
   <211> 966
   <212> DNA
   <213> Megathura crenulata
<400> 96
   ggcctaccgt actgggactg gactgaaccc atgacacaca ttccgggtct ggcaggaaac 60
   aaaacttatg tggattctca tggtgcatcc cacacaaatc cttttcatag ttcagtgatt 120
   gcatttgaag aaaatgctcc ccacaccaaa agacaaatag atcaaagact ctttaaaccc 180
   gctacctttg gacaccacac agacctgttc aaccagattt tgtatgcctt tgaacaagaa 240
   gattactgtg actttgaagt ccaatttgag attacccata acacgattca cgcttggaca 300
   ggaggaagcg aacatttctc aatgtcgtcc ctacattaca cagctttcga tcctttgttt 360
   tactttcacc attctaacgt tgatcgtctt tgggccgttt ggcaagcctt acagatgaga 420
   cggcataaac cctacagggc ccactgcgcc atatctctgg aacatatgca tctgaaacca 480
   ttcgcctttt catctcccct taacaataac gaaaagactc atgccaatgc catgccaaac 540
   aagatctacg actatgaaaa tgtcctccat tacacatacg aagatttaac atttggaggc 600
   atctctctgg aaaacataga aaagatgatc cacgaaaacc agcaagaaga cagaatatat 660
   gccggttttc tcctggctgg catacgtact tcagcaaatg ttgatatctt cattaaaact 720
   accgattccg tgcaacataa ggctggaaca tttgcagtgc tcggtggaag caaggaaatg 780
   aagtggggat ttgatcgcgt tttcaagttt gacatcacgc acgttttgaa agatctcgat 840
   ctcactgctg atggcgattt cgaagttact gttgacatca ctgaagtcga tggaactaaa 900
   cttgcatcca gtcttattcc acatgcttct gtcattcgtg agcatgcacg tggtaagctg 960
   aataga 966
<210> 97
   <211> 1242
   <212> DNA
   <213> Megathura crenulata
<400> 97
   gttaaatttg acaaagtgcc aaggagtcgt cttattcgaa aaaatgtaga ccgtttgagc 60
   cccgaggaga tgaatgaact tcgtaaagcc ctagccttac tgaaagagga caaaagtgcc 120
   ggtggatttc agcagcttgg tgcattccat ggggagccaa aatggtgtcc tagtcccgaa 180
   gcatctaaaa aatttgcctg ctgtgttcac ggcatgtctg tgttccctca ctggcatcga 240
   ctgttgacgg ttcagagtga aaatgctttg agacgacatg gctacgatgg agctttgccg 300
   tactgggatt ggacctctcc tcttaatcac cttcccgaac tggcagatca tgagaagtac 360
   gtcgaccctg aagatggggt agagaagcat aacccttggt tcgatggtca tatagataca 420
   gtcgacaaaa caacaacaag aagtgttcag aataaactct tcgaacagcc tgagtttggt 480
   cattatacaa gcattgccaa acaagtactg ctagcgttgg aacaggacaa tttctgtgac 540
   tttgaaatcc aatatgagat tgcccataac tacatccatg cacttgtagg aggcgctcag 600
   ccttatggta tggcatcgct tcgctacact gcttttgatc cactattcta cttgcatcac 660
   tctaatacag atcgtatatg ggcaatatgg caggctttac agaagtacag aggaaaaccg 720
   tacaacgttg ctaactgtgc tgttacatcg atgagagaac ctttgcaacc atttggcctc 780
   tctgccaata tcaacacaga ccatgtaacc aaggagcatt cagtgccatt caacgttttt 840
   gattacaaga ccaatttcaa ttatgaatat gacactttgg aatttaacgg tctctcaatc 900
   tctcagttga ataaaaagct cgaagcgata aagagccaag acaggttctt tgcaggcttc 960
   ctgttatctg gtttcaagaa atcatctctt gttaaattca atatttgcac cgatagcagc 1020
   aactgtcacc ccgctggaga gttttacctt ctgggtgatg aaaacgagat gccatgggca 1080
   tacgatagag tcttcaaata tgacataacc gaaaaactcc acgatctaaa gctgcatgca 1140
   gaagaccact tctacattga ctatgaagta tttgacctta aaccagcaag cctgggaaaa 1200
   gatttgttca agcagccttc agtcattcat gaaccaagaa ta                   1242
<210> 98
   <211> 1236
   <212> DNA
   <213> Megathura crenulata
<400> 98
   ggtcaccatg aaggcgaagt atatcaagct gaagtaactt ctgccaaccg tattcgaaaa 60
   aacattgaaa atctgagcct tggtgaactc gaaagtctga gagctgcctt cctggaaatt 120
   gaaaacgatg gaacttacga atcaatagct aaattccatg gtagccctgg tttgtgccag 180
   ttaaatggta accccatctc ttgttgtgtc catggcatgc caactttccc tcactggcac 240
   agactgtacg tggttgtcgt tgagaatgcc ctcctgaaaa aaggatcatc tgtagctgtt 300
   ccctattggg actggacaaa acgaatcgaa catttacctc acctgatttc agacgccact 360
   tactacaatt ccaggcaaca tcactatgag acaaacccat tccatcatgg caaaatcaca 420
   cacgagaatg aaatcactac tagggatccc aaggacagcc tcttccattc agactacttt 480
   tacgagcagg tcctttacgc cttggagcag gataacttct gtgatttcga gattcagttg 540
   gagatattac acaatgcatt gcattcttta cttggtggca aaggtaaata ttccatgtca 600
   aaccttgatt acgctgcttt tgatcctgtg ttcttccttc atcacgcaac gactgacaga 660
   atctgggcaa tctggcaaga ccttcagagg ttccgaaaac ggccataccg agaagcgaat 720
   tgcgctatcc aattgatgca cacgccactc cagccgtttg ataagagcga caacaatgac 780
   gaggcaacga aaacgcatgc cactccacat gatggttttg aatatcaaaa cagctttggt 840
   tatgcttacg ataatctgga actgaatcac tactcgattc ctcagcttga tcacatgctg 900
   caagaaagaa aaaggcatga cagagtattc gctggcttcc tccttcacaa tattggaaca 960
   tctgccgatg gccatgtatt tgtatgtctc ccaactgggg aacacacgaa ggactgcagt 1020
   catgaggctg gtatgttctc catcttaggc ggtcaaacgg agatgtcctt tgtatttgac 1080
   agactttaca aacttgacat aactaaagcc ttgaaaaaga acggtgtgca cctgcaaggg 1140
   gatttcgatc tggaaattga gattacggct gtgaatggat ctcatctaga cagtcatgtc 1200
   atccactctc ccactatact gtttgaggcc ggaaca                           1236
<210> 99
   <211> 1257
   <212> DNA
   <213> Megathura crenulata
<400> 99
   gattctgccc acacagatga tggacacact gaaccagtga tgattcgcaa agatatcaca 60
   caattggaca agcgtcaaca actgtcactg gtgaaagccc tcgagtccat gaaagccgac 120
   cattcatctg atgggttcca ggcaatcgct tccttccatg ctcttcctcc tctttgtcca 180
   tcaccagctg cttcaaagag gtttgcgtgc tgcgtccatg gcatggcaac gttcccacaa 240
   tggcaccgtc tgtacacagt ccaattccaa gattctctca gaaaacatgg tgcagtcgtt 300
   ggacttccgt actgggactg gaccctacct cgttctgaat taccagagct cctgaccgtc 360
   tcaactattc atgacccgga gacaggcaga gatataccaa atccatttat tggttctaaa 420
   atagagtttg aaggagaaaa cgtacatact aaaagagata tcaataggga tcgtctcttc 480
   cagggatcaa caaaaacaca tcataactgg tttattgagc aagcactgct tgctcttgaa 540
   caaaccaact actgcgactt cgaggttcag tttgaaatta tgcataatgg tgttcatacc 600
   tgggttggag gcaaggagcc ctatggaatt ggccatctgc attatgcttc ctatgatcca 660
   cttttctaca tccatcactc ccaaactgat cgtatttggg ctatatggca atcgttgcag 720
   cgtttcagag gactttctgg atctgaggct aactgtgctg taaatctcat gaaaactcct 780
   ctgaagcctt tcagctttgg agcaccatat aatcttaatg atcacacgca tgatttctca 840
   aagcctgaag atacattcga ctaccaaaag tttggataca tatatgacac tctggaattt 900
   gcagggtggt caattcgtgg cattgaccat attgtccgta acaggcagga acattcaagg 960
   gtctttgccg gattcttgct tgaaggattt ggcacctctg ccactgtcga tttccaggtc 1020
   tgtcgcacag cgggagactg tgaagatgca gggtacttca ccgtgttggg aggtgaaaaa 1080
   gaaatgcctt gggcctttga tcggctttac aagtacgaca taacagaaac cttagacaag 1140
   atgaaccttc gacatgacga aatcttccag attgaagtaa ccattacatc ctacgatgga 1200
   actgtactcg atagtggcct tattcccaca ccgtcaatca tctatgatcc tgctcat    1257
<210> 100
   <211> 1254
   <212> DNA
   <213> Megathura crenulata
<400> 100
   catgatatta gttcgcacca cctgtcgctc aacaaggttc gtcatgatct gagtacactg 60
   agtgagcgag atattggaag ccttaaatat gctttgagca gcttgcaggc agatacctca 120
   gcagatggtt ttgctgccat tgcatccttc catggtctgc ctgccaaatg taatgacagc 180
   cacaataacg aggtggcatg ctgtatccat ggaatgccta cattccccca ctggcacaga 240
   ctctacaccc tccaatttga gcaagctcta agaagacatg gctctagtgt agcagtaccc 300
   tactgggact ggacaaagcc aatacataat attccacatc tgttcacaga caaagaatac 360
   tacgatgtct ggagaaataa agtaatgcca aatccatttg cccgagggta tgtcccctca 420
   cacgatacat acacggtaag agacgtccaa gaaggcctgt tccacctgac atcaacgggt 480
   gaacactcag cgcttctgaa tcaagctctt ttggcgctgg aacagcacga ctactgcgat 540
   tttgcagtcc agtttgaagt catgcacaac acaatccatt acctagtggg aggacctcaa 600
   gtctattctt tgtcatccct tcattatgct tcatatgatc cgatcttctt catacaccac 660
   tcctttgtag acaaggtttg ggctgtctgg caggctcttc aagaaaagag aggccttcca 720
   tcagaccgtg ctgactgcgc tgttagtctg atgactcaga acatgaggcc tttccattac 780
   gaaattaacc ataaccagtt caccaagaaa catgcagttc caaatgatgt tttcaagtac 840
   gaactcctgg gttacagata cgacaatctg gaaatcggtg gcatgaattt gcatgaaatt 900
   gaaaaggaaa tcaaagacaa acagcaccat gtgagagtgt ttgcagggtt cctccttcac 960
   ggaattagaa cctcagctga tgtccaattc cagatttgta aaacatcaga agattgtcac 1020
   catggaggcc aaatcttcgt tcttgggggg actaaagaga tggcctgggc ttataaccgt 1080
   ttattcaagt acgatattac ccatgctctt catgacgcac acatcactcc agaagacgta 1140
   ttccatccct ctgaaccatt cttcatcaag gtgtcagtga cagccgtcaa cggaacagtt 1200
   cttccggctt caatcctgca tgcaccaacc attatctatg aacctggtct cggt       1254
<210> 101
   <211> 510
   <212> DNA
   <213> Megathura crenulata
<400> 101
   gaccatcacg aagatcatca ttcttcttct atggctggac atggtgtcag aaaggaaatc 60
   aacacactta ccactgcaga ggtggacaat ctcaaagatg ccatgagagc cgtcatggca 120
   gaccacggtc caaatggata ccaggctata gcagcgttcc atggaaaccc accaatgtgc 180
   cctatgccag atggaaagaa ttactcgtgt tgtacacatg gcatggctac tttcccccac 240
   tggcacagac tgtacacaaa acagatggaa gatgccttga ccgcccatgg tgccagagtc 300
   ggccttcctt actgggacgg gacaactgcc tttacagctt tgccaacttt tgtcacagat 360
   gaagaggaca atcctttcca tcatggtcac atagactatt tgggagtgga tacaactcgg 420
   tcgccccgag acaagttgtt caatgatcca gagcgaggat cagaatcgtt cttctacagg 480
   caggttctct tggctttgga gcagacagat                                  510
<210> 102
   <211> 942
   <212> DNA
   <213> Megathura crenulata
<400> 102
   ggcctgccct actgggattg gaccatgcca atgagtcatt tgccagaact ggctacaagt 60
   gagacctacc tcgatccagt tactggggaa actaaaaaca accctttcca tcacgcccaa 120
   gtggcgtttg aaaatggtgt aacaagcagg aatcctgatg ccaaactttt tatgaaacca 180
   acttacggag accacactta cctcttcgac agcatgatct acgcatttga gcaggaagac 240
   ttctgcgact ttgaagtcca atatgagctc acgcataatg caatacatgc atgggttgga 300
   ggcagtgaaa agtattcaat gtcttctctt cactacactg cttttgatcc tatattttac 360
   ctccatcact caaatgttga tcgtctctgg gccatttggc aagctcttca aatcaggaga 420
   ggcaagtctt acaaggccca ctgcgcctcg tctcaagaaa gagaaccatt aaagcctttt 480
   gcattcagtt ccccactgaa caacaacgag aaaacgtacc acaactctgt ccccactaac 540
   gtttatgact atgtgggagt tttgcactat cgatatgatg accttcagtt tggcggtatg 600
   accatgtcag aacttgagga atatattcac aagcagacac aacatgatag aacctttgca 660
   ggattcttcc tttcatatat tggaacatca gcaagcgtag atatcttcat caatcgagaa 720
   ggtcatgata aatacaaagt gggaagtttt gtagtacttg gtggatccaa agaaatgaaa 780
   tggggctttg atagaatgta caagtatgag atcactgagg ctctgaagac gctgaatgtt 840
   gcagtggatg atgggttcag cattactgtt gagatcaccg atgttgatgg atctccccca 900
   tctgcagatc tcattccacc tcctgctata atctttgaac gt                    942
<210> 103
   <211> 1248
   <212> DNA
   <213> Megathura crenulata
<400> 103
   gctgatgcca aagactttgg ccatagcaga aaaatcagga aagccgttga ttctctgaca 60
   gtcgaagaac aaacttcgtt gaggcgagct atggcagatc tacaggacga caaaacatca 120
   gggggtttcc agcagattgc agcattccac ggagaaccaa aatggtgtcc aagccccgaa 180
   gcggagaaaa aatttgcatg ctgtgttcat ggaatggctg ttttccctca ctggcacaga 240
   ttgctgacag ttcaaggaga aaatgctctg aggaaacatg gctttactgg tggactgccc 300
   tactgggact ggactcgatc aatgagcgcc cttccacatt ttgttgctga tcctacttac 360
   aatgatgcta tttccagcca ggaagaagat aacccatggc atcatggtca catagactct 420
   gttgggcatg atactacaag agatgtgcgt gatgatcttt atcaatctcc tggtttcggt 480
   cactacacag atattgcaaa acaagtcctt ctggcctttg agcaggacga tttctgtgat 540
   tttgaggtac aatttgaaat tgcccataat ttcatacatg ctctggttgg tggtaacgaa 600
   ccatacagta tgtcatcttt gaggtatact acatacgatc caatcttctt cttgcaccgc 660
   tccaatacag accgactttg ggccatttgg caagctttgc aaaaataccg ggggaaacca 720
   tacaacactg caaactgtgc cattgcatcc atgagaaaac cacttcagcc atttggtctt 780
   gatagtgtca taaatccaga tgacgaaact cgtgaacatt cggttccttt ccgagtcttc 840
   gactacaaga acaacttcga ctatgagtat gagagcctgg catttaatgg tctgtctatt 900
   gcccaactgg accgagagtt gcagagaaga aagtcacatg acagagtctt tgcaggattc 960
   cttcttcatg aaattggaca gtctgcactc gtgaaattct acgtttgcaa acacaatgta 1020
   tctgactgtg accattatgc tggagaattc tacattttgg gagatgaagc tgagatgcct 1080
   tggaggtatg accgtgtgta caagtacgag ataacacagc agctgcacga tttagatcta 1140
   catgttggag ataatttctt ccttaaatat gaagcctttg atctgaatgg cggaagtctt 1200
   ggtggaagta tcttttctca gccttcggtg attttcgagc cagctgca              1248
<210> 104
   <211> 1257
   <212> DNA
   <213> Megathura crenulata
<400> 104
   ggttcacacc aggctgatga atatcgtgag gcagtaacaa gcgctagcca cataagaaaa 60
   aatatccggg acctctcaga gggagaaatt gagagcatca gatctgcttt cctccaaatt 120
   caaaaagagg gtatatatga aaacattgca aagttccatg gaaaaccagg actttgtgaa 180
   catgatggac atcctgttgc ttgttgtgtc catggcatgc ccacctttcc ccactggcac 240
   agactgtacg ttcttcaggt ggagaatgcg ctcttagaac gagggtctgc agttgctgtt 300
   ccttactggg actggaccga gaaagctgac tctctgccat cattaatcaa tgatgcaact 360
   tatttcaatt cacgatccca gacctttgat cctaatcctt tcttcagggg acatattgcc 420
   ttcgagaatg ctgtgacgtc cagagatcct cagccagaac tatgggacaa taaggacttc 480
   tacgagaatg tcatgctggc tcttgagcaa gacaacttct gtgactttga gattcagctt 540
   gagctgatac acaacgccct tcattctaga cttggaggaa gggctaaata ctccctttcg 600
   tctcttgatt ataccgcatt tgatcctgta tttttccttc accatgcaaa cgttgacaga 660
   atctgggcca tctggcagga cttgcagaga tatagaaaga aaccatacaa tgaggctgac 720
   tgcgcagtca acgagatgcg taaacctctt caaccattta ataacccaga acttaacagt 780
   gattccatga cgcttaaaca caacctccca caagacagtt ttgattatca aaaccgcttc 840
   aggtaccaat atgataacct tcaatttaac cacttcagca tacaaaagct agaccaaact 900
   attcaggcta gaaaacaaca cgacagagtt tttgctggct ttattcttca caacattggg 960
   acatctgctg ttgtagatat ttatatttgc gttgaacaag gaggagaaca aaactgcaag 1020
   acaaaggcgg gttccttcac gattctgggg ggagaaacag aaatgccatt ccactttgac 1080
   cgcttgtaca aatttgacat aacgtctgct ctgcataaac ttggtgttcc cttggacgga 1140
   catggattcg acatcaaagt tgacgtcaga gctgtcaatg gatcgcatct tgatcaacac 1200
   atcctcaacg aaccgagtct gctttttgtt cctggtgaac gtaagaatat atattat 1257
<210> 105
   <211> 1239
   <212> DNA
   <213> Megathura crenulata
<400> 105
   gatgggcttt cacaacataa tcttgtgcga aaagaagtaa gctctcttac aacactggag 60
   aaacattttt tgaggaaagc tctcaagaac atgcaagcag atgattctcc agacggatat 120
   caagctattg cttctttcca cgctttgcct cctctttgtc caagtccatc tgctgcacat 180
   agacacgctt gttgcctcca tggtatggct accttccctc agtggcacag actctacaca 240
   gttcagttcg aagattcttt gaaacgacat ggttctattg tcggacttcc atattgggat 300
   tggctgaaac cgcagtctgc actccctgat ttggtgacac aggagacata cgagcacctg 360
   ttttcacaca aaaccttccc aaatccgttc ctcaaggcaa atatagaatt tgagggagag 420
   ggagtaacaa cagagaggga tgttgatgct gaacacctct ttgcaaaagg aaatctggtt 480
   tacaacaact ggttttgcaa tcaggcacta tatgcactag aacaagaaaa ttactgtgac 540
   tttgaaatac agttcgaaat tttgcataat ggaattcatt catgggttgg aggatcaaag 600
   acccattcaa taggtcatct tcattacgca tcatacgatc cactgttcta tatccaccat 660
   tcgcagacag atcgcatttg ggctatctgg caagctctcc aggagcacag aggtctttca 720
   gggaaggaag cacactgcgc cctggagcaa atgaaagacc ctctcaaacc tttcagcttt 780
   ggaagtccct ataatttgaa caaacgcact caagagttct ccaagcctga agacacattt 840
   gattatcacc gattcgggta tgagtatgat tccctcgaat ttgttggcat gtctgtttca 900
   agtttacata actatataaa acaacaacag gaagctgata gagtcttcgc aggattcctt 960
   cttaaaggat ttggacaatc agcatccgta tcgtttgata tctgcagacc agaccagagt 1020
   tgccaagaag ctggatactt ctcagttctc ggtggaagtt cagaaatgcc gtggcagttt 1080
   gacaggcttt acaagtacga cattacaaaa acgttgaaag acatgaaact gcgatacgat 1140
   gacacattta ccatcaaggt tcacataaag gatatagctg gagctgagtt ggacagcgat 1200
   ctgattccaa ctccttctgt tctccttgaa gaaggaaag 1239
<210> 106
   <211> 1251
   <212> DNA
   <213> Megathura crenulata
<400> 106
   catgggatca atgtacgtca cgttggtcgt aatcggattc gtatggaact atctgaactc 60
   accgagagag atctcgccag cctgaaatct gcaatgaggt ctctacaagc tgacgatggg 120
   gtgaacggtt atcaagccat tgcatcattc cacggtctcc cggcttcttg tcatgatgat 180
   gagggacatg agattgcctg ttgtatccac ggaatgccag tattcccaca ctggcacagg 240
   ctttacaccc tgcaaatgga catggctctg ttatctcacg gatctgctgt tgctattcca 300
   tactgggact ggaccaaacc tatcagcaaa ctgcctgatc tcttcaccag ccctgaatat 360
   tacgatcctt ggagggatgc agttgtcaat aatccatttg ctaaaggcta cattaaatcc 420
   gaggacgctt acacggttag ggatcctcag gacattttgt accacttgca ggacgaaacg 480
   ggaacatctg ttttgttaga tcaaactctt ttagccttag agcagacaga tttctgtgat 540
   tttgaggttc aatttgaggt cgtccataat gctattcact acttggtggg tggtcgacaa 600
   gtttatgctc tttcttctca acactatgct tcatatgacc cagccttctt tattcatcac 660
   tcctttgttg acaaaatatg ggcagtctgg caagctctgc aaaagaagag aaagcgtccc 720
   tatcataaag cggattgtgc tcttaacatg atgaccaaac caatgcgacc atttgcacac 780
   gatttcaatc acaatggatt cacaaaaatg cacgcagtcc ccaacactct atttgacttt 840
   caggaccttt tctacacgta tgacaactta gaaattgctg gcatgaatgt taatcagttg 900
   gaagcggaaa tcaaccggcg aaaaagccaa acaagagtct ttgccgggtt ccttctacat 960
   ggcattggaa gatcagctga tgtacgattt tggatttgca agacagctga cgactgccac 1020
   gcatctggca tgatctttat cttaggaggt tctaaagaga tgcactgggc ctatgacagg 1080
   aactttaaat acgacatcac ccaagctttg aaggctcagt ccatacaccc tgaagatgtg 1140
   tttgacactg atgctccttt cttcattaaa gtggaggtcc atggtgtaaa caagactgct 1200
   ctcccatctt cagctatccc agcacctact ataatctact cagctggtga a          1251
<210> 107
   <211> 1185
   <212> DNA
   <213> Megathura crenulata
<400> 107
   gatcatattg ctggcagtgg agtcaggaaa gacgtgacgt ctcttaccgc atctgagata 60
   gagaacctga ggcatgctct gcaaagcgtg atggatgatg atggacccaa tggattccag 120
   gcaattgctg cttatcacgg aagtcctccc atgtgtcaca tgcntgatgg tagagacgtt 180
   gcatgttgta ctcatggaat ggcatctttc cctcactggc acagactgtt tgtgaaacag 240
   atggaggatg cactggctgc gcatggagct cacattggca taccatactg ggattggaca 300
   agtgcgttta gtcatctgcc tgccctagtg actgaccacg agcacaatcc cttccaccac 360
   ggacatattg ctcatcggaa tgtggataca tctcgatctc cgagagacat gctgttcaat 420
   gaccccgaac acgggtcaga atcattcttc tatagacagg ttctcttggc tctagaacag 480
   acagacttct gccaatttga agttcagttt gaaataacac acaatgcaat ccactcttgg 540
   actggaggac atactccata tggaatgtca tcactggaat atacagcata tgatccactc 600
   ttttatctcc accattccaa cactgatcgt atctgggcca tctggcaggc actccagaaa 660
   tacagaggtt ttcaatacaa cgcagctcat tgcgatatcc aggttctgaa acaacctctt 720
   aaaccattca gcgagtccag gaatccaaac ccagtcacca gagccaattc tagggcagtc 780
   gattcatttg attatgagag actcaattat caatatgaca cacttacctt ccacggacat 840
   tctatctcag aacttgatgc catgcttcaa gagagaaaga aggaagagag aacatttgca 900
   gccttcctgt tgcacggatt tggcgccagt gctgatgttt cgtttgatgt ctgcacacct 960
   gatggtcatt gtgcctttgc tggaaccttc gcggtacttg gtggggagct tgagatgccc 1020
   tggtcctttg aaagattgtt ccgttacgat atcacaaagg ttctcaagca gatgaatctt 1080
   cactatgatt ctgagttcca ctttgagttg aagattgttg gcacagatgg aacagaactg 1140
   ccatcggatc gtatcaagag ccctaccatt gaacaccatg gagga                 1185
<210> 108
   <211> 309
   <212> DNA
   <213> Megathura crenulata
<400> 108
   ggtcacgatc acagtgaacg tcacgatgga tttttcagga aggaagtcgg ttccctgtcc 60
   ctggatgaag ccaatgacct taaaaatgca ctgtacaagc tgcagaatga tcagggtccc 120
   aatggatatg aatcaatagc cggttaccat ggctatccat tcctctgccc tgaacatggt 180
   gaagaccagt acgcatgctg tgtccacgga atgcctgtat ttccacattg gcacagactt 240
   catacaatcc agtttgagag agctctcaaa gaacatggtt ctcatttggg tctgccatac 300
   tgggactgg                                                         309

## Beschreibung

Die vorliegende Erfindung betrifft ein Nukleinsäuremolekül, umfassend eine für ein Hämocyanin bzw eine Hämocyanin-Domäne kodierende Nukleinsäuresequenz, mit mindestens einer Intronsequenz, Konstrukte, die solche Nukleinsäuremoleküle umfassen, die Nukleinsäuresequenzen oder die Konstrukte umfassende Wirtszellen, Verfahren zum Herstellen von Hämocyanin-Polypeptiden und rekombinante Hämocyanin-Polypeptide. Die Ausführungsformen der Erfindung sind in den Ansprüchen definiert.

Hämocyanin ist ein blaues Kupferprotein, das frei gelöst im Blut zahlreicher Mollusken und Arthropoden auftritt und den Sauerstoff transportiert. Von den Mollusken enthalten die Cephalopoden, Chitonen, die meisten Gastropoden sowie einige Bivalvia Hämocyanin. Hämocyanin ist bei den Arthropoden typisch für Arachniden, Xiphosuren, malakostrake Crustaceen und *Scutigera.* Zahlreiche Insektenarten weisen Proteine auf, die sich von Hämocyanin ableiten. Hämocyanine liegen extrazellulär vor und flottieren in der Hämolymphe.

Während das Arthropoden-Hämocyanin bei elektronenmikroskopischer Untersuchung einen Durchmesser von maximal 25 nm hat und eine Untereinheit ein Molekulargewicht von 75.000 Da aufweist, sind Molluskencyanine viel größer. So hat z.B. das Hämocyanin von *Megathura* einen Durchmesser von 35 nm und ist aus 2 Untereinheiten zusammengesetzt. Jede Untereinheit hat ein Molekulargewicht von ca. 400.000 Da und ist in acht sauerstoffbindende Domänen aufgeteilt, die jeweils ein Molekulargewicht von ca. 50.000 Da haben. Die Domänen unterscheiden sich immunologisch. Diese Domänen können durch limitierte Proteolyse aus der Untereinheit freigesetzt werden.

Das im Elektronenmikroskop sichtbare Hämocyanin der Gastropoden hat ein Molekulargewicht von ca. 8 Mio. Da und ist ein Di-Dekamer. Im Gegensatz hierzu ist das Hämocyanin der Cephalopoden als isoliertes Dekamer angeordnet, das sich auch in der Quartärstruktur deutlich vom Hämocyanin der Gastropoden unterscheidet.

Von besonderem immunologischen Interesse ist das Hämocyanin der kalifornischen Schlüssellochschnecke *Megathura crenulata,* einer "Keyhole Limpet". Das Hämocyanin wird deshalb auch als Keyhole Limpet Hemocyanin (KLH) bezeichnet. Hämocyanine sind sehr starke Antigene. Die Immunisierung eines Vertebraten führt zu einer bisher wenig verstandenen, unspezifischen Aktivierung des Immunsystems. Durch die allgemeine Aktivierung des Immunsystems ist es dann möglich, auch eine Immunreaktion gegenüber anderen, bisher tolerierten Fremdstrukturen zu erreichen. KLH wird vor allem als Hapten-Träger verwendet, um so die Bildung von Antikörpern gegen das Hapten zu erreichen.

Neben *Megathura crenulata* gehört auch das Seeohr *Haliotis tuberculata* zur im Hinblick auf die Evolution relativ alten Gruppe der Archaegastropoda. Es ist bekannt, dass auch *Haliotis* Hämocyanin produziert.

KLH ist ein Gemisch aus zwei unterschiedlichen Hämocyaninen, die als KLH1 und KLH2 bezeichnet werden. Die Untereinheit des KLH1 ist ein 390 kDa Polypeptid, das aus acht globulären Domänen besteht, die entsprechend ihrer Reihenfolge in der Untereinheit mit 1 a bis 1 h bezeichnet werden. KLH2 hingegen weist ein Molekulargewicht von 350 kDa auf und enthält nach neuesten Daten ebenfalls 8 Domänen, die als 2 a bis 2 h bezeichnet werden. *In vivo* bildet jede Art von Untereinheit Homo-Oligomere, wohingegen Hetero-Oligomere nicht beobachtet wurden.

Durch limitierte Proteolyse und gekreuzte Immunelektrophorese der Untereinheit von KLH1 und KLH2 wurden amino-, interne und carboxy-terminale Domänen erhalten, deren aminoterminale Sequenz bestimmt wurde (Söhngen et al., Eur. J. Biochem. 248 (1997), 602-614; Gebauer et al., Zoology 98(1994), 51-68). Die erhaltenen Sequenzen erlauben jedoch nicht den Entwurf sequenzspezifischer Primer und/oder Sonden, die für eine Hybridisierung mit genomischer DNA Erfolg versprechen. Obwohl beide KLH-Typen seit 1991 bzw. 1994 bekannt sind, konnte daher bisher keine Primärstruktur aufgeklärt werden.

Auf DNA-Ebene ist bisher in bezug auf Mollusken nur die cDNA-Sequenz der Hämocyanin-Untereinheit aus dem Cephalopoden *Octopus dofleini* bekannt (Miller et al., J. Mol. Biol. 278 (1998), 827-842). *Octopus dofleini* ist phylogenetisch von den Archaegastropoden sehr weit entfernt. Eine Hämocyanin-Gensequenz aus Mollusken ist bisher überhaupt nicht bekannt.

Wie von Miller et al. supra, beschrieben, ist es sowohl schwierig, eine einzige funktionelle Domäne (Funktionelle Einheit = Domäne; auch "funktionelle Domäne" genannt) zu isolieren als auch Gewebe zu erhalten, das zur Aufreinigung von mRNA für die cDNA-Sequenzierung geeignet ist.

Bei der Analyse des Hämocyanins aus *Megathura crenulata* besteht eine weitere Schwierigkeit darin, dass die Versuchstiere ein Alter von 4 bis 8 Jahren erreicht haben müssen, um ihnen erstmals Hämolymphe entnehmen zu können. Nach Entnahme der Hämolymphe wird Hämocyanin bei diesen Tieren nicht nachproduziert. Bisher ist nicht bekannt, wie die Hämocyaninsynthese stimuliert werden könnte. Darüber hinaus ist die Zucht von *Megathura* äußerst aufwendig, da hierfür spezielle Strömungsbecken erforderlich sind.

Es ist daher eine Aufgabe der vorliegenden Erfindung. Mittel und Wege bereitzustellen, um Hämocyanin und/oder Domänen davon in ausreichender Menge und kostengünstig produzieren zu können. Dies umfasst die weitere Aufgabe, ein Verfahren anzugeben, mit dem dieses Hämocyanin hergestellt werden kann.

Diese Aufgabe wird erfindungsgemäß gelöst durch
1. Ein Nukleinsäure-Molekül, umfassend eine Sequenz kodierend für ein Hämocyanin, eine Hämocyanin-Domäne oder ein funktionelles Fragment davon, wobei die Nukleinsäuresequenz ausgewählt ist aus:
   (a) Nukleinsäuresequenzen, die aus der Gruppe der nachfolgend angegebenen DNA-Sequenzen bzw. der ihnen entsprechenden RNA-Sequenzen ausgewählt sind oder diese enthalten:
      SEQ ID NO:9 (partielle HtH2 Domäne b),
      SEQ ID NO:10 (HtH2 Domäne c),
      SEQ ID NO:11 (HtH2 Domäne d),
      SEQ ID NO:12 (HtH2 Domäne e),
      SEQ ID NO:13 (HtH2 Domäne f),
      SEQ ID NO:14 (HtH2 Domäne g),
      SEQ ID NO:15 (HtH2 Domäne h),
      SEQ ID NO:20 (KLH2 Domäne b),
      SEQ ID NO:21 (KLH2 Domäne c),
      SEQ ID NO:22 (partielle KLH2 Domäne d),
      SEQ ID NO:23 (KLH2 Domäne g),
      SEQ ID NO:24 (partielle KLH2 Domäne h),
      SEQ ID NO:50 (partielle HtH2 Domäne a),
      SEQ ID NO:51 (HtH2 Domäne b'),
      SEQ ID NO:52 (HtH2 Domäne d'),
      SEQ ID NO:53 (HtH2 Domäne e'),
      SEQ ID NO:57 (KLH2 Domäne b'),
      SEQ ID NO:58 (KLH2 Domäne c'),
      SEQ ID NO:59 (KLH2 Domäne d'),
      SEQ ID NO:60 (KLH2 Domäne e),
      SEQ ID NO:61 (KLH2 Domäne f),
      SEQ ID NO:62 (KLH2 Domäne g'),
      SEQ ID NO:88 (partielle HtH2 Domäne a"),
      SEQ ID NO:89 (HtH2 Domäne b")
      SEQ ID NO:90 (HtH2 Domäne c"),
      SEQ ID NO:91 (HtH2 Domäne d"),
      SEQ ID NO:92 (HtH2 Domäne e"),
      SEQ ID NO:93 (HtH2 Domäne f'),
      SEQ ID NO:94 (HtH2 Domäne g"),
      SEQ ID NO:95 (HtH2 Domäne h"),
      SEQ ID NO:102 (KLH2 Domäne b"),
      SEQ ID NO:103 (KLH2 Domäne c"),
      SEQ ID NO:104 (KLH2 Domäne d"),
      SEQ ID NO:105 (KLH2 Domäne e"),
      SEQ ID NO:106 (KLH2 Domäne f'),
      SEQ ID NO: 107 (KLH2 Domäne g"),
      SEQ ID NO:108 (partielle KLH2 Domäne h"),
      SEQ ID NO: 157 (vollständige HtH2 Domäne a);
   (b) Nukleinsäuresequenzen, welche für ein Polypeptid kodieren umfassend wenigstens eine aus der folgenden Gruppe ausgewählte Aminosäuresequenz:
      SEQ ID NO:33 (partielle HtH2 Domäne b),
      SEQ ID NO:34 (HtH2 Domäne c),
      SEQ ID NO:35 (HtH2 Domäne d),
      SEQ ID NO:36 (HtH2 Domäne e),
      SEQ ID NO:37 (HtH2 Domäne f),
      SEQ ID NO:38 (HtH2 Domäne g),
      SEQ ID NO:39 (HtH2 Domäne h),
      SEQ ID NO:44 (KLH2 Domäne b),
      SEQ ID NO:45 (KLH2 Domäne c),
      SEQ ID NO:46 (partielle KLH2 Domäne d),
      SEQ ID NO:47 (KLH2 Domäne g),
      SEQ ID NO:48 (partielle KLH2 Domäne h),
      SEQ ID NO:65 (partielle HtH2 Domäne a),
      SEQ ID NO: 156 (vollständige HtH2 Domäne a),
      SEQ ID NO:66 (HtH2 Domäne b'),
      SEQ ID NO:67 (HtH2 Domäne d'),
      SEQ ID NO:68 (HtH2 Domäne e'),
      SEQ ID NO:74 (KLH2 Domäne b'),
      SEQ ID NO:75 (KLH2 Domäne c'),
      SEQ ID NO:76 (KLH2 Domäne d'),
      SEQ ID NO:77 (KLH2 Domäne e),
      SEQ ID NO:78 (KLH2 Domäne f),
      SEQ ID NO:79 (KLH2 Domäne g'),
      SEQ ID NO:158 (teilweise KLH2 Domäne h)
   (c) Nukleinsäuresequenzen, die für Polypeptide kodieren, das wenigstens 60% identisch zu einer Sequenz von Ausführungsform 1b ist, wobei die Sequenzidentität über 90 Aminosäuren berechnet wird.
2. Nukleinsäure-Molekül nach Ausführungsform 1, **dadurch gekennzeichnet**, dass die Intronsequenz ausgewählt ist aus:
   (i) Nukleinsäuresequenzen, die aus der Gruppe der nachfolgend angegebenen DNA-Sequenzen bzw. der ihnen entsprechenden RNA-Sequenzen ausgewählt sind oder diese enthalten:
      SEQ ID NO:147 (Intron 2B/2C von KLH2),
      SEQ ID NO:148 (Intron 2C/2D von KLH2),
      SEQ ID NO:149 (Intron 2D/2E von KLH2),
      SEQ ID NO:150 (Intron 2E/2F von KLH2),
      SEQ ID NO:1 51 (Intron 2F von KLH2),
      SEQ ID NO:152 (Intron 2F-2/2G von KLH2),
      SEQ ID NO:153 (Intron 2G-1/2G-2 von KLH2),
      SEQ ID NO:154 (Intron 2G-2/2G-3 von KLH2),
      SEQ ID NO:155 (Intron 2G/2H von KLH2);
   (ii) Nukleinsäuresequenzen, die mit dem Gegenstrang einer Nukleinsäuresequenz nach (i) hybridisieren;
   (iii) Nukleinsäuresequenzen, die wenigstens 60 % identisch zu einer der unter (i) angegebenen Nukleinsäuresequenzen sind;
   (iv) Varianten der unter (i) bis (iii) angegebenen Sequenzen, wobei die Varianten gegenüber den unter (i) unter (iii) angegebenen Sequenzen Additionen, Deletionen, Insertionen oder Inversionen aufweisen; und
   (v) Kombinationen mehrerer der unter (i) bis (iv) angegebenen DNA-Sequenzen.
3. Nukleinsäuremolekül gemäß einer der Ausführungsformen 1 und 2, **dadurch gekennzeichnet**, dass es ein Desoxyribonukleinsäuremolekül ist.
4. Konstrukt, umfassend ein Nukleinsäuremolekül gemäß einer der Ausführungsformen 1 bis 3.
5. Konstrukt gemäß Ausführungsform 4, weiterhin umfassend einen zur Expressionskontrolle geeigneten Promotor, wobei die für ein Hämocyanin, eine Hämocyanin-Domäne oder ein funktionelles Fragment davon kodierende Nukleinsäuresequenz unter der Kontrolle des Promotors steht.
6. Konstrukt gemäß Ausführungsform 4 oder 5, weiterhin umfassend eine für ein Antigen kodierende Nukleinsäuresequenz, die direkt mit der für ein Hämocyanin, eine Hämocyanin-Domäne oder ein funktionelles Fragment davon kodierenden Nukleinsäuresequenz verbunden ist.
7. Konstrukt gemäß Ausführungsform 6, wobei das Antigen ausgewählt ist aus: Tumorantigenen, Virusantigenen und Antigenen bakterieller oder parasitärer Pathogene.
8. Konstrukt gemäß einer der Ausführungsformen 4 bis 7, wobei das Konstrukt wenigstens einen Teil eines Vektors enthält, wobei der Vektor ausgewählt ist aus: Bakteriophagen, Adenoviren, Vacciniaviren, Baculoviren, SV40-Virus und Retroviren.
9. Konstrukt gemäß einer der Ausführungsformen 4 bis 8, wobei das Konstrukt weiterhin eine His-Tag-kodierende Nukleinsäuresequenz umfasst und die Expression des Konstrukts zur Bildung eines Fusionsproteins mit einem His-Tag führt.
10. Wirtszelle, enthaltend ein Konstrukt gemäß einer der Ausführungsformen 4 bis 9, wobei die Wirtszelle eine zur Expression des Konstrukts geeignete prokaryontische oder eukaryontische Zelle ist.
11. Wirtszelle gemäß Ausführungsform 10, **dadurch gekennzeichnet**, dass die prokaryontische Wirtszelle ausgewählt ist aus E. coli und Bacillus subtilis.
12. Wirtszelle gemäß Ausführungsform 10, **dadurch gekennzeichnet**, dass die eukaryontische Wirtszelle ausgewählt ist aus Hefezellen, Pflanzenzellen, Insektenzellen und Säugerzellen, bevorzugt aus CHO-Zellen, COS-Zellen und HeLa-Zellen.

Im Nachfolgenden werden einige Begriffe näher erläutert, um klarzustellen, wie sie im Zusammenhang der vorliegenden Anmeldung verstanden werden sollen.

Der Begriff "Hämocyanin", so, wie er nachfolgend in der Beschreibung verwendet wird, umfasst vollständiges Hämocyanin, Hämocyanin-Domänen und/oder Fragmente, Hämocyanin-Mutanten und Fusionsproteine. In bezug auf die Fusionsproteine sind insbesondere solche umfasst, bei denen die Fusion Hämocyanin und Antigene umfasst.

Unter "Domänen" werden funktionelle Teilsequenzen der Hämocyanin-Untereinheiten verstanden, die beispielsweise durch limitierte Proteolyse voneinander abgetrennt werden können. Weiterhin können sie unterschiedliche immunologische Eigenschaften aufweisen.

Mit den "immunologischen Eigenschaften wenigstens einer Domäne von Hämocyanin" ist die Eigenschaft eines Polypeptids gemeint, in gleicher Weise wie wenigstens eine Domäne von Hämocyanin eine immunologische Antwort des Empfängers zu induzieren, der mit dem Polypeptid immunisiert wird. Unter "immunologischer Antwort" werden hier T- und/oder B-Zell-Antworten gegen Hämocyanin-Epitope verstanden, wie beispielsweise eine Antikörperproduktion. Die immunologische Reaktion kann beispielsweise beobachtet werden durch Immunisieren eines Säugers, wie z.B. einer Maus, einer Ratte oder eines Kaninchens mit dem entsprechenden Polypeptid und Vergleich der Immunantwort auf das zur Immunisierung verwendete Polypeptid mit der Immunantwort auf natürliche Hämocyanine.

Der Begriff "Intronsequenz" steht sowohl für eine dazwischenliegende Sequenz in einem eurkaryotischen Gen als auch für die korrespondierende dazwischenliegende Sequenz im RNA-Transkript. Die Intronsequenz(en) und die kodierende(n) Sequenz(en) werden gemeinsam transkribiert; der Introntranskript bzw. die Introntranskripte werden dann entfernt, um die funktionelle RNA zu erhalten.

Der Begriff "Antigen" umfasst erfindungsgemäß sowohl Haptene, als auch schwache und starke Antigene. Haptene sind dadurch charakterisiert, dass sie Substanzen niedriger Molekülmasse (kleiner als 4000 Da) sind, jedoch ohne Kopplung an ein Trägermolekül nicht in der Lage sind, eine immunologische Reaktion auszulösen. Schwache Antigene sind Substanzen, die selbst bereits eine immunologische Reaktion auslösen können, deren Potential, eine immunologische Reaktion auslösen zu können, durch Kopplung mit einem TrägerMolekül auf Protein- und/oder DNA-Ebene, noch erhöht werden kann.

"His-Tag" bedeutet eine Sequenz von wenigstens 6 Histidin-Aminosäuren, die durch entsprechende Klonierung und Fusion mit einer exprimierbaren Sequenz zu einem Fusionsprotein mit wenigstens 6 His-Resten am NH₂-Terminus führt, das leicht durch Komplexierung mit einer Ni²⁺-Säule aufgereinigt werden kann.

"Klonierung" soll alle im Stand der Technik bekannten Klonierungsmethoden umfassen, die hier zum Einsatz kommen könnten, die jedoch nicht alle im einzelnen beschrieben werden, weil sie zum selbstverständlichen Handwerkszeug des Fachmanns gehören.

"Varianten" einer Nukleinsäure umfassen Additionen, Deletionen, Insertionen oder Inversionen und kodieren für ein Polypeptid, das die immunologischen Eigenschaften wenigstens einer Domäne von Hämocyanin aufweist. Varianten können künstlich oder natürlich sein. Ein Beispiel für natürliche Varianten stellen allelische Varianten dar.

Unter "rekombinanter Expression in einer geeigneten Wirtszelle" sollen alle im Stand der Technik bekannten Expressionsmethoden in bekannten Expressionssystemen verstanden werden, die hier zum Einsatz kommen könnten, jedoch nicht alle im einzelnen beschrieben werden, weil sie zum selbstverständlichen Handwerkszeug des Fachmanns gehören:

Die im erfindungsgemäßen Nukleinsäuremolekül enthaltene Nukleinsäuresequenz kann genomische DNA oder synthetische DNA sein, wobei unter synthetischen DNA-Sequenzen auch solche verstanden werden, die modifizierte Internukleosid-Bindungen enthalten. Weiter kann es sich bei den Nukleinsäuresequenzen um RNA-Sequenzen handeln, was z.B. für die Expression mittels rekombinanter Vektorsysteme erforderlich sein kann. Die Nukleinsäuresequenzen sind beispielsweise erhältlich durch Verwenden einer nachweisbar markierten Sonde, die einer der angegebenen Sequenzen oder einem Fragment bzw. deren Gegenstrang entspricht, zum Screening von cDNA/genomischen DNA-Bibliotheken aus Mollusken oder Arthropoden. Die der cDNA-Bibliothek zugrundeliegende mRNA ist vorzugsweise aus Mollusken-Geweben zu erhalten, die Hämocyanin besonders stark exprimieren, wie z.B. Mantel-Gewebe aus Gastropoden und Branchialdrüsengewebe aus Cephalopoden.

Die Identifizierung positiver genomischer DNA-Klone erfolgt gemäß Standardverfahren. Vgl. Maniatis et al., Molecular Cloning (1989) Cold Spring Harbor Laboratory Press.

Stringente Hybridisierungsbedingungen sind z.B. 68°C über Nacht in 0,5 x SSC; 1% Blockierungsreagenz (Boehringer Mannheim); 0,1 % Natriumlaurylsarcosinat und nachfolgendem Waschen mit 2 x SSC, 0,1 %SDS.

In einer weiteren bevorzugten Ausführungsform werden Nukleinsäuresequenzen bereitgestellt, die mindestens eine Intronsequenz, die wenigstens 60 % homolog zu einer der unter (i) angegebenen Nukleinsäuresequenzen ist, umfassen. Bevorzugt ist (bzw. sind) die Intronsequenz(en) wenigstens 80% homolog zu einer der unter 2(a) angegebenen Nukleinsäuresequenzen. Besonders bevorzugt ist (bzw. sind) die Intronsequenz(en) wenigstens 90 % homolog zu einer der unter 2(a) angegebenen Nukleinsäuresequenzen. Insbesondere ist (bzw. sind) die Intronsequenz(en) wenigstens 95% homolog zu einer der unter 2(a) angegebenen Nukleinsäuresequenzen.

Erfindungsgemäß bedeutet der Ausdruck "Homologie" Homologie auf DNA-Ebene, die gemäß bekannter Verfahren, z.B. der computergestützten Sequenzvergleiche (Basic local alignment search tool, S.F. Altschul et al., J. Mol. Biol. 215 (1990), 403-410) bestimmt werden kann.

Der dem Fachmann bekannte Ausdruck "Homologie" bezeichnet den Grad der Verwandtschaft zwischen zwei oder mehr Nukleinsäuremolekülen, der durch die Übereinstimmung zwischen den Sequenzen bestimmt wird. Der Prozentsatz der "Homologie" ergibt sich aus dem Prozentsatz identischer Bereiche in zwei oder mehr Sequenzen unter Berücksichtigung von Lücken oder anderen Sequenzbesonderheiten.

Die Homologie miteinander verwandter Nukleinsäuremoleküle kann mit Hilfe bekannter Verfahren bestimmt werden. In der Regel werden spezielle Computerprogramme mit den besonderen Anforderungen Rechnung tragenden Algorithmen eingesetzt.

Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, sind jedoch nicht eingeschränkt auf, das GCG Programmpaket, einschließlich GAP (Devereux, J., et al., Nucleic Acids Research 12 (12): 387 (1984); Genetics Computer Group University of Wisconsin, Madison, (WI)); BLASTP, BLASTN und FASTA (Altschul, S. et al., J. Mol. Biol. 215:403-410 (1990)). Das BLASTX Programm kann vom National Centre for Biotechnology Information (NCBI) und aus weiteren Quellen bezogen werden (BLAST Handbuch, Altschul S., et al., NCB NLM NIH Bethesda MD 20894; Altschul, S. et al., J. Mol. Biol. 215:403-410 (1990)). Auch der bekannte Smith Waterman-Algorithmus kann zur Bestimmung von Homologien verwendet werden.

Bevorzugte Parameter für den Nukleinsäuresequenz-Vergleich umfassen die nachstehenden:

| | |
|---|---|
| Algorithmus: | Needleman und Wunsch, J. Mol. Biol 48:443-453 (1970) |
| Vergleichsmatrix: | Übereinstimmung (matches) = + 10 Nichtübereinstimmung (mismatch) = 0 |
| Lücken-Wert (Gap Penalty): | 50 |
| Lückenlängen-Wert: | |
| (Gap Length Penalty): | 3 |

Das GAP-Programm ist auch zur Verwendung mit den vorstehenden Parametern geeignet. Die vorstehenden Parameter sind die Fehler-Parameter (default parameters) für Nukleinsäuresequenz-Vergleiche.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermittelte Übereinstimmung von 60 % wird im Rahmen dieser Anmeldung als 60 % Homologie bezeichnet. Entsprechendes gilt für höhere Homologiegrade.

In einer bevorzugten Ausführungsform ist die erfindungsgemäße DNA-Sequenz eine Kombination mehrerer der unter (a) bis (f) angegebenen DNA-Sequenzen und mindestens einer Intronsequenz, die durch dem Fachmann bekannte Fusion und gegebenenfalls Klonierung erhalten werden können. Diese Kombinationen sind von besonderem Interesse, da die davon kodierten Polypeptide besonders immunogen sind. Insbesondere sind Kombinationen bevorzugt, die mehrere oder alle Domänen in der in der Untereinheit natürlicherweise vorkommenden Reihenfolge (a bis h) aufweisen. Besonders bevorzugt sind dabei Ausführungsformen, in denen die für die Domänen kodierenden Nukleinsäuresequenzen direkt im Raster aneinandergekoppelt sind, nachdem die Intronsequenz bzw. die Intronsequenzen entfernt wurden.

Weiterhin werden Konstrukte bereitgestellt, die die erfindungsgemäßen Nukleinsäuremoleküle umfassen. In einer bevorzugten Ausführungsform umfasst das erfindungsgemäße Konstrukt einen zur Expression geeigneten Promotor, wobei die Nukleinsäuresequenz unter der Kontrolle des Promotors steht. Die Wahl des Promotors hängt vom zur Expression verwendeten Expressionssystem ab. Generell sind konstitutive Promotoren bevorzugt, jedoch sind auch induzierbare Promotoren wie z.B. der Metallothionein-Promotor möglich.

In einer weiterhin bevorzugten Ausführungsform umfasst das Konstrukt ferner eine Antigen-kodierende Nukleinsäuresequenz, die direkt mit der erfindungsgemäßen Hämocyanin-Nukleinsäure verbunden ist. Die Antigen-kodierende Sequenz kann sowohl 5' als auch 3' relativ zur Hämocyanin-Sequenz oder auch an beiden Enden gelegen sein. Sie schließt im gleichen Leseraster entweder unmittelbar an die Hämocyanin-Sequenz an oder ist durch einen Nukleinsäure-Linker unter Wahrung des Leserasters mit ihr verbunden. Durch die Fusion der Antigen-kodierenden Sequenz mit der Hämocyanin-Sequenz ist die Bildung eines Fusionsproteins beabsichtigt, in dem die Antigen-kodierende Sequenz kovalent mit der Hämocyanin-Sequenz verbunden ist. Das erfindungsgemäße Antigen ist hierbei ein medizinisch relevantes Antigen, das beispielsweise ausgewählt ist aus: Tumorantigenen, Virusantigenen und Antigenen bakterieller oder parasitärer Pathogene. Tumorantigene können hierbei beispielsweise Rb und p53 sein. Vorzugsweise stammen die Virusantigene aus immunologisch relevanten Viren, wie z.B. Influenza-Virus, Hepatitis-Virus und HIV. Pathogenantigene sind unter anderem solche aus Säugerpathogenen, insbesondere humanpathogenen Organismen, wie z.B. Plasmodium. Bakterielle Antigene können z.B. von *Klebsiella, Pseudomonas, E. coli, Vibrio cholerae, Chlamydia, Streptococcen* oder *Staphylococcen* stammen.

In einer weiteren bevorzugten Ausführungsform umfasst das Konstrukt ferner wenigstens ein Teil eines Vektors, insbesondere regulatorische Regionen, wobei der Vektor ausgewählt ist aus : Bacteriophagen wie λ-Derivaten, Adenoviren, Vacciniaviren, Baculoviren, SV40-Viren und Retroviren, vorzugsweise MoMuLV (Moloney Murine Leukemia Virus).

Ferner ist ein Konstrukt bevorzugt, das zusätzlich eine His-Tag-kodierende DNA-Sequenz umfasst, die bei Expression des Konstrukts zur Bildung eines Fusionsproteins mit einem His-Tag am NH₂-Terminus des Hämocyanins führt, welches die Aufreinigung des Proteins an einer Nickel-Säule durch Chelat-Bildung erleichtert.

Ferner stellt die Erfindung Wirtszellen bereit, die das Konstrukt enthalten und die zur Expression des Konstruktes geeignet sind. Im Stand der Technik sind zahlreiche prokaryontische und eukaryontische Expressionssysteme bekannt, wobei die Wirtszellen beispielsweise ausgewählt sind aus prokaryontischen Zellen wie *E. coli* oder *B. subtilis*, aus eukaryontischen Zellen wie Hefezellen, Pflanzenzellen, Insektenzellen und Säugerzellen, z.B. CHO-Zellen, COS-Zellen oder HeLa-Zellen, sowie Derivaten davon. Im Stand der Technik sind beispielsweise bestimmte CHO-Produktionslinien bekannt, deren Glykosylierungsmuster im Vergleich zu CHO-Zellen verändert sind. Die durch die Verwendung Glykosylierungs-defizienter oder Glykosylierungs-verringerter Wirtszellen erhaltenen Hämocyanine verfügen möglicherweise über zusätzliche Epitope, die bei vollständiger Glykosylierung ansonsten dem Immunsystem des Empfängers nicht zugänglich sind, so dass Hämocyanine mit verringerter Glykosylierung unter Umständen eine erhöhte Immunogenizität aufweisen. Aus mit dem erfindungsgemäßen Konstrukt transformierten Pflanzenzellen können transgene Pflanzen oder Pflanzenzellkulturen hergestellt werden, die Hämocyanin-Polypeptide produzieren, beispielsweise Tabak-, Kartoffel-, Tomaten-, Zuckerrüben-, Sojabohnen-, Kaffee-, Erbsen-, Bohnen-, Raps-, Baumwoll-, Reis- oder Maispflanzen bzw. -pflanzenzellkulturen.

Gegenstand der vorliegenden Erfindung ist weiter ein Verfahren zum Herstellen eines Hämocyanin-Polypeptids. Dazu wird das erfindungsgemäße Nukleinsäuremolekül und/oder das Konstrukt in einer geeigneten Wirtszelle exprimiert und das Protein aus der Wirtszelle oder dem Medium mittels üblicher Verfahren isoliert. Ausführungsformen der Erfindung sind
13. Verfahren zum Herstellen eines Hämocyanin-Polypeptides, wobei das Nukleinsäuremolekül gemäß einer der Ausführungsformen 1 bis 3 und/oder das Konstrukt gemäß einer der Ausführungsformen4 bis 9 in einer geeigneten Wirtszelle exprimiert wird und das Protein gegebenenfalls isoliert wird.
14. Verfahren gemäß Ausführungsform 13, **dadurch gekennzeichnet,** dass das hergestellte Hämocyanin-Polypeptid natürlich oder chemisch modifiziert wird.
15. Verfahren gemäß Ausführungsform 14, **dadurch gekennzeichnet,** dass die Modifikation eine Quervernetzung oder eine kovalente Bindung an ein Antigen ist.
16. Verfahren gemäß einer der Ausführungsformen 13 bis 15, **dadurch gekennzeichnet,** dass die Expression in einer Wirtszelle gemäß einer der Ausführungsformen10 bis 12 durchgeführt wird.

Dem Fachmann sind zahlreiche Verfahren zur Expression von DNA-Sequenzen bekannt; vergleiche Recombinant Gene Expression Protocols in Methods in Molecular Biology, Band 62, Humana Press Totowa New Jersey (1995). Die Expression kann sowohl konstitutiv als auch induzierbar sein, wobei Induktoren wie beispielsweise IPTG und Zn²⁺ dem Fachmann bekannt sind. Das hergestellte Hämocyanins kann, falls ein His-Tag an den NH₂-Terminus des Hämocyanin fusioniert wurde, durch Chelat-Bildung an einer Nickel-Säule aufgereinigt werden. Verfahren zum Aufreinigen von Hämocyanin, insbesondere KLH, finden sich in Harris et al., Micron 26 (1995), 201-212. Vorzugsweise wird das Hämocyanin durch lonenaustausch-Chromatographie und/oder Gelfiltrationschromatographie aufgereinigt Die Durchführung dieser Maßnahmen ist dem Fachmann bekannt.

In einer weiteren bevorzugten Ausführungsform wird das erfindungsgemäß hergestellte Hämocyanin modifiziert. Die Modifikationen umfassen hierbei die Di-, Oligo- und Polymerisierung des monomeren Ausgangsprodukts beispielsweise durch Quervernetzung, z.B. durch Dicyclohexylcarbodiimid oder Pegylierung oder Assoziation (self assembly). Die somit hergestellten Di-, Oligo- und Polymere können voneinander durch Gelfiltration abgetrennt werden. Insbesondere beabsichtigt ist die Bildung von Dekameren, Didekameren oder Multi-Dekameren. Weitere Modifikationen umfassen Seitenketten-Modifikationen, beispielsweise von ε-Amino-Lysin-Resten des Hämocyanins, oder Amino- bzw. Carboxyterminale Modifikationen. Besonders bevorzugt ist die Modifikation des Hämocyanins durch kovalente Bindung an ein Antigen, wobei das Antigen stöchiometrisch oder nicht-stöchiometrisch mit dem Hämocyanin umgesetzt sein kann. Das Antigen ist vorzugsweise ausgewählt aus Tumorantigenen, Virusantigenen und Pathogenenantigenen wie oben ausgeführt. Weitere Modifikationen umfassen posttranslationale Ereignisse, z.B. die Glykosylierung oder die partielle oder vollständige Deglykosylierung des Proteins.

In einer bevorzugten Ausführungsform ist das erhaltene Hämocyanin bei rekombinanter Expression in Prokaryonten oder Glykosylierungs-defizienten Eukaryonten nicht-glykosyliert. Ebenfalls in Betracht gezogen wird erfindungsgemäß Hämocyanin, das durch rekombinante Expression in zur Glykosylierung fähigen Eukaryonten wie Hefezellen, Pflanzenzellen, Insektenzellen oder Säugerzellen, wie CHO-Zellen oder HeLa-Zellen, glykosyliert ist.

In einer weiteren Ausführungsform werden Hämocyanin-Polypeptide zur Verfügung gestellt, die eine Aminosäuresequenz umfassen, wobei die Aminosäuresequenz von einer oder mehreren der erfindungsgemäßen Nukleinsäuremoleküle kodiert wird:
17. Hämocyanin-Polypeptid, umfassend eine Aminosäuresequenz, die von einem oder mehreren der Nukleinsäuremoleküle nach einer der Ausführungsformen1 bis 3 kodiert wird.
18. Hämocyanin-Polypeptid gemäß Ausführungsform 17, umfassend wenigstens eine aus der folgenden Gruppe ausgewählte Aminosäuresequenz:
   SEQ ID NO:33 (partielle HtH2 Domäne b),
   SEQ ID NO:34 (HtH2 Domäne c),
   SEQ ID NO:35 (HtH2 Domäne d),
   SEQ ID NO:36 (HtH2 Domäne e),
   SEQ ID NO:37 (HtH2 Domäne f),
   SEQ ID NO:38 (HtH2 Domäne g),
   SEQ ID NO:39 (HtH2 Domäne h),
   SEQ ID NO:44 (KLH2 Domäne b),
   SEQ ID NO:45 (KLH2 Domäne c),
   SEQ ID NO:46 (partielle KLH2 Domäne d),
   SEQ ID NO:47 (KLH2 Domäne g),
   SEQ ID NO:48 (partielle KLH2 Domäne h),
   SEQ ID NO:65 (partielle HtH2 Domäne a),
   SEQ ID NO:156 (vollständige HtH2 Domäne a),
   SEQ ID NO:66 (HtH2 Domäne b'),
   SEQ ID NO:67 (HtH2 Domäne d'),
   SEQ ID NO:68 (HtH2 Domäne e'),
   SEQ ID NO:74 (KLH2 Domäne b'),
   SEQ ID NO:75 (KLH2 Domäne c'),
   SEQ ID NO:76 (KLH2 Domäne d'),
   SEQ ID NO:77 (KLH2 Domäne e),
   SEQ ID NO:78 (KLH2 Domäne f),
   SEQ ID NO:79 (KLH2 Domäne g'),
   SEQ ID NO:158 (teilweise KLH2 Domäne h),
   oder
   (b) ein Hämocyanin-Polypeptid, dessen Sequenz über einen Teilbereich von mindestens 90 Aminosäuren mindestens 60% identisch mit einer der Aminosäuresequenzen von (a) ist.
19. Rekombinantes Hämocyanin-Polypeptid, erhältlich durch das Verfahren gemäß einer der Ausführungsformen13 bis 16 oder Modifikationen davon, wobei die Modifikationen
   a) Di-, Oligo- und Polymere des durch das Verfahren gemäß einer der Ausführungsformen13 bis 16 erhaltenen Hämocyanin-Polypeptids sind,
   b) Seitenkettenmodifikationen davon,
   c) Fusionsproteine, erhalten durch Binden an ein Antigen oder
   d) Polypeptide, erhalten durch posttranslationale Ereignisse.
20. Rekombinantes Hämocyanin-Polypeptid gemäß Ausführungsform 18, **dadurch gekennzeichnet**, dass das Hämocyanin-Polypeptid entweder die Sequenzen SEQ ID NO: 44 bis 48 oder die Sequenzen SEQ ID NO:44 bis 46, 77, 78, 47, 48 umfasst und KLH2 aus *Megathura crenulata* ist, wobei jeweils die Sequenz mit SEQ ID NO:44 durch SEQ ID NO:74, SEQ ID NO:45 durch SEQ ID NO:75, SEQ ID NO:46 durch SEQ ID NO:76 und/oder SEQ ID NO:47 durch SEQ ID NO:79 ersetzt sein kann.
21. Rekombinantes Hämocyanin-Polypeptid gemäß Ausführungsform 17 bis 20, **dadurch gekennzeichnet**, dass es kovalent an Viren, Virenbestandteile, Bakterien, Bakterienbestandteile, DNA, DNA-Bestandteile, anorganische oder organische Moleküle wie z. B. Kohlenhydrate Peptide und/oder Glykoproteine gebunden ist.
22. Rekombinantes Hämocyanin-Polypeptid gemäß einer der Ausführungsformen17 bis 21, **dadurch gekennzeichnet**, dass das Hämocyanin-Polypeptid nicht-glykosyliert ist.
23. Rekombinantes Hämocyanin-Polypeptid gemäß einer der Ausführungsformen 17 bis 21, **dadurch gekennzeichnet**, dass das Hämocyanin-Polypeptid glykosyliert ist.
24. Pharmazeutische Zusammensetzung, enthaltend ein Nukleinsäuremolekül gemäß einer der Ausführungsformen 1 bis 3 und/oder ein Konstrukt gemäß einer der Ausführungsformen4 bis 9 und physiologisch verträgliche Zusatzmittel.
25. Pharmazeutische Zusammensetzung gemäß Ausführungsform 24, **dadurch gekennzeichnet**, dass sie zur gentherapeutischen Behandlung von Tumoren verwendet wird.
26. Pharmazeutische Zusammensetzung, enthaltend ein Hämocyanin-Polypeptid nach einer der Ausführungsformen 17 bis 23 und physiologisch verträgliche Zusatzmittel.
27. Pharmazeutische Zusammensetzung gemäß Ausführungsform 26, **dadurch gekennzeichnet**, dass sie als Antiparasitenmittel, Antivirusmittel oder als Antitumormittel verwendet wird.
28. Pharmazeutische Zusammensetzung nach Ausführungsform 26, **dadurch gekennzeichnet**, dass sie zum Behandeln einer der folgenden Erkrankungen verwendet wird: Schistosomiasis, Bluthochdruck, Oberflächen-Hamblasenkarzinomen, Epithelkarzinomen, Ovarialkarzinom, Mammakarzinom, Bronchialkarzinom und Kolonrektalkarzinom.
29. Pharmazeutische Zusammensetzung nach Ausführungsform 24, **dadurch gekennzeichnet**, dass sie als Impfstoff verwendet wird.
30. Pharmazeutische Zusammensetzung gemäß Ausführungsform 25, **dadurch gekennzeichnet**, dass sie zur Kokain-Missbrauchsvorsorge verwendet wird.
31. Verwendung von Hämocyanin-Polypeptid gemäß einer der Ausführungsformen 17 bis 23 als Trägerstoff für Arzneimittel.
32. Liposom, umfassend ein Nukleinsäuremolekül gemäß einer der Ausführungsformen 1 bis 3, ein Konstrukt gemäß einer der Ausführungsformen4 bis 9 und/oder ein Hämocyanin-Polypeptid gemäß einer der Ausführungsformen17 bis 23.
33. Liposom gemäß Ausführungsform 32, **dadurch gekennzeichnet**, dass das Liposom weiterhin Zellerkennungsmoleküle umfasst.
34. Antikörper, erhältlich durch Immunisieren eines Versuchstieres mit einem rekombinanten Hämocyanin-Polypeptid, das aus wenigstens einer der aus der folgendenden Aminosäuresequenzen ausgewählt ist:
   SEQ ID NO:33 (partielle HtH2 Domäne b),
   SEQ ID NO:34 (HtH2 Domäne c),
   SEQ ID NO:35 (HtH2 Domäne d),
   SEQ ID NO:36 (HtH2 Domäne e),
   SEQ ID NO:37 (HtH2 Domäne f),
   SEQ ID NO:38 (HtH2 Domäne g),
   SEQ ID NO:39 (HtH2 Domäne h),
   SEQ ID NO:44 (KLH2 Domäne b),
   SEQ ID NO:45 (KLH2 Domäne c),
   SEQ ID NO:46 (partielle KLH2 Domäne d),
   SEQ ID NO:47 (KLH2 Domäne g),
   SEQ ID NO:48 (partielle KLH2 Domäne h),
   SEQ ID NO:65 (partielle HtH2 Domäne a),
   SEQ ID NO:156 (vollständige HtH2 Domäne a),
   SEQ ID NO:66 (HtH2 Domäne b'),
   SEQ ID NO:67 (HtH2 Domäne d'),
   SEQ ID NO:68 (HtH2 Domäne e'),
   SEQ ID NO:74 (KLH2 Domäne b'),
   SEQ ID NO:75 (KLH2 Domäne c'),
   SEQ ID NO:76 (KLH2 Domäne d'),
   SEQ ID NO:77 (KLH2 Domäne e),
   SEQ ID NO:78 (KLH2 Domäne f),
   SEQ ID NO:79 (KLH2 Domäne g'),
   SEQ ID NO:158 (teilweise KLH2 Domäne h).
35. Screening-Verfahren zum Identifizieren von Tumor-spezifischer DNA in einer Zelle umfassend:
   a) das Inkontaktbringen zellulärer DNA und/oder zellulären Proteins mit einer Sonde umfassend die Nukleinsäuresequenz gemäß einer der Ausführungsformen 1 bis 3 und/oder des Antikörpers gemäß Ausführungsform 34 und
   b) das Nachweisen der spezifischen Bindung.
36. Screening-Verfahren gemäß Ausführungsform 35, **dadurch gekennzeichnet**, dass der nachzuweisende Tumor Harnblasenkarzinom, Epithelialkarzinom, Ovarialkarzinom, Mammakarzinom, Bronchialkarzinom oder Kolonrektalkarzinom ist.

Hämocyanin-Polypeptide, deren Sequenz über einen Teilbereich von mindestens 90 Aminosäuren mindestens 60% oder 70%, vorzugsweise mindestens 80%, besonders bevorzugt mindestens 90% oder 95% Homologie zu einer der Aminosäuresequenzen gemäß SEO ID NO:33 bis 48 und SEQ ID NO:63 bis 79 aufweist, werden von der Erfindung ebenfalls umfasst.

In diesem Zusammenhang bezieht sich der Ausdruck "mindestens 70%, vorzugsweise mindestens 80%, besonders bevorzugt mindestens 90% Homologie" auf Übereinstimmung auf der Ebene der Aminosäuresequenz, die gemäß bekannter Verfahren, z.B. der computergestützten Sequenzvergleiche (Basic local alignment search tool, S.F. Altschul et al., J. Mol. Biol. 215 (1990). 403-410) bestimmt werden kann.

Der dem Fachmann bekannte Ausdruck "Homologie" bezeichnet hier den Grad der Verwandtschaft zwischen zwei oder mehrerer Polypeptid-Molekülen, der durch die Übereinstimmung zwischen den Sequenzen bestimmt wird, wobei unter Übereinstimmung sowohl identische Übereinstimmung als auch konservativer Aminosäure-Austausch zu verstehen ist. Der Prozentsatz der "Homologie" ergibt sich aus dem Prozentsatz übereinstimmender Bereiche in zwei oder mehr Sequenzen unter Berücksichtigung von Lücken oder anderen Sequenzbesonderheiten.

Der Begriff "konservativer Aminosäure-Austausch" bezieht sich auf einen Austausch eines Aminosäurerestes durch einen anderen Aminosäurerest, wobei der Austausch nicht zu einer Änderung der Polarität oder Ladung führt. Ein Beispiel für einen konservativen Aminosäure-Austausch ist der Austausch eines unpolaren Aminosäurerestes durch einen anderen unpolaren Aminosäurerest.

Die Homologie miteinander verwandter Polypeptid-Moleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, sind jedoch nicht eingeschränkt auf, das GCG-Programmpaket, einschließlich GAP (Devereux, J., et al., Nucleic Acids Research 12 (12): 387 (1984); Genetics Computer Group University of Wisconsin, Madison, (WI)); BLASTP, BLASTN und FASTA (Altschul, S. et al., J. Molec Biol 215:403/410 (1990)). Das BLAST X Programm kann vom National Centre for Biotechnology Information (NCBI) und aus weiteren Quellen bezogen werden (BLAST Handbuch, Altschul S., *et al.*, NCB NLM NIH Bethesda MD 20894; Altschul, S., et al., J. Mol. 215:403/410 (1990)). Auch der bekannte Smith Waterman-Algorithmus kann zur Bestimmung von Homologie verwendet werden.

Bevorzugte Parameter für den Sequenz-Vergleich umfassen die nachstehenden:

| | |
|---|---|
| Algorithmus: | Needleman und Wunsch, J. Mol. Biol 48:443-453 (1970) |
| Vergleichsmatrix: | BLOSUM 62 von Henikoff und Henikoff, Proc. Natl. Acad. Sci. USA 89:10915-10919 (1992) |
| Lücken-Wert (Gap Penalty): | 12 |
| Lückenlängen-Wert | |
| (Gap Length Penalty): | 4 |
| Schwellenwert der Ähnlichkeit: | 0 |

Das GAP-Programm ist auch zur Verwendung mit den vorstehenden Parametern geeignet. Die vorstehenden Parameter sind die Standardparameter (default parameters) für Aminosäuresequenz-Vergleiche, wobei Lücken an den Enden den Homologie-Wert nicht verringern. Bei sehr kurzen Sequenzen im Vergleich zur Referenzsequenz kann es weiterhin notwendig sein, den Erwartungswert auf bis zu 100.000 (expected value) zu erhöhen und gegebenenfalls die Wortlänge (word size) auf bis zu 2 zu verkleinern.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermittelte Übereinstimmung von 60 % wird im Rahmen dieser Anmeldung als 60 % Homologie bezeichnet. Entsprechendes gilt für höhere Homologiegrade.

In einer weiteren Ausführungsform stellt die Erfindung Hämocyanin-Polypeptide, erhältlich durch das rekombinante Herstellungsverfahren oder Modifikationen davon, bereit.

Bevorzugt sind Hämocyanin-Polypeptide, der oben dargestellten Ausführungsformen, die jede der Sequenzen SEQ ID NO: 25 bis 32 umfassen, wobei die Sequenz mit SEQ ID NO:25 durch SEQ ID NO:63 und/oder SEQ ID NO:32 durch SEQ ID NO:64 ersetzt sein kann. Bevorzugt sind ebenfalls Hämocyanin-Polypeptide, die entweder die Sequenzen SEQ ID NO: 33 bis 39 oder die Sequenzen SEQ ID NO:65, 66, 34-39 umfassen, wobei SEQ ID NO:35 durch SEQ ID NO:67 und/oder SEQ ID NO:36 durch SEQ ID NO:68 ersetzt sein kann. Besonders bevorzugt handelt es sich bei diesen Hämocyanin-Polypeptiden um Hämocyanin 2 aus *Haliotis tuberculata.*

Weiterhin ist insbesondere Hämocyanin 2 aus *Haliotis tuberculata* bevorzugt, das ein scheinbares Molekulargewicht von 370 kDa in SDS-PAGE PAGE unter reduzierenden Bedingungen aufweist. Die Hämocyanine sind durch das in den Beispielen beschriebene selektive Dissoziationsverfahren aus Gesamt-Hämocyanin aus *Haliotis tuberculata* erhältlich.

Weiterhin bevorzugt sind Hämocyanin-Polypeptide, der ober dargestellten Ausführungsformen, die jede der Sequenzen SEQ ID NO: 40 bis 43 oder die Sequenzen SEQ ID NO:40 bis 43 und SEQ ID NO:71 bis 73 umfassen, wobei jeweils die Sequenz mit SEQ ID NO:40 durch SEQ ID NO:66 und/oder SEQ ID NO:43 durch SEQ ID NO:70 ersetzt sein kann. Bevorzugt sind ebenfalls Hämocyanin-Polypeptide, die entweder jede der Sequenzen SEQ ID NO: 44 bis 48 oder die Sequenzen SEQ ID NO:44 bis 46, 77, 78, 47, 48 umfassen, wobei jeweils die Sequenz mit SEQ ID NO:44 durch SEQ ID NO:74, SEQ ID NO:45 durch SEQ ID NO:75, SEQ ID NO:46 durch SEQ ID NO:76 und/oder SEQ ID NO:47 durch SEQ ID NO:79 ersetzt sein kann.

Besonders bevorzugt handelt es sich bei diesen Hämocyanin-Polypeptiden um vollständiges Hämocyanin 2 (KLH2) aus *Megathura crenulata.*

Weiterhin wird nicht-glykosyliertes und glykosyliertes Hämocyanin-Polypeptid, erhältlich durch Expression in zur Glykosylierung fähigen bzw. unfähigen Wirtszellen, bereitgestellt. Je nach vorgesehener Verwendung des Hämocyanin-Polypeptids kann das Glykosylierungsmuster von Hefe, inbesondere methylotropher Hefe, von Pflanzenzellen oder von COS- oder HeLa-Zellen bevorzugt sein.

Die Erfindung betrifft weiterhin pharmazeutische Zusammensetzungen, die die erfindungsgemäßen Nukleinsäuremoleküle und physiologisch verträgliche Zusatzstoffe, die im Stand der Technik bekannt sind, enthalten. Vorzugsweise werden die pharmazeutischen Zusammensetzungen zur unspezifischen Immunstimulierung in Form einer Gentherapie eingesetzt, wobei nach Transformation mit einem geeigneten Vektor Hämocyanin-Polypeptide exprimiert werden und zur Antigenisierung des Gewebes dienen.

Insbesondere sieht die vorliegende Erfindung die Verwendung eines erfindungsgemäßen Nukleinsäuremoleküls, das mit einer Antigen-kodierenden DNA-Sequenz verbunden ist, zur spezifischen Immunisierung gegen dieses Antigen vor. Die Immunisierung beruht hierbei, ohne an diese Theorie gebunden zu sein, auf der unspezifischen Stimmulierung des Immunsystems durch Hämocyanin-Polypeptid-Epitope und die weitergehende spezifische Immunisierung durch Erkennung von Antigen-Epitopen durch das Immunsystem.

Eine solche Immunisierung ist besonders wertvoll im Hinblick auf Pathogen-Antigene, ganz besonders aber im Hinblick auf Tumorantigene. Die Anwendbarkeit der erfindungsgemäßen pharmazeutischen Zusammensetzung zur Behandlung von Tumorerkrankungen ergibt sich auch aus der Kreuzreaktivität der gebildeten Hämocyanin-spezifischen Antikörper mit Kohlenhydratresten, die auf der Oberfläche von Tumoren auftreten, wie z.B. dem Thomsen-Friedenreich-Antigen, das bei der Mehrzahl von humanen Tumoren wie Epithelialkarzinomen, Ovarialkarzinom, Kolonrektalkarzinom, Mammakarzinom, Bronchialkarzinom und Hamblasenkarzinom auftritt.

Weiterhin können die erfindungsgemäßen pharmazeutischen Zusammensetzungen zum Behandeln von parasitären Erkrankungen wie Schistosomiasis und für die Kokain-Mißbrauchsvorsorge eingesetzt werden.

Als weitere Ausführungsform der vorliegenden Erfindung werden pharmazeutische Zusammensetzungen zur Verfügung gestellt, die ein erfindungsgemäßes Hämocyanin-Polypeptid in Verbindung mit einem oder mehreren physiologisch verträglichen Zusatzmitteln enthalten. Wie oben bereits erwähnt, kann ein solches Hämocyanin-Poly-peptid aus einer vollständigen Hämocyanin-Untereinheit, aus einer oder mehreren Domänen sowie aus einem oder mehreren Fragmenten solcher Domänen bestehen, vorausgesetzt, dass diese Fragmente noch die immunologischen Eigenschaften wenigstens einer Domäne eines Hämocyanins aufweist. Eine solche pharmazeutische Zusammensetzung eignet sich durch die entweder unspezifische Immunstimulation, die allein auf das Hämocyanin zurückzuführen ist, oder durch die spezifische Immunreaktion auf mit dem Hämocyanin assoziierte Antigene z.B. als Antiparasitenmittel, Antivirusmittel oder Antitumormittel. So kann sie z.B. zum Behandeln von Schistosomiasis, Epithelialkarzinomen, Ovarialkarzinorn, Kolonrektalkarzinom, Mammakarzinom, Bronchialkarzinom und Hamblasenkarzinomen eingesetzt werden, eignet sich jedoch auch zum Behandeln von Bluthochdruck. Die Behandlung von Bluthochdruck wird erreicht, indem eine Immunisierung mit Hilfe von erfindungsgemäßen Hämocyanin-β-adrenergen-Rezeptorpeptid-Konstrukten und/oder Fusionsproteinen durchgeführt wird.

In einer weiteren Ausführungsform werden die erfindungsgemäßen pharmazeutischen Zusammensetzungen als Impfstoffe verwendet. Sie können somit einen wertvollen Beitrag zur Prophylaxe von durch bekannte Pathogene verursachte Erkrankungen leisten. Dies gilt insbesondere für pharmazeutische Zusammensetzungen, in denen ein Hämocyanin-Polypeptid an ein Virus, Virusbestandteil, abgetötete Bakterien, Bakterienbestandteile, insbesondere Oberflächenproteine aus Virus- oder Bakterienhüllen, DNA, DNA-Bestandteile, anorganische oder organische Moleküle, z.B. Kohlenhydrate, Peptide und/oder Glykoproteine gebunden ist.

Gemäß einer weiteren bevorzugten Ausführungsform wird die erfindungsgemäße pharmazeutische Zusammensetzung zur Kokain-Mißbrauchsvorsorge verwendet.

Zur Applikation sowohl der erfindungsgemäßen Nukleinsäuremoleküle als auch der Hämocyanin-Polypeptide eignen sich insbesondere Liposomen. Dementsprechend umfasst die vorliegende Erfindung Liposomen, die ein erfindungsgemäßes Nukleinsäuremolekül, ein erfindungsgemäßes Konstrukt oder ein erfindungsgemäßes Hämocyanin-Polypeptid umfassen.

Dem Fachmann sind verschiedene Verfahren zum Herstellen von Liposomen, die für pharmazeutische Zwecke verwendbar sind, bekannt. Die Selektivität der die erfindungsgemäßen Nukleinsäuremoleküle oder Hämocyanin-Polypeptide enthaltenden Liposomen kann durch den zusätzlichen Einbau von Zellerkennungsmolekülen in die Liposomen erhöht werden, die selektiv an Zielzellen binden. Hierzu eignen sich insbesondere Rezeptorliganden, die an Rezeptoren der Zielzellen binden, oder, besonders im Fall von Tumoren, Antikörper, die gegen Oberflächenantigene der jeweils anvisierten Zielzellen gerichtet sind.

Die erfindungsgemäßen Hämocyanin-Polypeptide sind außerdem als Trägermolekül für Arzneistoffe, wie z. B. Cytostatika, vorgesehen. Die Vergrößerung des Molekulargewichtes verlängert die physiologische Halbwertszeit der Arzneistoffe erheblich, da der Verlust durch Ultrafiltration in der Niere deutlich verringert ist.

Die Zubereitung der Impfstoffe erfolgt nach dem Fachmann bekannten Verfahren; in einigen Ausführungsformen ist die zusätzliche Verwendung von Adjuvanzien wie z. B. Freundsches Adjuvanz oder Polysacchariden vorgesehen.

Die Erfindung stellt ferner Antikörper bereit, die spezifisch mit dem erfindungsgemäßen Hämocyanin-Polypeptid reagieren und erhältlich sind durch Immunisieren eines Versuchstieres mit einem Hämocyanin-Polypeptid. Polyklonale Antikörper können durch Immunisieren beispielsweise von Kaninchen und anschließendem Gewinnen von Antiseren erhalten werden. Monoklonale Antikörper können gemäß Standardverfahren durch Immunisieren von z.B. Mäusen, Gewinnen und Immortalisieren der Milzzellen und Klonieren der Hybridome, die für Hämocyanin spezifische Antikörper produzieren, erhalten werden.

Weiterhin wird ein Screening-Verfahren zum Identifizieren von Tumor-spezifischer DNA in einer Zelle bereitgestellt, das die Schritte umfasst:
a) das Inkontaktbringen zellulärer DNA und/oder zellulären Proteins mit einer Sonde umfassend das erfindungsgemäße Nukleinsäuremolekül und/oder den erfindungsgemäßen Antikörper und
b) das Nachweisen der spezifischen Bindung.

Vorzugsweise ist der nachzuweisende Tumor ein Harnblasenkarzinom, Epithelialkarzinom, Ovarialkarzinom, Mammakarzinom, Bronchialkarzinom oder Kolonrektalkarzinom.

Es ist beabsichtigt, mit den nachfolgenden Figuren und Beispielen die Erfindung zu erläutern, diese jedoch in keiner Weise einzuschränken. Dem Fachmann sind aufgrund der Beschreibung und der Beispiele weitere Ausführungsformen zugänglich, die ebenfalls umfasst sind.

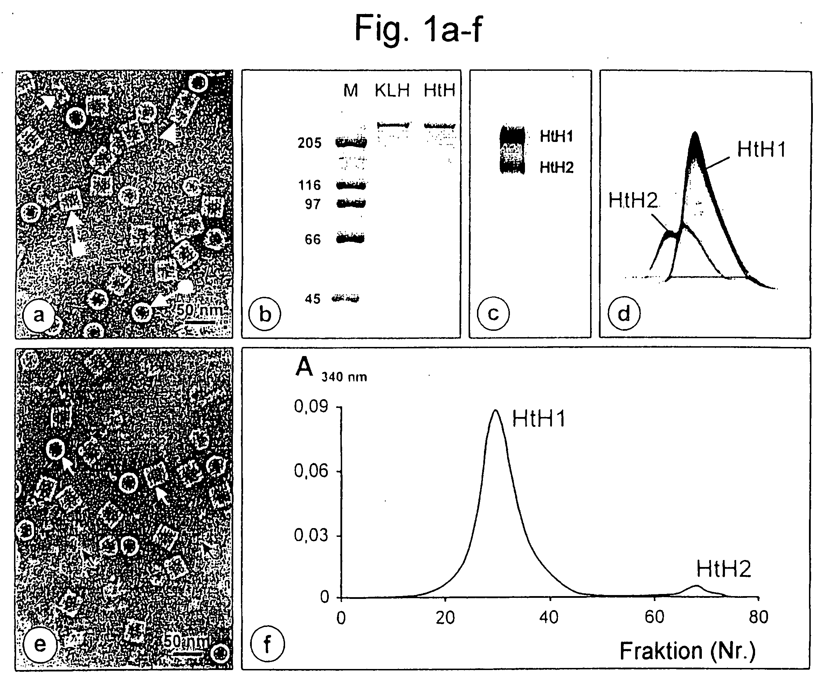

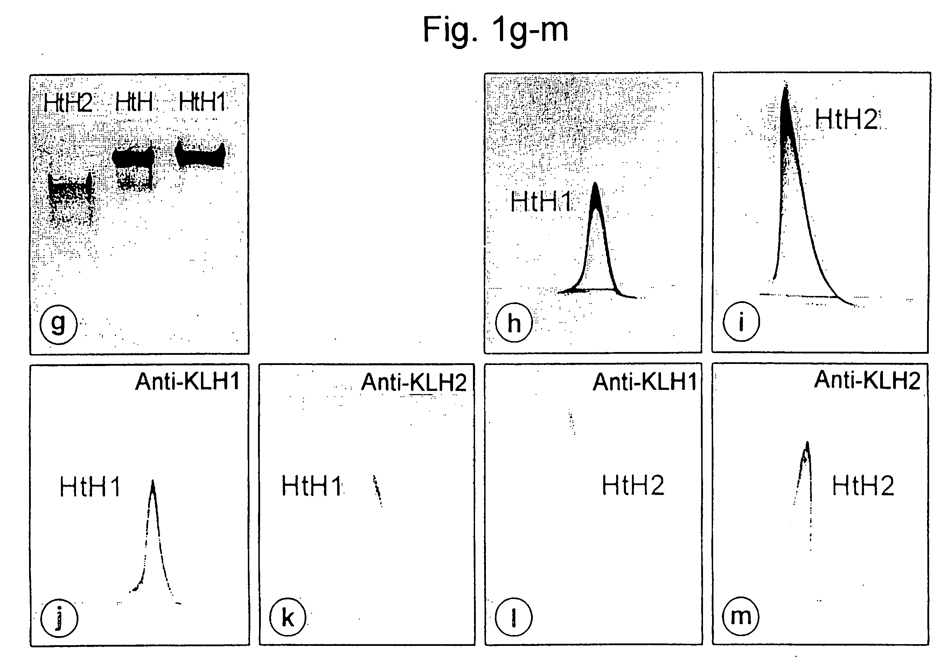

Fig. 1 zeigt die Charakterisierung und Aufreinigung von *Haliotis tuberculata* Hämocyanin (HtH):
(a) Elektronenmikroskopie von negativ gefärbtem Gesamt-HtH, das durch UItrazentrifugation von Zell-freier Hämolymphe aufgereinigt wurde;
(b) SDS-Polyacryamid-Gelelektrophorese (7,5 % Polyacrylamid) von HtH1 im Vergleich zu KLH (MW 370 kDa);
(c) Native Polyacrylamid-Gelektrophorese (5 % Polyacrylamid) der HtH-Untereinheiten-Präparation, wobei die Anode am unteren Rand liegt;
(d) Gekreuzte Immunektrophorese der beiden HtH-Untereinheiten unter Verwendung von anti-HtH-Antikörpem aus Kaninchen;
(e) Elektronenmikroskopie der verbleibenden HtH1-Didekamere (weiße Pfeile) nach der selektiven Dissoziation von HtH2 (schwarze Pfeile);
(f) Elutionsprofil der Gelfiltrationschromatographie (Biogel A15m) in Gegenwart von Ammoniummolybdat/Polyethylenglykol-Lösung (pH 5,9) nach der selektiven Dissoziation von HtH2 in seine Untereineinheit und nachfolgender Anreicherung von HtH1 durch Ultrazentrifugation;
(g) Native Polyacrylamid-Gelelektrophorese (6,5 % Polyacrylamid) von durch Gelchromatographie aufgereinigten HtH1- und HtH2-Untereinheiten im Vergleich zum Ausgangsmaterial;
(h,i) Gekreuzte Immunelektrophorese von chromatographisch aufgereinigten HtH-Untereinheiten; und
(j,m) Gekreuzte Immunelektrophorese der aufgereinigten HtH-Untereinheiten unter Verwendung von anti-KLH-Antikörpern aus Kaninchen, die für KLH1 bzw. KLH2 spezifisch sind.

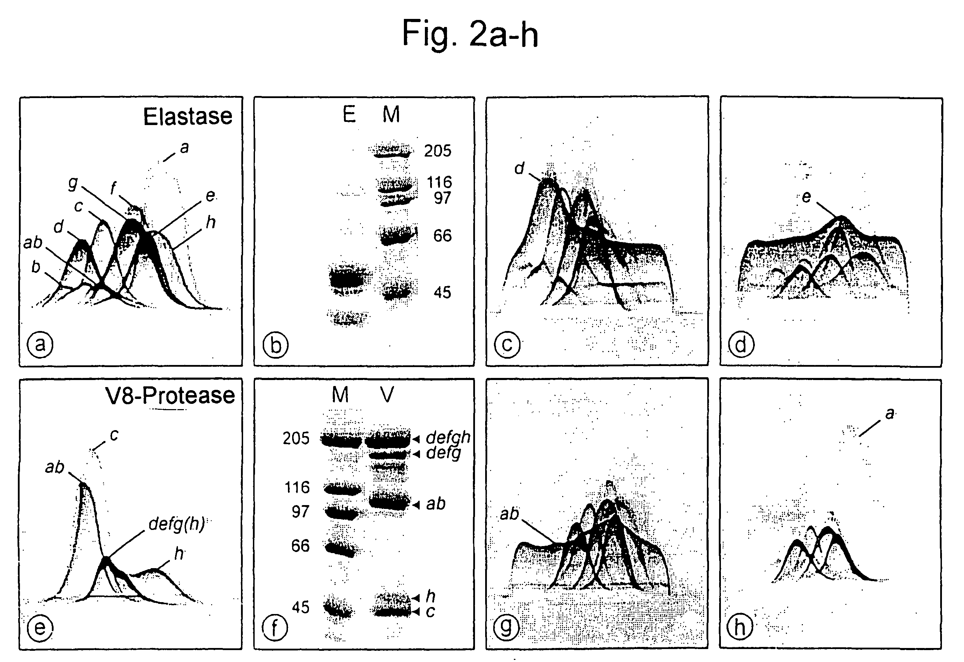

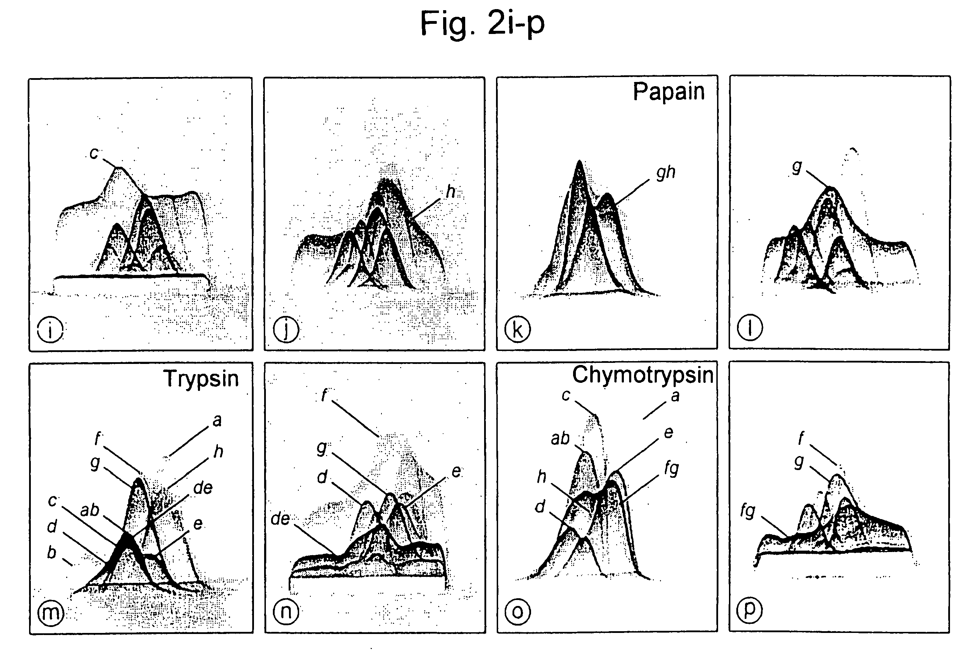

Fig. 2 zeigt die Untersuchung der Untereinheiten-Organisation von HtH1, wobei für die Immunelektrophorese anti-HtH1-Antikörper aus Kaninchen verwendet wurden und die Anode sich auf der linken Seite befand:
(a) Gekreuzte Immunelelektrophorese nach limitierter Proteolyse von HtH1 mit Hilfe von Elastase;
(b) SDS-Polyacrylamid-Gelelektrophorese (7,5 % Polyacrylamid) von Elastasegespaltener HtH1-Untereinheit;
(c,d,g-j,l,n,p) Gekreuzte Immunelektrophorese der Elastase-Spaltprodukte der HtH1-Untereinheit;
(e) Gekreuzte Immunelelektrophorese nach limitierter Proteolyse von HtH1 mit Hilfe von V8 Protease;
(f) SDS-Polyacrylamid-Gelelektrophorese (7,5 % Polyacrylamid) von V8 Protease-gespaltener HtH1-Untereinheit und
(k,m,o) Gekreuzte Immunelelektrophorese nach limitierter Proteolyse von HtH1 mit Hilfe der drei angegebenen Proteasen.

Fig. 3 zeigt die Auftrennung von proteolytischen Spaltprodukten der Untereinheit HtH1 mit Hilfe von HPLC.

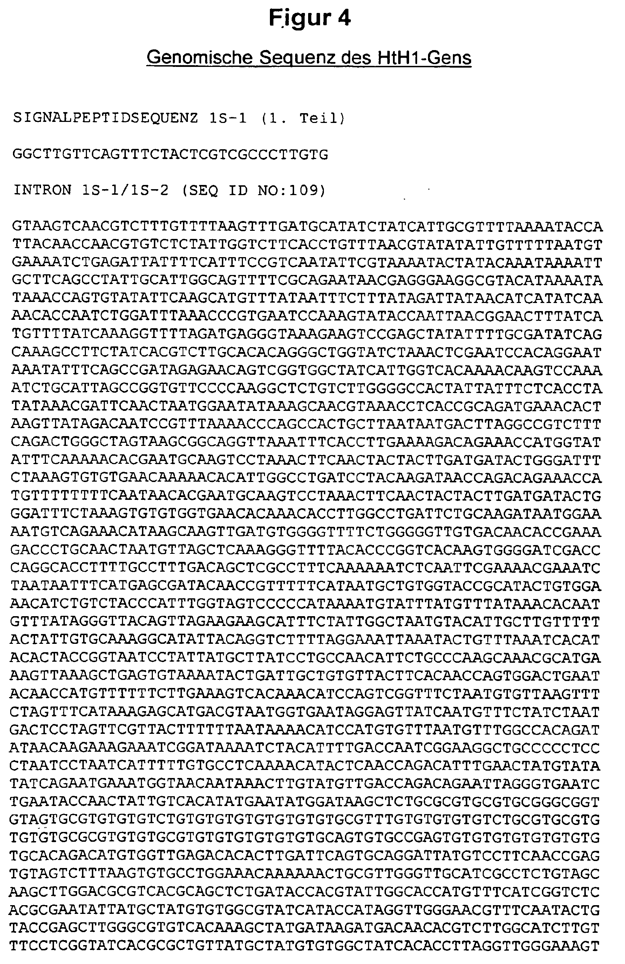

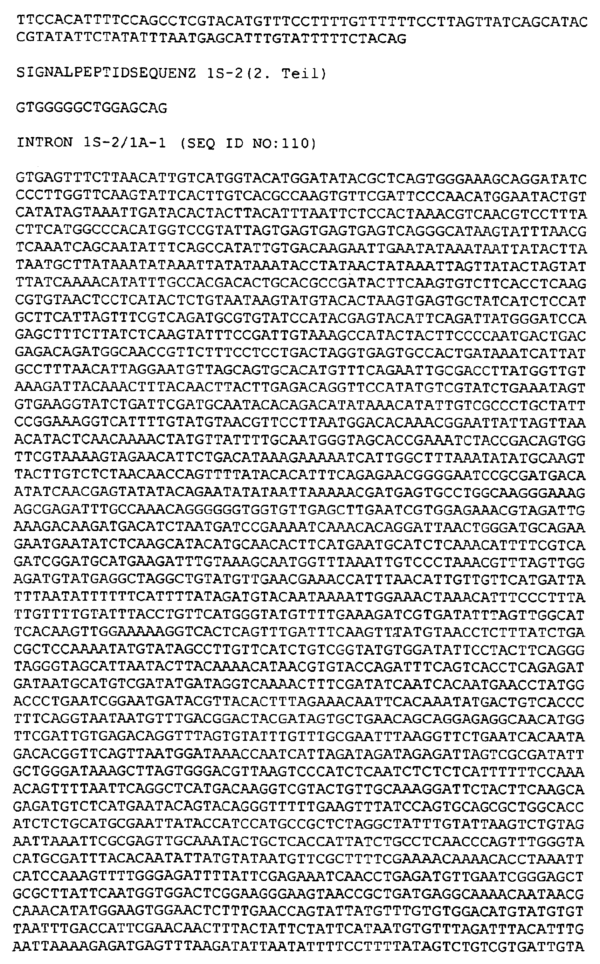

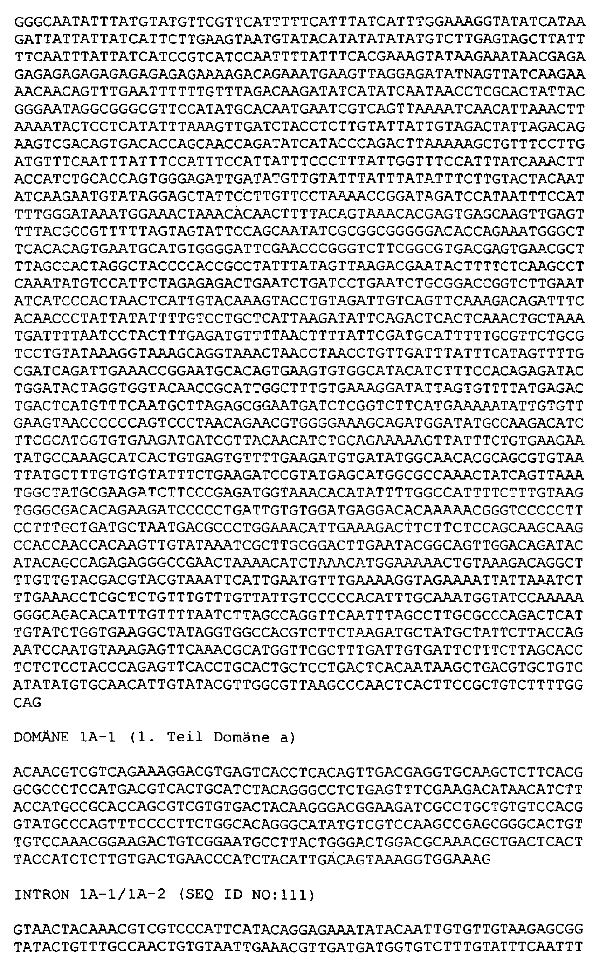

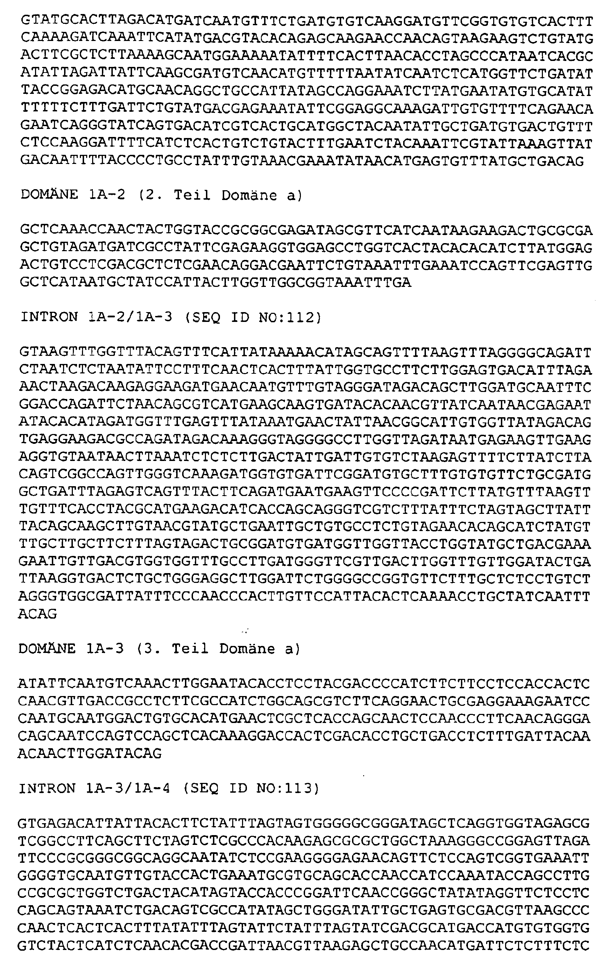

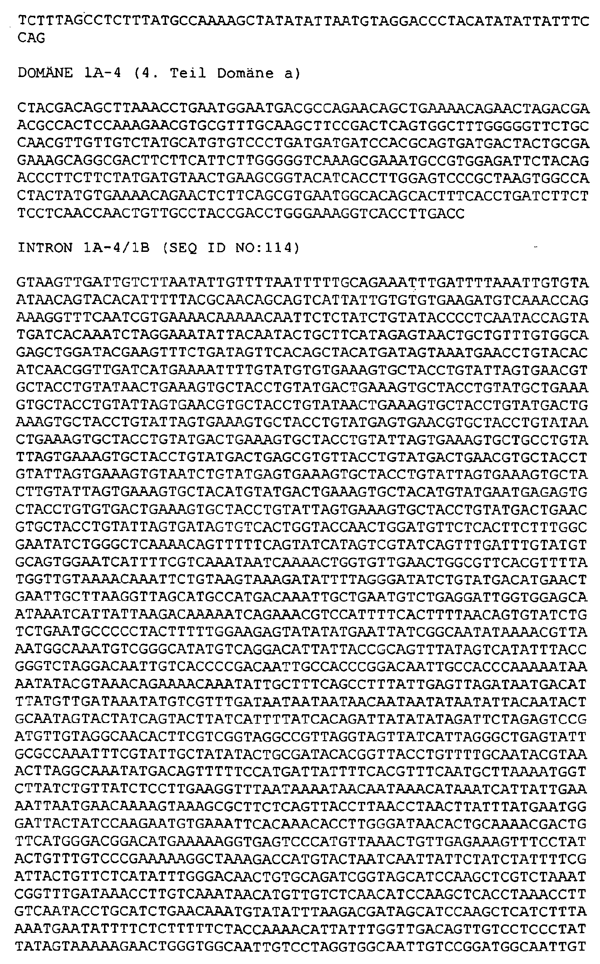

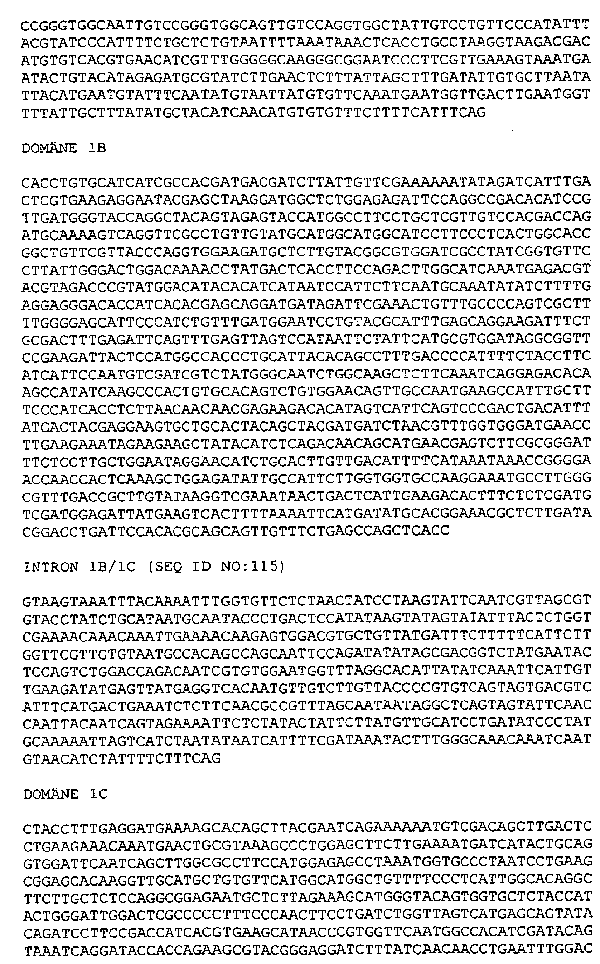

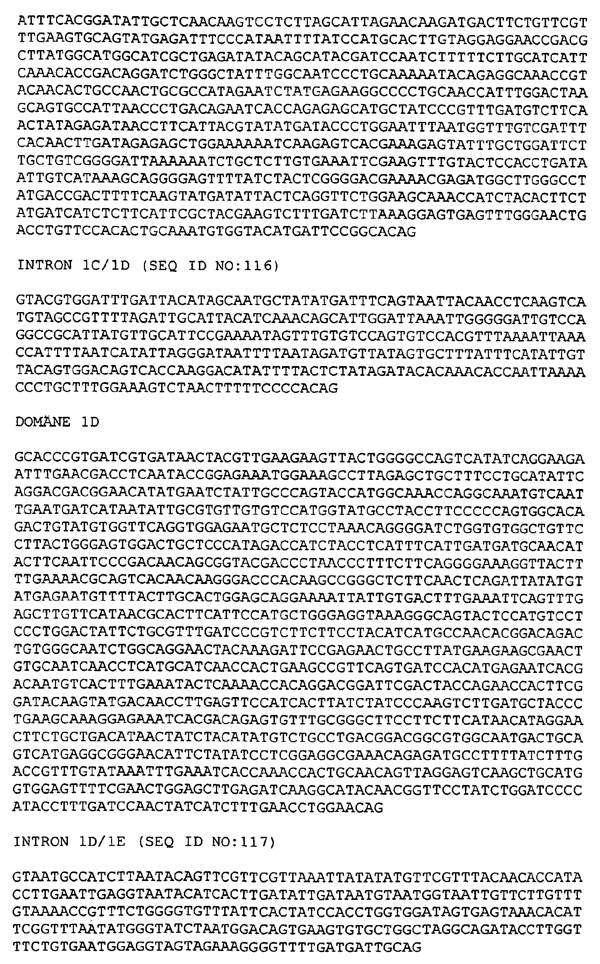

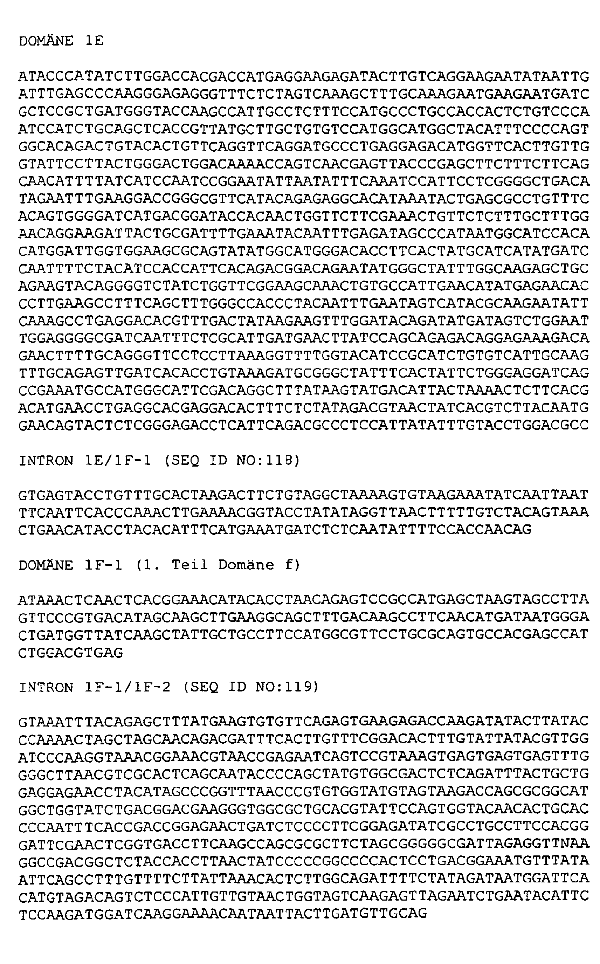

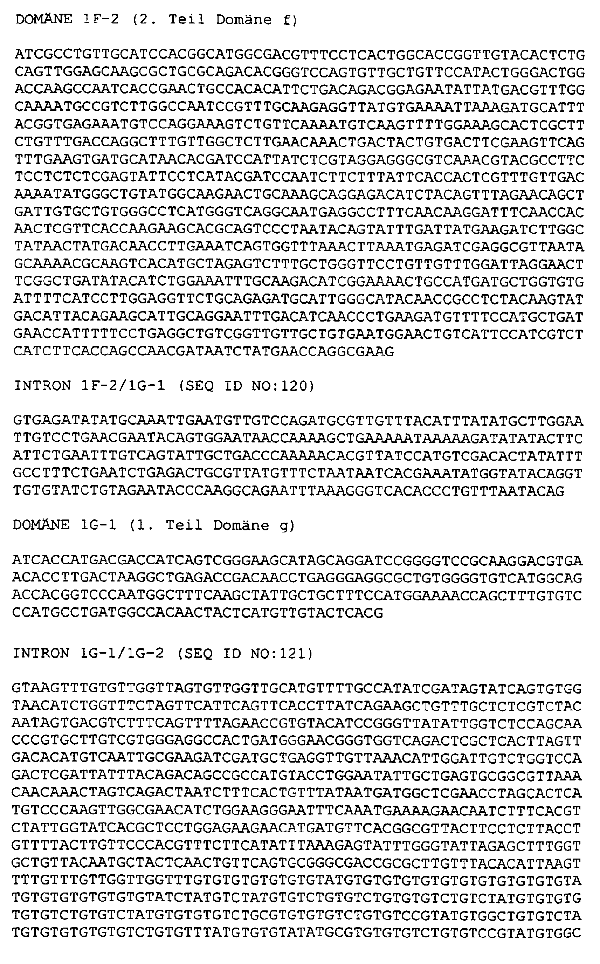

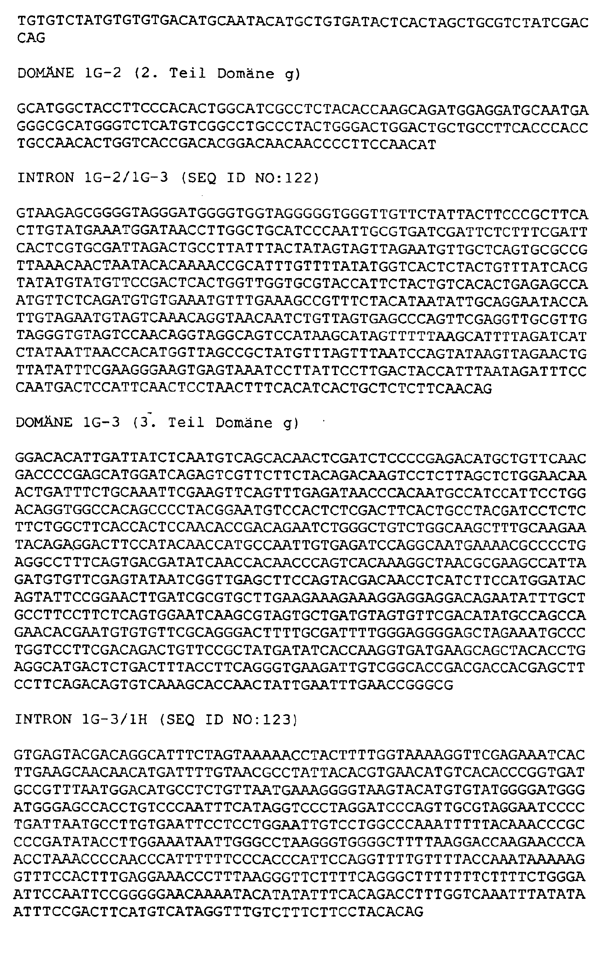

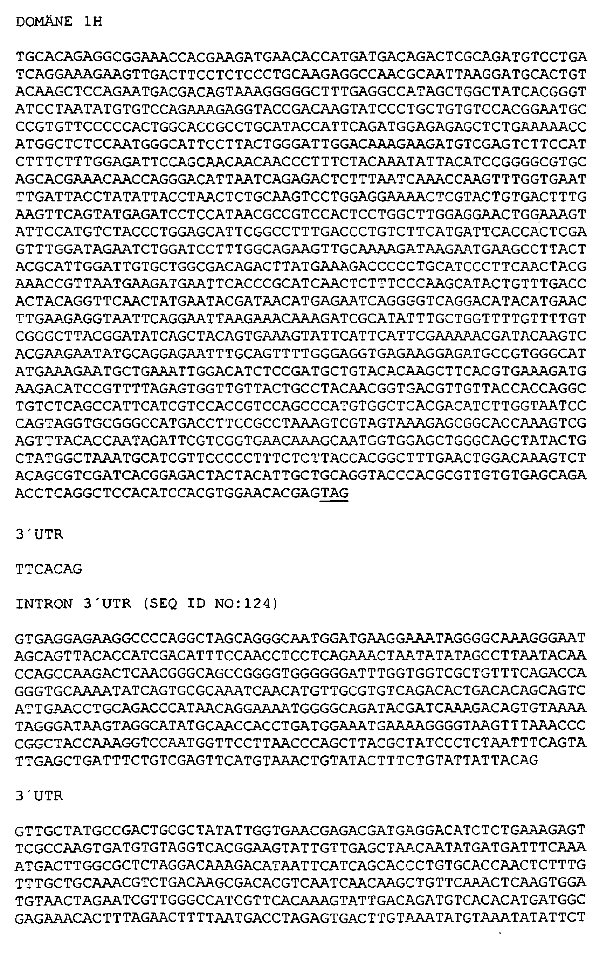

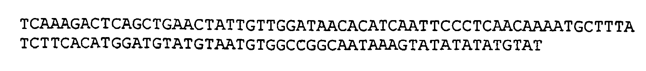

Fig. 4 zeigt die genomische Sequenz des HtH1-Gens.

Fig. 5 zeigt die abgeleitete Primärstruktur des HtH1-Proteins.

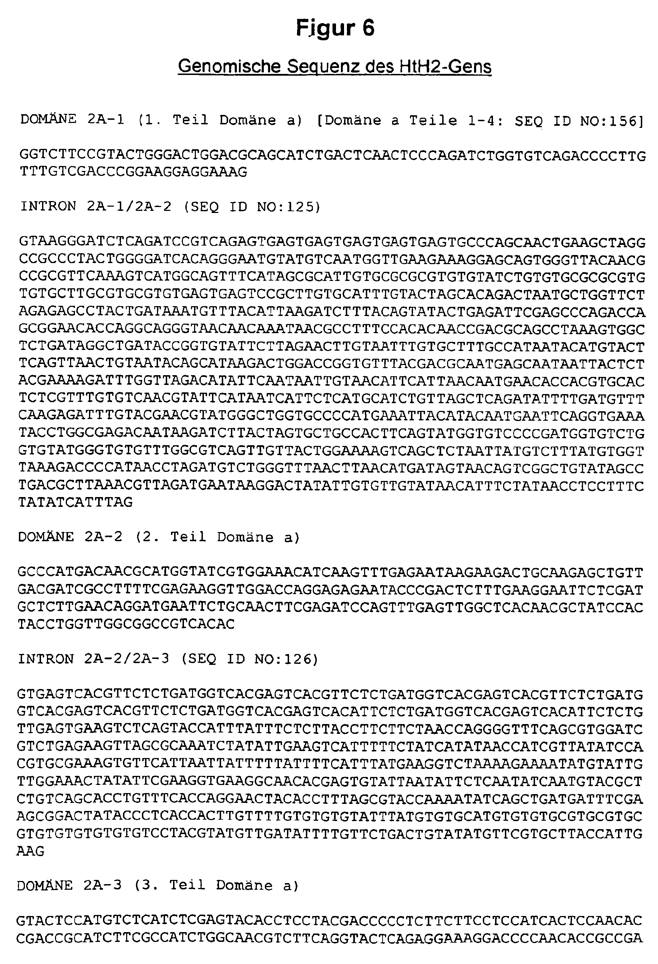

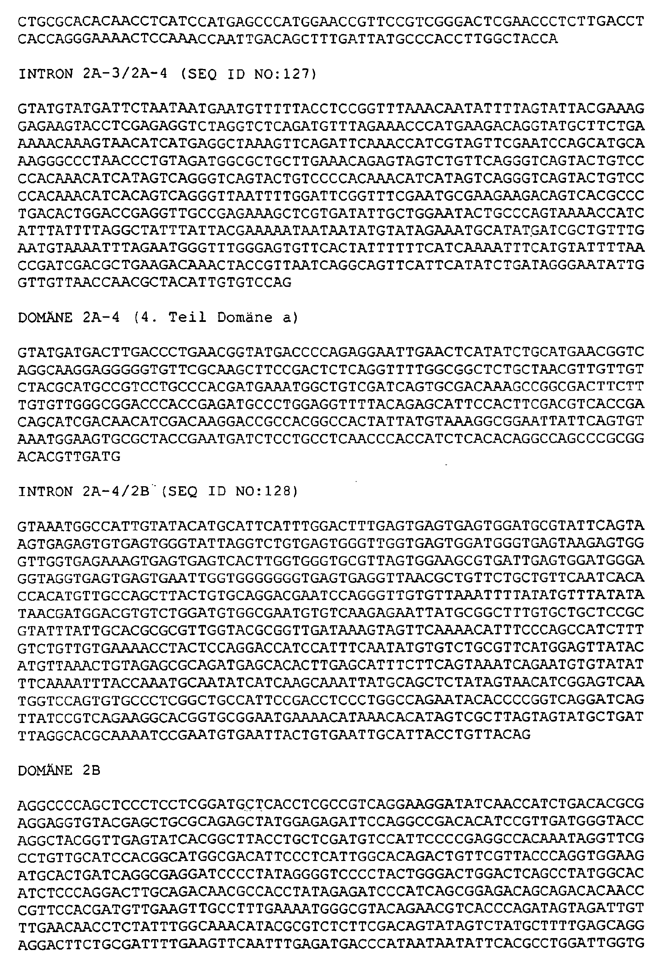

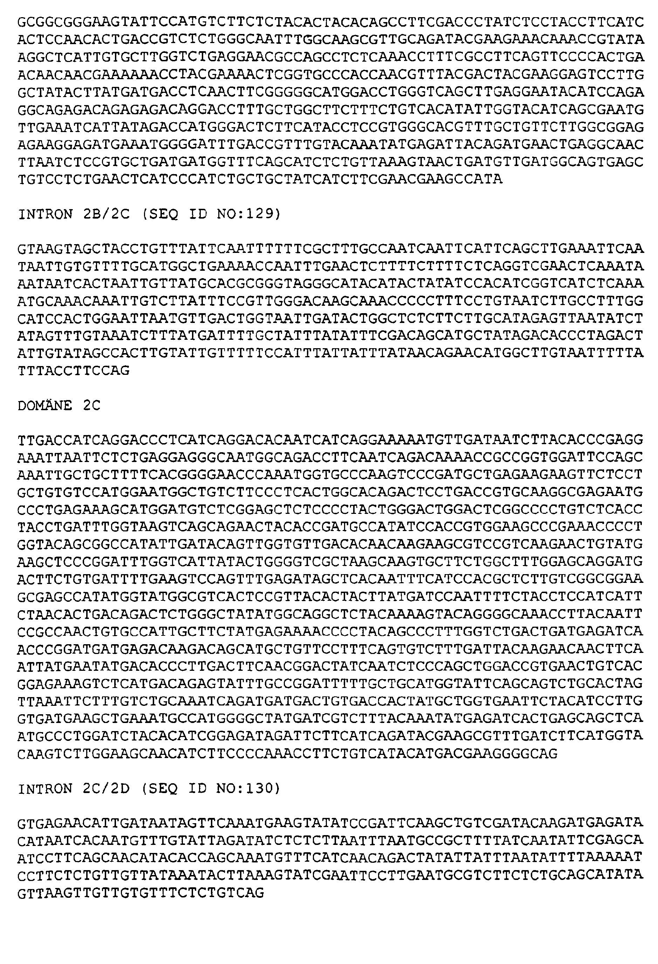

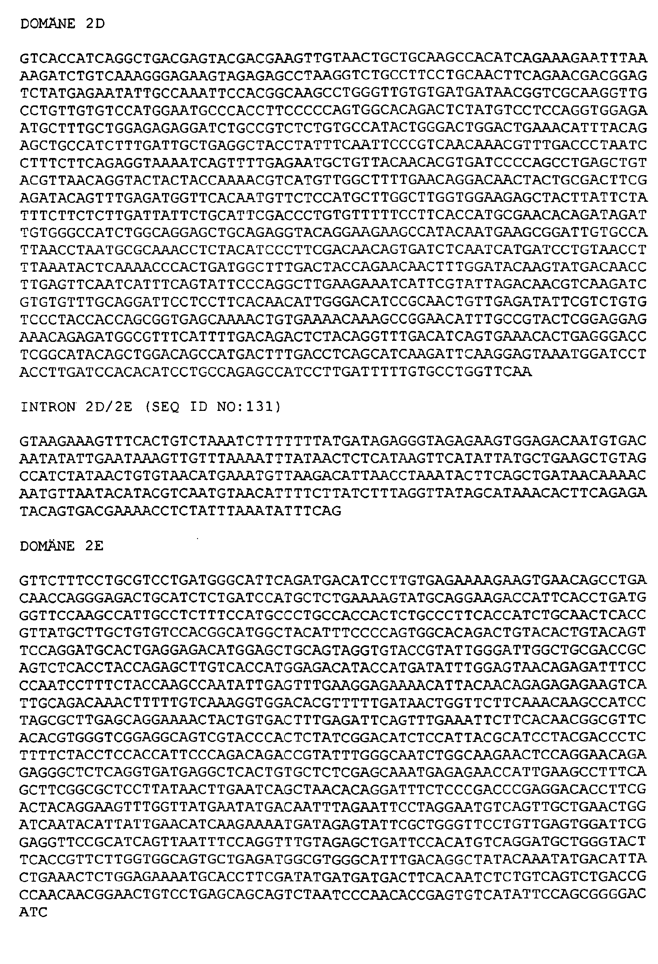

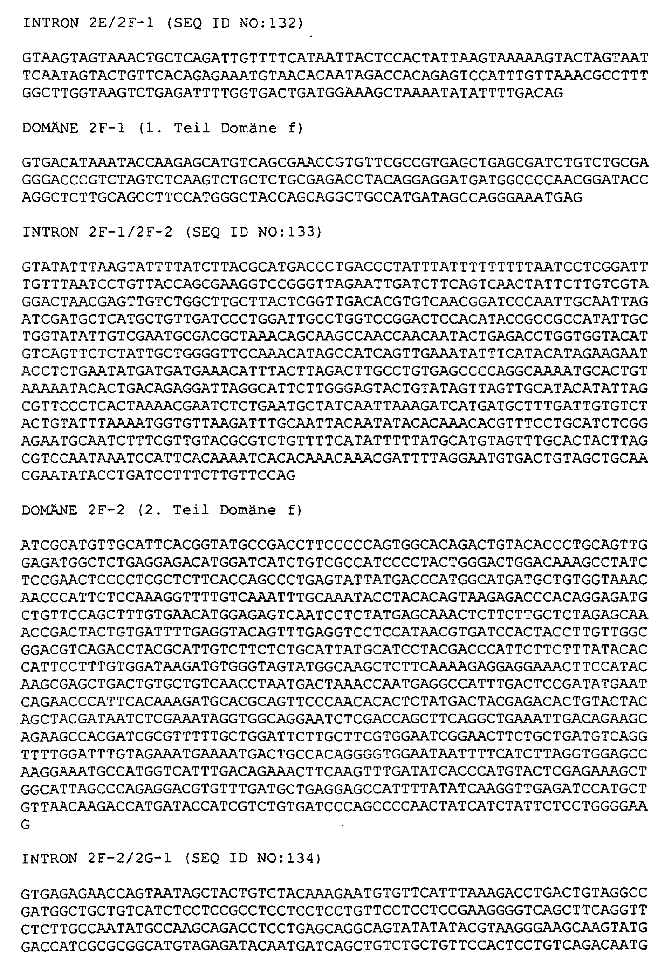

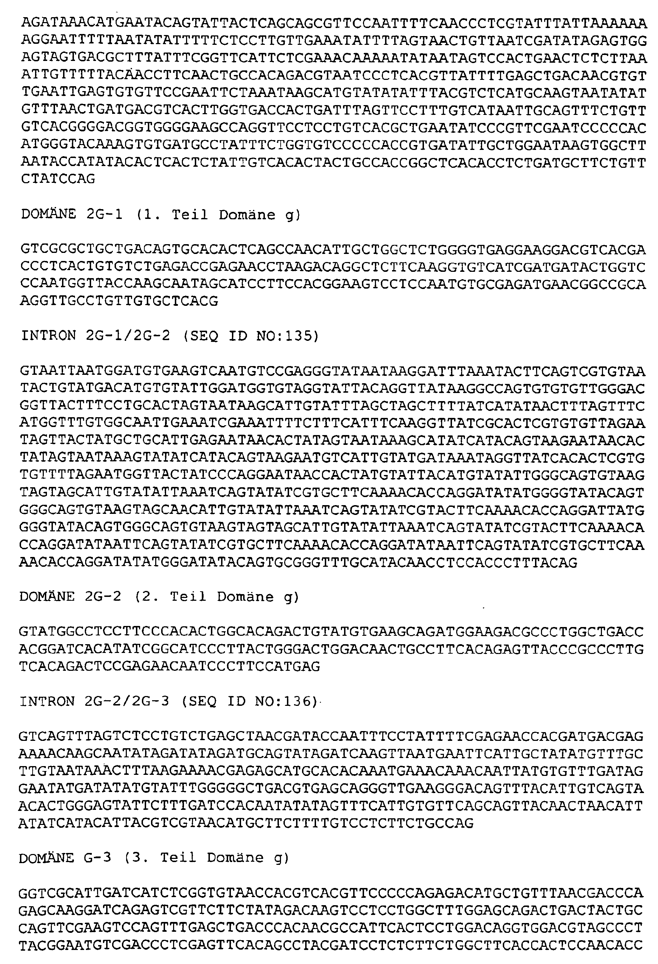

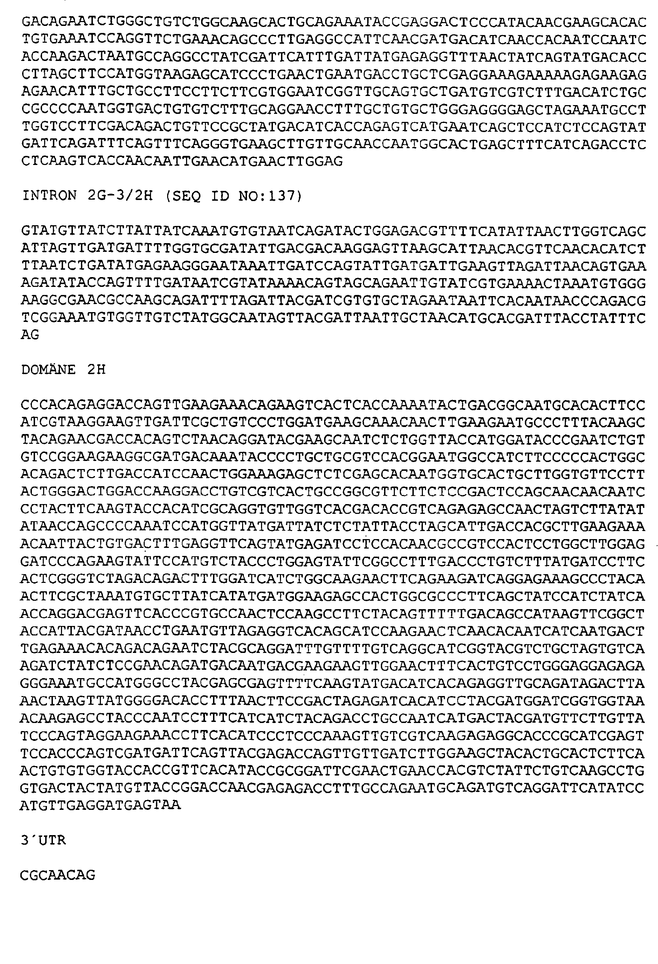

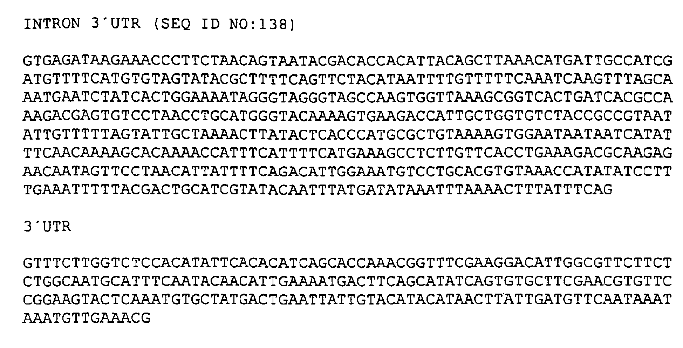

Fig. 6 zeigt die genomische Sequenz des HtH2-Gens.

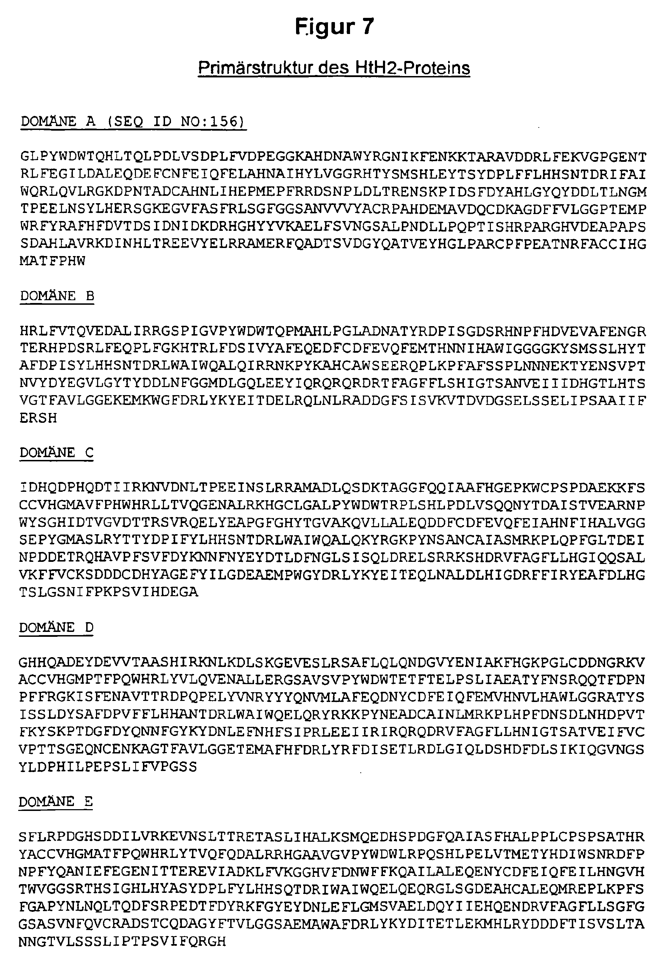

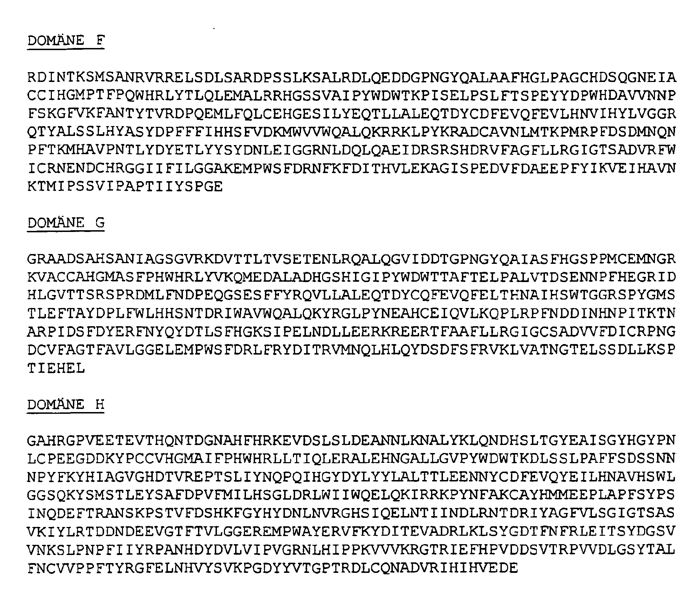

Fig. 7 zeigt die abgeleitete Primärstruktur des HtH2-Proteins.

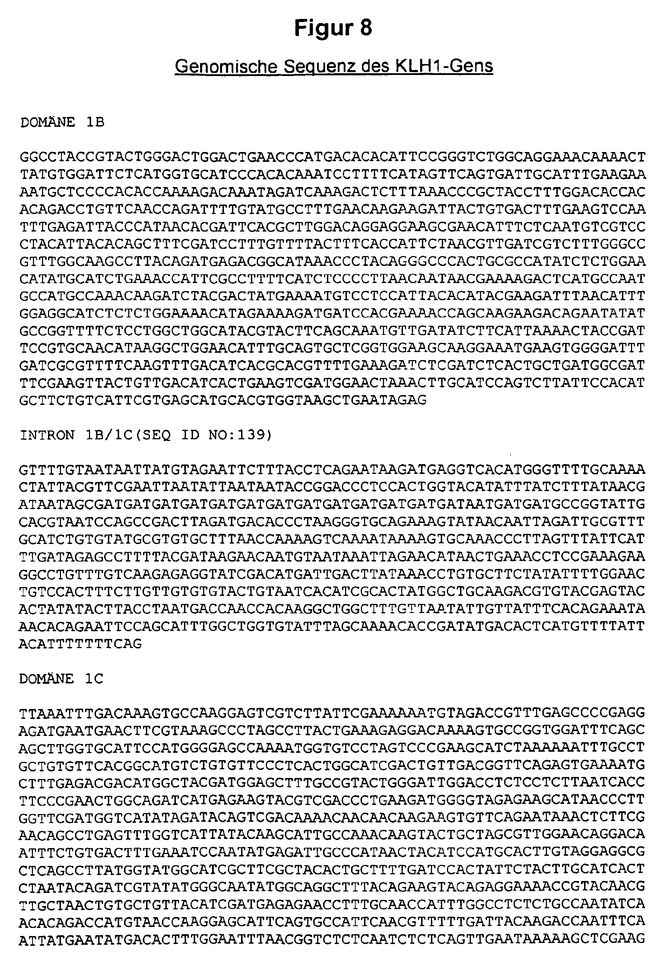

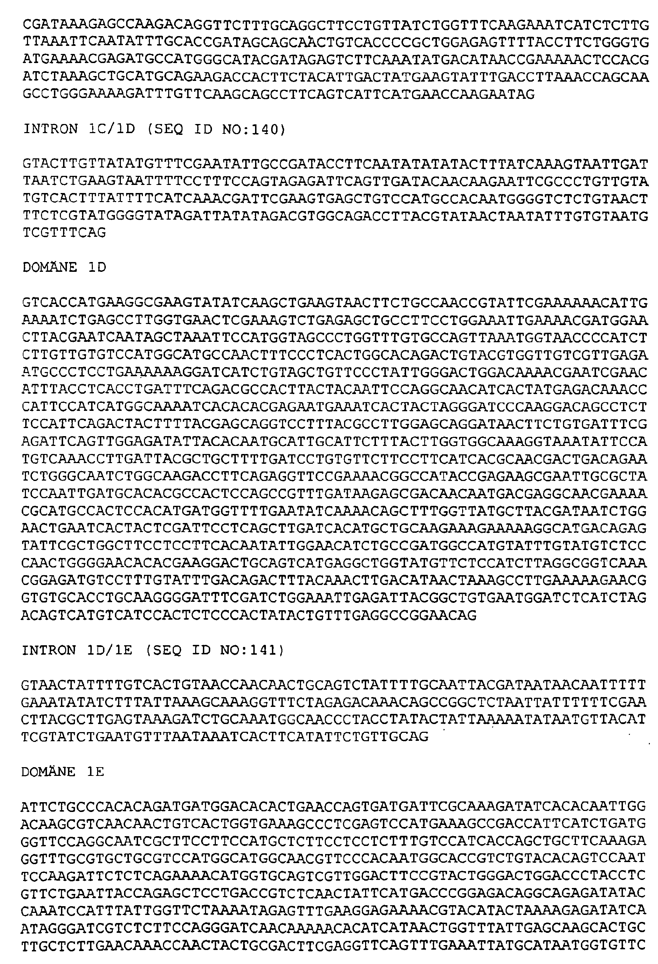

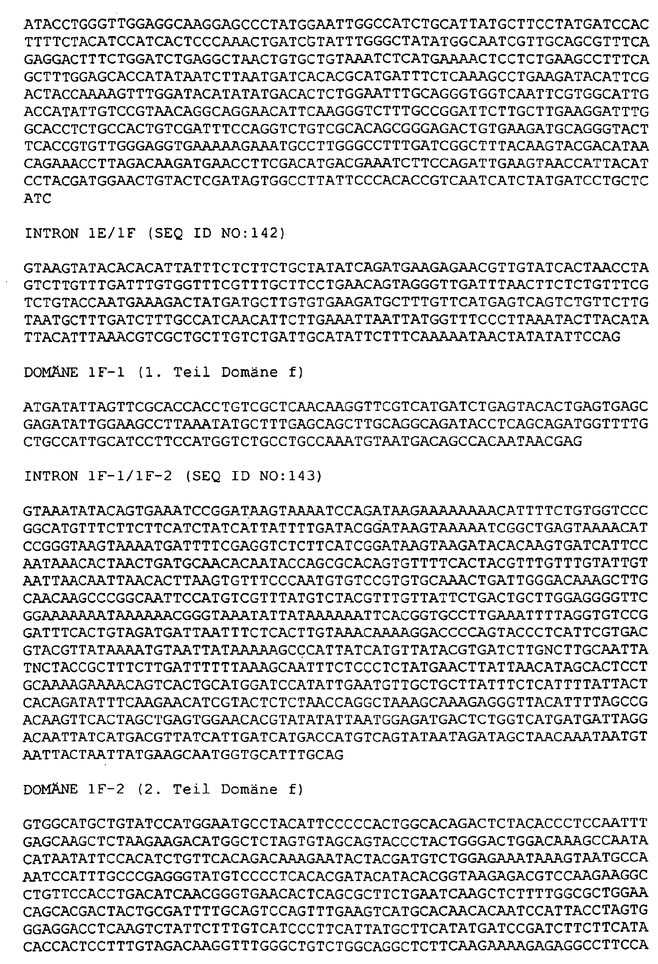

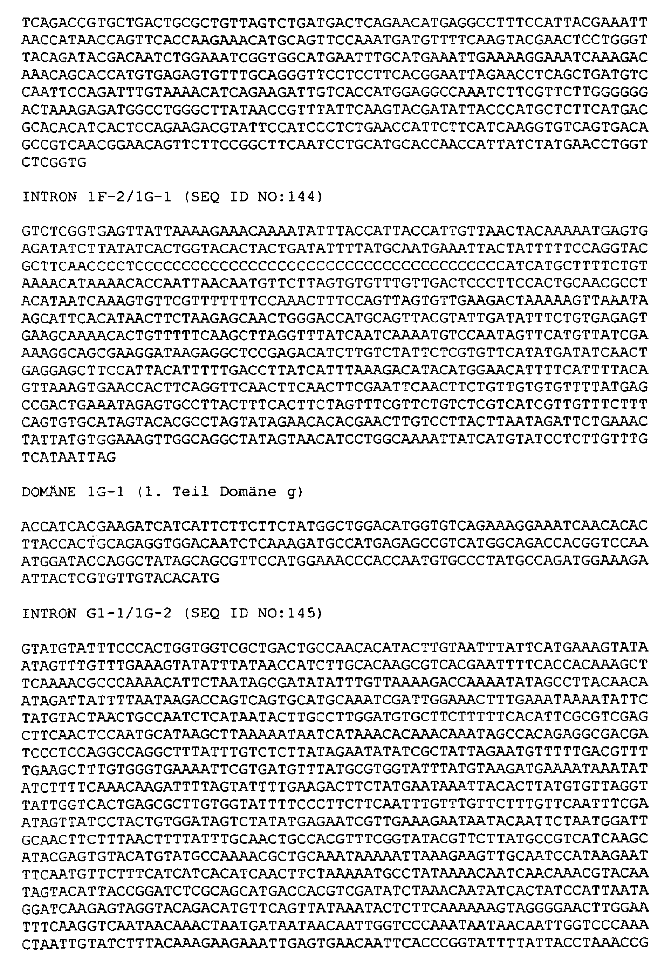

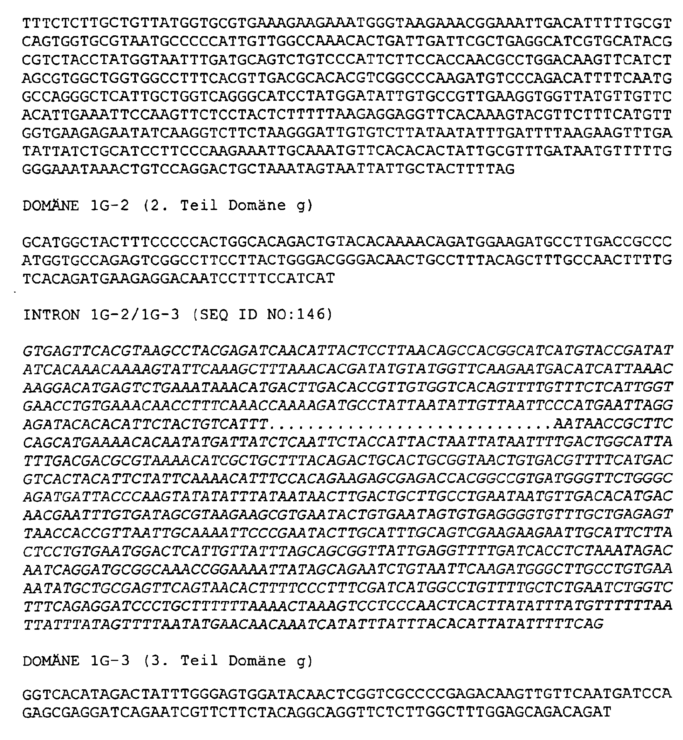

Fig. 8 zeigt die genomische Sequenz des KLH1-Gens.

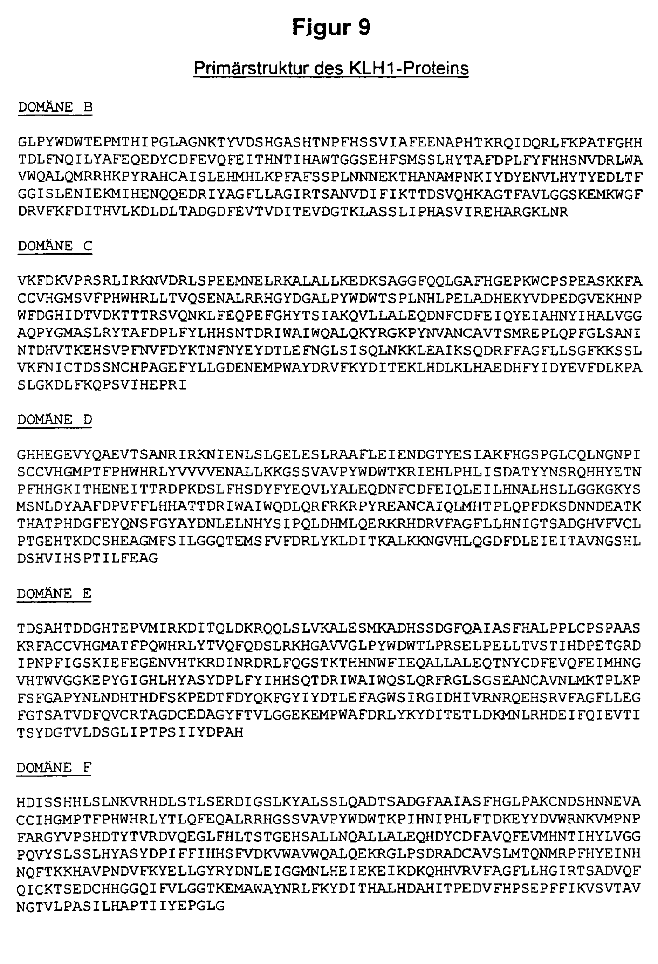

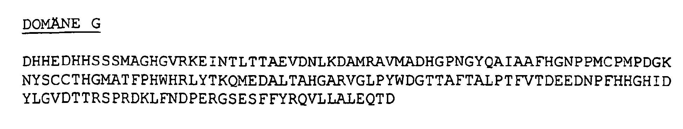

Fig. 9 zeigt die abgeleitete Primärstruktur des KLH1-Proteins.

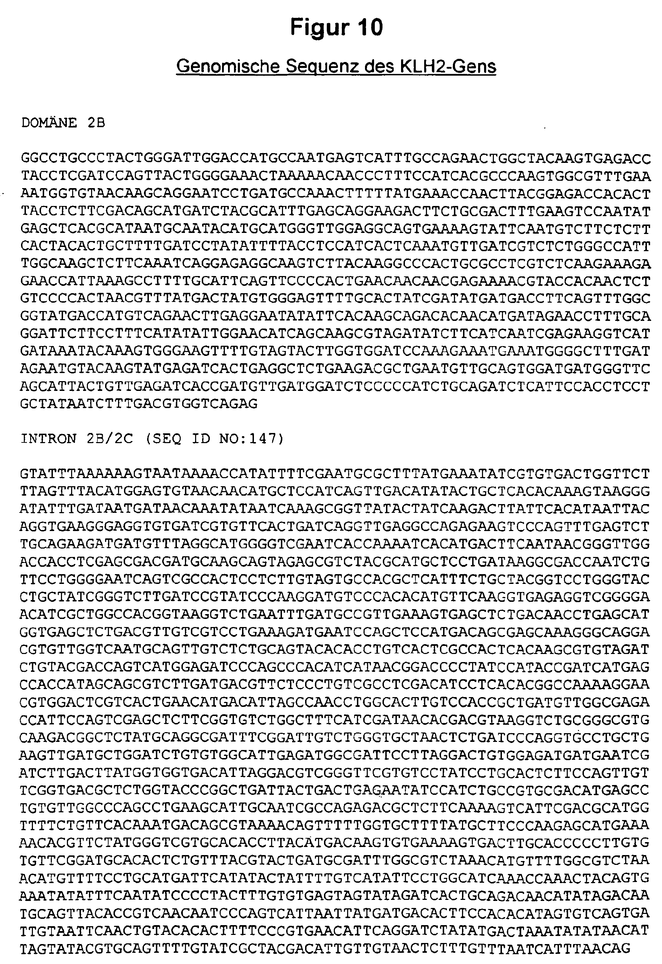

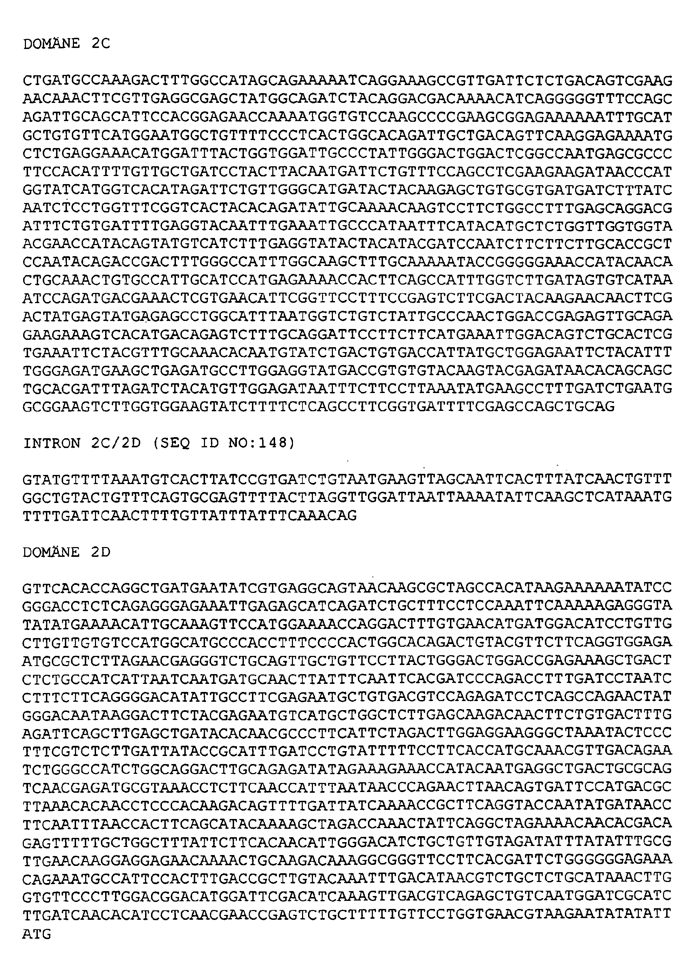

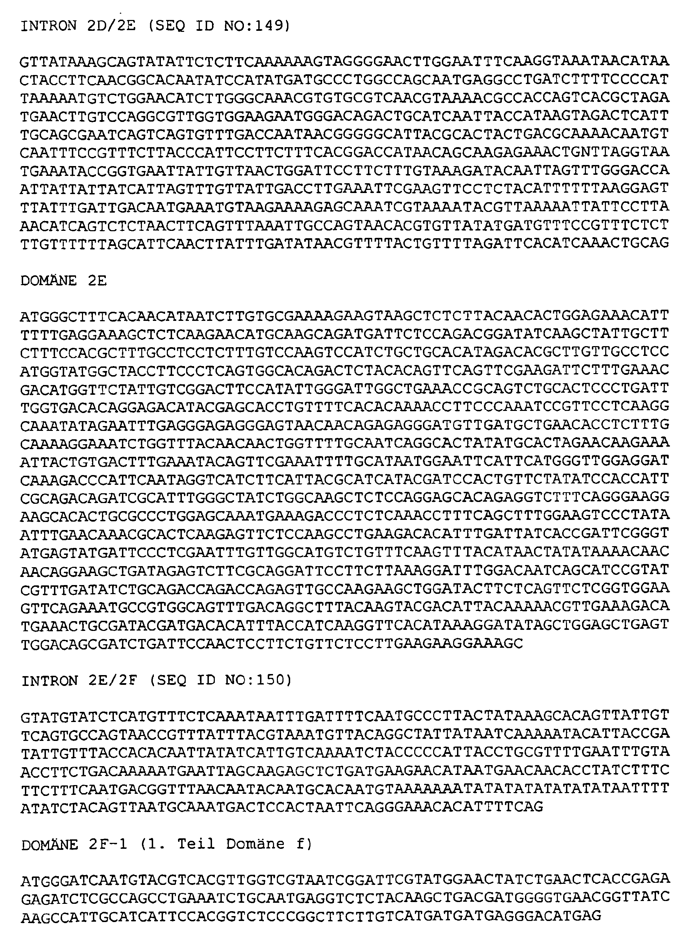

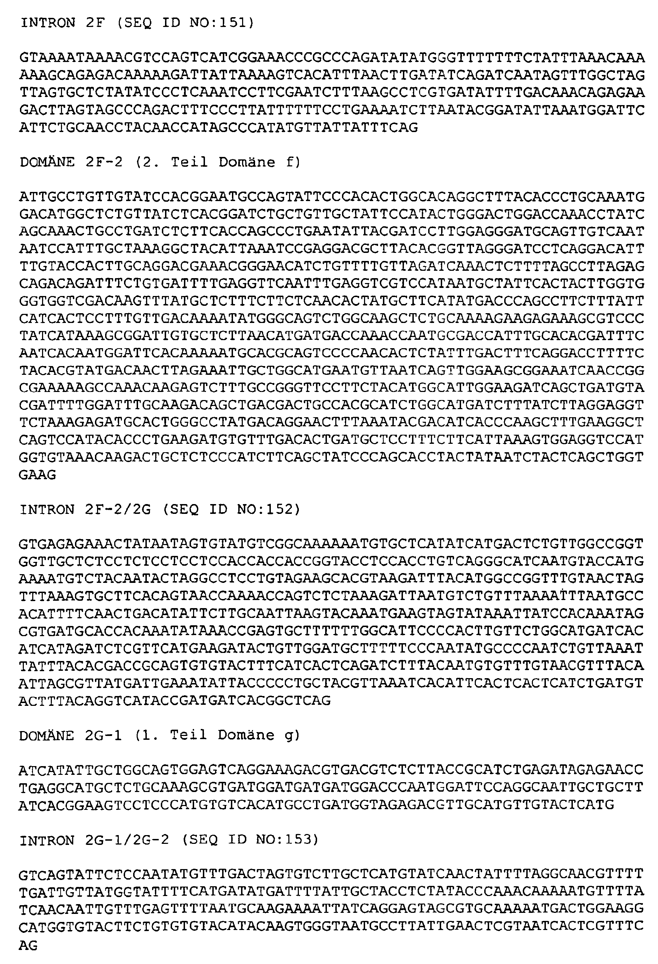

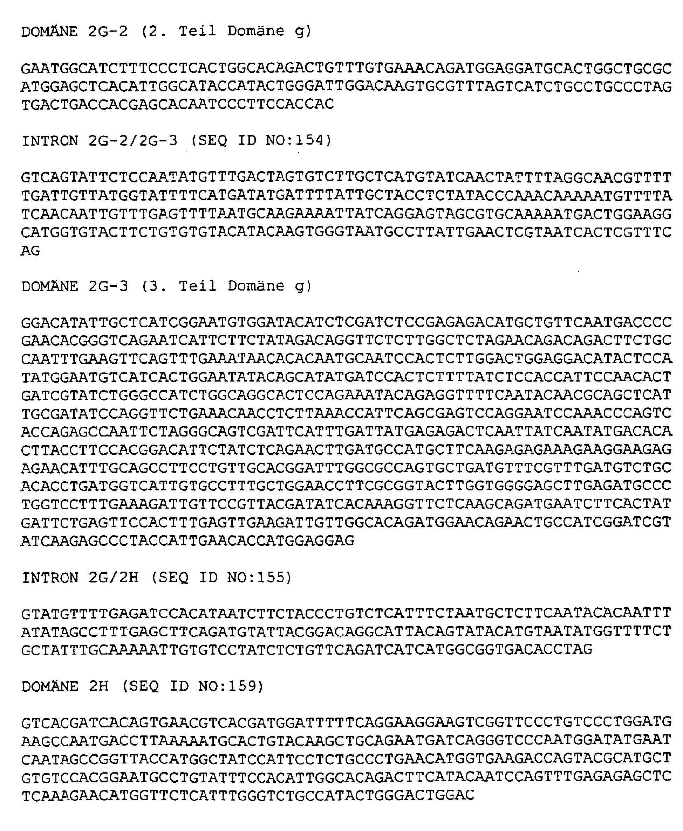

Fig. 10 zeigt die genomische Sequenz des KLH2-Gens.

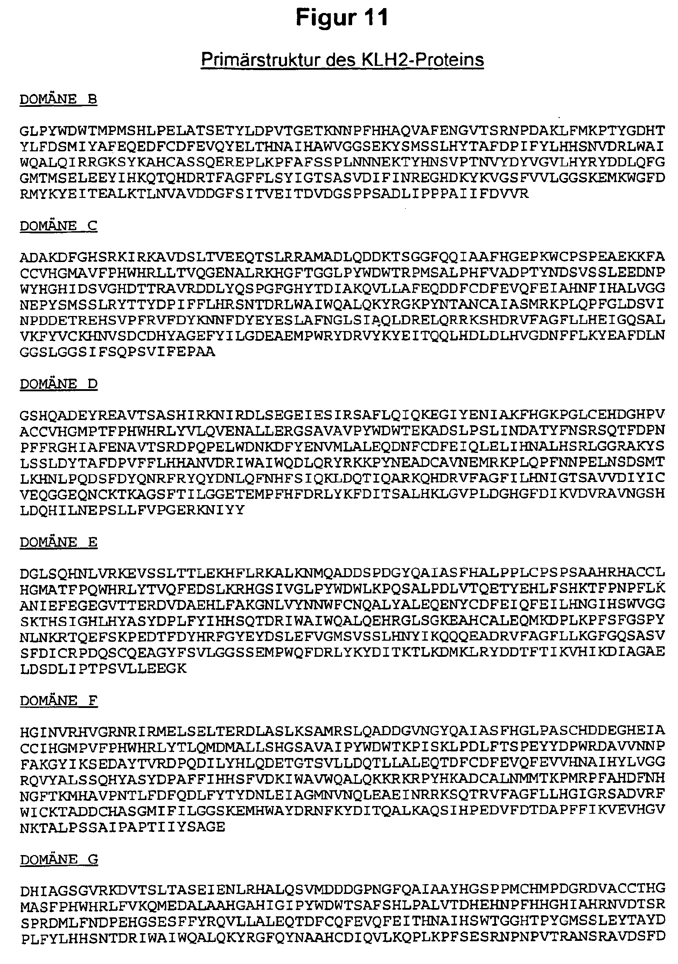

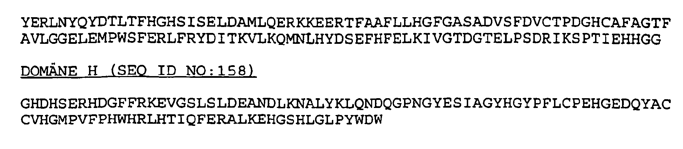

Fig. 11 zeigt die abgeleitete Primärstruktur des KLH2-Proteins.

### BEISPIELE, die die vorl. Erfindung erläutern

### Material und Methoden

### 1. Gewinnen der Hämolymphe und Isolieren von Hämocyanin

Individuen des europäischen Seeohrs *Haliotis tuberculata* aus dem Bereich der französischen Atlantikküste wurden von S.M.E.L (Blainville sur Mer, Frankreich) und der Firma Biosyn (Fellbach, Deutschland) bereitgestellt. Die Tiere wurden in einem 300 l Seewasser-Aquarium bei 17° C gehalten und mit Braunalgen gefüttert. Zur Entnahme der Hämolymphe wurden die Seeohren in einem verschlossenen Plastiksack auf Eis gestellt. Nach einer Stunde waren große Volumina an Hämolymphe durch ihre Haut sezemiert worden. Es stellte sich heraus, dass das durch dieses Verfahren erhaltene Hämocyanin identisch ist mit dem Hämocyanin, das durch Einschneiden einer Mulde in den Fuß heruntergekühlter Meeresschnecken unter Verwendung einer Skalpellklinge gesammelt werden konnte. Die Blutzellen wurden von der Hämolymphe durch Zentrifugation bei 800 g über 30 min bei 4° C abgetrennt. Das gesamte Hämocyanin wurde sofort danach durch präparative Ultrazentrifugation bei 30000 g über 4 Stunden bei 4 °C sedimentiert. Der Überstand wurde verworfen und das blaue Hämocyanin-Pellet wurde über Nacht in "Stabilisierungs-Puffer" (0,05 M Tris, 5 mM CaCl₂, 5 mM MgCl₂, 0,15 M NaCl, 1 mM PMSF, pH 7,4) suspendiert und bei 4 ° C gelagert.

Intaktes HtH1 wurde unter Verwendung des von Harris et al., 1995, supra, beschriebenen Verfahrens aus dem gesamten HtH durch selektive Dissoziation von HtH2 in Ammoniummolybdat/Polyethylenglykol (1%/0,2%)-Lösung, pH 5,9 und nachfolgender Ultrazentrifugation erhalten. Das entstandene, teilweise aufgereinigte HtH1-Pellet wurde aufgelöst und durch Gelfiltration auf einer Biogel A15m-Vorrichtung zur Homogenität aufgereinigt. Der letzte Schritt ergab geringe Mengen von aufgereinigtem HtH2. Natives HtH1 und HtH2 wurde durch Dialyse gegen "Dissoziationspuffer" (0,13 M Glycin/NaOH, pH 9,6) bei 4 ° C über Nacht quantitativ in die Untereinheiten dissoziiert; die Gegenwart von EDTA war nicht erforderlich. 1 mM PMSF wurde bei jeder Stufe der Aufreinigung hinzugefügt, um die Proteolyse zu hemmen.

### 2. Elektronenmikroskopie

Konventionelles "negative staining" wurde mit dem Einzel-Tropfen-Verfahren (Harris und Horne in Harris, J.R. (Herausgeber) Electron microscopy in biology, (1991), IRL Press Oxford, S. 203-228) durchgeführt. Kohlenstoffträger-Filme wurde anfänglich über 20 Sekunden glühentladen, um sie hydrophil und für das Protein adsorptiv zu machen. Man läßt die Proteinproben an den Kohlenstoffilmen über 60 Sekunden adsorbieren. Sodann werden die Puffersalze durch sequentielles Waschen mit vier aufeinanderfolgenden 20 µl Wassertropfen entfernt. Die Gitter werden schließlich mit einem 20 µl Tropfen 5 % wäßrigen Ammoniummolybdats, enthaltend 1 % Trehalose (pH 7,0) negativ gefärbt und bei Raumtemperatur trocknen gelassen. Für die elektronenmikroskopische Untersuchung wird ein Zeiss EM 900 Transmissionselektronenmikroskop verwendet.

### 3. Polyacrylamid-Gel-Elektrophorese und Immunelektrophorese

SDS-Polyacrylamid-Gel-Elektrophorese (SDS-PAGE) wurde gemäß des Verfahrens von Laemmli (Nature 227 (1970), 670-685) durchgeführt. Zur nativen PAGE wurde ein alkalisches System gemäß Markl et al. (1979) J. Comp. Physiol. 133 B, 167-175 mit einem 0,33 M Tris/Borat, pH 9,6 als Gelpuffer und 0,065 M Tris/Borat, pH 9,6 als Elektrodenpuffer verwendet. Gekreuzte und "crossed-line" lmmunelektrophorese (lE) wurden gemäß Weeke (Scand. J. Immunol. 2 (1973), Suppl. 1, 47-56) bzw. Kroll (Scand. J. Immunol. 2, Suppl. 1 (1973), 79-81) durchgeführt. Kaninchenantikörper wurden von Charles River Deutschland (Kisslegg, Deutschland) gegen dissoziiertes Gesamt-HtH und aufgereinigtes HtH1 erzeugt. Das lmmunisierungsverfahren wurde durchgeführt gemäß Markl und Winter (J. Comp. Physiol. 159B (1989), 139-151).

### 4. Limitierte Proteolyse und Isolierung der Fragmente

Die limitierte Proteolyse wurde bei 37 ° C in 0,13 M Glycin/NaOH, pH 9,6 durch Hinzufügen eines der nachstehenden Enzyme (Sigma, Deisenhofen, Deutschland), die in 0,1 M NH₄HCO₃, pH 8,0 gelöst waren, durchgeführt: *Staphylococcus aureus* V8-Protease Typ XVII (8400), Papain Typ II aus Papaya-Milch (P-3125), Rinder-Pankreas-Elastase Typ IV (E-0258), Chymotrypsin und Trypsin. Die Hämocyanin-Konzentration lag zwischen 1 und 10 mg/ml. Die Endkonzentration des Enzyms betrug 2 % (Gewicht/Gewicht). Die Proteolyse wurde nach 5 Stunden durch Einfrieren auf -20 ° C beendet. Das HPLC-Verfahren wurde auf einer Vorrichtung von Applied Biosystems (BAI, Bensheim, Deutschland) durchgeführt, die mit einem Modell-1000S-Dioden-Array-Detektor ausgestattet war. Die proteolytischen Fragmente wurden auf kleine Mono-Q-Anionaustauscher-Säule aufgetragen (Pharmacia, Freiburg, Deutschland), die mit 0,02 M Tris/HCl, pH 8,0 äquilibriert worden war und mit einem linearen Natriumchlorid-Gradienten (0,0 M - 0,5 M CaCl) im gleichen Puffer bei einer Flußrate von 1 ml/min eluiert wurden. Alternativ wurden die proteolytischen Fragmente durch Ausschneiden der Banden aus nativen PAGE-Gelen (Markl.et al., 1979) J. Comp. Physiol. 133 B,167-175 isoliert, nachdem sie zuvor mit dem Roti-White-System (Roth, Karlsruhe, Deutschland) gemäß Femandez-Patron et al. (1995) Anal. Biochem. 224, 203-211 invers-gefärbt worden waren. Zur nachfolgenden Spaltung mit einem zweiten Enzym wurden die isolierten Fragmente zuerst Übemacht gegen 0,13 M Glycin/NaOH, pH 9,6 dialysiert, um NaCl zu entfernen.

### 5. Aminosäurensequenzanalyse

Die durch das HPLC-Verfahren erhaltenen Proteine wurden in SDS-enthaltendem Probenpuffer denaturiert und durch SDS-PAGE (Laemmli, 1970, supra; 7,5 % Polyacrylamid) aufgetrennt. Um die Blockierung des NH₂-Terminus zu verhindern wurde 0,6 % (Gewicht/ Gewicht) Thioglykolsäure zum Kathodenpuffer (Walsh et al., Biochemistry 27 (1988), 6867-6876) hinzugefügt. Die Proteinbanden wurden durch Elektro-Transfer auf ProBlot-Membranen (Applied Biosystems, Deutschland) in einer vertikalen Blotting-Kammer übertragen (25 mM Borat-Puffer, pH 8,8, enthaltend 2 mM EDTA; 10 min/100 mA, 15 min/200 mA, 12 h/300 mA). Der Nachweis der einzelnen Polypeptide auf den Membranen wurde mit der Ponceau-S-Färbung durchgeführt. Die interessierenden Polypeptid-Banden wurden ausgeschnitten und in einer 477A-Protein-Sequenzier-Vorrichtung von Applied Biosystems sequenziert. Die Mengen der auf die Sequenziervorrichtung aufgetragenen Polypeptide lag im unteren pmol-Bereich.

### 6. cDNA-Klonierung und Sequenzanalyse

Eine Lambda-cDNA-Expressionsbibliothek wurde aus Poly(A⁺)-RNA aus *Haliotis* Mantelgewebe unter Verwendung des Vektors Lambda ZAP Express^{®} gemäß den Herstelleranweisungen (Stratagene, Heidelberg, Deutschland) hergestellt. Die Klone wurden unter Verwendung von HtH-spezifischen Kaninchenantikörpern isoliert. Die Nukleotidsequenzierung wurde auf beiden Strängen unter Verwendung des Taq Dye deoxy Terminator®-Systems durchgeführt. Die Anordung der Sequenzen wurde mit der Software CLUSTAL W (1.7)® und TREEVIEW ®(Thompson et al., Nucl. Acids Res. 22 (1994), 4673-4680) durchgeführt.

### Beispiel 1:

### Isolierung von HtH und Auftrennung zweier unterschiedlicher Typen (HtH1 und HtH2)

Die Hämolymphe wurde aus adulten Seeohren gewonnen. Die Blutzellen wurden durch Zentrifugation entfernt und das Hämocyanin wurde sodann durch Ultrazentrifugation sedimentiert. Das blaue Hämocyanin-Pellet wurde in "Stabilisierungspuffer" (pH 7,4) wieder aufgelöst und in der Elektronenmikroskopie untersucht (Figur 1a). Es bestand hauptsächlich aus typischen Di-Dekameren, begleitet von einem kleinen Anteil an Dekameren und Tridekameren. Die Denaturierung in 2 % SDS in Gegenwart reduzierender Substanzen und anschließender SDS-PAGE-Trennung ergab eine einzige Bande, die dem Polypeptid mit einem scheinbaren Molekulargewicht von 370 kDa entsprach, welches nur geringfügig unterhalb des scheinbaren Untereinheiten-Gewichts von KLH (Figur 1b) liegt. Die vollständige Dissoziation der Oligomere wie der Di-Dekamere in die nativen Polypeptide (Untereinheiten) wurde erreicht durch Übernacht-Dialyse von HtH gegen "Dissoziationspuffer" (pH 9,6). Natives PAGE-Verfahren, das auf diese Proben angewendet wurde, ergab eine Haupt- und eine Neben-Komponente (Figur 1c). Die gekreuzte lmmunelektrophorese (gekreuzte IE) unter Verwendung von gegen aufgereinigtes Gesamt-HtH-erzeugten polyklonalen Kaninchen-Antikörpern zeigte zwei Komponenten, die immunologisch unterschiedlich sind, jedoch die klassische Reaktion einer teilweise immunologischen Identität aufweisen (Figur 1d). Ihre präparative Isolierung (Figur 1e-i) zeigte, dass sie die Untereinheiten von zwei unterschiedlichen HtH-Typen darstellen, bezeichnet als HtH1 und HtH2, und die Muster der nativen PAGE- und gekreuzten IE-Verfahren konnten jeder einzelnen zugeordnet werden (Figur 1 c, d).

Die Auftrennung von HtH1 und HtH2 wurde gemäß des Verfahrens zur selektiven Dissoziation gemäß Harris et al., 1995, supra, durchgeführt. In Ammoniummolybdat/Polyethylenglykol war HtH1 im Oligomeren-Zustand (Di-Dekamere) vollständig stabil, wohingegen HtH2 vollständig in die Untereinheiten dissoziiert (Figur 1e). Dies ermöglichte die quantitative Sedimentation von HtH1 in der Ultrazentrifuge, wohin gegen der größte Teil des HtH2 im Überstand verblieb. Aus dem wieder aufgelösten Pellet wurden große Mengen an HtH1 durch Gelfiltrationschromatographie zur Homogenität aufgereinigt, welche auch geringe Mengen an reinem HtH2 ergab (Figur 1f). Die Fraktionen wurden durch native PAGE (Figur 1g) und gekreuzte IE (Figur 1h, i) untersucht. Das Verfahren der selektiven Dissoziation von HtH2 entfernte sämtliche Tri-Dekamere aus den Proben, welches nahelegt, dass die letzteren aus HtH2, jedoch nicht aus HtH1 aufgebaut sind (Figur 1 e). Das selektive Dissoziationsverhalten von HtH2 und auch die Fähigkeit, Aggregate zu bilden, die größer als *in vivo* -Di-Dekamere sind, entspricht den Eigenschaften von KLH2. Umgekehrt ähnelt die Stabilität von HtH1 unter diesen Bedingungen und seine Unfähigkeit, sich zu größeren Aggregaten als Di-Dekameren zu assemblieren, dem Verhalten von KLH1. Diese Verwandtschaft wird weiter belegt durch die Reaktion von Anti-KLH1 bzw. Anti-KLH2 Antikörpern gegen die beiden HtH-Typen (Figur 1j-m).

### Beispiel 2:

### Analyse der Organisation der HtH1-Untereinheit

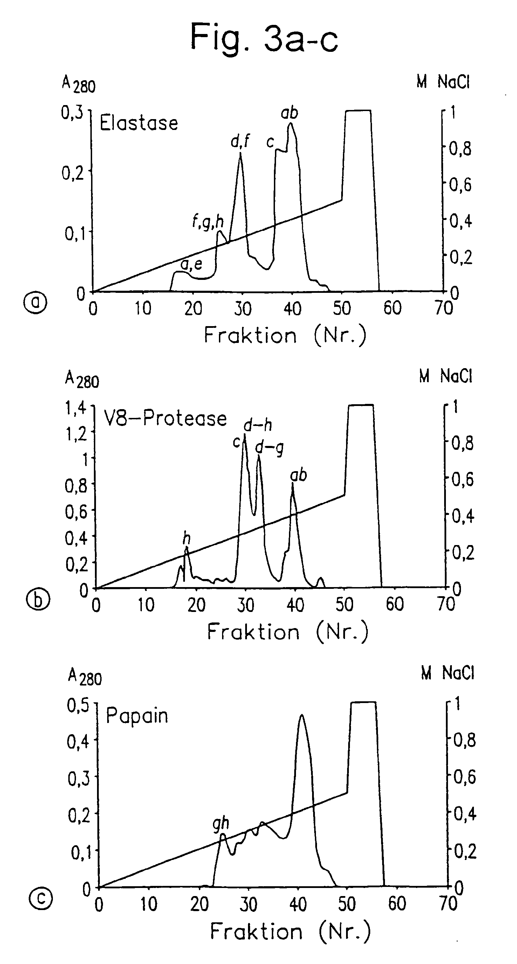

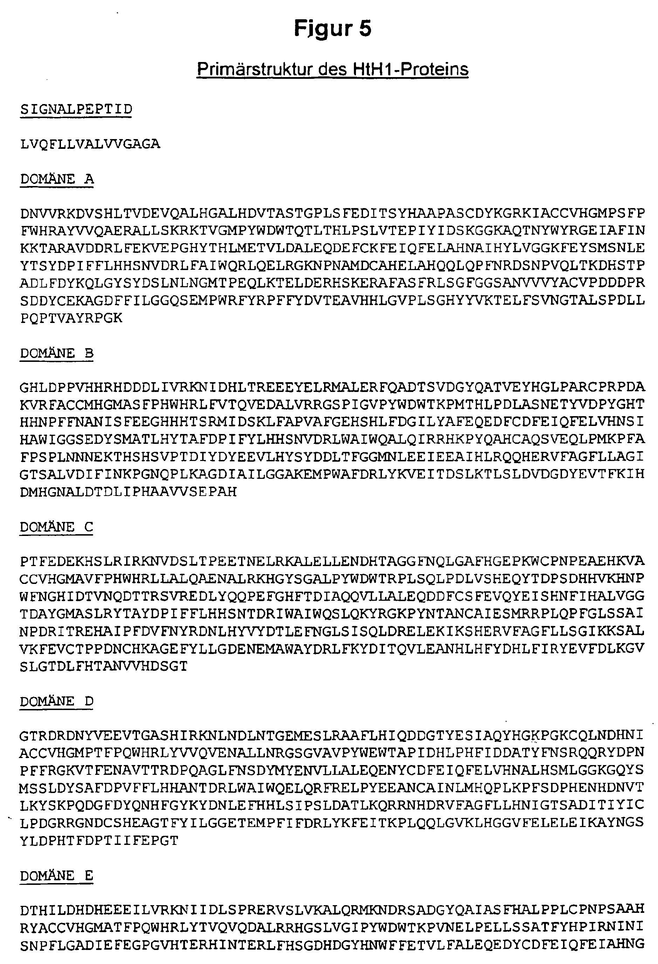

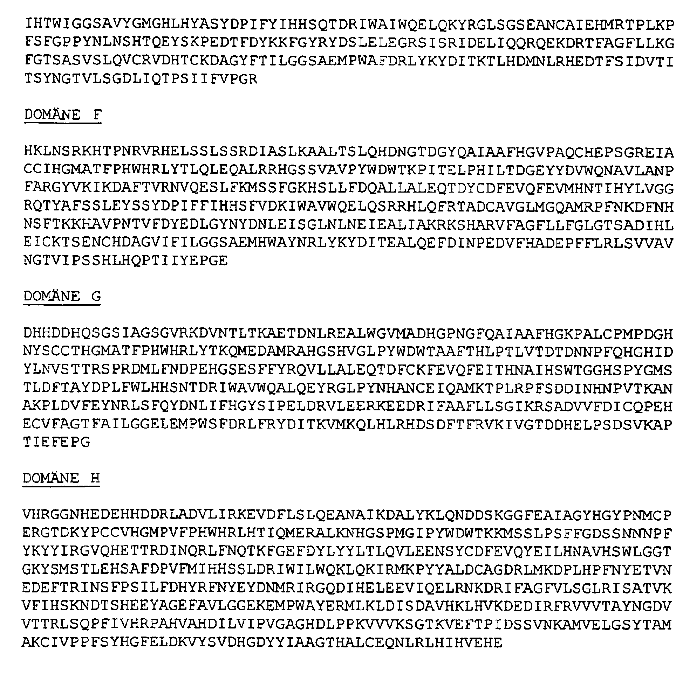

Die acht funktionellen Einheiten (FU's, häufig als "funktionelle Domänen" bezeichnet), die eine Mollusken-Hämocyanin-Untereinheit bilden, unterscheiden sich in der Primärstruktur und weisen keine immunologische Kreuz-Reaktivität auf, wie sich aus gekreuzter IE ergibt. Im Fall der aufgereinigten HtH1 Untereinheit (Figur 1g, h) wurden geringe Konzentrationen fünf unterschiedlicher Proteasen (Elastase, V8-Protease, Papain, Trypsin und Chymotrypsin) verwendet, welche die Peptid-Bindungen zwischen benachbarten FU's von KLH1 und KLH2 gespalten hatten (Gebauer et al., 1994, supra, Söhngen et al., 1997, supra). Die Spaltprodukte wurden durch gekreuzte IE und SDS-PAGE (Fig. 2) untersucht. Elastase-Behandlung erzeugt acht einzelne FU's, abgeleitet aus der Anzahl an unterschiedlichen Immunpräzipitations-Gipfeln in der gekreuzten IE (Fig. 2a) und mit dem scheinbaren Molekulargewicht von ca. 50 kDa des Hauptteils der Spaltprodukte in SDS-PAGE (Fig. 2b). Ein weiterer Präzipitationsgipfel wurde als FU-Dimer erkannt, welches durch unvollständige Spaltung des Segments ab (Fig. 2a) entstand. Durch HPLC-Verfahren mit einer Mono-Q-Säule (Fig. 3a) wurden zwei der Elastase-Spaltprodukte in einer ausreichenden Reinheit erhalten, um durch "crossed-line-lE" ihre klare Zuordnung zu zwei der acht Präzipitationsgipfel (Fig. 2c,d) zu ermöglichen. Die anderen vier Proteasen wiesen unterschiedliche Spaltmuster auf, die aus Gemischen einzelner FU's und größerer Fragmente, enthaltend zwei, drei oder mehrere FU's bestanden (z.B. Fig. 2 e,f). Viele von ihnen waren durch das HPLC-Verfahren in ausreichender Menge angereichert (Fig. 3b-e), um ihre Identifikation in ihren entsprechenden SDS-PAGE- und gekreuzten IE-Mustern zu ermöglichen. Eine Anzahl dieser Komponenten wurde N-terminal durch Blot-Transfer von SDS-Gelen auf Pro-Blot^{®}-Membrane (Tabelle 1) sequenziert. Die Resultate wurden mit N-terminalen Sequenzen verglichen, die aus dem scheinbar orthologen Protein in *Megathura crenulata,* KLH1 (Tabelle I), erhalten worden waren, von dem die vollständige FU-Anordnung verfügbar ist (Söhngen et al., 1997, supra; vgl. Fig. 5b). Das Ergebnis des gesamten Ansatzes führte zur Bestimmung der vollständigen FU-Anordnung innerhalb der HtH1-Untereinheit (Fig. 2a).

Insbesondere ergab die Spaltung der HtH1-Untereinheit (1-abcdefgh) mit V8-Protease vier Präzipitationsgipfel in der gekreuzten IE (Fig. 2e). Das SDS-PAGE-Verfahren zeigte fünf unterschiedliche Fragmente (Fig. 2f): 220 kDa (5 FU's), 185 kDa(4 FU's), 100 kDa (2 FU's), 55 kDa(1 FU) und 46 kDa(1 FU). Das 100 kDa-Fragment wurde durch das HPLC-Verfahren (Fig. 3b) isoliert und durch N-terminale Sequenzierung als 1-ab identifiziert, da die Sequenz identisch zu derjenigen der intakten Untereinheit war (Tabelle 1). In dem "crossed-line" IE-Verfahren verschmolz 1-ab mit drei Präzipitationsgipfeln des Elastase-Spaltmusters. Aufgrund der Auswertung ergab sich, dass sie die Fragmente 1-ab, 1-a bzw. 1-b darstellen (Fig. 2g). Jedoch verblieb es unklar, welcher Gipfel 1-a und welcher 1-b darstellt. In einem zweiten Schritt wurde das HPLC-aufgereinigte 1-ab durch Elastase in seine Kom-ponenten-FU's gespalten, aus denen eine durch das native PAGE-Gel-Streifenverfahren eluiert werden konnte und dem Elastase-Muster durch das "crossed-line" IE-Verfahren (Fig. 2h) zugeordnet und N-terminal sequenziert wurde. Diese Komponente wies die gleiche N-terminale Sequenz wie die gesamte Untereinheit auf und war deshalb identisch zu 1-a. Somit ist die zweite FU des 100 kDa-Fragments 1-b (Fig. 2a; Tabelle I). Ebenso wurden HPLC-aufgereinigtes 1-c und 1-h erhalten (Fig. 3b), durch N-terminale-Sequenzähnlichkeiten zu den entsprechenden FU's in KLH1 (Tabelle 1) identifiziert und durch das "crossed-line" IE-Verfahren ihren entsprechenden Präzipitationsgipfeln in dem Elastase-Muster zugeordnet (Fig. 2i,j). Weiterhin wurden 1-a, 1-b, 1-c und 1-h identifiziert (Fig. 2a). Unter Verwendung von Papain zur Untereinheiten-Spaltung wurden fünf unterschiedliche Gipfel in dem gekreuzten IE-Verfahren (Fig. k) erhalten. Aus einer solchen Probe wurde ein 100 kDa-Fragment (2 FU's) durch das HPLC-Verfahren (Fig. 3c) aufgereinigt, welches gemäß des "crossed-line" IE-Verfahrens das bereits identifizierte FU 1-h und eine der vier noch nicht identifizierten FU's enthielt und deshalb 1-gh darstellen muß (Figs. 2k, 3c). Tatsächlich wies dieses Fragment eine N-terminale Sequenz auf, die Ähnlichkeiten zu KLH1-g (Tabelle I) zeigte. Zur weiteren Bestätigung wurde das HPLC-aufgereinigte Fragment 1-gh mit Elastase in seine Bestandteil-FU's gespalten, aus denen 1-g aufgereinigt und durch N-terminale Sequenzierung identifiziert wurde. Es wurde durch "crossed-line" IE-Verfahren seinem Gipfel in dem Elastase-Spaltmuster zugeordnet (Fig. 2l).

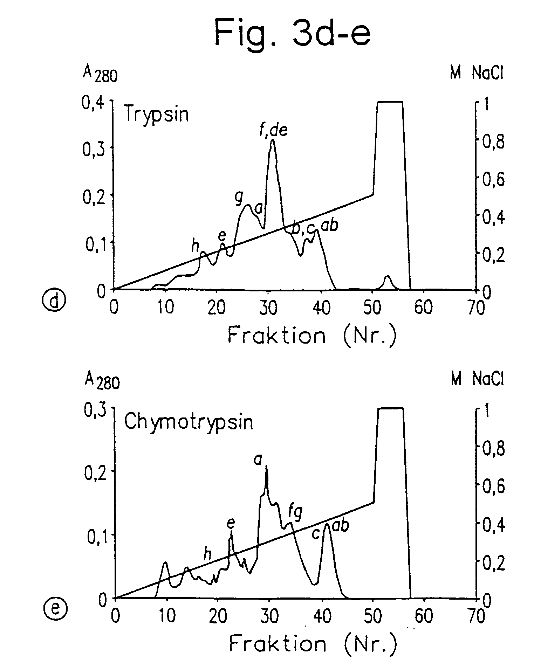

Das 220 kDa-Fragment aus der V8-Protease-Spaltung (Fig. 2e, f) wurde HPLC-aufgereinigt (Fig. 3b) und fusionierte im "crossed-line" IE-Verfahren mit 1-h, 1-g und drei noch nicht identifizierten Gipfeln des Elastase-Spaltmusters. Weiterhin wurde das 185 kDa-Fragment in ausreichender Reinheit erhalten (Fig. 2e, f; 3b), und es wurde gezeigt, dass sie die gleichen Komponenten mit Ausnahme von 1-h enthielten. Dies legte nahe, dass das 22 kDa und das 185 kDa-Fragment 1-defgh bzw. 1-defg sind. Tatsächlich war die N-terminale Sequenz praktisch identisch und zeigte weiterhin Ähnlichkeit zu KLH1-d (Tabelle 1). Die Spaltung der HtH1-Untereinheit mit Trypsin ergab eine Vielzahl an Komponenten in dem Molekulargewichtsbereich von ein bzw. zwei FU's (Fig. 2m). Mehrere der Komponenten wurden in HPLC-Fraktionen angereichert (Fig. 3d). Ein 100 kDa-Fragment erwies sich als besonders nützlich, da es die gleich N-terminale Sequenz wie das Fragment 1-defg aus der V8-Protease-Spaltung aufwies (Tabelle 1); deshalb sollte das 100 kDa-Fragment 1-de darstellen. In dem "crossed-lined" IE-Verfahren fusionierte diese Komponente mit zwei der drei noch nicht identifizierten FU-Gipfeln des Elastase-Spaltmusters (Fig. 2n), welches deshalb 1-d und 1-e darstellen sollte, und somit eine einzige Möglichkeit für 1-f übrig ließ. Das "crossed-line" IE-Verfahren zeigte auch, dass in der 1-de Fraktion weiterhin FU 1-f vorhanden war (Fig. 2n). Die Identifikation von 1-f bestätigte durch Spaltung der Untereinheit mit Chymotrypsin (Fig. 2o) und nachfolgendem HPLC-Verfahren (Fig. 3e). Diese Spaltung ergab unter anderem ein 95 kDa-Fragment (2 FU's) welches im "crossed-line"-IE-Verfahren mit 1-g und einem zweiten Gipfel (Fig. 2p) fusionierte und deshalb entweder 1-gh (welches ausgeschlossen werden konnte, da 1-h bereits identifiziert war), oder 1-fg (welches sinnvoll erscheint aufgrund des weiteren betreffenden Peaks, der zu dem verbleibenden Kandidaten identisch war) sein könnte. Tatsächlich zeigte dieses Fragment eine neue N-terminale Sequenz, welche in gewisser Weise zu KLH1-f ähnlich ist (Tabelle I). Das letzte Problem bestand nun darin, die zwei verbleibenden FU-Gipfel 1-d bzw. 1-e zuzuordnen. Dies wurde unter Verwendung von HPLC-isolierten FU's aus Proben gelöst, in denen die Untereinheit mit Elastase gespalten worden war. (Fig. 2c, d; 3a). Die saurere Komponente in dem gekreuzten IE-Verfahren war 1-d abgeleitet aus seiner N-terminalen Sequenz, welche identisch ist mit der von 1-defgh (Fig. 2c; Tabelle 1), wohingegen die basischere Komponente des 1-d/1-g-Paares eine neue N-terminale Sequenz (Tabelle 1) aufwies und deshalb 1-e (Fig. 2a) sein mußte. Somit war die Struktur der funktionellen Einheiten der Untereinheit HtH1 aufgeklärt.

### Beispiel 3:

Vergleich der Molekulargewichte und N-terminalen Sequenzen der biochemisch isolierten funktionellen Einheiten (FU's) aus HtH1 und KLH1. Die unterschiedlichen FU's, jede mit einer intakten binukleären Kupfer-Bindungsstelle, wurden durch limitierte Proteolyse als globuläre Segmente aus ihrer größeren Einheit freigesetzt; vgl. Abschnitt "Isolierung und Analyse der Einheiten aus HtH1". Die KLH1-Daten wurden aus Söhngen et al., supra, entnommen. Die Zuordnung als tatsächliche Einheit erfolgte aufgrund des Molekulargewichtes und des immunologischen Verhaltens (vgl. Fig. 2). Das ungewöhnlich niedrige Molekulargewicht von isoliertem HtH1-d könnte bedeuten, dass ein großes Peptid C-terminal abgespalten wurde.

**TABELLE I**

| Funktionelle Einheit | Masse (kDa) | N-terminale Sequenz |
|---|---|---|
| HtH1-a | 53 | DNVVRKDVSHLTDDEVQ |
| KLH1-a | 50 | ENLVRKDVER**L** |
| HtH1-b | 48 | ? |
| KLH1-b | 45 | ? |
| HtH1-C | 46 | FEDEKHSLRIRKNVDSLTPEENTNERLR |
| KLH1-C | 45 | KVPRSRL**I**RKNVDR**L**TPSE |
| HtH1-d | 40 | VEEVTGASHIRKNLND**L**NTGEM |
| KLH1-d | 50 | EVTSANR**I**RKNIENLS |
| HtH1-e | 49 | ILDHDHEEEILVRKNIID**L**SP |
| KLH1-e | 50 | ? |
| HtH1-f | 50 | KLNSRKHTPNRVRHELSSLSSRDIASLKA |
| KLH1-f | 45 | HHLSXNKVRHDLSTL |
| HtH1-g | 45 | DHQSGSIAGSGVRKDVNTLTKAETDNLRE |
| KLH1-g | 45 | SSMAGHFVRKDINTLTP |
| HtH1-h | 55 | DEHHDDRLADVLIRKEVDFLSLQEANAIKD |
| KLH1-h | 60 | HEDHHEDILVRKNIHS**L** |

### Beispiel 4:

### Klonierung von Hämocyanin-cDNA

1. Zur Klonierung der cDNA von Hämocyanin wurde mRNA aus dem Mantelgewebe des jeweiligen Mollusken isoliert. Der erste cDNA-Strang wurde durch reverse Transkription mit Oligo(dT) als Primer erhalten. Der zweite Strang wurde durch konventionelle Synthese mit random Primem erhalten. Die so erhaltene cDNA wurde in einen Lambda-Expressionsvektor kloniert unter Bildung einer cDNA-Expressionsbibliothek. Unter Verwendung eines anti-Hämocyanin-Antikörpers wurde die Bibliothek unter geeeigneten Bedingungen abgesucht, wobei positive Klone erhalten wurden. Diese positiven Klone wurden isoliert, sequenziert und charakterisiert.
2. Aus dem N-terminalen Bereich eines erhaltenen positiven Klons wurde eine cDNA-Sonde hergestellt, mit der die cDNA-Bibliothek abgesucht wurde. Die erhaltenen positiven Klone werden wiederum isoliert, sequenziert und charakterisiert.
3. Um noch weiter 5' gelegene Sequenzen zu erhalten, wurde eine weitere Expressionsbibliothek aus cDNA hergestellt, die mit Hilfe einer Kombination von Hämocyanin-spezifischen und "random"-Primem erhalten wurde. Diese cDNA-Bibliothek wurde mit cDNA-Sonden, die den "N-terminalen" Bereichen der unter (2.) erhaltenen positiven Klone entsprechen, abgesucht. Die erhaltenen positiven Klone wurden isoliert, sequenziert und charakterisiert.

### Beispiel 5:

### Klonierung von Hämocyanin-Genen

Genomische DNA wurde gemäß Standardverfahren isoliert. Die PCR-Reaktion wurde mit Hilfe von Hämocyanin-spezifischen Primern durchgeführt, um die Genabschnitte der interessierenden Hämocyanine zu amplifizieren. Die erhaltenen Amplifikationsprodukte wurden in einen geeigneten Vektor (beispielsweise pGem T oder pGem T easy (Promega, Mannheim) kloniert, sequenziert und charakterisiert.

### Beispiel 6:

### Rekombinante Expression von Hämocyanin

Mit einem cDNA-Klon, der die kodierende Sequenz für HtH-1d enthält, wurde eine PCR-Reaktion durchgeführt, um spezifisch die kodierende Sequenz der Domäne 1 d zu amplifizieren. Als Primer wurden synthetisch hergestellte Oligonukleotide verwendet.
Primer 1 (stromaufwärts) umfasst sechs Nukleotide des Endes der Domäne HtH-1c, eine Sacl-Schnittstelle und 12 Nukleotide des Endes der Domäne HtH-1d.
Primer 2 (stromabwärts) umfasst sechs Nukleotide des Anfangs der Domäne HtH1-e, eine Sa*l*l-Schnittstelle und eine HtH1-d spezifische Sequenz.

| | | | |
|---|---|---|---|
| PCR-Bedingungen: | 2 | min | 95°C |
| | 30 | sec | 95°C |
| | 30 | sec | 55°C |
| | 1 | min | 72°C |
| | 35 | Zyklen | |
| | 10 | min | 72°C |

Das Amplifikat wurde in dem pGEM T easy PCR Klonierungsvektor (Promega) in XL-1 Blue (Stratagene) kloniert. Nach Isolation des rekombinanten Plasmids und Restriktion mit S*ac*l und *Sal*l konnte die cDNA der Domäne 1 d isoliert werden. Der Expressionsvektor pQE30 (Qiagen) wurde ebenfalls mit den entsprechenden Enzymen restringiert.

Anschließend wurde die Ligation zwischen der HtH-1d-cDNA (restringiert mit S*ac*l und *Sal*l*)* und pQE (restringiert mit S*ac*I und S*al*l) durchgeführt. Somit ist eine gerichtete Klonierung der cDNA, kodierend für HtH-1d, in einen Expressionsvektor möglich. Die Expression von HtH1-d in pQE in XL-1 Blue erfolgt gemäß Herstelleranleitung. Die Expression weiterer HtH1-, HtH2- oder KLH1- oder KLH2-Domänen kann analog erfolgen.

## Patentansprüche

1. Nukleinsäure-Molekül, umfassend
(i) eine Sequenz kodierend für ein Hämocyanin, eine Hämocyanin-Domäne oder eine Variante nach Anspruch 1 (c)
(ii) und mindestens eine Intronsequenz,
wobei die Nukleinsäuresequenz ausgewählt ist aus:
(a) Nukleinsäuresequenzen, die aus der Gruppe der nachfolgend angegebenen DNA-Sequenzen bzw. der ihnen entsprechenden RNA-Sequenzen ausgewählt sind oder diese enthalten:
SEQ ID NO:9 (partielle HtH2 Domäne b),
SEQ ID NO:10 (HtH2 Domäne c),
SEQ ID NO:11 (HtH2 Domäne d),
SEQ ID NO:12 (HtH2 Domäne e),
SEQ ID NO:13 (HtH2 Domäne f),
SEQ ID NO::14 (HtH2 Domäne g),
SEQ ID NO:15 (HtH2 Domäne h),
SEQ ID NO:20 (KLH2 Domäne b),
SEQ ID NO:21 (KLH2 Domäne c),
SEQ ID NO:22 (partielle KLH2 Domäne d),
SEQ ID NO:23 (KLH2 Domäne g),
SEQ ID NO:24 (partielle KLH2 Domäne h),
SEQ ID NO:50 (partielle HtH2 Domäne a),
SEQ ID NO:51 (HtH2 Domäne b'),
SEQ ID NO:52 (HtH2 Domäne d'),
SEQ ID NO:53 (HtH2 Domäne e'),
SEQ ID NO:57 (KLH2 Domäne b'),
SEQ ID NO:58 (KLH2 Domäne c'),
SEQ ID NO:59 (KLH2 Domäne d'),
SEQ ID NO:60 (KLH2 Domäne e),
SEQ ID NO:61 (KLH2 Domäne f),
SEQ ID NO:62 (KLH2 Domäne g'),
SEQ ID NO:88 (partielle HtH2 Domäne a"),
SEQ ID NO:89 (HtH2 Domäne b")
SEQ ID NO:90 (HtH2 Domäne c"),
SEQ ID NO:91 (HtH2 Domäne d"),
SEQ ID NO:92 (HtH2 Domäne e"),
SEQ ID NO:93 (HtH2 Domäne f'),
SEQ ID NO:94 (HtH2 Domäne g"),
SEQ ID NO:95 (HtH2 Domäne h"),
SEQ ID NO:102 (KLH2 Domäne b"),
SEQ ID NO:103 (KLH2 Domäne c"),
SEQ ID NO:104 (KLH2 Domäne d"),
SEQ ID NO:105 (KLH2 Domäne e"),
SEQ ID NO:106 (KLH2 Domäne f'),
SEQ ID NO:107 (KLH2 Domäne g"),
SEQ ID NO:108 (partielle KLH2 Domäne h"),
SEQ ID NO:157 (vollständige HtH2 Domäne a);
(b) Nukleinsäuresequenzen, welche für ein Polypeptid kodieren umfassend wenigstens eine aus der folgenden Gruppe ausgewählte Aminosäuresequenz:
SEQ ID NO:33 (partielle HtH2 Domäne b),
SEQ ID NO:34 (HtH2 Domäne c),
SEQ ID NO:35 (HtH2 Domäne d),
SEQ ID NO:36 (HtH2 Domäne e),
SEQ ID NO:37 (HtH2 Domäne f),
SEQ ID NO:38 (HtH2 Domäne g),
SEQ ID NO:39 (HtH2 Domäne h),
SEQ ID NO:44 (KLH2 Domäne b),
SEQ ID NO:45 (KLH2 Domäne c),
SEQ ID NO:46 (partielle KLH2 Domäne d),
SEQ ID NO:47 (KLH2 Domäne g),
SEQ ID NO:48 (partielle KLH2 Domäne h),
SEQ ID NO:65 (partielle HtH2 Domäne a),
SEQ ID NO:156 (vollständige HtH2 Domäne a),
SEQ ID NO:66 (HtH2 Domäne b'),
SEQ ID NO:67 (HtH2 Domäne d'),
SEQ ID NO:68 (HtH2 Domäne e'),
SEQ ID NO:74 (KLH2 Domäne b'),
SEQ ID NO:75 (KLH2 Domäne c'),
SEQ ID NO:76 (KLH2 Domäne d'),
SEQ ID NO:77 (KLH2 Domäne e),
SEQ ID NO:78 (KLH2 Domäne f),
SEQ ID NO:79 (KLH2 Domäne g'),
SEQ ID NO:158 (teilweise KLH2 Domäne h)
(c) Nukleinsäuresequenzen, die für Polypeptide kodieren, die wenigstens 60% identisch zu einer Sequenz von Anspruch 1 b sind, wobei die Sequenzidentität über 90 Aminosäuren berechnet wird.

2. Nukleinsäure-Molekül nach Anspruch 1, **dadurch gekennzeichnet, dass** die Intronsequenz ausgewählt ist aus:
(i) Nukleinsäuresequenzen, die aus der Gruppe der nachfolgend angegebenen DNA-Sequenzen bzw. der ihnen entsprechenden RNA-Sequenzen ausgewählt sind oder diese enthalten:
SEQ ID NO:147 (Intron 2B/2C von KLH2),
SEQ ID NO:148 (Intron 2C/2D von KLH2),
SEQ ID NO:149 (Intron 2D/2E von KLH2),
SEQ ID NO:150 (Intron 2E/2F von KLH2),
SEQ ID NO:151 (Intron 2F von KLH2),
SEQ ID NO:152 (Intron 2F-2/2G von KLH2),
SEQ ID NO:153 (Intron 2G-1/2G-2 von KLH2),
SEQ ID NO:154 (Intron 2G-2/2G-3 von KLH2),
SEQ ID NO:155 (Intron 2G/2H von KLH2);
(ii) Nukleinsäuresequenzen, die mit dem Gegenstrang einer Nukleinsäuresequenz nach (i) hybridisieren;
(iii) Nukleinsäuresequenzen, die wenigstens 60 % identisch zu einer der unter (i) angegebenen Nukleinsäuresequenzen sind;
(iv) Varianten der unter (i) bis (iii) angegebenen Sequenzen, wobei die Varianten gegenüber den unter (i) unter (iii) angegebenen Sequenzen Additionen, Deletionen, Insertionen oder Inversionen aufweisen; und
(v) Kombinationen mehrerer der unter (i) bis (iv) angegebenen DNA-Sequenzen.

3. Nukleinsäuremolekül gemäß einem der Ansprüche 1 und 2, **dadurch gekennzeichnet, dass** es ein Desoxyribonukleinsäuremolekül ist.

4. Konstrukt, umfassend ein Nukleinsäuremolekül gemäß einem der Ansprüche 1 bis 3.

5. Konstrukt gemäß Anspruch 4, weiterhin umfassend einen zur Expressionskontrolle geeigneten Promotor, wobei die für ein Hämocyanin, eine Hämocyanin-Domäne oder ein funktionelles Fragment davon kodierende Nukleinsäuresequenz unter der Kontrolle des Promotors steht.

6. Konstrukt gemäß Anspruch 4 oder 5, weiterhin umfassend eine für ein Antigen kodierende Nukleinsäuresequenz, die direkt mit der für ein Hämocyanin, eine Hämocyanin-Domäne oder ein funktionelles Fragment davon kodierenden Nukleinsäuresequenz verbunden ist.

7. Konstrukt gemäß Anspruch 6, wobei das Antigen ausgewählt ist aus: Tumorantigenen, Virusantigenen und Antigenen bakterieller oder parasitärer Pathogene.

8. Konstrukt gemäß einem der Ansprüche 4 bis 7, wobei das Konstrukt wenigstens einen Teil eines Vektors enthält, wobei der Vektor ausgewählt ist aus: Bakteriophagen, Adenoviren, Vacciniaviren, Baculoviren, SV40-Virus und Retroviren.

9. Konstrukt gemäß einem der Ansprüche 4 bis 8, wobei das Konstrukt weiterhin eine His-Tag-kodierende Nukleinsäuresequenz umfasst und die Expression des Konstrukts zur Bildung eines Fusionsproteins mit einem His-Tag führt.

10. Wirtszelle, enthaltend ein Konstrukt gemäß einem der Ansprüche 4 bis 9, wobei die Wirtszelle eine zur Expression des Konstrukts geeignete prokaryontische oder eukaryontische Zelle ist.

11. Wirtszelle gemäß Anspruch 10, **dadurch gekennzeichnet, dass** die prokaryontische Wirtszelle ausgewählt ist aus E. coli und Bacillus subtilis.

12. Wirtszelle gemäß Anspruch 10, **dadurch gekennzeichnet, dass** die eukaryontische Wirtszelle ausgewählt ist aus Hefezellen, Pflanzenzellen, Insektenzellen und Säugerzellen, bevorzugt aus CHO-Zellen, COS-Zellen und HeLa-Zellen.

13. Verfahren zum Herstellen eines Hämocyanin-Polypeptides, wobei das Nukleinsäuremolekül gemäß einem der Ansprüche 1 bis 3 und/oder das Konstrukt gemäß einem der Ansprüche 4 bis 9 in einer geeigneten Wirtszelle exprimiert wird und das Protein gegebenenfalls isoliert wird.

14. Verfahren gemäß Anspruch 13, **dadurch gekennzeichnet, dass** das hergestellte Hämocyanin-Polypeptid natürlich oder chemisch modifiziert wird.

15. Verfahren gemäß Anspruch 14, **dadurch gekennzeichnet, dass** die Modifikation eine Quervernetzung oder eine kovalente Bindung an ein Antigen ist.

16. Verfahren gemäß einem der Ansprüche 13 bis 15, **dadurch gekennzeichnet, dass** die Expression in einer Wirtszelle gemäß einem der Ansprüche 10 bis 12 durchgeführt wird.

17. Hämocyanin-Polypeptid, umfassend eine Aminosäuresequenz, die von einem oder mehreren der Nukleinsäuremoleküle nach einem der Ansprüche 1 bis 3 kodiert wird.

18. Hämocyanin-Polypeptid gemäß Anspruch 17, umfassend wenigstens eine aus der folgenden Gruppe ausgewählte Aminosäuresequenz:
SEQ ID NO:33 (partielle HtH2 Domäne b),
SEQ ID NO:34 (HtH2 Domäne c),
SEQ ID NO:35 (HtH2 Domäne d),
SEQ ID NO:36 (HtH2 Domäne e),
SEQ ID NO:37 (HtH2 Domäne f),
SEQ ID NO:38 (HtH2 Domäne g),
SEQ ID NO:39 (HtH2 Domäne h),
SEQ ID NO:44 (KLH2 Domäne b),
SEQ ID NO:45 (KLH2 Domäne c),
SEQ ID NO:46 (partielle KLH2 Domäne d),
SEQ ID NO:47 (KLH2 Domäne g),
SEQ ID NO:48 (partielle KLH2 Domäne h),
SEQ ID NO:65 (partielle HtH2 Domäne a),
SEQ ID NO:156 (vollständige HtH2 Domäne a),
SEQ ID NO:66 (HtH2 Domäne b'),
SEQ ID NO:67 (HtH2 Domäne d'),
SEQ ID NO:68 (HtH2 Domäne e'),
SEQ ID NO:74 (KLH2 Domäne b'),
SEQ ID NO:75 (KLH2 Domäne c'),
SEQ ID NO:76 (KLH2 Domäne d'),
SEQ ID NO:77 (KLH2 Domäne e),
SEQ ID NO:78 (KLH2 Domäne f),
SEQ ID NO:79 (KLH2 Domäne g'),
SEQ ID NO:158 (teilweise KLH2 Domäne h),
oder
(b) ein Hämocyanin-Polypeptid, dessen Sequenz über einen Teilbereich von mindestens 90 Aminosäuren mindestens 60% identisch mit einer der Aminosäuresequenzen von (a) ist.

19. Rekombinantes Hämocyanin-Polypeptid, erhältlich durch das Verfahren gemäß einem der Ansprüche 13 bis 16 oder Modifikationen davon, wobei die Modifikationen
a) Di-, Oligo- und Polymere des durch das Verfahren gemäß einem der Ansprüche 13 bis 16 erhaltenen Hämocyanin-Polypeptids sind,
b) Seitenkettenmodifikationen davon,
c) Fusionsproteine, erhalten durch Binden an ein Antigen oder
d) Polypeptide, erhalten durch posttranslationale Ereignisse.

20. Rekombinantes Hämocyanin-Polypeptid gemäß Anspruch 18, **dadurch gekennzeichnet, dass** das Hämocyanin-Polypeptid entweder die Sequenzen SEQ ID NO: 44 bis 48 oder die Sequenzen SEQ ID NO:44 bis 46, 77, 78, 47, 48 umfasst und KLH2 aus Me*gathura crenulata* ist, wobei jeweils die Sequenz mit SEQ ID NO:44 durch SEQ ID NO:74, SEQ ID NO:45 durch SEQ ID NO:75, SEQ ID NO:46 durch SEQ ID NO:76 und/oder SEQ ID NO:47 durch SEQ ID NO:79 ersetzt sein kann.

21. Rekombinantes Hämocyanin-Polypeptid gemäß Anspruch 17 bis 20, **dadurch gekennzeichnet, dass** es kovalent an Viren, Virenbestandteile, Bakterien, Bakterienbestandteile, DNA, DNA-Bestandteile, anorganische oder organische Moleküle wie z. B. Kohlenhydrate Peptide und/oder Glykoproteine gebunden ist.

22. Rekombinantes Hämocyanin-Polypeptid gemäß einem der Ansprüche 17 bis 21, **dadurch gekennzeichnet, dass** das Hämocyanin-Polypeptid nicht-glykosyliert ist.

23. Rekombinantes Hämocyanin-Polypeptid gemäß einem der Ansprüche 17 bis 21, **dadurch gekennzeichnet, dass** das Hämocyanin-Polypeptid glykosyliert ist.

24. Pharmazeutische Zusammensetzung, enthaltend ein Nukleinsäuremolekül gemäß einem der Ansprüche 1 bis 3 und/oder ein Konstrukt gemäß einem der Ansprüche 4 bis 9 und physiologisch verträgliche Zusatzmittel.

25. Pharmazeutische Zusammensetzung gemäß Anspruch 24, **dadurch gekennzeichnet, dass** sie zur gentherapeutischen Behandlung von Tumoren verwendet wird.

26. Pharmazeutische Zusammensetzung, enthaltend ein Hämocyanin-Polypeptid nach einem der Ansprüche 17 bis 23 und physiologisch verträgliche Zusatzmittel.

27. Pharmazeutische Zusammensetzung gemäß Anspruch 26, **dadurch gekennzeichnet, dass** sie als Antiparasitenmittel, Antivirusmittel oder als Antitumormittel verwendet wird.

28. Pharmazeutische Zusammensetzung nach Anspruch 26, **dadurch gekennzeichnet, dass** sie zum Behandeln einer der folgenden Erkrankungen verwendet wird: Schistosomiasis, Bluthochdruck, Oberflächen-Hamblasenkarzinomen, Epithelkarzinomen, Ovarialkarzinom, Mammakarzinom, Bronchialkarzinom und Kolonrektalkarzinom.

29. Pharmazeutische Zusammensetzung nach Anspruch 24, **dadurch gekennzeichnet, dass** sie als Impfstoff verwendet wird.

30. Pharmazeutische Zusammensetzung gemäß Anspruch 25, **dadurch gekennzeichnet, dass** sie zur Kokain-Missbrauchsvorsorge verwendet wird.

31. Verwendung von Hämocyanin-Polypeptid gemäß einem der Ansprüche 17 bis 23 als Trägerstoff für Arzneimittel.

32. Liposom, umfassend ein Nukleinsäuremolekül gemäß einem der Ansprüche 1 bis 3, ein Konstrukt gemäß einem der Ansprüche 4 bis 9 und/oder ein Hämocyanin-Polypeptid gemäß einem der Ansprüche 17 bis 23.

33. Liposom gemäß Anspruch 32, **dadurch gekennzeichnet, dass** das Liposom weiterhin Zellerkennungsmoleküle umfasst.

34. Antikörper, erhältlich durch Immunisieren eines Versuchstieres mit einem rekombinanten Hämocyanin-Polypeptid, das aus wenigstens einer der aus der folgendenden Aminosäuresequenzen ausgewählt ist:
SEQ ID NO:33 (partielle HtH2 Domäne b),
SEQ ID NO:34 (HtH2 Domäne c),
SEQ ID NO:35 (HtH2 Domäne d),
SEQ ID NO:36 (HtH2 Domäne e),
SEQ ID NO:37 (HtH2 Domäne f),
SEQ ID NO:38 (HtH2 Domäne g),
SEQ ID NO:39 (HtH2 Domäne h),
SEQ ID NO:44 (KLH2 Domäne b),
SEQ ID NO:45 (KLH2 Domäne c),
SEQ ID NO:46 (partielle KLH2 Domäne d),
SEQ ID NO:47 (KLH2 Domäne g),
SEQ ID NO:48 (partielle KLH2 Domäne h),
SEQ ID NO:65 (partielle HtH2 Domäne a),
SEQ ID NO: 156 (vollständige HtH2 Domäne a),
SEQ ID NO:66 (HtH2 Domäne b'),
SEQ ID NO:67 (HtH2 Domäne d'),
SEQ ID NO:68 (HtH2 Domäne e'),
SEQ ID NO:74 (KLH2 Domäne b'),
SEQ ID NO:75 (KLH2 Domäne c'),
SEQ ID NO:76 (KLH2 Domäne d'),
SEQ ID NO:77 (KLH2 Domäne e),
SEQ ID NO:78 (KLH2 Domäne f),
SEQ ID NO:79 (KLH2 Domäne g'),
SEQ ID NO: 158 (teilweise KLH2 Domäne h).

35. Screening-Verfahren zum Identifizieren von Tumor-spezifischer DNA in einer Zelle umfassend:
a) das Inkontaktbringen zellulärer DNA und/oder zellulären Proteins mit einer Sonde umfassend die Nukleinsäuresequenz gemäß einem der Ansprüche 1 bis 3 und/oder des Antikörpers gemäß Anspruch 34 und
b) das Nachweisen der spezifischen Bindung.

36. Screening-Verfahren gemäß Anspruch 35, **dadurch gekennzeichnet, dass** der nachzuweisende Tumor Harnblasenkarzinom, Epithelialkarzinom, Ovarialkarzinom, Mammakarzinom, Bronchialkarzinom oder Kolonrektalkarzinom ist.

## Claims

1. Nucleic acid molecule comprising
(i) a sequence which codes for a haemocyanin, a haemocyanin domain or a variant according to claim 1(c)
(ii) and at least one intron sequence,
the nucleic acid sequence being selected from:
(a) nucleic acid sequences which are selected from the group consisting of the DNA sequences shown below or the corresponding RNA sequences or which contain these:
SEQ ID NO:9 (partial HtH2 domain b),
SEQ ID NO:10 (HtH2 domain c),
SEQ ID NO:11 (HtH2 domain d),
SEQ ID NO:12 (HtH2 domain e),
SEQ ID NO:13 (HtH2 domain f),
SEQ ID NO:14 (HtH2 domain g),
SEQ ID NO: 15 (HtH2 domain h),
SEQ ID NO:20 (KLH2 domain b),
SEQ ID NO:21 (KLH2 domain c),
SEQ ID NO:22 (partial KLH2 domain d),
SEQ ID NO:23 (KLH2 domain g),
SEQ ID NO:24 (partial KLH2 domain h),
SEQ ID NO:50 (partial HtH2 domain a),
SEQ ID NO:51 (HtH2 domain b'),
SEQ ID NO:52 (HtH2 domain d'),
SEQ ID NO:53 (HtH2 domain e'),
SEQ ID NO:57 (KLH2 domain b'),
SEQ ID NO:58 (KLH2 domain c'),
SEQ ID NO:59 (KLH2 domain d'),
SEQ ID NO:60 (KLH2 domain e),
SEQ ID NO:61 (KLH2 domain f),
SEQ ID NO:62 (KLH2 domain g'),
SEQ ID NO:88 (partial HtH2 domain a"),
SEQ ID NO:89 (HtH2 domain b"),
SEQ ID NO:90 (HtH2 domain c"),
SEQ ID NO:91 (HtH2 domain d"),
SEQ ID NO:92 (HtH2 domain e"),
SEQ ID NO:93 (HtH2 domain f"),
SEQ ID NO:94 (HtH2 domain g"),
SEQ ID NO:95 (HtH2 domain h"),
SEQ ID NO:102 (KLH2 domain b"),
SEQ ID NO:103 (KLH2 domain c"),
SEQ ID NO:104 (KLH2 domain d"),
SEQ ID NO:105 (KLH2 domain e"),
SEQ ID NO:106 (KLH2 domain f"),
SEQ ID NO:107 (KLH2 domain g"),
SEQ ID NO:108 (partial KLH2 domain h"),
SEQ ID NO:157 (complete HtH2 domain a);
(b) nucleic acid sequences which code for a polypeptide, comprising at least one amino acid sequence selected from the following group:
SEQ ID NO:33 (partial HtH2 domain b),
SEQ ID NO:34 (HtH2 domain c),
SEQ ID NO:35 (HtH2 domain d),
SEQ ID NO:36 (HtH2 domain e),
SEQ ID NO:37 (HtH2 domain f),
SEQ ID NO:38 (HtH2 domain g),
SEQ ID NO:39 (HtH2 domain h),
SEQ ID NO:44 (KLH2 domain b),
SEQ ID NO:45 (KLH2 domain c),
SEQ ID NO:46 (partial KLH2 domain d),
SEQ ID NO:47 (KLH2 domain g),
SEQ ID NO:48 (partial KLH2 domain h),
SEQ ID NO:65 (partial HtH2 domain a),
SEQ ID NO:156 (complete HtH2 domain a),
SEQ ID NO:66 (HtH2 domain b'),
SEQ ID NO:67 (HtH2 domain d'),
SEQ ID NO:68 (HtH2 domain e'),
SEQ ID NO:74 (KLH2 domain b'),
SEQ ID NO:75 (KLH2 domain c'),
SEQ ID NO:76 (KLH2 domain d'),
SEQ ID NO:77 (KLH2 domain e),
SEQ ID NO:78 (KLH2 domain f),
SEQ ID NO:79 (KLH2 domain g'),
SEQ ID NO:158 (partial KLH2 domain h),
(c) nucleic acid sequences coding for polypeptides, which are at least 60% identical to a sequence of claim 1b, the sequence identity being calculated over 90 amino acids.

2. Nucleic acid molecule according to claim 1, **characterized in that** the intron sequence is selected from:
(i) nucleic acid sequences which are selected from the group consisting of the DNA sequences shown below or the corresponding RNA sequences or which contain these:
SEQ ID NO:147 (KLH2 intron 2B/2C),
SEQ ID NO:148 (KLH2 intron 2C/2D),
SEQ ID NO:149 (KLH2 intron 2D/2E),
SEQ ID NO:150 (KLH2 intron 2E/2F),
SEQ ID NO:151 (KLH2 intron 2F),
SEQ ID NO:152 (KLH2 intron 2F-2/2G),
SEQ ID NO:153 (KLH2 intron 2G-1/2G-2),
SEQ ID NO:154 (KLH2 intron 2G-2/2G-3),
SEQ ID NO:155 (KLH2 intron 2G/2H);
(ii) nucleic acid sequences which hybridize with the counter-strand of a nucleic acid sequence according to (i);
(iii) nucleic acid sequences which are at least 60% identical to one of the nucleic acid sequences described under (i);
(iv) variants of the sequences described under (i) to (iii), wherein the variants contain additions, deletions, insertions or inversions with respect to the sequences described under (i) to (iv); and
(v) combinations of the DNA sequences described under (i) to (iv).

3. Nucleic acid molecule according to one of claims 1 and 2, **characterized in that** it is a deoxyribonucleic acid molecule.

4. Construct comprising a nucleic acid molecule according to one of claims 1 to 3.

5. Construct according to claim 4, furthermore comprising a promoter which is suitable for expression control, the nucleic acid sequence which codes for a haemocyanin, a haemocyanin domain or a functional fragment thereof being under the control of the promoter.

6. Construct according to claim 4 or 5, furthermore comprising a nucleic acid sequence which codes for an antigen and is coupled directly to the nucleic acid sequence which codes for a haemocyanin, a haemocyanin domain or a functional fragment thereof.

7. Construct according to claim 6, wherein the antigen is selected from: tumour antigens, virus antigens and antigens of bacterial or parasitic pathogens.

8. Construct according to one of claims 4 to 7, wherein the construct comprises at least a part of a vector, the vector being selected from: bacteriophages, adenoviruses, vaccinia viruses, baculoviruses, SV40 virus and retroviruses.

9. Construct according to one of claims 4 to 8, wherein the construct furthermore comprises a His tag-coding nucleic acid sequence and the expression of the construct leads to the formation of a fusion protein with a His tag.

10. Host cell containing a construct according to one of claims 4 to 9, wherein the host cell is a prokaryotic or eukaryotic cell suitable for expression of the construct.

11. Host cell according to claim 10, **characterized in that** the prokaryotic host cell is selected from E. coli and Bacillus subtilis.

12. Host cell according to claim 10, **characterized in that** the eukaryotic host cell is selected from yeast cells, plant cells, insect cells and mammalian cells, preferably from CHO cells, COS cells and HeLa cells.

13. Process for the preparation of a haemocyanin polypeptide, wherein the nucleic acid molecule according to one of claims 1 to 3 and/or the construct according to one of claims 4 to 9 is expressed in a suitable host cell and the protein is isolated, if appropriate.

14. Process according to claim 13, **characterized in that** the haemocyanin polypeptide prepared is modified naturally or chemically.

15. Process according to claim 14, **characterized in that** the modification is a crosslinking or a covalent bonding to an antigen.

16. Process according to one of claims 13 to 15, **characterized in that** the expression is carried out in a host cell according to one of claims 10 to 12.

17. Haemocyanin polypeptide, comprising an amino acid sequence which is coded by one or more of the nucleic acid molecules according to one of claims 1 to 3.

18. Haemocyanin polypeptide according to claim 17, comprising at least one amino acid sequence selected from the following group:
SEQ ID NO:33 (partial HtH2 domain b),
SEQ ID NO:34 (HtH2 domain c),
SEQ ID NO:35 (HtH2 domain d),
SEQ ID NO:36 (HtH2 domain e),
SEQ ID NO:37 (HtH2 domain f),
SEQ ID NO:38 (HtH2 domain g),
SEQ ID NO:39 (HtH2 domain h),
SEQ ID NO:44 (KLH2 domain b),
SEQ ID NO:45 (KLH2 domain c),
SEQ ID NO:46 (partial KLH2 domain d),
SEQ ID NO:47 (KLH2 domain g),
SEQ ID NO:48 (partial KLH2 domain h),
SEQ ID NO:65 (partial HtH2 domain a),
SEQ ID NO:156 (complete HtH2 domain a),
SEQ ID NO:66 (HtH2 domain b'),
SEQ ID NO:67 (HtH2 domain d'),
SEQ ID NO:68 (HtH2 domain e'),
SEQ ID NO:74 (KLH2 domain b'),
SEQ ID NO:75 (KLH2 domain c'),
SEQ ID NO:76 (KLH2 domain d'),
SEQ ID NO:77 (KLH2 domain e),
SEQ ID NO:78 (KLH2 domain f),
SEQ ID NO:79 (KLH2 domain g'),
SEQ ID NO:158 (partial KLH2 domain h),
or
(b) a haemocyanin polypeptide the sequence of which is, over a part of at least 90 amino acids, at least 60% identical to one of the amino sequences of (a).

19. Recombinant haemocyanin polypeptide, obtainable by the process according to one of claims 13 to 16 or modifications thereof, wherein the modifications
a) are di-, oligo- and polymers of the haemocyanin polypeptide obtained by the process according to one of claims 13 to 16,
b) side chain modifications thereof,
c) fusion proteins obtained by bonding to an antigen, or
d) polypeptides obtained by posttranslational events.

20. Recombinant haemocyanin polypeptide according to claim 18, **characterized in that** the haemocyanin polypeptide comprises either the sequences SEQ ID NO: 44 to 48 or the sequences SEQ ID NO:44 to 46, 77, 78, 47, 48 and is KLH2 from *Megathura crenulata,* it being possible in each case for the sequence with SEQ ID NO:44 to be replaced by SEQ ID NO:74, SEQ ID NO:45 to be replaced by SEQ ID NO:75, SEQ ID NO:46 to be replaced by SEQ ID NO:76 and/or SEQ ID NO:47 to be replaced by SEQ ID NO:79.

21. Recombinant haemocyanin polypeptide according to claims 17 to 20, **characterized in that** it is bonded covalently to viruses, virus constituents, bacteria, bacteria constituents, DNA, DNA constituents, inorganic or organic molecules, such as carbohydrates, peptides and/or glycoproteins.

22. Recombinant haemocyanin polypeptide according to one of claims 17 to 21, **characterized in that** the haemocyanin polypeptide is non-glycosylated.

23. Recombinant haemocyanin polypeptide according to one of claims 17 to 21, **characterized in that** the haemocyanin polypeptide is glycosylated.

24. Pharmaceutical composition, comprising a nucleic acid molecule according to one of claims 1 to 3 and/or a construct according to one of claims 4 to 9 and physiologically tolerated additives.

25. Pharmaceutical composition according to claim 24, **characterized in that** it is used for gene therapy treatment of tumours.

26. Pharmaceutical composition, comprising a haemocyanin polypeptide according to one of claims 17 to 23 and physiologically tolerated additives.

27. Pharmaceutical composition according to claim 26, **characterized in that** it is used as an antiparasitic composition, antivirus composition or as an antitumour composition.

28. Pharmaceutical composition according to claim 26, **characterized in that** it is used for treatment of one of the following diseases: schistosomiasis, high blood pressure, surface bladder carcinomas, epithelial carcinomas, ovarian carcinoma, mammary carcinoma, bronchial carcinoma and colorectal carcinoma.

29. Pharmaceutical composition according to claim 24, **characterized in that** it is used as a vaccine.

30. Pharmaceutical composition according to claim 25, **characterized in that** it is used for prevention of cocaine abuse.

31. Use of a haemocyanin polypeptide according to one of claims 17 to 23 as a carrier substance for medicaments.

32. Liposome, comprising a nucleic acid molecule according to one of claims 1 to 3, a construct according to one of claims 4 to 9 and/or a haemocyanin polypeptide according to one of claims 17 to 23.

33. Liposome according to claim 32, **characterized in that** the liposome furthermore comprises cell recognition molecules.

34. Antibodies, obtainable by immunization of a test animal with a recombinant haemocyanin polypeptide selected from at least one of the following amino acid sequences:
SEQ ID NO:33 (partial HtH2 domain b),
SEQ ID NO:34 (HtH2 domain c),
SEQ ID NO:35 (HtH2 domain d),
SEQ ID NO:36 (HtH2 domain e),
SEQ ID NO:37 (HtH2 domain f),
SEQ ID NO:38 (HtH2 domain g),
SEQ ID NO:39 (HtH2 domain h),
SEQ ID NO:44 (KLH2 domain b),
SEQ ID NO:45 (KLH2 domain c),
SEQ ID NO:46 (partial KLH2 domain d),
SEQ ID NO:47 (KLH2 domain g),
SEQ ID NO:48 (partial KLH2 domain h),
SEQ ID NO:65 (partial HtH2 domain a),
SEQ ID NO:156 (complete HtH2 domain a),
SEQ ID NO:66 (HtH2 domain b'),
SEQ ID NO:67 (HtH2 domain d'),
SEQ ID NO:68 (HtH2 domain e'),
SEQ ID NO:74 (KLH2 domain b'),
SEQ ID NO:75 (KLH2 domain c'),
SEQ ID NO:76 (KLH2 domain d'),
SEQ ID NO:77 (KLH2 domain e),
SEQ ID NO:78 (KLH2 domain f),
SEQ ID NO:79 (KLH2 domain g'),
SEQ ID NO:158 (partial KLH2 domain h),

35. Screening method for identification of tumour-specific DNA in a cell, comprising:
a) bringing cell DNA and/or cell protein into contact with a probe comprising the nucleic acid sequence according to one of claims 1 to 3 and/or the antibody according to claim 34 and
b) detecting the specific binding.

36. Screening method according to claim 35, **characterized in that** the tumour to be detected is a bladder carcinoma, epithelial carcinoma, ovarian carcinoma, mammary carcinoma, bronchial carcinoma or colorectal carcinoma.

## Revendications

1. Molécule d'acide nucléique, comprenant
(i) une séquence codant pour une hémocyanine, un domaine d'hémocyanine ou une variante selon la revendication 1(c)
(ii) et au moins une séquence d'introns, la séquence d'acide nucléique étant choisie parmi :
(a) des séquences d'acide nucléique qui sont choisies dans le groupe des séquences d'ADN indiquées ci-après ou leurs séquences d'ARN correspondantes, ou contenant celles-ci :
SEQ ID N° : 9 (domaine b partiel de HtH2),
SEQ ID N° : 10 (domaine c de HtH2),
SEQ ID N° : 11 (domaine d de HtH2),
SEQ ID N° : 12 (domaine e de HtH2),
SEQ ID N° : 13 (domaine f de HtH2),
SEQ ID N° : 14 (domaine g de HtH2),
SEQ ID N° : 15 (domaine h de HtH2),
SEQ ID N° : 20 (domaine b de KLH2),
SEQ ID N° : 21 (domaine c de KLH2),
SEQ ID N° : 22 (domaine d partiel de KLH2),
SEQ ID N° : 23 (domaine g de KLH2),
SEQ ID N° : 24 (domaine h partiel de KLH2),
SEQ ID N° : 50 (domaine a partiel de HtH2),
SEQ ID N° : 51 (domaine b' de HtH2),
SEQ ID N° : 52 (domaine d' de HtH2),
SEQ ID N° : 53 (domaine e' de HtH2),
SEQ ID N° : 57 (domaine b' de KLH2),
SEQ ID N° : 58 (domaine c' de KLH2),
SEQ ID N° : 59 (domaine d' de KLH2),
SEQ ID N° : 60 (domaine e de KLH2),
SEQ ID N° : 61 (domaine f de KLH2),
SEQ ID N° : 62 (domaine g' de KLH2),
SEQ ID N° : 88 (domaine a " partiel de HtH2),
SEQ ID N° : 89 (domaine b " de HtH2),
SEQ ID N° : 90 (domaine c" de HtH2),
SEQ ID N° : 91 (domaine d " de HtH2),
SEQ ID N° : 92 (domaine e " de HtH2),
SEQ ID N° : 93 (domaine f " de HtH2),
SEQ ID N° : 94 (domaine g " de HtH2),
SEQ ID N° : 95 (domaine h " de HtH2),
SEQ ID N° : 102 (domaine b " de KLH2),
SEQ ID N° : 103 (domaine c " de KLH2),
SEQ ID N° : 104 (domaine d " de KLH2),
SEQ ID N° : 105 (domaine e " de KLH2),
SEQ ID N° : 106 (domaine f " de KLH2),
SEQ ID N° : 107 (domaine g " de KLH2),
SEQ ID N° : 108 (domaine h " partiel de KLH2),
SEQ ID N° : 157 (domaine a complet de HtH2),
(b) des séquences d'acide nucléique qui codent pour un polypeptide, comprenant au moins une séquence d'acides aminés choisie dans le groupe suivait :
SEQ ID N° : 33 (domaine b partiel de HtH2),
SEQ ID N° : 34 (domaine c de HtH2),
SEQ ID N° : 35 (domaine d de HtH2),
SEQ ID N° : 36 (domaine e de HtH2),
SEQ ID N° : 37 (domaine f de HtH2),
SEQ ID N° : 38 (domaine g de HtH2),
SEQ ID N° : 39 (domaine h de HtH2),
SEQ ID N° : 44 (domaine b de KLH2),
SEQ ID N° : 45 (domaine c de KLH2),
SEQ ID N° : 46 (domaine d partiel de KLH2),
SEQ ID N° : 47 (domaine g de KLH2),
SEQ ID N° : 48 (domaine h partiel de KLH2),
SEQ ID N° : 65 (domaine a de HtH2),
SEQ ID N° : 156 (domaine a complet de HtH2),
SEQ ID N° : 66 (domaine b' de HtH2),
SEQ ID N° : 67 (domaine d' de HtH2),
SEQ ID N° : 68 (domaine e' de HtH2),
SEQ ID N° : 74 (domaine b' de KLH2),
SEQ ID N° : 75 (domaine c' de KLH2),
SEQ ID N° : 76 (domaine d' de KLH2),
SEQ ID N° : 77 (domaine e de KLH2),
SEQ ID N° : 78 (domaine f de KLH2),
SEQ ID N° : 79 (domaine g' de KLH2),
SEQ ID N° : 158 (domaine h partiel de KLH2),
(c) des séquences d'acide nucléique qui codent pour les polypeptides qui sont au moins identiques à 60 % à une séquence selon la revendication 1b, l'identité de séquence étant calculée sur 90 acides aminés.

2. Molécule d'acide nucléique selon la revendication 1, **caractérisée en ce que** la séquence d'intron est choisie parmi :
(i) des séquences d'acide nucléique qui sont choisies dans le groupe des séquences d'ADN indiquées ci-après ou leurs séquences d'ARN correspondantes ou qui contiennent celles-ci
SEQ ID N° : 147 (Intron 2B/2C de KLH2),
SEQ ID N° : 148 (Intron 2C/2D de KLH2),
SEQ ID N° : 149 (Intron 2D/2E de KLH2),
SEQ ID N° : 150 (Intron 2E/2F de KLH2),
SEQ ID N° : 151 (Intron 2F de KLH2),
SEQ ID N° : 152 (Intron 2F-2/2G de KLH2),
SEQ ID N° : 153 (Intron 2G-1/2G-2 de KLH2),
SEQ ID N° : 154 (Intron 2G-2/2G-3 de KLH2),
SEQ ID N° : 155 (intron 2G/2H de KLH2),
(ii) des séquences d'acide nucléique qui s'hybrident avec le brin opposé d'une séquence d'acide nucléique selon le point (i);
(iii) des séquences d'acide nucléique qui sont au moins à 60 % identiques à l'une des séquences d'acide nucléique indiquée au point (i);
(iv) des variantes des séquences indiquées aux points (i) à (iii), les variantes présentant par rapport aux séquences indiquées aux points (i) à (iii), des additions, délétions, insertions ou inversions ; et
(v) des combinaisons de plusieurs des séquences ADN indiquées aux points (i) à (iv).

3. Molécule d'acide nucléique selon l'une quelconque des revendications 1 et 2, **caractérisée en ce qu'**elle est une molécule d'acide désoxyribonucléique.

4. Construction comprenant une molécule d'acide nucléique selon l'une quelconque des revendications 1 à 3.

5. Construction selon la revendication 4, comprenant en outre un promoteur approprié pour le contrôle de l'expression, la séquence d'acide nucléique codant pour une hémocyanine, un domaine d'hémocyanine ou un fragment fonctionnel de celle-ci étant sous le contrôle du promoteur.

6. Construction selon la revendication 4 ou 5, comprenant en outre une séquence d'acide nucléique codant pour un antigène, qui est reliée directement à la séquence d'acide nucléique codant pour une hémocyanine, un domaine d'hémocyanine ou un fragment fonctionnel de celle-ci.

7. Construction selon la revendication 6, l'antigène étant choisi parmi : des antigènes tumoraux, des antigènes viraux et des antigènes d'agents pathogènes bactériens ou parasitaires.

8. Construction selon l'une quelconque des revendications 4 à 7, la construction contenant au moins une partie d'un vecteur, le vecteur étant choisi parmi les bactériophages, les adénovirus, les virus de la vaccine, les baculovirus, le virus SV40 et les rétrovirus.

9. Construction selon l'une quelconque des revendications 4 à 8, la construction comprenant en outre une séquence d'acide nucléique codant pour His-Tag et l'expression de la construction entraînant la formation d'une protéine de fusion avec un His-Tag.

10. Cellule hôte contenant une construction selon l'une quelconque des revendications 4 à 9, la cellule hôte étant une cellule procaryote ou eucaryote appropriée pour l'expression de la construction.

11. Cellule hôte selon la revendication 10, **caractérisée en ce que** la cellule hôte procaryote est choisie parmi *E. coli* et *Bacillus subtilis.*

12. Cellule hôte selon la revendication 10, **caractérisée en ce que** la cellule hôte eucaryote est choisie parmi les cellules de levure, les cellules de plantes, les cellules d'insectes et les cellules de mammifères, de préférence, les cellules CHO, les cellules COS et les cellules HeLa.

13. Procédé de production d'un polypeptide d'hémocyanine, la molécule d'acide nucléique selon l'une des revendications 1 à 3 et/ou la construction selon l'une quelconque des revendications 4 à 9 étant exprimée dans une cellule hôte appropriée et la protéine étant éventuellement isolée.

14. Procédé selon la revendication 13, **caractérisé en ce que** le polypeptide d'hémocyanine produit est modifié naturellement ou chimiquement.

15. Procédé selon la revendication 14, **caractérisé en ce que** la modification est une réticulation croisée ou une liaison covalente à un antigène.

16. Procédé selon l'une quelconque des revendications 13 à 15, **caractérisé en ce que** l'expression est réalisée dans une cellule hôte selon l'une quelconque des revendications 10 à 12.

17. Polypeptide d'hémocyanine, comprenant une séquence d'acides aminés qui est codée par une ou plusieurs des molécules d'acide nucléique selon l'une quelconque des revendications 1 à 3.

18. Polypeptide d'hémocyanine selon la revendication 17, comprenant au moins une séquence d'acides aminés choisie dans le groupe suivant :
SEQ ID N° : 33 (domaine b partiel de HtH2),
SEQ ID N° : 34 (domaine c de HtH2),
SEQ ID N° : 35 (domaine d de HtH2),
SEQ ID N° : 36 (domaine e de HtH2),
SEQ ID N° : 37 (domaine f de HtH2),
SEQ ID N° : 38 (domaine g de HtH2),
SEQ ID N° : 39 (domaine h de HtH2),
SEQ ID N° : 44 (domaine b de KLH2),
SEQ ID N° : 45 (domaine c de KLH2),
SEQ ID N° : 46 (domaine d partiel de KLH2),
SEQ ID N° : 47 (domaine g de KLH2),
SEQ ID N° : 48 (domaine h partiel de KLH2),
SEQ ID N° : 65 (domaine a de HtH2),
SEQ ID N° : 156 (domaine a complet de HtH2),
SEQ ID N° : 66 (domaine b' de HtH2),
SEQ ID N° : 67 (domaine d' de HtH2),
SEQ ID N° : 68 (domaine e' de HtH2),
SEQ ID N° : 74 (domaine b' de KLH2),
SEQ ID N° : 75 (domaine c' de KLH2),
SEQ ID N° : 76 (domaine d' de KLH2),
SEQ ID N° : 77 (domaine e de KLH2),
SEQ ID N° : 78 (domaine f de KLH2),
SEQ ID N° : 79 (domaine g' KLH2),
SEQ ID N° : 158 (domaine h partiel de KLH2),
ou
(b) un polypeptide d'hémocyanine dont la séquence est identique à au moins 60 % sur une partie d'au moins 90 acides aminés, aux séquences d'acides aminés du point (a).

19. Polypeptide d'hémocyanine recombinant, pouvant être obtenu par le procédé selon l'une quelconque des revendications 13 à 16 ou leurs modifications, les modifications étant
a) des di-, oligo- et polymères du polypeptide d'hémocyanine obtenu selon l'une quelconque des revendications 13 à 16,
b) des modifications de chaînes latérales de ceux-ci,
c) des protéines de fusion obtenues par liaison à un antigène, ou
d) des polypeptides obtenus par des évènements post-translationnels.

20. Polypeptide d'hémocyanine recombinant selon la revendication 18, **caractérisé en ce que** le polypeptide d'hémocyanine comprend soit les séquences SEQ ID N° : 44 à 48 soit les séquences SEQ ID N° : 44 à 46, 77, 78, 47, 48 et KLH2 est issu de *Megathura crenulata,* où respectivement la séquence avec SEQ ID N° : 44 peut être substituée par SEQ ID N° : 74, SEQ ID N° : 45 par SEQ ID N° : 75, SEQ ID N° : 46 par SEQ ID N° : 76 et/ou SEQ ID N° : 47 par SEQ ID N° : 79.

21. Polypeptide d'hémocyanine recombinant selon la revendication 17 à 20, **caractérisé en ce qu'**il est lié de façon covalente à des virus, des composants viraux, des bactéries, des composants bactériens, un ADN, des composants d'ADN, des molécules anorganiques ou organiques comme par exemple, un peptide et/ou une glycoprotéine d'hydrate de carbone.

22. Polypeptide d'hémocyanine recombinant selon l'une quelconque des revendications 17 à 21, **caractérisé en ce que** le polypeptide d'hémocyanine n'est pas glycosylé.

23. Polypeptide d'hémocyanine recombinant selon l'une quelconque des revendications 17 à 21, **caractérisé en ce que** le polypeptide d'hémocyanine est glycosylé.

24. Composition pharmaceutique contenant une molécule d'acide nucléique selon l'une quelconque des revendications 1 à 3 et/ou une construction selon l'une quelconque des revendications 4 à 9, et des additifs physiologiquement acceptables.

25. Composition pharmaceutique selon la revendication 24, **caractérisée en ce qu'**elle est utilisée pour le traitement par thérapie génique de tumeurs.

26. Composition pharmaceutique, contenant un polypeptide d'hémocyanine selon l'une quelconque des revendications 17 à 23 et des additifs physiologiquement acceptables.

27. Composition pharmaceutique selon la revendication 26, **caractérisée en ce qu'**elle est utilisée comme agent antiparasitaire, antiviral ou antitumoral.

28. Composition pharmaceutique selon la revendication 26, **caractérisée en ce qu'**elle est utilisée pour le traitement de l'une des maladies suivantes : la schistosomiase, l'hypertension, les carcinomes superficiels de la vessie, les carcinomes épithéliaux, le carcinome ovarien, le carcinome mammaire, le carcinome bronchique et le carcinome colorectal.

29. Composition pharmaceutique selon la revendication 24, **caractérisée en ce qu'**elle est utilisée comme vaccin.

30. Composition pharmaceutique selon la revendication 25, **caractérisée en ce qu'**elle est utilisée en prévention d'une toxicomanie à la cocaïne.

31. Utilisation de polypeptide d'hémocyanine selon l'une quelconque des revendications 17 à 23 comme véhicule d'un médicament.

32. Liposome, comprenant une molécule d'acide nucléique selon l'une quelconque des revendications 1 à 3, une construction selon l'une quelconque des revendications 4 à 9 et/ou un polypeptide d'hémocyanine selon l'une quelconque des revendications 17 à 23.

33. Liposome selon la revendication 32, **caractérisé en ce que** le liposome comprend en outre des molécules de reconnaissance cellulaire.

34. Anticorps pouvant être obtenus par immunisation d'un animal de laboratoire, qui est choisi parmi au moins une des séquences d'acides aminés suivantes :
SEQ ID N° : 33 (domaine b partiel de HtH2),
SEQ ID N° : 34 (domaine c de HtH2),
SEQ ID N° : 35 (domaine d de HtH2),
SEQ ID N° : 36 (domaine e de HtH2),
SEQ ID N° : 37 (domaine f de HtH2),
SEQ ID N° : 38 (domaine g de HtH2),
SEQ ID N° : 39 (domaine h de HtH2),
SEQ ID N° : 44 (domaine b de KLH2),
SEQ ID N° : 45 (domaine c de KLH2),
SEQ ID N° : 46 (domaine d partiel de KLH2),
SEQ ID N° : 47 (domaine g de KLH2),
SEQ ID N° : 48 (domaine h partiel de KLH2),
SEQ ID N° : 65 (domaine a partiel de HtH2),
SEQ ID N° : 156 (domaine a complet de HtH2),
SEQ ID N° : 66 (domaine b' de HtH2),
SEQ ID N° : 67 (domaine d' de HtH2),
SEQ ID N° : 68 (domaine e' de HtH2),
SEQ ID N° : 74 (domaine b' de KLH2),
SEQ ID N° : 75 (domaine c' de KLH2),
SEQ ID N° : 76 (domaine d' de KLH2),
SEQ ID N° : 77 (domaine e de KLH2),
SEQ ID N° : 78 (domaine f de KLH2),
SEQ ID N° : 79 (domaine g' KLH2),
SEQ ID N° : 158 (domaine h partiel de KLH2).

35. Procédé de criblage pour identifier un ADN spécifique à une tumeur dans une cellule, comprenant :
a) la mise en contact d'ADN cellulaire et/ou de protéine cellulaire avec une sonde comprenant la séquence d'acide nucléique selon l'une quelconque des revendications 1 à 3 et/ou de l'anticorps selon la revendication 34 et
b) la détection de la liaison spécifique.

36. Procédé de criblage selon la revendication 35, **caractérisé en ce que** la tumeur à détecter est un carcinome de la vessie, un carcinome épithélial, un carcinome ovarien, un carcinome mammaire, un carcinome bronchique ou un carcinome colorectal.
